# EUROPEAN PATENT APPLICATION

(11) **EP 4 778 593 A2**
(43) Date of publication of application: **22.07.2026**
(21) Application number: 26181328.1
(22) Date of filing: 07.11.2022
(51) Int. Cl.: A61P 37/00

(54) **HETEROCYCLIC COMPOUNDS AND USES THEREOF**

(30) Priority: 08.11.2021 US 202163276927 P
(62) Divisional of application: 22843901.4
(71) Applicant: Ventus Therapeutics U.S., Inc., Waltham, MA 02453 (US)
(72) Inventor: BEVERIDGE, Ramsey, Québec, H9R 2L3 (CA); BURCH, Jason, Québec, H9S 4Z1 (CA); FADER, Lee, Ontario, K0B 1K0 (CA); BOILY, Marc-Olivier, Québec, H7K 1P4 (CA); ST-ONGE, Miguel, Québec, J7T 2L5 (CA); DORICH, Stéphane, Québec, H9R 1H6 (CA); CHAGNON, Felix, Québec, H8R 3K6 (CA); DURET, Guillaume, Québec, H3T 1G1 (CA); LEGER, Serge, Québec, J7W 3M1 (CA)
(74) Representative: J A Kemp LLP

(57) **Abstract**

The present disclosure relates to compounds of Formula (I): 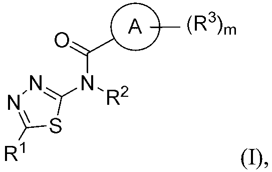 wherein Ring A, R¹, R², R³, and m are described herein. The present disclosure relates to the use of the compounds of Formula (I), and pharmaceutically acceptable salts, hydrates, solvates, prodrugs, stereoisomers, labeled isotopes, or tautomers thereof, in the treatment of cGAS-related diseases and disorders.

## Description

### RELATED APPLICATIONS

This application claims priority to, and the benefit of, U.S. Provisional Application No. 63/276,927 filed November 8, 2021, the contents of which is hereby incorporated by reference in its entirety.

### FIELD OF DISCLOSURE

The present disclosure is directed to inhibitors of cyclic GMP-AMP synthase. The inhibitors described herein can be useful in the treatment of diseases or disorders associated with cyclic GMP-AMP synthase. In particular, the disclosure is concerned with compounds and pharmaceutical compositions inhibiting cyclic GMP-AMP synthase, methods of treating diseases or disorders associated with cyclic GMP-AMP synthase, and methods of synthesizing these compounds.

### BACKGROUND

Aberrant accumulation of cytosolic DNA induces type I interferons and other cytokines that are important for antimicrobial defense but can also induce autoimmunity. This DNA signaling pathway requires the stimulator of interferon genes (STING) adapter protein and the transcription factors NF-κB and IRF3, but the mechanism of DNA sensing was unclear until recently. It is now understood that mammalian cytosolic extracts synthesize cyclic GMP-AMP (cGAMP) in vitro from ATP and GTP in the presence of DNA rather than RNA (WO 2014099824). DNA transfection or DNA virus infection of mammalian cells also trigger the production of cGAMP. cGAMP binds to STING, leading to IRF3 activation and induction of interferon-β (IFNβ). Thus, cGAMP is the first cyclic dinucleotide in metazoans, and cGAMP functions as an endogenous secondary messenger that induces interferon production in response to cytosolic DNA.

cGAMP synthase (cGAS) is an enzyme that intervenes in the synthesis of cyclic GMP-AMP and belongs to the nucleotidyltransferase family. Overexpression of cGAS activates the transcription factor IRF3 and induces IFNβ in a STING-dependent manner. Knockdown of cGAS inhibits IRF3 activation and IFNβ induction by DNA transfection or DNA virus infection. cGAS binds to DNA in the cytoplasm and catalyzes cGAMP synthesis. These findings indicate that cGAS is a cytosolic DNA sensor that induces interferons by producing the second messenger cGAMP.

The critical role of cGAS in cytosolic DNA sensing has been established in different pathogenic bacteria, viruses, and retroviruses (US 20210155625). Additionally, cGAS is essential in various other biological processes, such as cellular senescence and recognition of ruptured micronuclei in the surveillance of potential cancer cells.

There is a need for therapeutic agents that target cGAS. Small molecule inhibitors that are specific for cGAS would be of great value in treating diseases that arise from inappropriate cGAS activity and the resulting undesired type I interferon activity. This present disclosure is intended to fill this unmet need associated with current cGAS inhibition therapy.

### SUMMARY

In some aspects, the present disclosure provides a compound of Formula (I): 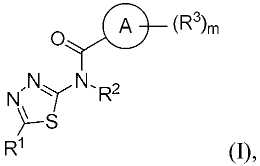 or a pharmaceutically acceptable salt, hydrate, solvate, prodrug, stereoisomer, labeled isotope, or tautomer thereof, wherein:
Ring A is a 5- to 10-membered heteroaryl or 3- to 10-membered heterocyclyl;
R' is H, -SR⁴, -S(O)R⁴, -S(O)₂R⁴, -N(R⁴)₂, -CN, C₁-C₆ alkyl, C₆-C₁₀ aryl, or 5- to 10-membered heteroaryl, wherein the alkyl, aryl or heteroaryl is optionally substituted with one or more R⁵, provided R¹ is not pyridinyl when Ring A is 1-isoquinolinyl;
R² is H or C₁-C₆ alkyl;
each R³ is independently halogen, -CN, -OR⁸, -NH₂, -NH(R⁶), -N(R⁶)(R⁷), -NHC(O)R⁸, - CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or
two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷, or
two geminal R³, together with the carbon atom to which they attached, form an oxo;
R⁴ is H, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl;
each R⁵ is independently -OH, halogen, -C(O)OH, C₁-C₆ alkyl, C₁-C₆ alkoxy, C₂-C₆ alkenyl, or C₂-C₆ alkynyl, wherein the alkyl, alkoxy, alkenyl, or alkynyl is optionally substituted with halogen, OH, or NH₂;
each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, - (CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl), or
two R⁶, together with the atoms to which they are attached, form a C₃-C₈ cycloalkyl, C₆-C₁₀ aryl, 5- to 10-membered heteroaryl, or 3- to 10-membered heterocyclyl, or
two geminal R⁶, together with the carbon atom to which they are attached, form an oxo;
each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, or
two R⁷, together with the atoms to which they are attached, form a C₃-C₈ cycloalkyl, C₆-C₁₀ aryl, 5- to 10-membered heteroaryl, or 3- to 10-membered heterocyclyl, or
two geminal R⁷, together with carbon atom to which they are attached, form oxo;
R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, or heteroaryl is optionally substituted with one or more R⁹;
R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl);
R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, heteroaryl, or aryl is optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, 3- to 10-membered heterocyclyl, 5- to 10-membered heteroaryl, or C₆-C₁₀ aryl;
m is an integer from 0 to 4;
n is an integer from 0 to 6; and
p is an integer from 0 to 6.

In some aspects, the present disclosure provides a pharmaceutical composition comprising a compound of Formula (I), or a pharmaceutically acceptable salt, hydrate, solvate, prodrug, stereoisomer, labeled isotope, or tautomer thereof and a pharmaceutically acceptable carrier. The pharmaceutical acceptable carrier may further include an excipient, diluent, or surfactant.

In some aspects, the present disclosure provides a method of treating a disease or disorder associated with modulation of cGAS, wherein the method comprises administering to a subject in need thereof a compound of Formula (I), or a pharmaceutically acceptable salt, hydrate, solvate, prodrug, stereoisomer, labeled isotope, or tautomer thereof, or pharmaceutical composition thereof.

In some aspects, the present disclosure provides a method of inhibiting cGAS, wherein the method comprises administering to a subject in need thereof a compound of Formula (I), or a pharmaceutically acceptable salt, hydrate, solvate, prodrug, stereoisomer, labeled isotope, or tautomer thereof, or pharmaceutical composition thereof.

In some aspects, the present disclosure provides a method of treating or preventing a disease or disorder disclosed herein, wherein the method comprises administering to a subject in need thereof a compound of Formula (I), or a pharmaceutically acceptable salt, hydrate, solvate, prodrug, stereoisomer, labeled isotope, or tautomer thereof, or pharmaceutical composition thereof.

In some aspects, the present disclosure provides a method of treating a disease or disorder disclosed herein in a subject in need thereof, comprising administering to the subject a therapeutically effective amount of a compound of the present disclosure or a pharmaceutically acceptable salt, hydrate, solvate, prodrug, stereoisomer, labeled isotope, or tautomer thereof, or a pharmaceutical composition of the present disclosure.

In some aspects, the present disclosure provides a compound of Formula (I), or pharmaceutically acceptable salts, hydrates, solvates, prodrugs, stereoisomers, labeled isotopes, or tautomers thereof, or pharmaceutical compositions thereof, for use in the manufacture of a medicament for inhibiting cGAS.

In some aspects, the present disclosure provides a compound of Formula (I), or pharmaceutically acceptable salts, hydrates, solvates, prodrugs, stereoisomers, labeled isotopes, or tautomers thereof, or pharmaceutical compositions thereof, for use in the manufacture of a medicament for treating or preventing a disease or disorder disclosed herein.

In some aspects, the present disclosure provides use of a compound of the present disclosure or a pharmaceutically acceptable salt, hydrate, solvate, prodrug, stereoisomer, labeled isotope, or tautomer thereof in the manufacture of a medicament for treating a disease or disorder disclosed herein.

In some aspects, the present disclosure provides a compound of the present disclosure or a pharmaceutically acceptable salt, hydrate, solvate, prodrug, stereoisomer, labeled isotope, or tautomer thereof, for use in treating or preventing a disease or disorder disclosed herein.

In some aspects, the present disclosure provides a compound of the present disclosure or a pharmaceutically acceptable salt, hydrate, solvate, prodrug, stereoisomer, labeled isotope, or tautomer thereof, for use in treating a disease or disorder disclosed herein.

In some aspects, the present disclosure provides an intermediate as described herein, being suitable for use in a method for preparing a compound as described herein (e.g., the intermediate is selected from the intermediates described in Examples).

Unless otherwise defined, all technical and scientific terms used herein have the same meaning as commonly understood by one of ordinary skill in the art to which this disclosure belongs. In the specification, the singular forms also include the plural unless the context clearly dictates otherwise. Although methods and materials similar or equivalent to those described herein can be used in the practice or testing of the present disclosure, suitable methods and materials are described below. All publications, patent applications, patents and other references mentioned herein are incorporated by reference. The references cited herein are not admitted to be prior art to the claimed disclosure. In the case of conflict, the present specification, including definitions, will control. In addition, the materials, methods and examples are illustrative only and are not intended to be limiting. In the case of conflict between the chemical structures and names of the compounds disclosed herein, the chemical structures will control.

Other features and advantages of the disclosure will be apparent from the following detailed description and claims.

### DETAILED DESCRIPTION

The present disclosure relates to compounds and compositions that are capable of inhibiting cGAS. The disclosure features methods of treating, preventing, or ameliorating a disease or disorder in which cGAS plays a role by administering to a patient in need thereof a therapeutically effective amount of a compound of Formula (I), or a pharmaceutically acceptable salt, hydrate, solvate, prodrug, stereoisomer, labeled isotope, or tautomer thereof. The methods of the present disclosure can be used in the treatment of a variety of cGAS-mediated diseases and disorders by inhibiting the activity of cGAS. The present disclosure also relates to processes for the preparation of these compounds, to pharmaceutical compositions comprising them, and to their use in the treatment of disorders in which cGAS is implicated including, but not limited to inflammation, an auto-immune disease, a cancer, an infection, a disease or disorder of the central nervous system, a metabolic disease, a cardiovascular disease, a respiratory disease, a kidney disease, a liver disease, an ocular disease, a skin disease, a lymphatic disease, a rheumatic disease, a psychological disease, graft versus host disease, or allodynia.

In some aspects, the present disclosure provides a compound of Formula (I): 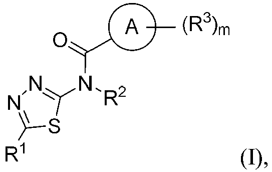 or a pharmaceutically acceptable salt, hydrate, solvate, prodrug, stereoisomer, labeled isotope, or tautomer thereof, wherein Ring A, R¹, R², R³, and m are described herein.

The details of the disclosure are set forth in the accompanying description below. Although methods and materials similar or equivalent to those described herein can be used in the practice or testing of the present disclosure, illustrative methods and materials are now described. Other features, objects, and advantages of the disclosure will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this disclosure belongs. All patents and publications cited in this specification are incorporated herein by reference in their entireties.

### Definitions

The articles "a" and "an" are used in this disclosure to refer to one or more than one *(i.e.,* to at least one) of the grammatical object of the article. By way of example, "an element" means one element or more than one element.

The term "and/or" is used in this disclosure to mean either "and" or "or" unless indicated otherwise.

The term "optionally substituted" is understood to mean that a given chemical moiety (e.g., an alkyl group) can (but is not required to) be bonded other substituents (e.g., heteroatoms). For instance, an alkyl group that is optionally substituted can be a fully saturated alkyl chain *(i.e.,* a pure hydrocarbon). Alternatively, the same optionally substituted alkyl group can have substituents different from hydrogen. For instance, it can, at any point along the chain be bounded to a halogen atom, a hydroxyl group, or any other substituent described herein. Thus, the term "optionally substituted" means that a given chemical moiety has the potential to contain other functional groups but does not necessarily have any further functional groups. Suitable substituents used in the optional substitution of the described groups include, without limitation, halogen, oxo, -OH, -CN, -COOH, - CH₂CN, -O-(C₁-C₆) alkyl, (C₁-C₆) alkyl, (C₁-C₆) alkoxy, (C₁-C₆) haloalkyl, (C₁-C₆) haloalkoxy, -O-(C₂-C₆) alkenyl, -O-(C₂-C₆) alkynyl, (C₂-C₆) alkenyl, (C₂-C₆) alkynyl, -OH, -OP(O)(OH)₂, - OC(O)(C₁-C₆) alkyl, -C(O)(C₁-C₆) alkyl, -OC(O)O(C₁-C₆) alkyl, -NH₂, -NH((C₁-C₆) alkyl), -N((C₁-C₆) alkyl)₂, -NHC(O)(C₁-C₆) alkyl, -C(O)NH(C₁-C₆) alkyl, -S(O)₂(C₁-C₆) alkyl, -S(O)NH(C₁-C₆) alkyl, and S(O)N((C₁-C₆) alkyl)₂. The substituents can themselves be optionally substituted. "Optionally substituted" as used herein also refers to substituted or unsubstituted whose meaning is described below.

As used herein, the term "substituted" means that the specified group or moiety bears one or more suitable substituents wherein the substituents may connect to the specified group or moiety at one or more positions. For example, an aryl substituted with a cycloalkyl may indicate that the cycloalkyl connects to one atom of the aryl with a bond or by fusing with the aryl and sharing two or more common atoms.

As used herein, the term "unsubstituted" means that the specified group bears no substituents.

Unless otherwise specifically defined, the term "aryl" refers to cyclic, aromatic hydrocarbon groups that have 1 to 3 aromatic rings, including monocyclic or bicyclic groups such as phenyl, biphenyl, or naphthyl. Where containing two aromatic rings (bicyclic, etc.), the aromatic rings of the aryl group may be joined at a single point (*e.g.,* biphenyl), or fused (*e.g.,* naphthyl). The aryl group may be optionally substituted by one or more substituents, *e.g.*, 1 to 5 substituents, at any point of attachment. Exemplary substituents include, but are not limited to, -H, -halogen, -O-(C₁-C₆) alkyl, (C₁-C₆) alkyl, -O-(C₂-C₆) alkenyl, -O-(C₂-C₆) alkynyl, (C₂-C₆) alkenyl, (C₂-C₆) alkynyl, -OH, - OP(O)(OH)₂, -OC(O)(C₁-C₆) alkyl, -C(O)(C₁-C₆) alkyl, -OC(O)O(C₁-C₆) alkyl, -NH₂, NH((C₁-C₆) alkyl), N((C₁-C₆) alkyl)₂, -S(O)₂-(C₁-C₆) alkyl, -S(O)NH(C₁-C₆) alkyl, and -S(O)N((C₁-C₆) alkyl)₂. The substituents can themselves be optionally substituted. Furthermore, when containing two fused rings, the aryl groups herein defined may have a saturated or partially unsaturated ring fused with a fully unsaturated aromatic ring. Exemplary ring systems of these aryl groups include, but are not limited to, phenyl, biphenyl, naphthyl, anthracenyl, phenalenyl, phenanthrenyl, indanyl, indenyl, tetrahydronaphthalenyl, tetrahydrobenzoannulenyl, and the like.

Unless otherwise specifically defined, "heteroaryl" means a monovalent monocyclic or polycyclic aromatic radical of 5 to 24 ring atoms, containing one or more ring heteroatoms selected from N, O, S, P, Se, or B, the remaining ring atoms being C. Heteroaryl as herein defined also means a bicyclic heteroaromatic group wherein the heteroatom is selected from N, O, S, P, Se, or B. Heteroaryl as herein defined also means a tricyclic heteroaromatic group containing one or more ring heteroatoms selected from N, O, S, P, Se, or B. The aromatic radical is optionally substituted independently with one or more substituents described herein. Examples include, but are not limited to, furyl, thienyl, pyrrolyl, pyridyl, pyrazolyl, pyrimidinyl, imidazolyl, isoxazolyl, oxazolyl, oxadiazolyl, pyrazinyl, indolyl, thiophen-2-yl, quinolinyl, benzopyranyl, isothiazolyl, thiazolyl, thiadiazole, indazole, benzimidazolyl, thieno[3,2-b]thiophene, triazolyl, triazinyl, imidazo[1,2-b]pyrazolyl, furo[2,3-c]pyridinyl, imidazo[1,2-a]pyridinyl, indazolyl, pyrrolo[2,3-c]pyridinyl, pyrrolo[3,2-c]pyridinyl, pyrazolo[3,4-c]pyridinyl, thieno[3,2-c]pyridinyl, thieno[2,3-c]pyridinyl, thieno[2,3-b]pyridinyl, benzothiazolyl, indolyl, indolinyl, indolinonyl, dihydrobenzothiophenyl, dihydrobenzofuranyl, benzofuran, chromanyl, thiochromanyl, tetrahydroquinolinyl, dihydrobenzothiazine, quinolinyl, isoquinolinyl, 1,6-naphthyridinyl, benzo[de]isoquinolinyl, pyrido[4,3-b][1,6]naphthyridinyl, thieno[2,3-b]pyrazinyl, quinazolinyl, tetrazolo[1,5-a]pyridinyl, [1,2,4]triazolo[4,3-a]pyridinyl, isoindolyl, pyrrolo[2,3-b]pyridinyl, pyrrolo[3,4-b]pyridinyl, pyrrolo[3,2-b]pyridinyl, imidazo[5,4-b]pyridinyl, pyrrolo[1,2-a]pyrimidinyl, tetrahydro pyrrolo[1,2-a]pyrimidinyl, 3,4-dihydro-2H-1λ²-pyrrolo[2,1-b]pyrimidine, dibenzo[b,d] thiophene, pyridin-2-one, furo[3,2-c]pyridinyl, furo[2,3-c]pyridinyl, 1H-pyrido[3,4-b][1,4] thiazinyl, benzoxazolyl, benzisoxazolyl, furo[2,3-b]pyridinyl, benzothiophenyl, 1,5-naphthyridinyl, furo[3,2-b]pyridine, [1,2,4]triazolo[1,5-a]pyridinyl, benzo [1,2,3]triazolyl, imidazo[1,2-a]pyrimidinyl, [1,2,4]triazolo[4,3-b]pyridazinyl, benzo[c][1,2,5]thiadiazolyl, benzo[c][1,2,5]oxadiazole, 1,3-dihydro-2H-benzo[d]imidazol-2-one, 3,4-dihydro-2H-pyrazolo [1,5-b][1,2]oxazinyl, 4,5,6,7-tetrahydropyrazolo[1,5-a]pyridinyl, thiazolo[5,4-d]thiazolyl, imidazo[2,1-b][1,3,4]thiadiazolyl, thieno[2,3-b]pyrrolyl, 3H-indolyl, and derivatives thereof. Furthermore, when containing two or more fused rings, the heteroaryl groups defined herein may have one or more saturated or partially unsaturated ring fused with a fully unsaturated aromatic ring, *e.g.,* a 5-membered heteroaromatic ring containing 1 to 3 heteroatoms selected from N, O, S, P, Se, or B, or a 6-membered heteroaromatic ring containing 1 to 3 nitrogens, wherein the saturated or partially unsaturated ring includes 0 to 4 heteroatoms selected from N, O, S, P, Se, or B, and is optionally substituted with one or more oxo. In heteroaryl ring systems containing more than two fused rings, a saturated or partially unsaturated ring may further be fused with a saturated or partially unsaturated ring described herein. Exemplary ring systems of these heteroaryl groups include, for example, indolinyl, indolinonyl, dihydrobenzothiophenyl, dihydrobenzofuran, chromanyl, thiochromanyl, tetrahydroquinolinyl, dihydrobenzothiazine, 3,4-dihydro-1H-isoquinolinyl, 2,3-dihydrobenzofuranyl, benzofuranonyl, indolinyl, oxindolyl, indolyl, 1,6-dihydro-7H-pyrazolo[3,4-c]pyridin-7-onyl, 7,8-dihydro-6H-pyrido[3,2-b]pyrrolizinyl, 8H-pyrido[3,2-b]pyrrolizinyl, 1,5,6,7-tetrahydrocyclopenta[b]pyrazolo[4,3-e]pyridinyl, 7,8-dihydro-6H-pyrido[3,2-b]pyrrolizine, pyrazolo[1,5-a]pyrimidin-7(4H)-only, 3,4-dihydropyrazino[1,2-a]indol-1(2H)-onyl, or benzo[*c*][1,2]oxaborol-1(*3H*)-olyl.

"Halogen" or "halo" refers to fluorine, chlorine, bromine, or iodine.

"Alkyl" refers to a straight or branched chain saturated hydrocarbon containing 1-12 carbon atoms. Examples of a (C₁-C₆) alkyl group include, but are not limited to, methyl (-CH₃), ethyl (-CH₂CH₃), propyl, butyl, pentyl, hexyl, isopropyl, isobutyl, sec-butyl, tert-butyl, isopentyl, neopentyl, and isohexyl.

"Alkoxy" refers to a straight or branched chain saturated hydrocarbon containing 1-12 carbon atoms containing a terminal "O" in the chain, *i.e.,* -O(alkyl). Examples of alkoxy groups include without limitation, methoxy, ethoxy, propoxy, butoxy, t-butoxy, or pentoxy groups.

"Alkenyl" refers to a straight or branched chain unsaturated hydrocarbon containing 2-12 carbon atoms. The "alkenyl" group contains at least one double bond in the chain. The double bond of an alkenyl group can be unconjugated or conjugated to another unsaturated group. Examples of alkenyl groups include ethenyl, propenyl, n-butenyl, iso-butenyl, pentenyl, or hexenyl. An alkenyl group can be unsubstituted or substituted. Alkenyl, as herein defined, may be straight or branched.

"Alkynyl" refers to a straight or branched chain unsaturated hydrocarbon containing 2-12 carbon atoms. The "alkynyl" group contains at least one triple bond in the chain. Examples of alkenyl groups include ethynyl, propargyl, n-butynyl, iso-butynyl, pentynyl, or hexynyl. An alkynyl group can be unsubstituted or substituted.

The term "alkylene" or "alkylenyl" refers to a divalent alkyl radical. Any of the above-mentioned monovalent alkyl groups may be an alkylene by abstraction of a second hydrogen atom from the alkyl. As herein defined, alkylene may also be a C₁-C₆ alkylene. An alkylene may further be a C₁-C₄ alkylene. Typical alkylene groups include, but are not limited to, -CH₂-, -CH(CH₃)-, - C(CH₃)₂-, -CH₂CH₂-, -CH₂CH(CH₃)-, -CH₂C(CH₃)₂-, -CH₂CH₂CH₂-, -CH₂CH₂CH₂CH₂-, and the like.

"Cycloalkyl" means a saturated or partially unsaturated hydrocarbon monocyclic or polycyclic (*e.g.,* fused, bridged, or spiro rings) system having 3 to 10 carbon atoms (*e.g.,* C₃-C₁₂, C₃-C₁₀, or C₃-C₈). Examples of cycloalkyl groups include, without limitations, cyclopropyl, cyclobutyl, cyclopentyl, cyclohexyl, cycloheptanyl, cyclooctanyl, norboranyl, norborenyl, bicyclo[2.2.2]octanyl, bicyclo[2.2.2]octenyl, decahydronaphthalenyl, octahydro-1H-indenyl, cyclopentenyl, cyclohexenyl, cyclohexa-1,4-dienyl, cyclohexa-1,3-dienyl, 1,2,3,4-tetrahydronaphthalenyl, octahydropentalenyl, 3a,4,5,6,7,7a-hexahydro-1H-indenyl, 1,2,3,3a-tetrahydropentalenyl, bicyclo[3.1.0]hexanyl, bicyclo[2.1.0]pentanyl, spiro[3.3]heptanyl, bicyclo[2.2.1]heptanyl, bicyclo[2.2.1]hept-2-enyl, bicyclo[2.2.2]octanyl, 6-methylbicyclo[3.1.1]heptanyl, 2,6,6-trimethylbicyclo[3.1.1]heptanyl, adamantyl, and derivatives thereof. In the case of polycyclic cycloalkyl, only one of the rings in the cycloalkyl needs to be non-aromatic.

"Heterocyclyl", "heterocycle" or "heterocycloalkyl" refers to a saturated or partially unsaturated 3-10 membered monocyclic, 7-12 membered bicyclic (fused, bridged, or spiro rings), or 11-14 membered tricyclic ring system (fused, bridged, or spiro rings) having one or more heteroatoms (such as O, N, S, P, Se, or B), *e.g.,* 1 or 1-2 or 1-3 or 1-4 or 1-5 or 1-6 heteroatoms, or *e.g.,* 1, 2, 3, 4, 5, or 6 heteroatoms, independently selected from the group consisting of nitrogen, oxygen and sulfur, unless specified otherwise. Examples of heterocycloalkyl groups include, but are not limited to, piperidinyl, piperazinyl, pyrrolidinyl, dioxanyl, tetrahydrofuranyl, isoindolinyl, indolinyl, imidazolidinyl, pyrazolidinyl, oxazolidinyl, isoxazolidinyl, triazolidinyl, oxiranyl, azetidinyl, oxetanyl, thietanyl, 1,2,3,6-tetrahydropyridinyl, tetrahydropyranyl, dihydropyranyl, pyranyl, morpholinyl, tetrahydrothiopyranyl, 1,4-diazepanyl, 1,4-oxazepanyl, 2-oxa-5-azabicyclo[2.2.1]heptanyl, 2,5-diazabicyclo[2.2.1]heptanyl, 2-oxa-6-azaspiro[3.3]heptanyl, 2,6-diazaspiro[3.3]heptanyl, 1,4-dioxa-8-azaspiro[4.5]decanyl, 1,4-dioxaspiro[4.5]decanyl, 1-oxaspiro[4.5]decanyl, 1-azaspiro[4.5]decanyl, 3'H-spiro[cyclohexane-1,1'-isobenzofuran]-yl, 7H-spiro[cyclohexane-1,5'-furo[3,4-b]pyridin]-yl, 3'H-spiro[cyclohexane-1,1'-furo[3,4-c]pyridin]-yl, 3-azabicyclo[3.1.0]hexanyl, 3-azabicyclo[3.1.0]hexan-3-yl, 1,4,5,6-tetrahydropyrrolo[3,4-c]pyrazolyl, 3,4,5,6,7,8-hexahydropyrido[4,3-d]pyrimidinyl, 4,5,6,7-tetrahydro-1H-pyrazolo[3,4-c]pyridinyl, 5,6,7,8-tetrahydropyrido[4,3-d]pyrimidinyl, 2-azaspiro[3.3]heptanyl, 2-methyl-2-azaspiro[3.3]heptanyl, 2-azaspiro[3.5]nonanyl, 2-methyl-2-azaspiro[3.5]nonanyl, 2-azaspiro[4.5]decanyl, 2-methyl-2-azaspiro[4.5]decanyl, 2-oxa-azaspiro[3.4]octanyl, 2-oxa-azaspiro[3.4]octan-6-yl, and the like.

The term "haloalkyl" as used herein refers to an alkyl group, as defined herein, which is substituted one or more halogen. Examples of haloalkyl groups include, but are not limited to, trifluoromethyl, difluoromethyl, pentafluoroethyl, trichloromethyl, *etc.*

The term "haloalkoxy" as used herein refers to an alkoxy group, as defined herein, which is substituted one or more halogen. Examples of haloalkoxy groups include, but are not limited to, trifluoromethoxy, difluoromethoxy, pentafluoroethoxy, trichloromethoxy, etc.

The term "cyano" as used herein means a substituent having a carbon atom joined to a nitrogen atom by a triple bond, *i.e.,* C≡N.

The term "amine" as used herein refers to primary (R-NH₂, R ≠ H), secondary (R₂-NH, R ≠ H) and tertiary (R₃-N, R ≠ H) amines. A substituted amine is intended to mean an amine where at least one of the hydrogen atoms has been replaced by the substituent.

The term "amino" as used herein means a substituent containing at least one nitrogen atom. Specifically, -NH₂, -NH(alkyl) or alkylamino, -N(alkyl)₂ or dialkylamino, amide, carbamide, urea, and sulfamide substituents are included in the term "amino".

The term "solvate" refers to a complex of variable stoichiometry formed by a solute and solvent. Such solvents for the purpose of the disclosure may not interfere with the biological activity of the solute. Examples of suitable solvents include, but are not limited to, water, MeOH, EtOH, and AcOH. Solvates wherein water is the solvent molecule are typically referred to as hydrates. Hydrates include compositions containing stoichiometric amounts of water, as well as compositions containing variable amounts of water.

The term "isomer" refers to compounds that have the same composition and molecular weight but differ in physical and/or chemical properties. The structural difference may be in constitution (geometric isomers) or in the ability to rotate the plane of polarized light (stereoisomers). With regard to stereoisomers, the compounds of Formula (I) may have one or more asymmetric carbon atom and may occur as racemates, racemic mixtures and as individual enantiomers or diastereomers.

The present disclosure also contemplates isotopically labelled compound(s) of Formula (I), also referred to herein as "labeled isotope(s)", *e.g.,* those labeled with ²H and ¹⁴C. Deuterated *(i.e.,* ²H or D) and carbon-14 (*i.e.*, ¹⁴C) isotopes are particularly preferred for their ease of preparation and detectability. Further, substitution with heavier isotopes such as deuterium may afford certain therapeutic advantages resulting from greater metabolic stability (*e.g.,* increased *in vivo* half-life or reduced dosage requirements) and hence may be preferred in some circumstances. Isotopically labeled compounds of Formula (I) can generally be prepared by following procedures analogous to those disclosed in the Schemes and/or in the Examples herein below, by substituting an appropriate isotopically labeled reagent for a non-isotopically labeled reagent.

The disclosure also includes pharmaceutical compositions comprising an effective amount of a disclosed compound and a pharmaceutically acceptable carrier. Representative "pharmaceutically acceptable salts" include, *e.g*., water-soluble and water-insoluble salts, such as acetate, amsonate (4,4-diaminostilbene-2,2-disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumerate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

A "patient" or "subject" is a mammal, *e.g*., a human, mouse, rat, guinea pig, dog, cat, horse, cow, pig, or non-human primate, such as a monkey, chimpanzee, baboon, or rhesus.

An "effective amount" when used in connection with a compound is an amount effective for treating or preventing a disease or disorder in a subject as described herein.

The term "carrier", as used in this disclosure, encompasses carriers, excipients, and diluents, and means a material, composition, or vehicle, such as a liquid or solid filler, diluent, excipient, solvent or encapsulating material, involved in carrying or transporting a pharmaceutical agent from one organ, or portion of the body, to another organ, or portion of the body of a subject.

The term "treating" with regard to a subject, refers to improving at least one symptom of the subject's disorder. Treating includes curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a disclosed compound or pharmaceutically acceptable salt of the disclosed compound or a composition to a subject, or administering a prodrug derivative or analog of the compound or pharmaceutically acceptable salt of the compound or composition to the subject, which can form an equivalent amount of active compound within the subject's body.

The term "prodrug," as used in this disclosure, means a compound which is convertible in vivo by metabolic means (*e.g*., by hydrolysis) to a disclosed compound.

### Compounds of the Present Disclosure

The present disclosure relates to compounds or pharmaceutically acceptable salts, hydrates, solvates, prodrugs, stereoisomers, labeled isotopes, or tautomers thereof, capable of inhibiting cGAS, which are useful for the treatment of diseases and disorders associated with modulation of cGAS. The disclosure further relates to compounds, or pharmaceutically acceptable salts, hydrates, solvates, prodrugs, stereoisomers, labeled isotopes, or tautomers thereof, which can be useful for inhibiting cGAS.

In some aspects, the present disclosure provides a compound of Formula (I): 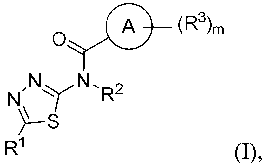 or a pharmaceutically acceptable salt, hydrate, solvate, prodrug, stereoisomer, labeled isotope, or tautomer thereof, wherein:
Ring A is a 5- to 10-membered heteroaryl or 3- to 10-membered heterocyclyl;
R¹ is H, -SR⁴, -S(O)R⁴, -S(O)₂R⁴, -N(R⁴)₂, -CN, C₁-C₆ alkyl, C₆-C₁₀ aryl, or 5- to 10-membered heteroaryl, wherein the alkyl, aryl or heteroaryl is optionally substituted with one or more R³, provided R¹ is not pyridinyl when Ring A is 1-isoquinolinyl;
R² is H or C₁-C₆ alkyl;
each R³ is independently halogen, -CN, -OR⁸, -NH₂, -NH(R⁶), -N(R⁶)(R⁷), -NHC(O)R⁸, - CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or
two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷, or
two geminal R³, together with the carbon atom to which they attached, form an oxo;
R⁴ is H, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl;
each R⁵ is independently -OH, halogen, -C(O)OH, C₁-C₆ alkyl, C₁-C₆ alkoxy, C₂-C₆ alkenyl, or C₂-C₆ alkynyl, wherein the alkyl, alkoxy, alkenyl, or alkynyl is optionally substituted with halogen, OH, or NH₂;
each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, - (CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl), or
two R⁶, together with the atoms to which they are attached, form a C₃-C₈ cycloalkyl, C₆-C₁₀ aryl, 5- to 10-membered heteroaryl, or 3- to 10-membered heterocyclyl, or
two geminal R⁶, together with the carbon atom to which they are attached, form an oxo;
each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, or
two R⁷, together with the atoms to which they are attached, form a C₃-C₈ cycloalkyl, C₆-C₁₀ aryl, 5- to 10-membered heteroaryl, or 3- to 10-membered heterocyclyl, or
two geminal R⁷, together with carbon atom to which they are attached, form oxo;
R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, or heteroaryl is optionally substituted with one or more R⁹;
R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl);
R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, heteroaryl, or aryl is optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, 3- to 10-membered heterocyclyl, 5- to 10-membered heteroaryl, or C₆-C₁₀ aryl;
m is an integer from 0 to 4;
n is an integer from 0 to 6; and
p is an integer from 0 to 6.

In some aspects, the present disclosure provides a compound of Formula (I), or a pharmaceutically acceptable salt, hydrate, solvate, prodrug, stereoisomer, or tautomer thereof, wherein:
Ring A is heteroaryl or heterocyclyl;
R¹ is **H,** -SR⁴, -S(O)R⁴, -S(O)₂R⁴, -N(R⁴)₂, -CN, C₁-C₆ alkyl, aryl, or heteroaryl, wherein the aryl or heteroaryl is optionally substituted with one or more R⁵, provided that R¹ is not pyridinyl when A is 1-isoquinolinyl;
R² is H or C₁-C₆ alkyl;
each R³ is independently halogen, -CN, -OR⁸, -NH₂, -NH(R⁶), -N(R⁶)(R⁷), -NHC(O)R⁸, - CO(OR⁸), -C(O)R⁸, -C(O)N(R⁸)₂, C₁-C₆ alkyl, C₃-C₈ cycloalkyl, heteroaryl, or heterocyclyl, wherein the alkyl, cycloalkyl, heterocyclyl or heteroaryl is optionally substituted with one or more R⁶,
or two R³, together with the intervening atoms, form a C₃-C₈ cycloalkyl, aryl, heterocyclyl, or heteroaryl, wherein the cycloalkyl, aryl, heterocyclyl, or heteroaryl is optionally substituted with one or more R⁷;
or two R³, together with the carbon atom to which they attached, form an oxo;
R⁴ is H, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl;
each R⁵ is independently -OH, halogen, -C(O)OH, C₁-C₆ alkyl, C₁-C₆ alkoxy, C₂-C₆ alkenyl, or C₂-C₆ alkynyl, wherein the alkyl, alkoxy, alkenyl, or alkynyl is optionally substituted with halogen, OH, or NH₂;
each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl,
or two R⁶, together with the intervening atoms, form a C₃-C₈ cycloalkyl, aryl, heterocyclyl, or heteroaryl,
or two R⁶, together with the carbon atom to which they are attached, form an oxo;
each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, heterocyclyl, heteroaryl, or aryl,
or two R⁷, together with the intervening atoms, form a C₃-C₈ cycloalkyl, aryl, heterocyclyl, or heteroaryl,
or two R⁷, together with carbon atom to which they are attached, form oxo;
each R⁸ is independently H, -OH, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, or heteroaryl is optionally substituted with one or more R⁹;
each R⁹ is independently -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₃ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl;
each R¹⁰ is independently C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, heteroaryl, or aryl is optionally substituted with one or more halogen, -OH, -CO(OH), -
(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl;
m is an integer from 0 to 4;
n is an integer from 0 to 6; and
p is an integer from 0 to 6.

In some embodiments, the compound of Formula (I), or a pharmaceutically acceptable salt, hydrate, solvate, prodrug, stereoisomer, labeled isotope, or tautomer thereof, is a compound of Formula (I-a):

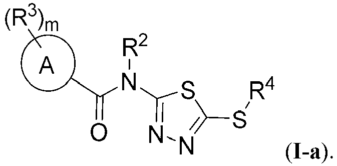

In some embodiments, the compound of Formula (I), or a pharmaceutically acceptable salt, hydrate, solvate, prodrug, stereoisomer, labeled isotope, or tautomer thereof, is a compound of Formula (I-a), wherein R⁴ is methyl.

In some embodiments, the compound of Formula (I), or a pharmaceutically acceptable salt, hydrate, solvate, prodrug, stereoisomer, labeled isotope, or tautomer thereof, is a compound of Formula (**I-a-1**) 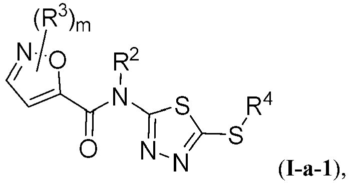 wherein m is 0, 1 or 2.

In some embodiments, the compound of Formula **(I),** or a pharmaceutically acceptable salt, hydrate, solvate, prodrug, stereoisomer, labeled isotope, or tautomer thereof, is a compound of Formula (**I-a-1**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-1-i**)

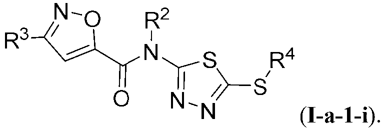

In some embodiments, the compound of Formula **(I),** or a pharmaceutically acceptable salt, hydrate, solvate, prodrug, stereoisomer, labeled isotope, or tautomer thereof, is a compound of Formula (**I-a-1-i**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-1-ii**) 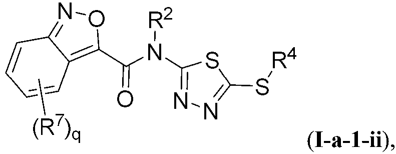 wherein q is 0, 1, 2, 3, or 4.

In some embodiments, the compound of Formula **(I),** or a pharmaceutically acceptable salt, hydrate, solvate, prodrug, stereoisomer, labeled isotope, or tautomer thereof, is a compound of Formula (**I-a-1-ii**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-2**) 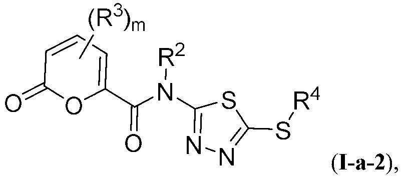 wherein m is 0, 1, 2, or 3.

In some embodiments, the compound of Formula **(I),** or a pharmaceutically acceptable salt, hydrate, solvate, prodrug, stereoisomer, labeled isotope, or tautomer thereof, is a compound of Formula (**I-a-2**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-2-i**): 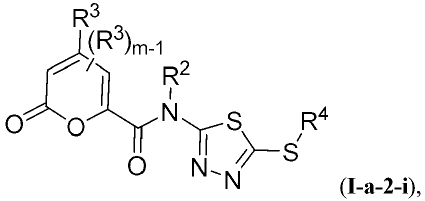 wherein m is 1, 2, or 3.

In some embodiments, the compound of Formula **(I),** or a pharmaceutically acceptable salt, hydrate, solvate, prodrug, stereoisomer, labeled isotope, or tautomer thereof, is a compound of Formula (**I-a-2-i**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-2-ii**) 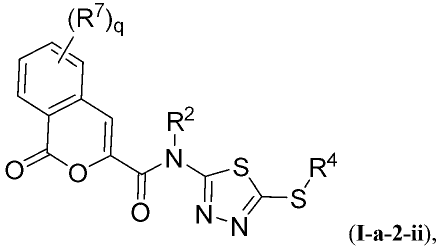 wherein q is 0, 1, 2, 3, or 4.

In some embodiments, the compound of Formula **(I),** or a pharmaceutically acceptable salt, hydrate, solvate, prodrug, stereoisomer, labeled isotope, or tautomer thereof, is a compound of Formula (**I-a-2-ii**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-5**) to Formula (**I-a-13**): 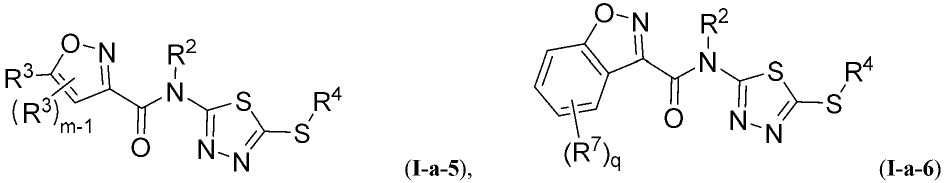 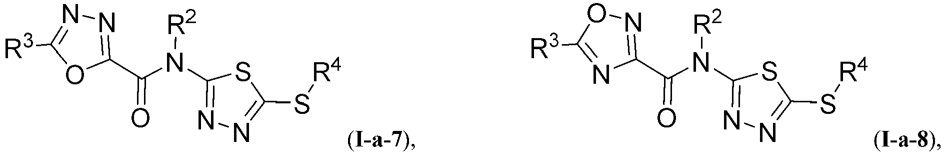 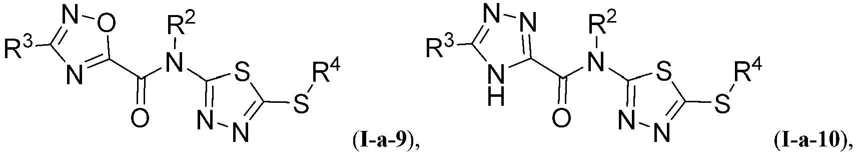 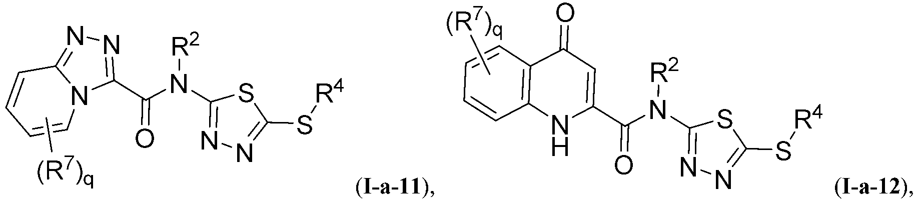 or 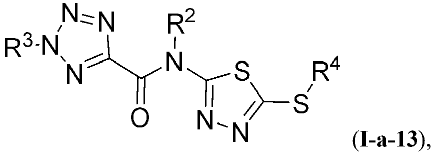 wherein q is 0, 1, 2, 3, or 4, and m is 0, 1, 2, or 3.

In some embodiments, the compound of Formula **(I),** or a pharmaceutically acceptable salt, hydrate, solvate, prodrug, stereoisomer, labeled isotope, or tautomer thereof, is a compound of Formula (**I-a-5**) to (**I-a-13**), wherein R⁴ is methyl.

In some embodiments, the compound of Formula **(I),** or a pharmaceutically acceptable salt, hydrate, solvate, prodrug, stereoisomer, labeled isotope, or tautomer thereof, is a compound of Formula (**I-a-14**) to Formula (**I-a-33**): 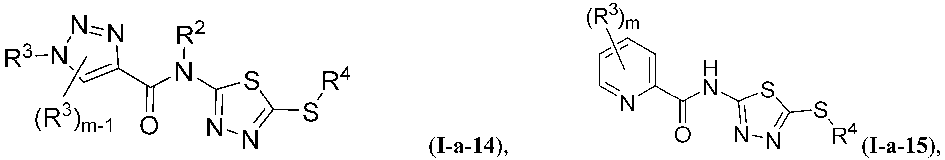 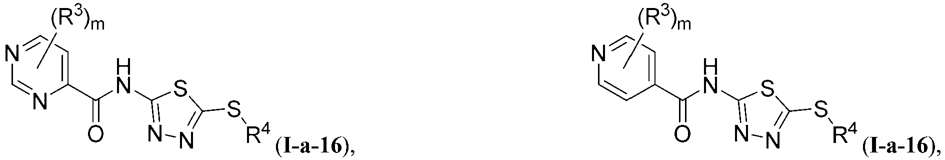 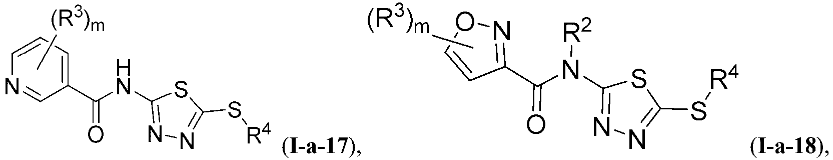 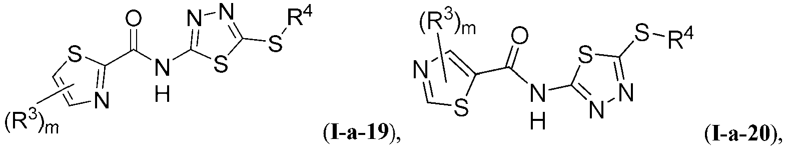 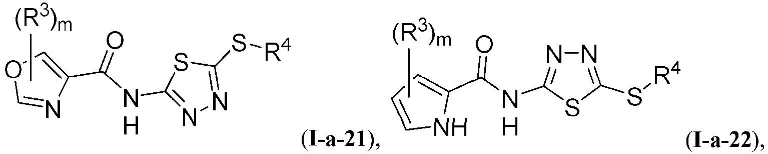 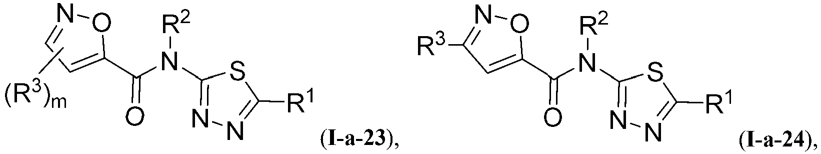 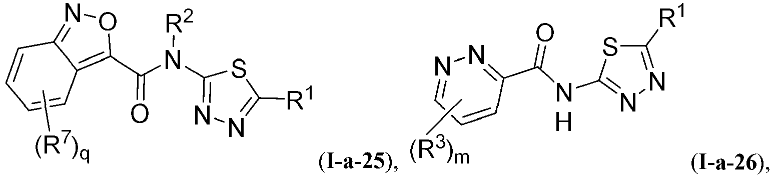 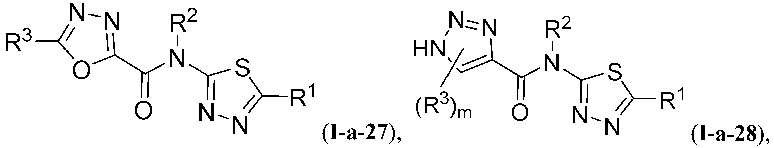 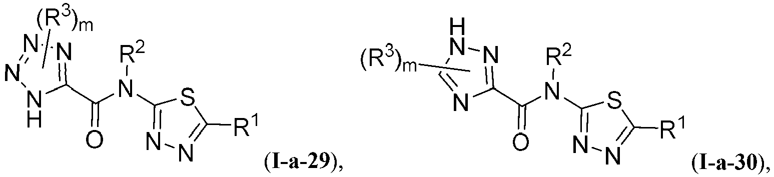 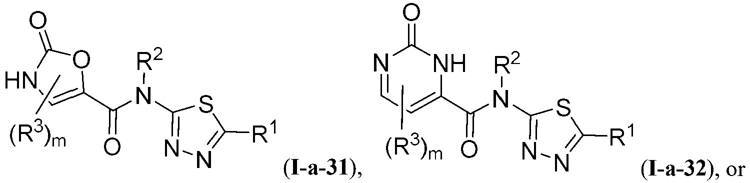 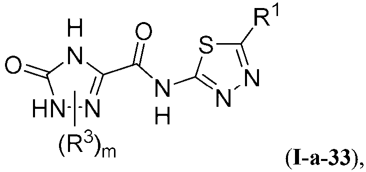 wherein q is 0, 1, 2, 3, or 4, and m is 0, 1, 2, or 3. It is understood that R³, if m >0, may replace the hydrogen of the NH group of Ring A.

In some embodiments, the compound of Formula **(I),** or a pharmaceutically acceptable salt, hydrate, solvate, prodrug, stereoisomer, labeled isotope, or tautomer thereof, is a compound of Formula (**I-a-14**) to (**I-a-33**), wherein R⁴ is methyl.

In some embodiments, R⁴ is methyl.

In some embodiments, R¹ is H, -SR⁴, -S(O)R⁴, -S(O)₂R⁴, -N(R⁴)₂, -CN, C₁-C₆ alkyl, C₆-C₁₀ aryl, or 5- to 10- membered heteroaryl, wherein the alkyl, aryl or heteroaryl is optionally substituted with one or more R⁵, provided R¹ is not pyridinyl when Ring A is 1-isoquinolinyl.

In some embodiments, R¹ is H, -SR⁴, -S(O)R⁴, -S(O)₂R⁴, -N(R⁴)₂, -CN, C₁-C₆ alkyl, C₆-C₁₀ aryl, or 5- to 10- membered heteroaryl, wherein the alkyl, aryl or heteroaryl is optionally substituted with one or more R⁵.

In some embodiments, R¹ is H, -SR⁴, -S(O)R⁴, -S(O)₂R⁴, -N(R⁴)₂, -CN, C₁-C₆ alkyl, C₆-C₁₀ aryl, or 5- to 10- membered heteroaryl.

In some embodiments, R¹ is H, -SR⁴, -S(O)R⁴, -S(O)₂R⁴, -N(R⁴)₂, -CN, C₁-C₆ alkyl, C₆-C₁₀ aryl, or 5- to 10- membered heteroaryl, wherein the alkyl, aryl or heteroaryl is substituted with one or more R⁵.

In some embodiments, R¹ is C₆-C₁₀ aryl optionally substituted with one or more R⁵.

In some embodiments, R¹ is C₆-C₁₀ aryl.

In some embodiments, R¹ is C₆-C₁₀ aryl substituted with one or more R⁵.

In some embodiments, R¹ is 5- to 10- membered heteroaryl optionally substituted with one or more R⁵.

In some embodiments, R¹ is 5- to 10- membered heteroaryl.

In some embodiments, R¹ is 5- to 10- membered heteroaryl substituted with one or more R⁵.

In some embodiments, R¹ is H, -SR⁴, -S(O)R⁴, -S(O)₂R⁴, -N(R⁴)₂, -CN, C₁-C₆ alkyl, aryl, or heteroaryl. In some embodiments, R¹ is H, -SR⁴, -S(O)R⁴, -S(O)₂R⁴, -N(R⁴)₂, -CN, C₁-C₆ alkyl, or aryl. In some embodiments, R¹ is H, -SR⁴, -S(O)R⁴, -S(O)₂R⁴, -N(R⁴)₂, -CN, or C₁-C₆ alkyl. In some embodiments, R¹ is H, -SR⁴, -S(O)R⁴, -S(O)₂R⁴, -N(R⁴)₂, or -CN. In some embodiments, R¹ is H, - SR⁴, -S(O)R⁴, -S(O)_{2R}⁴, or -N(R⁴)₂. In some embodiments, R¹ is H, -SR⁴, -S(O)R⁴, or -S(O)₂R⁴. In some embodiments, R¹ is H, -SR⁴, -or S(O)R⁴. In some embodiments, R¹ is H or -SR⁴. In some embodiments, R¹ is H. In some embodiments, R¹ is -SR⁴. In some embodiments, R¹ is -S(O)R⁴. In some embodiments, R¹ is -N(R⁴)₂. In other embodiments, R¹ is -CN. In some embodiments, R¹ is - S(O)₂R⁴. In some embodiments, R¹ is C₁-C₆ alkyl. In some embodiments, R¹ is aryl. In some embodiments, R¹ is heteroaryl.

In some embodiments, R¹ is aryl optionally substituted with one or more R⁵. In some embodiments, R¹ is heteroaryl optionally substituted with one or more R⁵.

In some embodiments, R¹ is hydrogen, -SCH₃, -SCH₂CH₃, -CH₃, -CHF₂, -CF₃, -CH₂CH₃, - (CH₂)₂CH₃, -(CH₂)₄CH₃, -CN, -CH₂CH₂OCH₃, -NH₂, -NH(CH₃), -N(CH₃)₂, -CH(CH₂CH₃), - CH(C₆H₆)(CH₂CH₃), 3-chlorophenyl, 3-fluorophenyl, 3-methoxyphenyl, 2-fluoropyridinyl. In some preferred embodiments, R¹ is -SCH₃.

In some embodiments, when R¹ is not pyridinyl when A is 1-isoquinolinyl.

In some embodiments, R² is C₁-C₆ alkyl. In some embodiments, R² is methyl (C₁). In some embodiments, R² is ethyl (C₂). In some embodiments, R² is n-propyl (C₃). In some embodiments, R² is isopropyl (C₃). In some embodiments, R² is n-butyl (C₄). In some embodiments, R² is tert-butyl (C₄). In some embodiments, R² is sec-butyl (C₄). In some embodiments, R² is isobutyl (C₄). In some embodiments, R² is n-pentyl (C₅). In some embodiments, R² is 3-pentanyl (C₅). In some embodiments, R² is amyl (C₅). In some embodiments, R² is neopentyl (C₅). In some embodiments, R² is 3-methyl-2-butanyl (C₅). In some embodiments, R² is tertiary amyl (C₅). In some embodiments, R² is n-hexyl (C₆).

In some embodiments, R₂ is H.

In some embodiments, R₂ is not methyl.

In some embodiments, R² is C₂-C₆ alkyl.

In some embodiments, R² is H or C₂-C₆ alkyl.

In some embodiments, Ring A is a 5- to 10-membered heteroaryl or 3- to 10-membered heterocyclyl.

In some embodiments, Ring A is a 5- to 10-membered heteroaryl.

In some embodiments, Ring A is a 3- to 10-membered heterocyclyl.

In some embodiments, Ring A is heteroaryl. In some embodiments, Ring A is heterocyclyl. In some embodiments, Ring A is 5- or 6-membered heterocyclyl. In some embodiments, Ring A is 5- or 6-membered heteroaryl.

In some embodiments, Ring A is 5-membered heterocyclyl.

In some embodiments, Ring A is 5-membered heterocyclyl comprising one heteroatom including, without limitation, tetrahydrofuranyl, dihydrofuranyl, tetrahydrothiophenyl, dihydrothiophenyl, pyrrolidinyl, dihydropyrrolyl and pyrrolyl-2,5-dione.

In some embodiments, Ring A is tetrahydrofuranyl. In some embodiments, Ring A is dihydrofuranyl. In some embodiments, Ring A is tetrahydrothiophenyl. In some embodiments, Ring A is dihydrothiophenyl. In some embodiments, Ring A is pyrrolidinyl. In some embodiments, Ring A is dihydropyrrolyl. In some embodiments, Ring A is pyrrolyl-2,5-dione.

In some embodiments, Ring A is 5-membered heterocyclyl comprising two heteroatoms including, without limitation, dioxolanyl, oxasulfuranyl, disulfuranyl, and oxazolidin-2-one.

In some embodiments, Ring A is dioxolanyl. In some embodiments, Ring A is oxasulfuranyl.

In some embodiments, Ring A is disulfuranyl. In some embodiments, Ring A is oxazolidin-2-one.

In some embodiments, Ring A is 5-membered heterocyclyl comprising three heteroatoms including, without limitation, triazolinyl, oxadiazolinyl, and thiadiazolinyl.

In some embodiments, Ring A is triazolinyl. In some embodiments, Ring A is oxadiazolinyl. In some embodiments, Ring A is thiadiazolinyl.

In some embodiments, Ring A is 6-membered heterocyclyl.

In some embodiments, Ring A is 6-membered heterocyclyl comprising one heteroatom include, without limitation, piperidinyl, tetrahydropyranyl, dihydropyridinyl, and thianyl.

In some embodiments, Ring A is piperidinyl. In some embodiments, Ring A is tetrahydropyranyl. In some embodiments, Ring A is dihydropyridinyl. In some embodiments, Ring A is thianyl.

In some embodiments, Ring A is 6-membered heterocyclyl comprising two heteroatoms including, without limitation, piperazinyl, morpholinyl, dithianyl, dioxanyl.

In some embodiments, Ring A is piperazinyl. In some embodiments, Ring A is morpholinyl. In some embodiments, Ring A is dithianyl. In some embodiments, Ring A is dioxanyl.

In some embodiments, Ring A is 6-membered heterocyclyl comprising three heteroatoms including, without limitation, triazinanyl.

In some embodiments, Ring A is triazinanyl.

In some embodiments, Ring A is 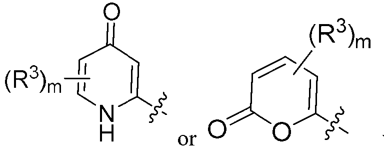 wherein m is 0, 1, 2, or 3.

In some embodiments, Ring A is 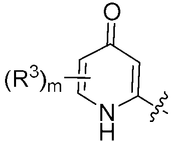 wherein m is 0, 1, 2, or 3.

In some embodiments, Ring A is 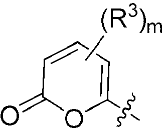 wherein m is 0, 1, 2, or 3.

In some embodiments, Ring A is 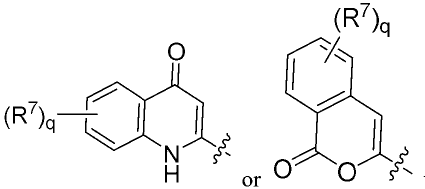 wherein q is 0, 1, 2, 3, or 4.

In some embodiments, Ring A is 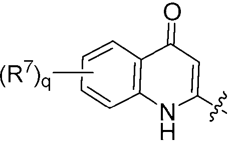 wherein q is 0, 1, 2, 3, or 4.

In some embodiments, Ring A is 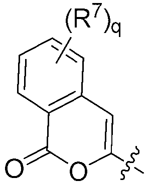 wherein q is 0, 1, 2, 3, or 4.

In some embodiments, q is an integer selected from 0 to 3. In some embodiments, q is an integer selected from 0 to 2. In some embodiments, q is an integer selected from 0 and 1. In some embodiments, q is an integer selected from 1 to 4. In some embodiments, q is an integer selected from 1 to 3. In some embodiments, q is an integer selected from 1 and 2. In some embodiments, q is an integer selected from 2 to 4. In some embodiments, q is an integer selected from 2 and 3.

In some embodiments, q is 0. In some embodiments, q is 1. In some embodiments, q is 2. In some embodiments, q is 3. In some embodiments, q is 4.

In some embodiments, Ring A is 5-membered heteroaryl.

In some embodiments, Ring A is 5-membered heteroaryl comprising one heteroatom include, without limitation, pyrrolyl, furanyl and thiophenyl.

In some embodiments, Ring A is pyrrolyl. In some embodiments, Ring A is furanyl. In some embodiments, Ring A is thiophenyl.

In some embodiments, Ring A is 5-membered heteroaryl comprising two heteroatoms including, without limitation, imidazolyl, pyrazolyl, oxazolyl, isoxazolyl, thiazolyl, and isothiazolyl.

In some embodiments, Ring A is imidazolyl. In some embodiments, Ring A is pyrazolyl. In some embodiments, Ring A is oxazolyl. In some embodiments, Ring A is isoxazolyl. In some embodiments, Ring A is thiazolyl. In some embodiments, Ring A is isothiazolyl.

In some embodiments, Ring A is 5-membered heteroaryl comprising three heteroatoms including, without limitation, triazolyl, oxadiazolyl, and thiadiazolyl.

In some embodiments, Ring A is triazolyl. In some embodiments, Ring A is oxadiazolyl. In some embodiments, Ring A is thiadiazolyl.

In some embodiments, Ring A is 5-membered heteroaryl comprising four heteroatoms including, without limitation, tetrazolyl.

In some embodiments, Ring A is tetrazolyl.

In some embodiments, Ring A is 6-membered heteroaryl.

In some embodiments, Ring A is 6-membered heteroaryl comprising one heteroatom including, without limitation, pyridinyl.

In some embodiments, Ring A is pyridinyl.

In some embodiments, Ring A is 6-membered heteroaryl comprising two heteroatoms including, without limitation, pyridazinyl, pyrimidinyl, and pyrazinyl.

In some embodiments, Ring A is pyridazinyl. In some embodiments, Ring A is pyrimidinyl.

In some embodiments, Ring A is pyridazinyl, pyrimidinyl, and pyrazinyl.

In some embodiments, Ring A is 6-membered heteroaryl comprising three or four heteroatoms including, without limitation, triazinyl and tetrazinyl, respectively.

In some embodiments, Ring A is triazinyl. In some embodiments, Ring A is tetrazinyl.

In some embodiments, Ring A is 5-membered heteroaryl comprising at least two heteroatoms independently selected from N, O, and S.

In some embodiments, Ring A is 5-membered heteroaryl comprising two heteroatoms independently selected from N, O, and S.

In some embodiments, Ring A is 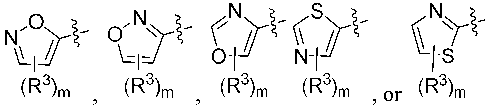 wherein m is 0, 1 or 2.

In some embodiments, Ring A is

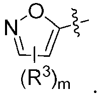

In some embodiments, Ring A is

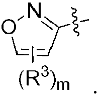

In some embodiments, Ring A is

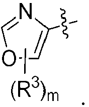

In some embodiments, Ring A is 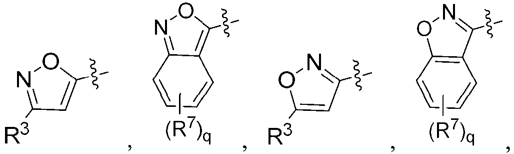 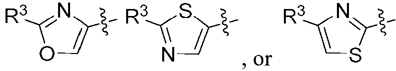 wherein q is 0, 1, 2, 3 or 4.

In some embodiments, Ring A is

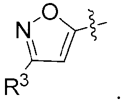

In some embodiments, Ring A is

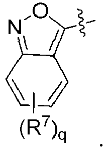

In some embodiments, Ring A is

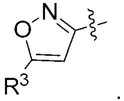

In some embodiments, Ring A is

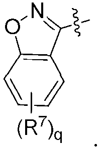

In some embodiments, Ring A is

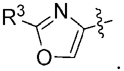

In some embodiments, Ring A is 5-membered heteroaryl comprising three heteroatoms independently selected from N, O, and S.

In some embodiments, Ring A is 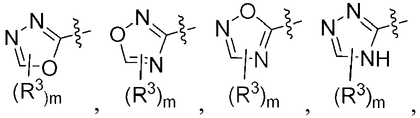 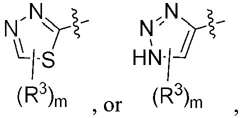 wherein m is 0 or 1.

In some embodiments, Ring A is

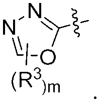

In some embodiments, Ring A is

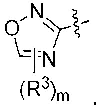

In some embodiments, Ring A is

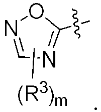

In some embodiments, Ring A is 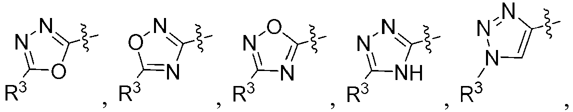 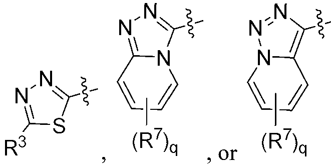 wherein q is 0, 1, 2, 3 or 4.

In some embodiments, Ring A is

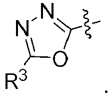

In some embodiments, Ring A is

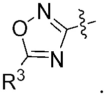

In some embodiments, Ring A is

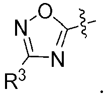

In some embodiments, Ring A is 5-membered heteroaryl comprising three heteroatoms independently selected from N and O.

In some embodiments, Ring A is

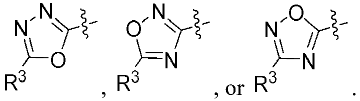

In some embodiments, Ring A is 5- to 6-membered heteroaryl comprising one or four heteroatoms independently selected from N, O, and S.

In some embodiments, Ring A is 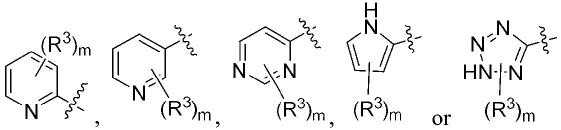 wherein m is 0, 1, 2, 3 or 4. It is understood that R³, if m >0, may replace the hydrogen of the NH group of Ring A.

In some embodiments, each R³ is independently halogen, -CN, -OR⁸, -NH₂, -NH(R⁶), -N(R⁶)(R⁷), -NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶.

In some embodiments, each R³ is independently halogen, -CN, -OR⁸, -NH₂, -NH(R⁶), -N(R⁶)(R⁷), -NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl.

In some embodiments, each R³ is independently halogen, -CN, -OR⁸, -NH₂, -NH(R⁶), -N(R⁶)(R⁷), -NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is substituted with one or more R⁶.

In some embodiments, each R³ is independently halogen, -CN, OR⁸, -NH₂, NH(R⁶), -N(R⁶)(R⁷), -NHC(O)R⁸, -CO(OR⁸), -C(O)R⁸, -C(O)N(R⁸)₂, C₁-C₆ alkyl, C₃-C₈ cycloalkyl, heteroaryl or heterocyclyl; wherein the alkyl, cycloalkyl, heterocyclyl or heteroaryl is optionally substituted with one or more R⁶. In some embodiments, each R³ independently is halogen, -CN, OR⁸, -NH₂, NH(R⁶), - N(R⁶)(R⁷), -NHC(O)R⁸, -CO(OR⁸), -C(O)R⁸, -C(O)N(R⁸)₂, C₁-C₆ alkyl, C₃-C₈ cycloalkyl, or heteroaryl. In some embodiments, each R³ independently is halogen, -CN, OR⁸, -NH₂, NH(R⁶), -N(R⁶)(R⁷), - NHC(O)R⁸, -CO(OR⁸), -C(O)R⁸, C₁-C₆ alkyl, or C₃-C₈ cycloalkyl. In some embodiments, each R³ independently is halogen, -CN, OR⁸, -NH₂, NH(R⁶), -N(R⁶)(R⁷), -NHC(O)R⁸, -CO(OR⁸), -C(O)R⁸, - C(O)N(R⁸)₂, or C₁-C₆ alkyl. In some embodiments, each R³ independently is halogen, -CN, OR⁸, -NH₂, NH(R⁶), -N(R⁶)(R⁷), -NHC(O)R⁸, -CO(OR⁸), or -C(O)R⁸, -C(O)N(R⁸)₂. In some embodiments, each R³ independently is halogen, -CN, OR⁸, -NH₂, NH(R⁶), -N(R⁶)(R⁷), -NHC(O)R⁸, or -C(O)OR⁸. In some embodiments, each R³ independently is halogen, -CN, OR⁸, -NH₂, NH(R⁶), -N(R⁶)(R⁷), or -NHC(O)R⁸. In some embodiments, each R³ independently is halogen, -CN, OR³, -NH₂, or N(H)R⁶. In some embodiments, each R³ independently is halogen -CN, OR⁸, or -NH₂,. In some embodiments, each R³ independently is halogen, -CN, or OR⁸. In some embodiments, each R³ independently is halogen or - CN. In some embodiments, each R³ is halogen. In some embodiments, each R³ is -CN. In some embodiments, each R³ is OR⁸. In some embodiments, each R³ is -NH₂. In some embodiments, each R³ is N(H)R⁶. In some embodiments, each R³ is -N(R⁶)(R⁷). In some embodiments, each R³ independently is -NHC(O)R⁸. In some embodiments, each R³ is -C(O)OR⁸. In some embodiments, each R³ is -C(O)R⁸. In some embodiments, each R³ independently is -C(O)N(R⁸)₂. In some embodiments, each R³ is C₁-C₆ alkyl. In some embodiments, each R³ is C₃-C₈ cycloalkyl. In some embodiments, each R³ is heteroaryl. In some embodiments, each R³ is C₁-C₆ alkyl optionally substituted with one or more R⁶. In some embodiments, each R³ is C₃-C₈ cycloalkyl optionally substituted with one or more R⁶. In some embodiments, each R³ is heteroaryl optionally substituted with one or more R⁶.

In some embodiments, R³ is not halogen or two geminal R³, together with carbon atom to which they are attached, do not form oxo.

In some embodiments, R³ is not halogen.

In some embodiments, two geminal R³, together with carbon atom to which they are attached, do not form oxo.

In some embodiments, each R³ is independently -CN, -OR⁸, -NH₂, -NH(R⁶), -N(R⁶)(R⁷), - NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷.

In some embodiments, R³ is not -SR⁸ or -S(O)₂R⁸.

In some embodiments, each R³ is independently halogen, -CN, -OR⁸, -NH₂, -NH(R⁶), -N(R⁶)(R⁷), -NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or
two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷, or
two geminal R³, together with the carbon atom to which they attached, form an oxo.

In some embodiments, two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷.

In some embodiments, two R³, together with the atoms to which they are attached, form a C₃-C₈ cycloalkyl, C₆-C₁₀ aryl, 5- to 10-membered heteroaryl, or 3- to 10-membered heterocyclyl.

In some embodiments, two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is substituted with one or more R⁷.

In some embodiments, two R³, together with the atoms to which they are attached, form a C₃-C₈ cycloalkyl optionally substituted with one or more R⁷.

In some embodiments, two R³, together with the atoms to which they are attached, form a C₃-C₈ cycloalkyl.

In some embodiments, two R³, together with the atoms to which they are attached, form a C₃-C₈ cycloalkyl substituted with one or more R⁷.

In some embodiments, two R³, together with the atoms to which they are attached, form a C₆-C₁₀ aryl optionally substituted with one or more R⁷.

In some embodiments, two R³, together with the atoms to which they are attached, form a C₆-C₁₀ aryl.

In some embodiments, two R³, together with the atoms to which they are attached, form a C₆-C₁₀ aryl substituted with one or more R⁷.

In some embodiments, two R³, together with the atoms to which they are attached, form a 5- to 10-membered heteroaryl optionally substituted with one or more R⁷.

In some embodiments, two R³, together with the atoms to which they are attached, form a 5- to 10-membered heteroaryl.

In some embodiments, two R³, together with the atoms to which they are attached, form a 5- to 10-membered heteroaryl substituted with one or more R⁷.

In some embodiments, two R³, together with the atoms to which they are attached, form a 3- to 10- membered heterocyclyl optionally substituted with one or more R⁷.

In some embodiments, two R³, together with the atoms to which they are attached, form a 3- to 10- membered heterocyclyl.

In some embodiments, two R³, together with the atoms to which they are attached, form a 3- to 10- membered heterocyclyl substituted with one or more R⁷.

In some embodiments, two R³, together with the intervening atoms, form a C₃-C₈ cycloalkyl, aryl, heterocyclyl, or heteroaryl, wherein the cycloalkyl, aryl, heterocyclyl, or heteroaryl is optionally substituted with one or more R⁷. In some embodiments, two R³, together with the intervening atoms, form a C₃-C₈ cycloalkyl, aryl, or heterocyclyl. In some embodiments, two R³, together with the intervening atoms, form a C₃-C₈ cycloalkyl or aryl. In some embodiments, two R³, together with the intervening atoms, form a C₃-C₈ cycloalkyl. In some embodiments, two R³, together with the intervening atoms, form an aryl. In some embodiments, two R³, together with the intervening atoms, form a heterocyclyl. In some embodiments, two R³, together with the intervening atoms, form a heteroaryl. In some embodiments, two R³, together with the intervening atoms, form a C₃-C₈ cycloalkyl optionally substituted with one or more R⁷. In some embodiments, two R³, together with the intervening atoms, form an aryl optionally substituted with one or more R⁷. In some embodiments, two R³, together with the intervening atoms, form a heterocyclyl optionally substituted with one or more R⁷. In some embodiments, two R³, together with the intervening atoms, form a heteroaryl optionally substituted with one or more R⁷.

In some embodiments, two geminal R³, together with the carbon atom to which they attached, form an oxo.

In some embodiments, two R³, together with the intervening atoms, form 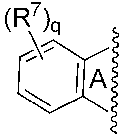 wherein q is 0, 1, 2, 3 or 4.

In some embodiments, R⁴ is H, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl.

In some embodiments, R⁴ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl.

In some embodiments, R⁴ is H.

In some embodiments, R⁴ is C₆-C₁₀ aryl.

In some embodiments, R⁴ is 5- to 10-membered heteroaryl.

In some embodiments, R⁴ is 3- to 10-membered heterocyclyl.

In some embodiments, R⁴ is C₁-C₆ alkyl. In some embodiments, R⁴ is methyl (C₁). In some embodiments, R⁴ is ethyl (C₂). In some embodiments, R⁴ is n-propyl (C₃). In some embodiments, R⁴ is isopropyl (C₃). In some embodiments, R⁴ is n-butyl (C₄). In some embodiments, R⁴ is tert-butyl (C₄). In some embodiments, R⁴ is sec-butyl (C₄). In some embodiments, R⁴ is isobutyl (C₄). In some embodiments, R⁴ is n-pentyl (C₅). In some embodiments, R⁴ is 3-pentanyl (C₅). In some embodiments, R⁴ is amyl (C₅). In some embodiments, R⁴ is neopentyl (C₅). In some embodiments, R⁴ is 3-methyl-2-butanyl (C₅). In some embodiments, R⁴ is tertiary amyl (C₅). In some embodiments, R⁴ is n-hexyl (C₆).

In some embodiments, R⁴ is H, C₁-C₆ alkyl, C₁-C₆ alkoxy, C₂-C₆ alkenyl, C₂-C₆ alkynyl, C₃-C₃ cycloalkyl, heterocyclyl, heteroaryl, or aryl. In some embodiments, R⁴ is H, C₁-C₆ alkyl, C₁-C₆ alkoxy, C₂-C₆ alkenyl, C₂-C₆ alkynyl, C₃-C₃ cycloalkyl, heterocyclyl, or heteroaryl. In some embodiments, R⁴ is H, C₁-C₆ alkyl, C₁-C₆ alkoxy, C₂-C₆ alkenyl, C₂-C₆ alkynyl, C₃-C₃ cycloalkyl, or heterocyclyl. In some embodiments R⁴ is H, C₁-C₆ alkyl, C₁-C₆ alkoxy, C₂-C₆ alkenyl, C₂-C₆ alkynyl, or C₃-C₃ cycloalkyl. In some embodiments, R⁴ is H, C₁-C₆ alkyl, C₁-C₆ alkoxy, C₂-C₆ alkenyl, or C₂-C₆ alkynyl. In some embodiments, R⁴ is H, C₁-C₆ alkyl, C₁-C₆ alkoxy, or C₂-C₆ alkenyl. In some embodiments, R⁴ is H, C₁-C₆ alkyl, or C₁-C₆ alkoxy. In some embodiments, R⁴ is H or C₁-C₆ alkyl. In some embodiments, R⁴ is H. In some embodiments, R⁴ is C₁-C₆ alkoxy. In some embodiments, R⁴ is C₂-C₆ alkenyl. In some embodiments, R⁴ is C₂-C₆ alkynyl. In some embodiments, R⁴ is C₃-C₈ cycloalkyl. In some embodiments, R⁴ is heterocyclyl. In some embodiments, R⁴ is heteroaryl. In some embodiments, R⁴ is aryl.

In some embodiments, each R⁵ is independently -OH, halogen, -C(O)OH, C₁-C₆ alkyl, C₁-C₆ alkoxy, C₂-C₆ alkenyl, C₂-C₆ alkynyl, wherein the alkyl, alkoxy, alkenyl, or alkynyl is optionally substituted with halogen, -OH, or -NH₂. In some embodiments, each R⁵ is halogen, -C(O)OH, C₁-C₆ alkyl, C₁-C₆ alkoxy, C₂-C₆ alkenyl, C₂-C₆ alkynyl, wherein the alkyl, alkoxy, alkenyl, or alkynyl is optionally substituted with halogen, OH, or NH₂.

In some embodiments, each R⁵ independently is -OH, halogen, -C(O)OH, C₁-C₆ alkyl, C₁-C₆ alkoxy, C₂-C₆ alkenyl, or C₂-C₆ alkynyl. In some embodiments, each R⁵ independently is halogen, - C(O)OH, C₁-C₆ alkyl, C₁-C₆ alkoxy, C₂-C₆ alkenyl, or C₂-C₆ alkynyl.

In some embodiments, each R⁵ independently is -OH.

In some embodiments, each R⁵ independently is halogen, -C(O)OH, C₁-C₆ alkyl, C₁-C₆ alkoxy, or C₂-C₆ alkenyl. In some embodiments, each R⁵ independently is halogen, -C(O)OH, C₁-C₆ alkyl, or C₁-C₆ alkoxy. In some embodiments, each R⁵ independently is halogen, -C(O)OH, or C₁-C₆ alkyl. In some embodiments, each R⁵ is independently halogen or -C(O)OH. In some embodiments, each R⁵ is halogen. In some embodiments, each R⁵ is C₁-C₆ alkyl. In some embodiments, each R⁵ is C₁-C₆ alkoxy. In some embodiments, each R⁵ is C₂-C₆ alkenyl. In some embodiments, each R⁵ is C₂-C₆ alkynyl. In some embodiments, each R⁵ is C₁-C₆ alkyl optionally substituted with halogen, -OH, or -NH₂. In some embodiments, each R⁵ is C₁-C₆ alkoxy optionally substituted with halogen, -OH, or -NH₂. In some embodiments, each R⁵ is C₂-C₆ alkenyl optionally substituted with halogen, -OH, or -NH₂. In some embodiments, each R⁵ is C₂-C₆ alkynyl optionally substituted with halogen, -OH, or -NH₂.

In some embodiments, R⁵ is not C₁-C₆ alkyl or C₁-C₆ alkoxy.

In some embodiments, each R⁵ is independently-OH, halogen, -C(O)OH, C₂-C₆ alkenyl, or C₂-C₆ alkynyl, wherein the alkenyl, or alkynyl is optionally substituted with halogen, OH, or NH₂.

In some embodiments, each R⁶ is independently halogen, OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, - (CH₂)ₙ-C(O)R⁸, -(CH₂).-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl.

In some embodiments, each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl, wherein the alkyl, alkoxy, alkenyl, alkynyl, cycloalkyl, aryl, heterocyclyl, or heteroaryl is optionally substituted with halogen, - OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl.

In some embodiments, each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl, wherein the alkyl, alkoxy, alkenyl, alkynyl, cycloalkyl, aryl, heterocyclyl, or heteroaryl is substituted with halogen, -OH, -NH₂, - NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently C₆-C₁₀ aryl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently C₆-C₁₀ aryl.

In some embodiments, each R⁶ is independently C₆-C₁₀ aryl substituted with halogen, -OH, - NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently 5- to 10- membered heteroaryl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, - (CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5-to 10-membered heteroaryl, 3- to 10-membered heterocyclyl, or -(C₁-C₆ alkyl)-(C₆-C₁₀ aryl).

In some embodiments, each R⁶ is independently 5- to 10- membered heteroaryl.

In some embodiments, each R⁶ is independently 5- to 10- membered heteroaryl substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently 3- to 10- membered heterocyclyl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, - (CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5-to 10-membered heteroaryl, 3- to 10-membered heterocyclyl, or -(C₁-C₆ alkyl)-(C₆-C₁₀ aryl).

In some embodiments, each R⁶ is independently 3- to 10- membered heterocyclyl.

In some embodiments, each R⁶ is independently 3- to 10- membered heterocyclyl substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, each R⁶ is independently halogen, -OH, oxo, - CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, or heteroaryl. In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, or heterocyclyl. In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, or C₃-C₈ cycloalkyl. In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, C₂-C₆ alkenyl, or C₂-C₆ alkynyl. In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, C₁-C₆ alkoxy, or C₂-C₆ alkenyl. In some embodiments, each R⁶ is independently halogen, -OH, oxo, -CO(OR⁸), -(CH₂)ₙ-NHC(O)-(CH₂)ₚ-OR⁸, -CN, C₁-C₆ alkyl, or C₁-C₆ alkoxy. In some embodiments, each R⁶ is independently halogen, -OH, oxo, -CO(OR⁸), -(CH₂)ₙ-NHC(O)-(CH₂)ₚ-OR⁸, -CN, or C₁-C₆ alkyl. In some embodiments, each R⁶ is independently halogen, - OH, oxo, -CO(OR⁸), -(CH₂)ₙ-NHC(O)-(CH₂)ₚ-OR⁸, or -CN. In some embodiments, each R⁶ is independently halogen, -OH, oxo, -CO(OR⁸), or -(CH₂)ₙ-NHC(O)-(CH₂)ₚ-OR⁸. In some embodiments, each R⁶ is independently halogen, -OH, oxo, or -CO(OR⁸). In some embodiments, each R⁶ is independently halogen, -OH, or oxo. In some embodiments, each R⁶ is independently halogen or -OH. In some embodiments, each R⁶ is halogen.

In some embodiments, each R⁶ is OH. In some embodiments, each R⁶ is oxo. In some embodiments, each R⁶ is -CO(OR⁸). In some embodiments, each R⁶ is -(CH₂)ₙ-NHC(O)-(CH₂)ₚ-OR⁸. In some embodiments, each R⁶ is CN. In some embodiments, each R⁶ is C₁-C₆ alkyl. In some embodiments, each R⁶ is C₁-C₆ alkoxy. In some embodiments, each R⁶ is C₂-C₆ alkenyl. In some embodiments, each R⁶ is C₂-C₆ alkynyl. In some embodiments, each R⁶ is C₃-C₈ cycloalkyl. In some embodiments, each R⁶ is heterocyclyl. In some embodiments, each R⁶ is heteroaryl. In some embodiments, each R⁶ is aryl.

In some embodiments, each R⁶ is C₁-C₆ alkyl optionally substituted with halogen, OH, NH₂, - NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, each R⁶ is C₁-C₆ alkyl optionally substituted with halogen, OH, NH₂, - NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is C₁-C₆ alkoxy optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is C₂-C₆ alkenyl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, - (CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is C₂-C₆ alkynyl optionally substituted with halogen, -OH, -NH₂, -NHC(O)OR⁸, -(CH₂)ₙ-NHC(O)R⁸, - (CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is C₃-C₈ cycloalkyl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is heterocyclyl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, - (CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is heteroaryl optionally substituted with halogen, -OH, -NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, - (CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl. In some embodiments, each R⁶ is aryl optionally substituted with halogen, -OH, -NH₂, - NHC(O)OR⁸, -(CH₂)ₙ-NHC(O)R⁸, -(CH₂)ₙ-NHC(O)-(CH₂)ₚ-OR⁸, -(CH₂)ₙ-NHR³, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl.

In some embodiments, two R⁶, together with the atoms to which they are attached, form a C₃-C₈ cycloalkyl, C₆-C₁₀ aryl, 5- to 10- membered heteroaryl, or 3- to 10- membered heterocyclyl.

In some embodiments, two R⁶, together with the atoms to which they are attached, form a C₆-C₁₀ aryl.

In some embodiments, two R⁶, together with the atoms to which they are attached, form a 5- to 10- membered heteroaryl.

In some embodiments, two R⁶, together with the atoms to which they are attached, form a 3- to 10- membered heterocyclyl.

In some embodiments, two R⁶, together with the intervening atoms, form a C₃-C₈ cycloalkyl, aryl, heterocyclyl, or heteroaryl. In some embodiments, two R⁶, together with the intervening atoms, form a C₃-C₈ cycloalkyl. In some embodiments, two R⁶, together with the intervening atoms, form an aryl. In some embodiments, two R⁶, together with the intervening atoms, form a heterocyclyl. In some embodiments, two R⁶, together with the intervening atoms, form a heteroaryl.

In some embodiments, two geminal R⁶, together with the carbon atom to which they are attached, form an oxo.

In some embodiments, each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, each R⁷ is independently C₆-C₁₀ aryl.

In some embodiments, each R⁷ is independently 5- to 10- membered heteroaryl.

In some embodiments, each R⁷ is independently 3- to 10- membered heterocyclyl.

In some embodiments, each R⁷ is independently halogen, -OH, -C(O)OR⁸, C₁-C₆ alkyl, C₁-C₆ alkoxy, C₂-C₆ alkenyl, is C₂-C₆ alkynyl, C₃-C₈ cycloalkyl, heterocyclyl, heteroaryl, or aryl. In some embodiments, each R⁷ is halogen. In some embodiments, each R⁷ is -OH. In some embodiments, each R⁷ is -C(O)OR⁸. In some embodiments, each R⁷ is C₁-C₆ alkyl. In some embodiments, each R⁷ is C₁-C₆ alkoxy. In some embodiments, each R⁷ is C₂-C₆ alkenyl. In some embodiments, each R⁷ is C₂-C₆ alkynyl. In some embodiments, each R⁷ is C₃-C₈ cycloalkyl. In some embodiments, each R⁷ is heterocyclyl. In some embodiments, each R⁷ is heteroaryl. In some embodiments, each R⁷ is aryl.

In some embodiments, two R⁷, together with the atoms to which they are attached, form a C₃-C₈ cycloalkyl, C₆-C₁₀ aryl, 5- to 10- membered heteroaryl, or 3- to 10- membered heterocyclyl.

In some embodiments, two R⁷, together with the atoms to which they are attached, form a C₆-C₁₀ aryl.

In some embodiments, two R⁷, together with the atoms to which they are attached, form a 5- to 10- membered heteroaryl.

In some embodiments, two R⁷, together with the atoms to which they are attached, form a 3- to 10- membered heterocyclyl.

In some embodiments, two R⁷, together with the intervening atoms, form a C₃-C₈ cycloalkyl, aryl, heterocyclyl, or heteroaryl. In some embodiments, two R⁷, together with the intervening atoms, form a C₃-C₈ cycloalkyl. In some embodiments, two R⁷, together with the intervening atoms, form an aryl. In some embodiments, two R⁷, together with the intervening atoms, form a heterocyclyl. In some embodiments, two R⁷, together with the intervening atoms, form a heteroaryl.

In some embodiments, two geminal R⁷, together with carbon atom to which they are attached, form oxo.

In some embodiments, R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl, wherein the alkyl, alkoxy, alkenyl, alkynyl, cycloalkyl, heterocyclyl, or heteroaryl is optionally substituted with one or more R⁹.

In some embodiments, R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl.

In some embodiments, R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10- membered heterocyclyl, wherein the alkyl, alkoxy, alkenyl, alkynyl, cycloalkyl, heterocyclyl, or heteroaryl is substituted with one or more R⁹.

In some embodiments, R⁸ is C₆-C₁₀ aryl optionally substituted with one or more R⁹.

In some embodiments, R⁸ is C₆-C₁₀ aryl.

In some embodiments, R⁸ is C₆-C₁₀ aryl substituted with one or more R⁹.

In some embodiments, R⁸ is 5- to 10- membered heteroaryl optionally substituted with one or more R⁹.

In some embodiments, R⁸ is 5- to 10- membered heteroaryl.

In some embodiments, R⁸ is 5- to 10- membered heteroaryl substituted with one or more R⁹.

In some embodiments, R⁸ is 3- to 10- membered heterocyclyl optionally substituted with one or more R⁹.

In some embodiments, R⁸ is 3- to 10- membered heterocyclyl.

In some embodiments, R⁸ is 3- to 10- membered heterocyclyl substituted with one or more R⁹.

In some embodiments, R⁸ is H, -OH, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, or heteroaryl is optionally substituted with one or more R⁹.

In some embodiments, R⁸ is H. In some embodiments, R⁸ is -OH. In some embodiments, R⁸ is C₁-C₆ alkyl. In some embodiments, R⁸ is C₁-C₆ alkoxy. In some embodiments, R⁸ is C₂-C₆ alkenyl. In some embodiments, R⁸ is C₂-C₆ alkynyl. In some embodiments, R⁸ is C₃-C₈ cycloalkyl. In some embodiments, R⁸ is, heteroaryl. In some embodiments, R⁸ is aryl. In some embodiments, R⁸ is C₁-C₆ alkyl optionally substituted with one or more R⁹. In some embodiments, R⁸ is C₁-C₆ alkoxy. In some embodiments, R⁸ is C₂-C₆ alkenyl optionally substituted with one or more R⁹. In some embodiments, R⁸ is C₂-C₆ alkynyl optionally substituted with one or more R⁹. In some embodiments, R⁸ is C₃-C₈ cycloalkyl optionally substituted with one or more R⁹. In some embodiments, R⁸ is heteroaryl optionally substituted with one or more R⁹. In some embodiments, R⁸ is aryl optionally substituted with one or more R⁹.

In some embodiments, R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₙ-NHC(O)R¹⁰, - (CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10-membered heterocyclyl.

In some embodiments, R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10- membered heteroaryl, or 3- to 10-membered heterocyclyl, wherein the alkyl, alkoxy, alkenyl, alkynyl, cycloalkyl, aryl, heterocyclyl, or heteroaryl is substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl).

In some embodiments, R⁹ is C₆-C₁₀ aryl optionally substituted with halogen, -OH, -NH₂, - NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is C₆-C₁₀ aryl.

In some embodiments, R⁹ is C₆-C₁₀ aryl substituted with halogen, -OH, -NH₂, -NHC(O)OR¹⁰, - (CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is 5- to 10-membered heteroaryl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, - (CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is 5- to 10-membered heteroaryl.

In some embodiments, R⁹ is 5- to 10-membered heteroaryl substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is 3- to 10- membered heterocyclyl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, - (CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is 3- to 10- membered heterocyclyl.

In some embodiments, R⁹ is 3- to 10- membered heterocyclyl substituted with halogen, -OH, - NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, C₆-C₁₀ aryl, 5- to 10-membered heteroaryl, 3- to 10- membered heterocyclyl, or -(C₁-C₆ alkyl)-(C₆-C₁₀ aryl).

In some embodiments, R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₃ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, - OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, - (CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl.

In some embodiments, R⁹ is -(CH₂)ₙ-NHC(O)-(CH₂)ₚ-OR¹⁰. In some embodiments, R⁹ is CN. In some embodiments, R⁹ is C₁-C₆ alkyl. In some embodiments, R⁹ is C₁-C₆ alkoxy. In some embodiments, R⁹ is C₂-C₆ alkenyl. In some embodiments, R⁹ is C₂-C₆ alkynyl. In some embodiments, R⁹ is C₃-C₈ cycloalkyl. In some embodiments, R⁹ is heterocyclyl. In some embodiments, R⁹ is heteroaryl. In some embodiments, R⁹ is aryl.

In some embodiments, R⁹ is C₁-C₆ alkyl optionally substituted with halogen, -OH, -NH₂, - NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl. In some embodiments, R⁹ is C₁-C₆ alkoxy optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, - (CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl. In some embodiments, R⁹ is C₂-C₆ alkenyl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, heteroaryl, heterocyclyl, or alkylaryl. In some embodiments, R⁹ is C₂-C₆ alkynyl optionally substituted with halogen, -OH, -NH₂, -NHC(O)OR¹⁰, - (CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl. In some embodiments, R⁹ is C₃-C₈ cycloalkyl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl. In some embodiments, R⁹ is heterocyclyl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, heteroaryl, heterocyclyl, or alkylaryl. In some embodiments, R⁹ is heteroaryl optionally substituted with halogen, -OH, -NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl. In some embodiments, R⁹ is aryl optionally substituted with halogen, -OH, - NH₂, -NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl.

In some embodiments, R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl, wherein the alkyl, alkoxy, alkenyl, alkynyl, cycloalkyl, heterocyclyl, heteroaryl, or aryl is optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl, wherein the alkyl, alkoxy, alkenyl, alkynyl, cycloalkyl, heterocyclyl, heteroaryl, or aryl is substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl.

In some embodiments, R¹⁰ is C₆-C₁₀ aryl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is C₆-C₁₀ aryl.

In some embodiments, R¹⁰ is C₆-C₁₀ aryl substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is 5- to 10-membered heteroaryl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl.

In some embodiments, R¹⁰ is 5- to 10-membered heteroaryl.

In some embodiments, R¹⁰ is 5- to 10-membered heteroaryl substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is 3- to 10- membered heterocyclyl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl.

In some embodiments, R¹⁰ is 3- to 10- membered heterocyclyl.

In some embodiments, R¹⁰ is 3- to 10- membered heterocyclyl substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10- membered heterocyclyl.

In some embodiments, R¹⁰ is C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, heteroaryl, or aryl is optionally substituted with one or more halogen, -OH, -CO(OH), - (CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl.

In some embodiments, R¹⁰ is C₁-C₆ alkyl. In some embodiments, R¹⁰ is C₁-C₆ alkoxy. In some embodiments, R¹⁰ is C₂-C₆ alkenyl. In some embodiments, R¹⁰ is C₂-C₆ alkynyl. In some embodiments, R¹⁰ is C₃-C₈ cycloalkyl. In some embodiments, R¹⁰ is heterocyclyl. In some embodiments, R¹⁰ is heteroaryl. In some embodiments, R¹⁰ is aryl.

In some embodiments, R¹⁰ is C₁-C₆ alkyl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is C₁-C₆ alkoxy optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is C₂-C₆ alkenyl optionally substituted with one or more halogen, - OH, -CO(OH), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is C₂-C₆ alkynyl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is C₃-C₈ cycloalkyl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-Cₐ cycloalkyl, heterocyclyl, heteroaryl, or aryl. In some embodiments, R¹⁰ is heterocyclyl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is heteroaryl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl. In some embodiments, R¹⁰ is aryl optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl.

In some embodiments, q is an integer selected from 0 to 4. In some embodiments, q is an integer selected from 0 to 3. In some embodiments, q is an integer selected from 0 to 2. In some embodiments, q is an integer selected from 0 and 1. In some embodiments, q is an integer selected from 1 to 4. In some embodiments, q is an integer selected from 1 to 3. In some embodiments, q is an integer selected from 1 and 2. In some embodiments, q is an integer selected from 2 to 4. In some embodiments, q is an integer selected from 2 and 3.

In some embodiments, q is 0. In some embodiments, q is 1. In some embodiments, q is 2. In some embodiments, q is 3. In some embodiments, q is 4.

In some embodiments, m is an integer selected from 0 to 4. In some embodiments, m is an integer selected from 0 to 3. In some embodiments, m is an integer selected from 0 to 2. In some embodiments, m is an integer selected from 0 and 1. In some embodiments, m is an integer selected from 1 to 4. In some embodiments, m is an integer selected from 1 to 3. In some embodiments, m is an integer selected from 1 and 2. In some embodiments, m is an integer selected from 2 to 4. In some embodiments, m is an integer selected from 2 and 3.

In some embodiments, m is 0. In some embodiments, m is 1. In some embodiments, m is 2. In some embodiments, m is 3. In some embodiments, m is 4.

In some embodiments, n is an integer selected from 0 to 6. In some embodiments, n is an integer selected from 0 to 5. In some embodiments, n is an integer selected from 0 to 4. In some embodiments, n is an integer selected from 0 to 3. In some embodiments, n is an integer selected from 0 to 2. In some embodiments, n is an integer selected from 0 and 1. In some embodiments, n is an integer selected from 1 to 6. In some embodiments, n is an integer selected from 1 to 5. In some embodiments, n is an integer selected from 1 to 4. In some embodiments, n is an integer selected from 1 to 3. In some embodiments, n is an integer selected from 1 and 2. In some embodiments, n is an integer selected from 2 to 6. In some embodiments, n is an integer selected from 2 to 5. In some embodiments, n is an integer selected from 2 to 4. In some embodiments, n is an integer selected from 2 and 3. In some embodiments, n is an integer selected from 3 to 6. In some embodiments, n is an integer selected from 3 to 5. In some embodiments, n is an integer selected from 3 and 4. In some embodiments, n is an integer selected from 4 to 6. In some embodiments, n is an integer selected from 4 and 5.

In some embodiments, n is 0. In some embodiments, n is 1. In some embodiments, n is 2. In some embodiments, n is 3. In some embodiments, n is 4. In some embodiments, n is 5. In some embodiments, n is 6.

In some embodiments, p is an integer selected from 0 to 6. In some embodiments, p is an integer selected from 0 to 5. In some embodiments, n is an integer selected from 0 to 4. In some embodiments, p is an integer selected from 0 to 3. In some embodiments, p is an integer selected from 0 to 2. In some embodiments, p is an integer selected from 0 and 1. In some embodiments, p is an integer selected from 1 to 6. In some embodiments, p is an integer selected from 1 to 5. In some embodiments, p is an integer selected from 1 to 4. In some embodiments, p is an integer selected from 1 to 3. In some embodiments, p is an integer selected from 1 and 2. In some embodiments, p is an integer selected from 2 to 6. In some embodiments, p is an integer selected from 2 to 5. In some embodiments, p is an integer selected from 2 to 4. In some embodiments, p is an integer selected from 2 and 3. In some embodiments, p is an integer selected from 3 to 6. In some embodiments, p is an integer selected from 3 to 5. In some embodiments, p is an integer selected from 3 and 4. In some embodiments, p is an integer selected from 4 to 6. In some embodiments, p is an integer selected from 4 and 5.

In some embodiments, p is 0. In some embodiments, p is 1. In some embodiments, p is 2. In some embodiments, p is 3. In some embodiments, p is 4. In some embodiments, p is 5. In some embodiments, p is 6.

In some embodiments, when Ring A is 5-membered heteroaryl and R¹ is -SR⁴, then R⁴ is -CH₃.

In some embodiments, when Ring A is 6-membered heteroaryl and R¹ is -SR⁴, then R⁴ is -CH₃.

In some embodiments, when Ring A is pyridinyl, then pyridinyl is substituted by at least one R³ and R³ is not halogen or two geminal R³, together with carbon atom to which they are attached, do not form oxo.

In some embodiments, when Ring A is pyridinyl, then pyridinyl is substituted by at least one R³ and each R³ is independently -CN, -OR⁸, -NH₂, -NH(R⁶), -N(R⁶)(R⁷), -NHC(O)R⁸, -CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷.

In some embodiments, when Ring A is pyrimidinyl substituted with at least one R³, then R³ is not -SR⁸ or -S(O)₂R⁸.

In some embodiments, when Ring A is pyrimidinyl substituted with at least one R³, then each R³ is independently halogen, -CN, -OR⁸, -NH₂, -NH(R⁶), -N(R⁶)(R⁷), -NHC(O)R⁸, -CO(OR⁸), -C(O)R⁸, - C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or
two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷, or
two geminal R³, together with the carbon atom to which they attached, form an oxo.

In some embodiments, when Ring A is 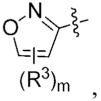 then R³ is a 5-membered heteroaryl substituted with at least one R⁶.

In some embodiments, when Ring A is 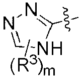 and R¹ is phenyl, then the phenyl is substituted with at least one R⁵.

In some embodiments, when Ring A is 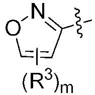 and m is 1 or 2, then R¹ is phenyl substituted by at least one R⁵ and R⁵ is not C₁-C₆ alkyl or C₁-C₆ alkoxy.

In some embodiments, when Ring A is 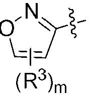 and m is 1 or 2, then R¹ is phenyl substituted by at least one R⁵ and each R⁵ is independently-OH, halogen, -C(O)OH, C₂-C₆ alkenyl, or C₂-C₆ alkynyl, wherein the alkenyl, or alkynyl is optionally substituted with halogen, OH, or NH₂.

In some embodiments, when Ring A is 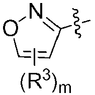 and R¹ is 6-membered heteroaryl, then R² is not methyl.

In some embodiments, when Ring A is 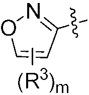 and R¹ is 6-membered heteroaryl, then R² is H or C₂-C₆ alkyl.

In some embodiments, the compound is not N-(5-(3-chlorophenyl)-1,3,4-thiadiazol-2-yl)-3-methylisoxazole-5-carboxamide.

In some embodiments, the compound is not N-(5-(4-chlorophenyl)-1,3,4-thiadiazol-2-yl)-3-methoxyisoxazole-5-carboxamide.

In some embodiments, the compound of Formula (I) is a compound selected from compounds provided in **Table 1.**

In some embodiments, the compound of Formula (I) is a compound selected from compounds provided in **Table 1,** or a pharmaceutically acceptable salt, hydrate, solvate, prodrug, stereoisomer, labeled isotope, or tautomer thereof.

In some embodiments, the compound of Formula (I) is a compound selected from compounds provided in **Table 1,** or a pharmaceutically acceptable salt thereof.

In some embodiments, the compound of Formula (I) is a compound selected from compounds provided in **Table 1,** or a hydrate thereof.

In some embodiments, the compound of Formula (I) is a compound selected from compounds provided in **Table 1,** or a solvate thereof.

In some embodiments, the compound of Formula (I) is a compound selected from compounds provided in **Table 1,** or prodrug thereof.

In some embodiments, the compound of Formula (I) is a compound selected from compounds provided in **Table 1,** or a stereoisomer thereof.

In some embodiments, the compound of Formula (I) is a compound selected from compounds provided in **Table 1,** or a labeled isotope thereof.

In some embodiments, the compound of Formula (I) is a compound selected from compounds provided in **Table 1,** or a tautomer thereof.

Stereochemistry arbitrarily assigned is designated with an Asterix (*).

| **Table 1.** | | | |
|---|---|---|---|
| **#** | **Compound Name/Structure** | **#** | **Compound Name/Structure** |
| 1 | methyl 5-((5-(2-chlorophenyl)-1,3,4-thiadiazol-2-yl)carbamoyl)isoxazole-3-carboxylate 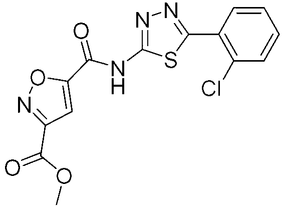 | 143 | *N*-(5-(2-(trifluoromethyl)phenyl)-1,3,4-thiadiazol-2-yl)benzo[c]isoxazole-3-carboxamide 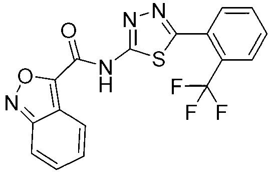 |
| 2 | 5-((5-(2-chlorophenyl)-1,3,4-thiadiazol-2-yl)carbamoyl)isoxazole-3-carboxylic acid 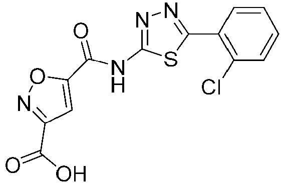 | 144 | *N*-[5-(2-bromophenyl)-1,3,4-thiadiazol-2-yl]- 2,1-benzoxazole-3-carboxamide 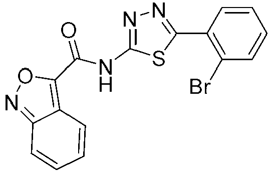 |
| 3 | *tert*-butyl (5-((5-(2-chlorophenyl)-1,3,4-thiadiazol-2-yl)carbamoyl)isoxazol-3-yl)carbamate 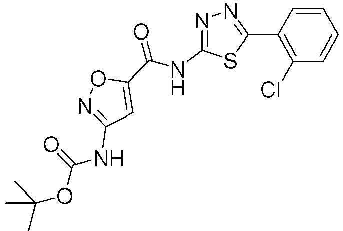 | 145 | *N*-(5-(1H-pyrazol-4-yl)-1,3,4-thiadiazol-2-yl)benzo[c]isoxazole-3-carboxamide 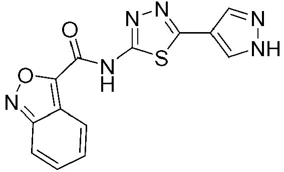 |
| 4 | 3-amino-*N-*(5-(chlorophenyl)-1,3,4-thiadiazol-2-yl)isoxazole-5-carboxamide 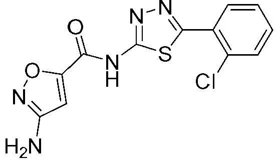 | 146 | *N*-(5-(1-methyl-1H-pyrazol-5-yl)-1,3,4-thiadiazol-2-yl)benzo[c]isoxazole-3-carboxamide 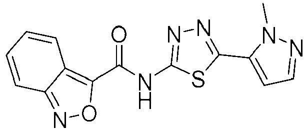 |
| 5 | 3-acetamido-*N*-(5-(2-chlorophenyl)-1,3,4-thiadiazol-2-yl)isoxazole-5-carboxamide 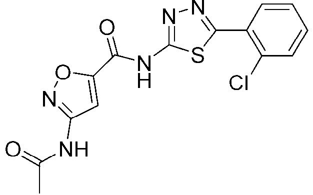 | 147 | *N*-(5-(2-(difluoromethyl)phenyl)-1,3,4-thiadiazol-2-yl)benzo[c]isoxazole-3-carboxamide 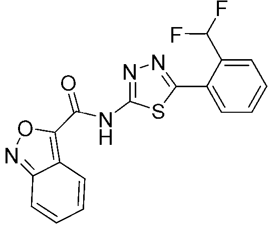 |
| 6 | *N*-(5-(2-chlorophenyl)-1,3,4-thiadiazol-2-yl)isoxazole-5-carboxamide 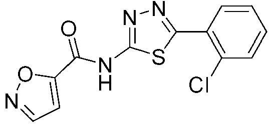 | 148 | *N*-(5-(pyrimidin-2-yl)-1,3,4-thiadiazol-2-yl)benzo[c]isoxazole-3-carboxamide 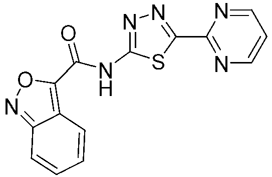 |
| 7 | *N*-(5-(2-chlorophenyl)-1,3,4-thiadiazol-2-yl)isoxazolo[4,3-h]quinoline-3-carboxamide 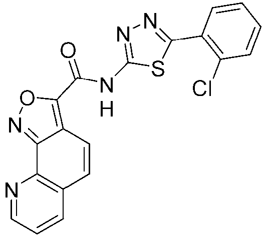 | 149 | *N*-(5-(methylthio)-1,3,4-thiadiazol-2-yl)-5-(2-phenylpiperidin-1-yl)-1,3,4-oxadiazole-2-carboxamide 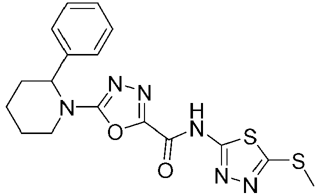 |
| 8 | *N*-(5-(2,6-difluorophenyl)-1,3,4-thiadiazol-2-yl)isoxazolo[4,3-h]quinoline-3-carboxamide 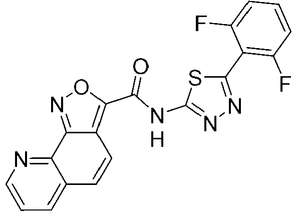 | 150 | 3-(Isoquinolin-1-yl)-*N*-(5-(methylthio)-1,3,4-thiadiazol-2-yl)isoxazole-5-carboxamide 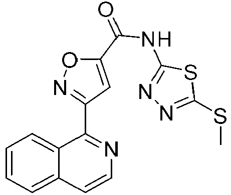 |
| 9 | *N*-(5-cyano-1,3,4-thiadiazol-2-yl)isoxazolo[4,3-h]quinoline-3-carboxamide 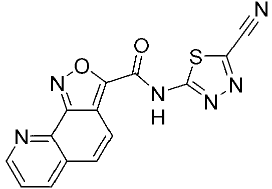 | 151 | 3-(2,3-Difluorophenyl)-*N*-(5-(methylthio)-1,3,4-thiadiazol-2-yl)isoxazole-5-carboxamide 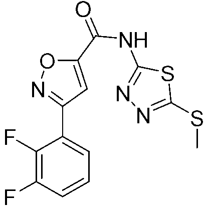 |
| 10 | *N*-(5-(pyridin-2-yl)-1,3,4-thiadiazol-2-yl)isoxazolo[4,3-h]quinoline-3-carboxamide 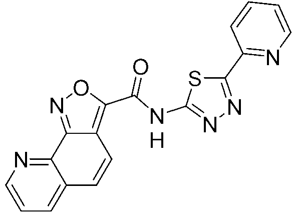 | 152 | *N*-(5-(Methylthio)-1,3,4-thiadiazol-2-yl)-3-(pyrimidin-2-yl)isoxazole-5-carboxamide 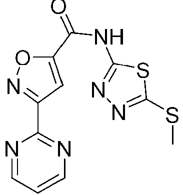 |
| 12 | *N*-(5-(trifluoromethyl)-1,3,4-thiadiazol-2-yl)isoxazolo[4,3-h]quinoline-3-carboxamide 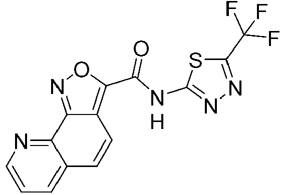 | 153 | *N*-(5-(Methylthio)-1,3,4-thiadiazol-2-yl)-3-(quinazolin-2-yl)isoxazole-5-carboxamide 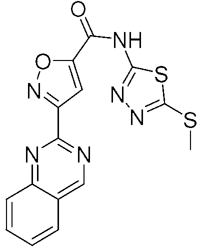 |
| 13 | N-(1,3,4-thiadiazol-2-yl)isoxazolo[4,3-h]quinoline-3-carboxamide 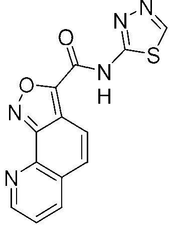 | 154 | *N*-(5-(2-Chlorophenyl)-1,3,4-thiadiazol-2-yl)-3-(pyrimidin-2-yl)isoxazole-5-carboxamide 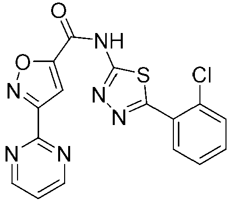 |
| 14 | *N*-(5-(dimethylamino)-1,3,4-thiadiazol-2-yl)isoxazolo[4,3-h]quinoline-3-carboxamide 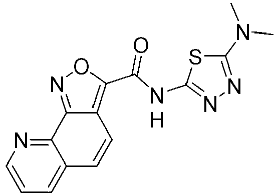 | 155 | *N*-(5*-*(2-Chlorophenyl)-1,3,4-thiadiazol-2-yl)-3-(quinazolin-2-yl)isoxazole-5-carboxamide 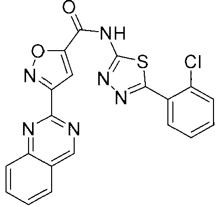 |
| 15 | methyl 5-((5-(methylthio)-1,3,4-thiadiazol-2-yl)carbamoyl)isoxazole-3-carboxylate 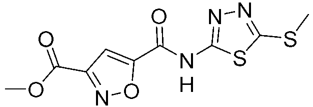 | 156 | *N*-(5-(2-Chlorophenyl)-1,3,4-thiadiazol-2-yl)-3-(5-fluoropyrimidin-2-yl)isoxazole-5-carboxamide 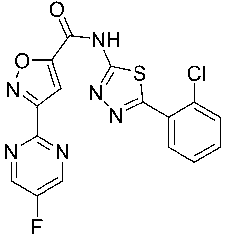 |
| 16 | 5-((5-(methylthio)-1,3,4-thiadiazol-2-yl)carbamoyl)isoxazole-3-carboxylic acid 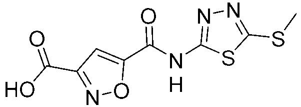 | 157 | *N*-(5-(2-Chlorophenyl)-1,3,4-thiadiazol-2-yl)-3-(4-methylpyrimidin-2-yl)isoxazole-5-carboxamide 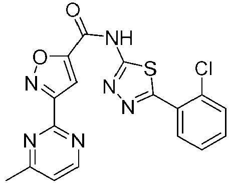 |
| 17 | 3-cyclopropyl-*N*-(5-(methylthio)-1,3,4-thiadiazol-2-yl)isoxazole-5-carboxamide 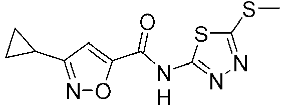 | 158 | *N*-(5-(2-Chlorophenyl)-1,3,4-thiadiazol-2-yl)-3-(5-methylpyrimidin-2-yl)isoxazole-5-carboxamide 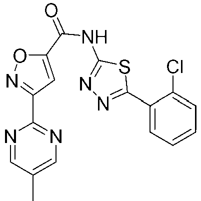 |
| 18 | *N*-(5-(difluoromethyl)-1,3,4-thiadiazol-2-yl)benzo[c]isoxazole-3-carboxamide 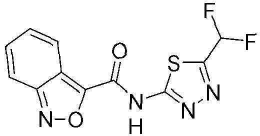 | 159 | 5-Benzyl-*N-*(5-(methylthio)-1,3,4-thiadiazol-2-yl)benzo[c]isoxazole-3-carboxamide 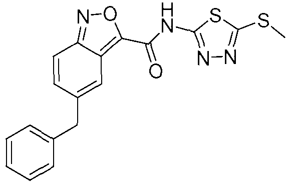 |
| 19 | *N*-(5-phenyl-1,3,4-thiadiazol-2-yl)benzo[c]isoxazole-3-carboxamide 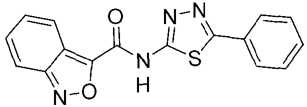 | 160 | 5-(4-Methoxybenzyl)-*N*-(5-(methylthio)-1,3,4-thiadiazol-2-yl)benzo[c]isoxazole-3-carboxamide 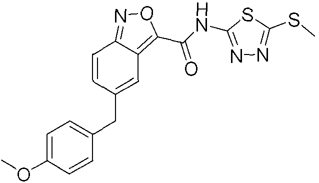 |
| 20 | *N*-(5-propyl-1,3,4-thiadiazol-2-yl)benzo[c]isoxazole-3-carboxamide 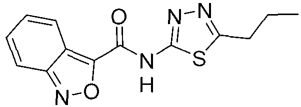 | 161 | 5-(3-methoxybenzyl)-*N*-(5-(methylthio)-1,3,4-thiadiazol-2-yl)benzo[c]isoxazole-3-carboxamide 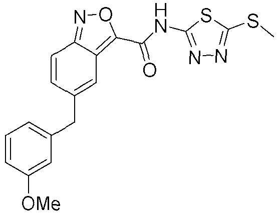 |
| 21 | *N*-(5-(pyridin-2-yl)-1,3,4-thiadiazol-2-yl)benzo[c]isoxazole-3-carboxamide 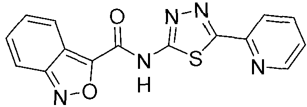 | 162 | *N*-(5-(methylthio)-1,3,4-thiadiazol-2-yl)-5-(phenylamino)benzo[c]isoxazole-3-carboxamide 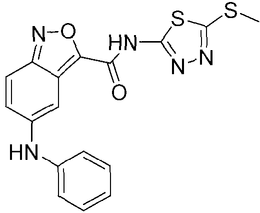 |
| 22 | *N*-(5-(2-chlorophenyl)-1,3,4-thiadiazol-2-yl)benzo[c]isoxazole-3-carboxamide 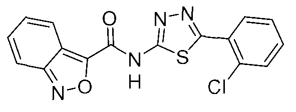 | 163 | *N*-(5-(methylthio)-1,3,4-thiadiazol-2-yl)-5-(phenylthio)benzo[c]isoxazole-3-carboxamide 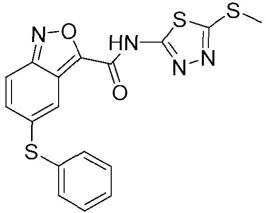 |
| 23 | *N*-(5-(3-chlorophenyl)-1,3,4-thiadiazol-2-yl)benzo[c]isoxazole-3-carboxamide 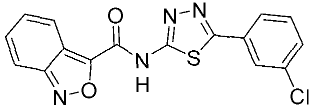 | 164 | *N*-(5-(methylthio)-1,3,4-thiadiazol-2-yl)-5-(phenylsulfonyl)benzo[c]isoxazole-3-carboxamide 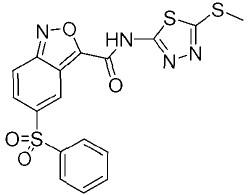 |
| 24 | *N*-(5-(2-methoxyphenyl)-1,3,4-thiadiazol-2-yl)benzo[c]isoxazole-3-carboxamide 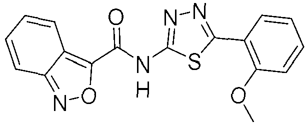 | 165 | *N*-(5-(methylthio)-1,3,4-thiadiazol-2-yl)-5-phenoxybenzo[c]isoxazole-3-carboxamide 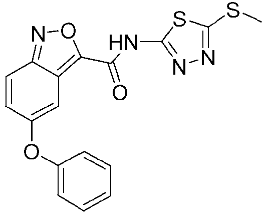 |
| 25 | *N*-(5-(3-fluorophenyl)-1,3,4-thiadiazol-2-yl)benzo[c]isoxazole-3-carboxamide 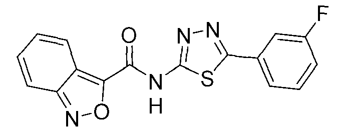 | 166 | 5-(hydroxy(phenyl)methyl)-*N*-(5-(methylthio)-1,3,4-thiadiazol-2-yl)benzo[c]isoxazole-3-carboxamide 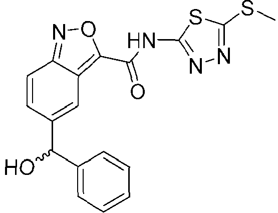 |
| 26 | *N*-(5-(2-fluorophenyl)-1,3,4-thiadiazol-2-yl)benzo[c]isoxazole-3-carboxamide 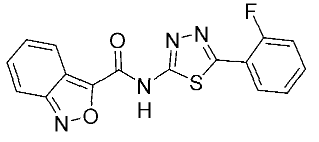 | 167 | 5-benzoyl-*N*-(5-(methylthio)-1,3,4-thiadiazol-2-yl)benzo[c]isoxazole-3-carboxamide 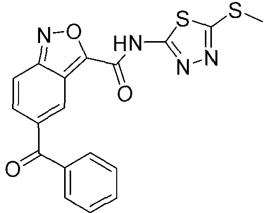 |
| 27 | *N*-(5-(o-tolyl)-1,3,4-thiadiazol-2-yl)benzo[c]isoxazole-3-carboxamide 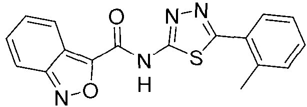 | 168 | 7-(hydroxymethyl)-*N*-(5-(methylthio)-1,3,4-thiadiazol-2-yl)benzo[c]isoxazole-3-carboxamide 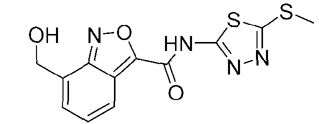 |
| 28 | *N*-(5-(m-tolyl)-1,3,4-thiadiazol-2-yl)benzo[c]isoxazole-3-carboxamide 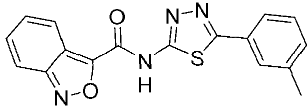 | 169 | *N*-(5-(2-chlorophenyl)-1,3,4-thiadiazol-2-yl)-3-(2-methoxyethyl)isoxazole-5-carboxamide 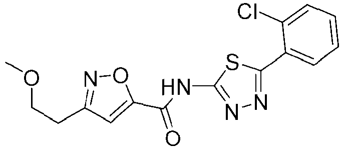 |
| 29 | *N*-(5-(p-tolyl)-1,3,4-thiadiazol-2-yl)benzo[c]isoxazole-3-carboxamide 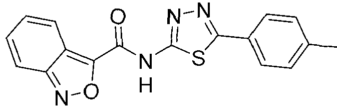 | 170 | *N*-(5-(2-chlorophenyl)-1,3,4-thiadiazol-2-yl)-3-(methoxymethyl)isoxazole-5-carboxamide 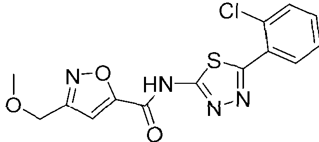 |
| 30 | *N*-(5-(3-hydroxyphenyl)-1,3,4-thiadiazol-2-yl)benzo[c]isoxazole-3-carboxamide 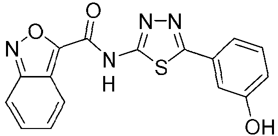 | 171 | *N*-(5-(methylthio)-1,3,4-thiadiazol-2-yl)-4,5-dihydrothiazolo[5',4':5,6]benzo[ 1,2-clisoxazole-3-carboxamide 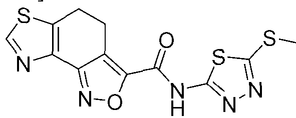 |
| 31 | *N*-(5-(2-chlorophenyl)-1,3,4-thiadiazol-2-yl)-3-methoxyisoxazole-5-carboxamide 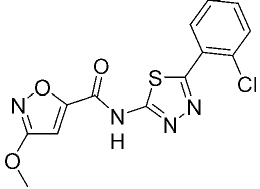 | 172 | 6-Methoxy-*N*-(5-(methylthio)-1,3,4-thiadiazol-2-yl)-4,5-dihydronaphtho[1,2-c]isoxazole-3-carboxamide 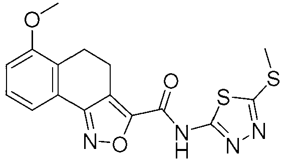 |
| 32 | *N*5-(5-(2-chlorophenyl)-1,3,4-thiadiazol-2-yl)isoxazole-3,5-dicarboxamide 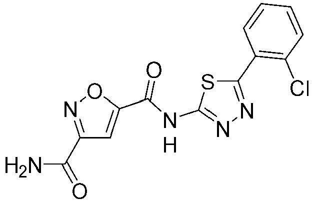 | 173 | *N*-(5-(Methylthio)-1,3,4-thiadiazol-2-yl)-4,5-dihydroisoxazolo[4,3-h]quinoline-3-carboxamide 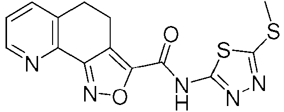 |
| 33 | *N*-(5-(2-chlorophenyl)-1,3,4-thiadiazol-2-yl)-3-ethylisoxazole-5-carboxamide 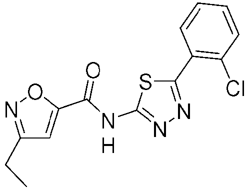 | 174 | 8-Methyl-*N*-(5-(methylthio)-1,3,4-thiadiazol-2-yl)-4,5-dihydroisoxazolo[4,3-*h*]quinazoline-3-carboxamide 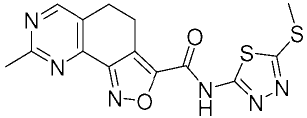 |
| 34 | *N*-(5-(2-chlorophenyl)-1,3,4-thiadiazol-2-yl)-3-(trifluoromethyl)isoxazole-5-carboxamide 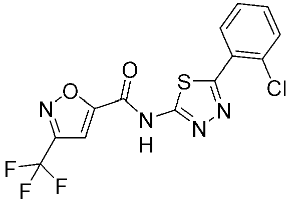 | 175 | *N*-(5-(2-chlorophenyl)-1,3,4-thiadiazol-2-yl)-3-(2-methoxyethoxy)isoxazole-5-carboxamide 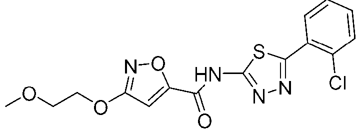 |
| 35 | *N*-(5-(2-chlorophenyl)-1,3,4-thiadiazol-2-yl)-3-(difluoromethyl)isoxazole-5-carboxamide 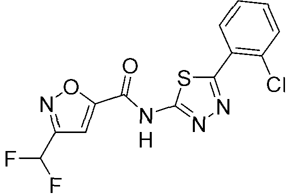 | 176 | 2-(2'-(6-((5-(methylthio)-1,3,4-thiadiazol-2-yl)carbamoyl)-2-oxo-2*H-*pyran-4-yl)-[1,1'-biphenyl]-4-yl)acetic acid 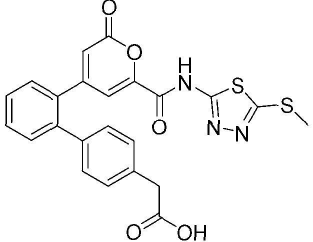 |
| 36 | *N*-(5-(2-chlorophenyl)-1,3,4-thiadiazol-2-yl)-3-methylisoxazole-5-carboxamide 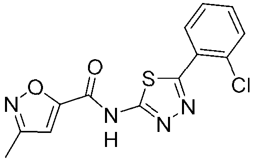 | 177 | 2-(2'-(6-((5-(methylthio)-1,3,4-thiadiazol-2-yl)carbamoyl)-2-oxo-2*H-*pyran-4-yl)-[1,1'-biphenyl]-3-yl)acetic acid 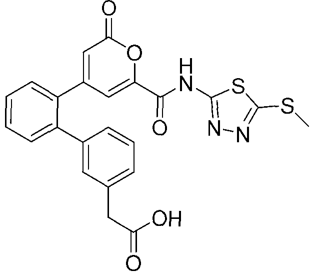 |
| 37 | *N*-(5-(2-chlorophenyl)-1,3,4-thiadiazol-2-yl)-3-cyanoisoxazole-5-carboxamide 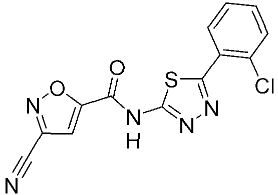 | 178 | 3-((5-(2-chlorophenyl)-1,3,4-thiadiazol-2-yl)carbamoyl)benzo[c]isoxazole-7-carboxylic acid 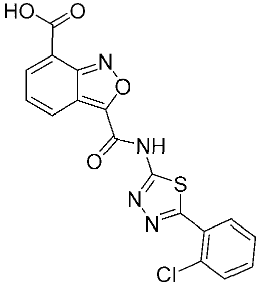 |
| 38 | *N*-(5-(2-chlorophenyl)-1,3,4-thiadiazol-2-yl)-3-(tetrahydro-2H-pyran-4-yl)isoxazole-5-carboxamide 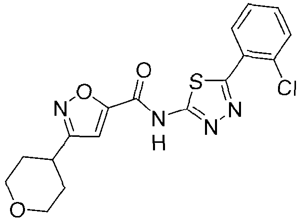 | 179 | *N*-(5-(2-chlorophenyl)-1,3,4-thiadiazol-2-yl)-3-(2-hydroxyethyl)isoxazole-5-carboxamide 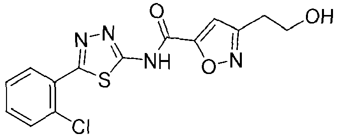 |
| 39 | *N*-(5-(2-chlorophenyl)-1,3,4-thiadiazol-2-yl)-3-cyclopropylisoxazole-5-carboxamide 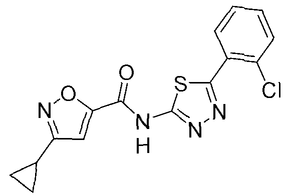 | 180 | *N*-(5-(methylthio)-1,3,4-thiadiazol-2-yl)-4,5-dihydroisoxazolo[4,5-h]quinoline-3-carboxamide 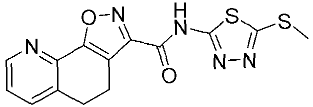 |
| 40 | *N*-(5-(2-chlorophenyl)-1,3,4-thiadiazol-2-yl)-3-isopropylisoxazole-5-carboxamide 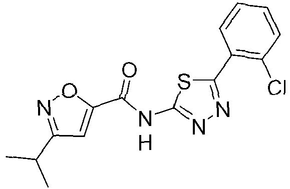 | 181 | 7-methyl-*N*-(5-(methylthio)-1,3,4-thiadiazol-2-yl)-4,5-dihydrothiazolo[5',4':5,6]benzo[ 1,2-c]isoxazole-3-carboxamide 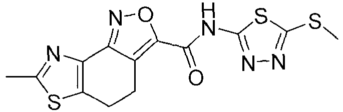 |
| 41 | *N*-(5-(2-chlorophenyl)-1,3,4-thiadiazol-2-yl)-3-(3-methyloxetan-3-yl)isoxazole-5-carboxamide 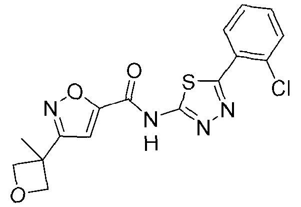 | 182 | 4-([1,1'-biphenyl]-2-yl)-*N*-(5-(methylthio)-1,3,4-thiadiazol-2-yl)-2-oxo-2*H*-pyran-6-carboxamide 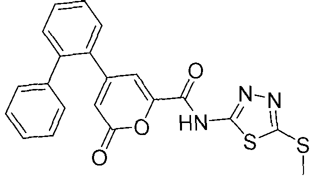 |
| 42 | 1-benzyl-N-(5-(methylthio)-1,3,4-thiadiazol-2-yl)-1*H*-1,2,3-triazole-4-carboxamide 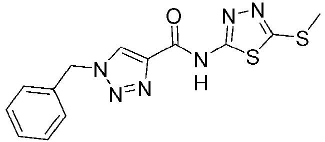 | Rac-183 | 5-((3,4-rac-*trans*-dihydroxypiperidin-1-yl)methyl)-*N*-(5-(methylthio)-1,3,4-thiadiazol-2-yl)benzo[c]isoxazole-3-carboxamide 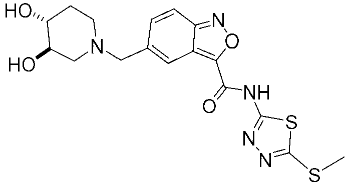 |
| 43 | *tert*-butyl 3-((5-(methylthio)-1,3,4-thiadiazol-2-yl)carbamoyl)-6,7-dihydroisoxazolo[4,3-c]pyridine-5(4*H*)-carboxylate 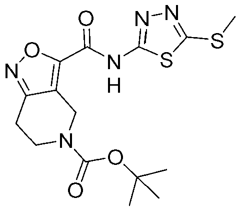 | 183 A | 5-(((3R,4R)-3,4-dihydroxypiperidin-1-yl)methyl)-N-(5-(methylthio)-1,3,4-thiadiazol-2-yl)benzo[c]isoxazole-3-carboxamide 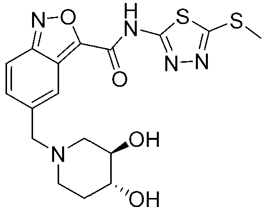 |
| 44 | 5-(3-chlorobenzyl)-*N*-(5-(methylthio)-1,3,4-thiadiazol-2-yl)-1,3,4-oxadiazole-2-carboxamide 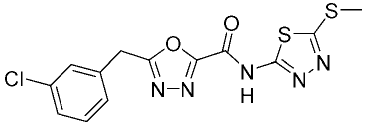 | 183B | 5-(((3S,4S)-3,4-dihydroxypiperidin-1-yl)methyl)-N-(5-(methylthio)-1,3,4-thiadiazol-2-yl)benzo[c]isoxazole-3-carboxamide 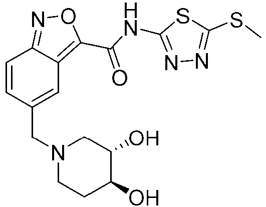 |
| 45 | 5-(2,6-difluorophenyl)-*N*-(5-(methylthio)-1,3,4-thiadiazol-2-yl)-1,3,4-oxadiazole-2-carboxamide 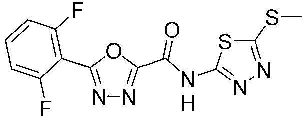 | 184 | 1-((3-((5-(methylthio)-1,3,4-thiadiazol-2-yl)carbamoyl)benzo[c]isoxazol-5-yl)methyl)-4-oxo-1,4-dihydroquinoline-3-carboxylic acid 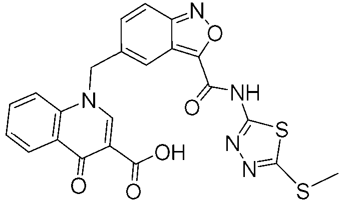 |
| 46 | 3-(2,6-difluorophenyl)*-N*-(5-(methylthio)-1,3,4-thiadiazol-2-yl)isoxazole-5-carboxamide 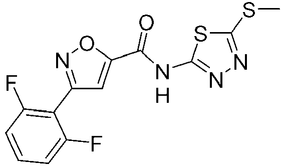 | 185 | 5-(benzo[d][1,3]dioxol-5-ylmethyl)-*N-*(5-(methylthio)-1,3,4-thiadiazol-2-yl)benzo[c]isoxazole-3-carboxamide 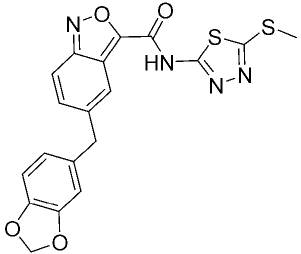 |
| 47 | 3-(2,5-difluorophenyl)-*N*-(5-(methylthio)-1,3,4-thiadiazol-2-yl)isoxazole-5-carboxamide 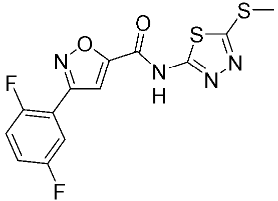 | 186 | 5-(3,4-dihydroxybenzyl)-*N*-(5-(methylthio)-1,3,4-thiadiazol-2-yl)benzo[c]isoxazole-3-carboxamide 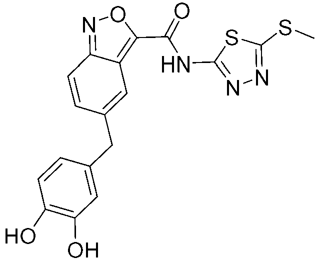 |
| 48 | 3-(2,4-difluorophenyl)-*N*-(5-(methylthio)-1,3,4-thiadiazol-2-yl)isoxazole-5-carboxamide 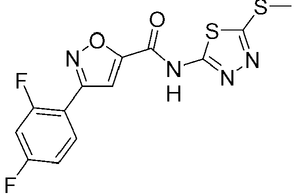 | 187 | 5-(3-hydroxy-4-methoxybenzyl)-*N*-(5-(methylthio)-1,3,4-thiadiazol-2-yl)benzo[c]isoxazole-3-carboxamide 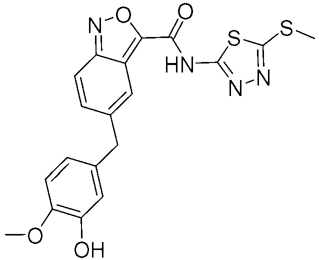 |
| 49 | 5-(2-chlorobenzyl)-*N*-(5-(methylthio)-1,3,4-thiadiazol-2-yl)-1,3,4-oxadiazole-2-carboxamide 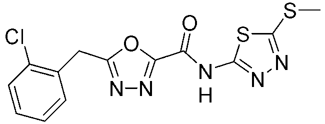 | 188 | 5-(4-hydroxybenzyl)-*N*-(5-(methylthio)-1,3,4-thiadiazol-2-yl)benzo[c]isoxazole-3-carboxamide 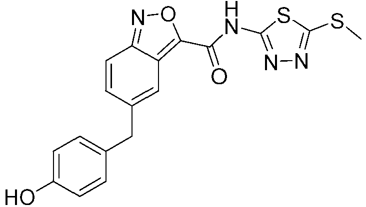 |
| 50 | 3-(4-chlorophenyl)-*N*-(5-(methylthio)-1,3,4-thiadiazol-2-yl)isoxazole-5-carboxamide 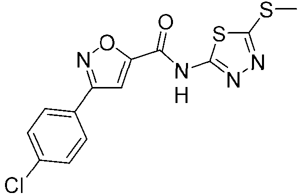 | 189 | 5-((5-Hydroxy-6-oxo-1,6-dihydropyridin-3-yl)methyl)-*N*-(5-(methylthio)-1,3,4-thiadiazol-2-yl)benzo[c]isoxazole-3-carboxamide 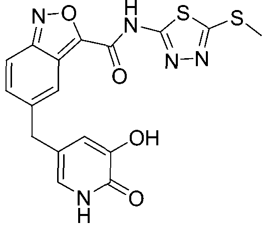 |
| 51 | *N*-(5-(2-chlorophenyl)-1,3,4-thiadiazol-2-yl)-8-hydroxy-4-oxo-1,4-dihydroquinoline-2-carboxamide 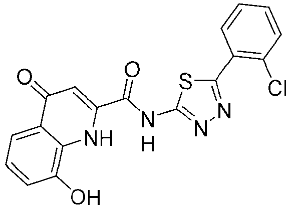 | 190 | *N*-(5-(methylthio)-1,3,4-thiadiazol-2-yl)-[1,2,3]triazolo[1,5-a]quinoline-3-carboxamide 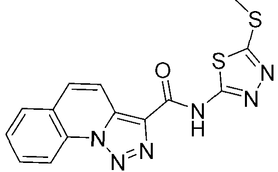 |
| 52 | *N*-(5-(2-chlorophenyl)-1,3,4-thiadiazol-2-yl)-3-(1-methyl-1H-pyrazol-4-yl)isoxazole-5-carboxamide 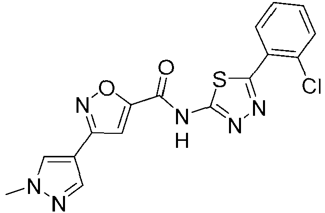 | 191 | 5-(hydroxymethyl)-*N*-(5-(methylthio)-1,3,4-thiadiazol-2-yl)benzo[c]isoxazole-3-carboxamide 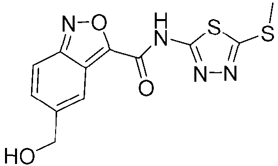 |
| 53 | *N*-(5-(2-chlorophenyl)-1,3,4-thiadiazol-2-yl)-3-(1,5-dimethyl-lH-pyrazol-4-yl)isoxazole-5-carboxamide 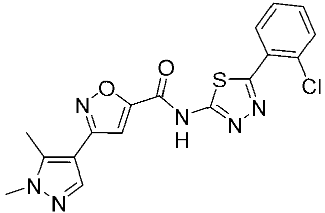 | 192 | *N*6,*N*6-dimethyl-*N*3-(5-(methylthio)-1,3,4-thiadiazol-2-yl)benzo[c]isoxazole-3,6-dicarboxamide 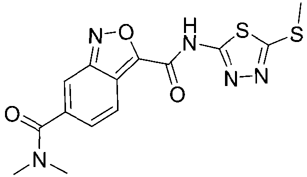 |
| 54 | *N*-(5-(2-chlorophenyl)-1,3,4-thiadiazol-2-yl)-3-(1,3-dimethyl-1H-pyrazol-4-yl)isoxazole-5-carboxamide 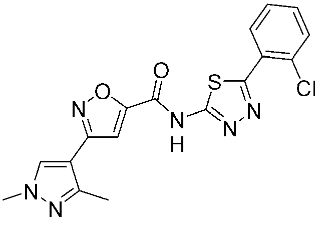 | 193 | *N*6-methyl-*N*3-(5-(methylthio)-1,3,4-thiadiazol-2-yl)benzo[c]isoxazole-3,6-dicarboxamide 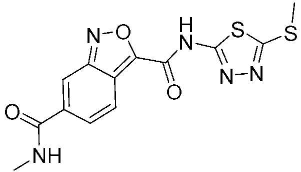 |
| 55 | *N*-(5-(2-chlorophenyl)-1,3,4-thiadiazol-2-yl)-3-hydroxyisoxazole-5-carboxamide 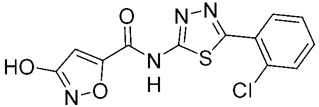 | 194 | 6-(hydroxymethyl)-*N*-(5-(methylthio)-1,3,4-thiadiazol-2-yl)benzo[c]isoxazole-3-carboxamide 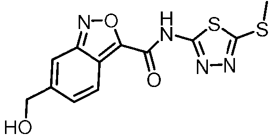 |
| 56 | *N*-(5-(2-chlorophenyl)-1,3,4-thiadiazol-2-yl)-6-hydroxypyridazine-3-carboxamide 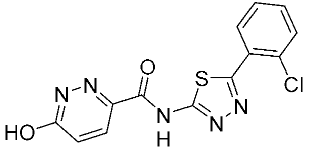 | 195 | *N*-(5-(methylthio)-1,3,4-thiadiazol-2-yl)-7-oxo-4,5,6,7-tetrahydrobenzo[c]isoxazole-3-carboxamide 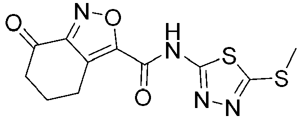 |
| 57 | *N*-(5-(2,3-dichlorophenyl)-1,3,4-thiadiazol-2-yl)-3-hydroxyisoxazole-5-carboxamide 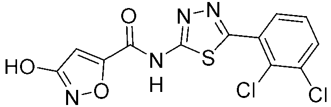 | 196 | 7-hydroxy-*N*-(5-(methylthio)-1,3,4-thiadiazol-2-yl)-4,5,6,7-tetrahydrobenzo[c]isoxazole-3-carboxamide 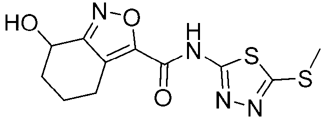 |
| 58 | *N*-(5-(2-chlorophenyl)-1,3,4-thiadiazol-2-yl)-1H-1,2,4-triazole-3-carboxamide 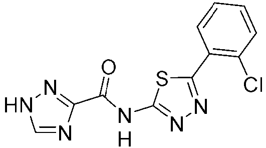 | 197 | *N*-(5-ethyl-1,3,4-thiadiazol-2-yl)isoxazolo[4,3-h]quinoline-3-carboxamide 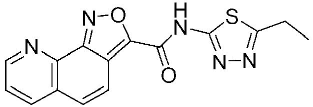 |
| 59 | *N*-(5-(2-chlorophenyl)-1,3,4-thiadiazol-2-yl)-1H-1,2,3-triazole-4-carboxamide 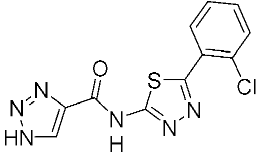 | 198 | 1-(3,3-dimethylbutyl)-*N*-(5-(methylthio)-1,3,4-thiadiazol-2-yl)-1*H*-1,2,3-triazole-4-carboxamide 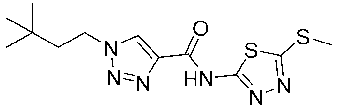 |
| 60 | *N*-(5-(2-chlorophenyl)-1,3,4-thiadiazol-2-yl)-1H-tetrazole-5-carboxamide 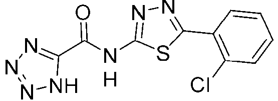 | 199 | 8-hydroxy-*N*-(5-(methylthio)-1,3,4-thiadiazol-2-yl)-4-oxo-1,4-dihydroquinoline-2-carboxamide 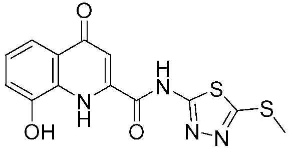 |
| 61 | *N*-(5-(2-chlorophenyl)-1,3,4-thiadiazol-2-yl)-2-oxo-2,3-dihydrooxazole-5-carboxamide 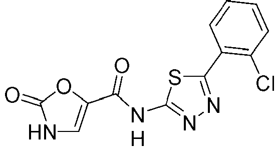 | 200 | 4-hydroxy-*N*-(5-(methylthio)-1,3,4-thiadiazol-2-yl)-6-phenylpicolinamide 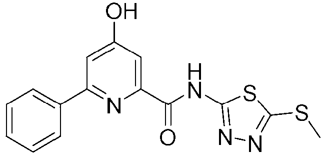 |
| 62 | *N*-(5-(2-chlorophenyl)-1,3,4-thiadiazol-2-yl)-2-oxo-2,3-dihydropyrimidine-4-carboxamide 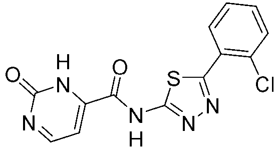 | 201 | *N*-(5-(methylthio)-1,3,4-thiadiazol-2-yl)-6-phenylpyrimidine-4-carboxamide 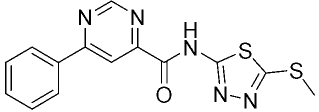 |
| 63 | *N*-(5-(2-chlorophenyl)-1,3,4-thiadiazol-2-yl)-5-oxo-4,5-dihydro-1H-1,2,4-triazole-3-carboxamide 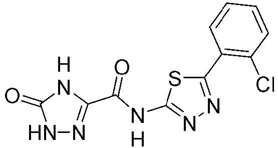 | 202 | *N*-(5-(methylthio)-1,3,4-thiadiazol-2-yl)-2-oxo-4-phenoxy-2H-pyran-6-carboxamide 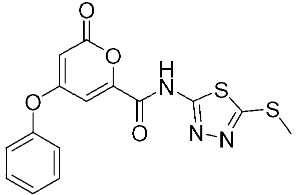 |
| 64 | *N*-(5-(2-chlorophenyl)-1,3,4-thiadiazol-2-yl)-3-hydroxy-2-oxo-2H-pyran-6-carboxamide 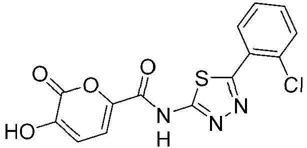 | 203 | 3-methyl-*N*-(5-(methylthio)-1,3,4-thiadiazol-2-yl)-2-oxo-4-phenyl-2*H-*pyran-6-carboxamide 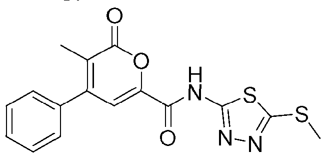 |
| 65 | *N*-(5-(methylthio)-1,3,4-thiadiazol-2-yl)-4-phenylthiazole-2-carboxamide 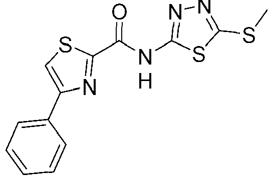 | 204 | 3-((5-(methylthio)-1,3,4-thiadiazol-2-yl)carbamoyl)benzo[c]isoxazole-6-carboxylic acid 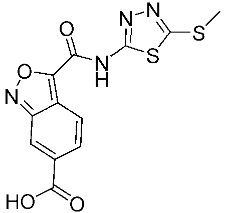 |
| 66 | *N*-(5-(methylthio)-1,3,4-thiadiazol-2-yl)-2-phenyl-2H-tetrazole-5-carboxamide 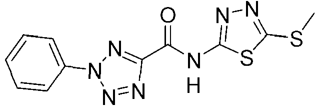 | 205 | *N*-(5-(2-chlorophenyl)-1,3,4-thiadiazol-2-yl)-2-oxo-4-phenyl-2*H-*pyran-6-carboxamide 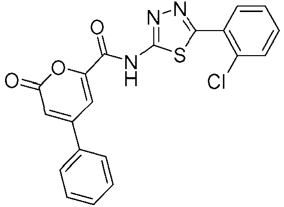 |
| 67 | *N*-(5-(methylthio)-1,3,4-thiadiazol-2-yl)-4,5,6,7-tetrahydrobenzo[c]isoxazole-3-carboxamide 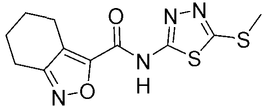 | 206 | *N*-(5-(2-chlorophenyl)-1,3,4-thiadiazol-2-yl)-2-oxo-4-(phenylamino)-2*H*-pyran-6-carboxamide 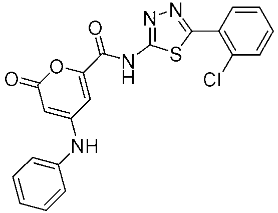 |
| 68 | 3-(2,3-dihydro-1*H*-inden-4-yl)-*N*-(5-(methylthio)-1,3,4-thiadiazol-2-yl)isoxazole-5-carboxamide 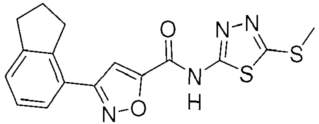 | 207 | *N*-(5-(methylthio)-1,3,4-thiadiazol-2-yl)-1-oxo-1*H*-isochromene-3-carboxamide 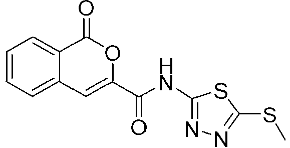 |
| 69 | *N*-(5-ethyl-1,3,4-thiadiazol-2-yl)benzo[c]isoxazole-3-carboxamide 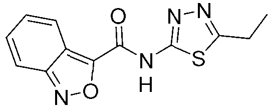 | 208 | *N*-[5-(methylsulfanyl)-1,3,4-thiadiazol-2-yl]-3-phenyl-1,2-oxazole-5-carboxamide 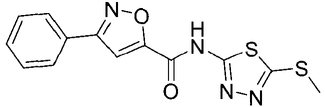 |
| 70 | *N*-(5-(ethylthio)-1,3,4-thiadiazol-2-yl)benzo[c]isoxazole-3-carboxamide 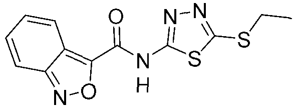 | 209 | 3-(3-chlorophenyl)-*N*-[5-(methylsulfanyl)-1,3,4-thiadiazol-2-yl]-1,2-oxazole-5-carboxamide 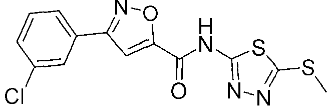 |
| 71 | *N*-(5-(methylthio)-1,3,4-thiadiazol-2-yl)-3-(pyrazin-2-yl)-1,2,4-oxadiazole-5-carboxamide 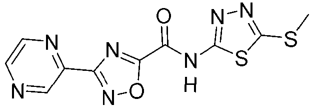 | 210 | 3-(4-methoxyphenyl)-*N*-[5-(methylsulfanyl)-1,3,4-thiadiazol-2-yl]-1,2-oxazole-5-carboxamide 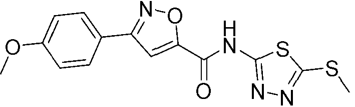 |
| 72 | *N*-(5-(methylthio)-1,3,4-thiadiazol-2-yl)-[2,3'-bipyridine]-5'-carboxamide 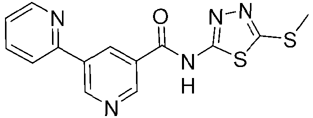 | 211 | 3-(3-fluorophenyl)-*N*-[5-(methylsulfanyl)-1,3,4-thiadiazol-2-yl]-1,2-oxazole-5-carboxamide 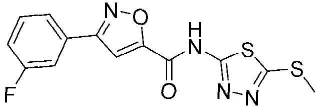 |
| 73 | *N*-(5-(methylthio)-1,3,4-thiadiazol-2-yl)-5-(3-phenylpyrrolidin-1-yl)-1,3,4-oxadiazole-2-carboxamide 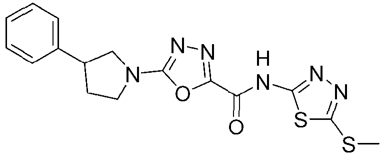 | 212 | 3-(3-methoxyphenyl)-*N*-[5-(methylsulfanyl)-1,3,4-thiadiazol-2-yl]-1,2-oxazole-5-carboxamide 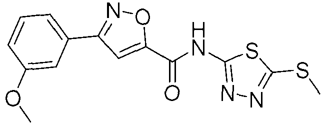 |
| 74 | *N*-(5-(methylthio)-1,3,4-thiadiazol-2-yl)-5-(2-phenylazetidin-1-yl)-1,3,4-oxadiazole-2-carboxamide 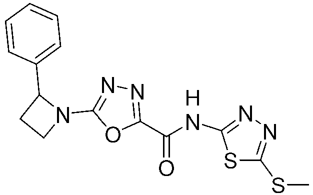 | 213 | *N*-[5-(methylsulfanyl)-1,3,4-thiadiazol-2-yl]-6-oxo-4-phenylpyran-2-carboxamide 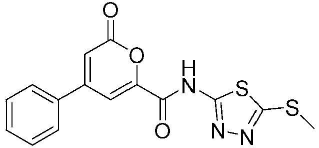 |
| 75 | *N*-(5-(methylthio)-1,3,4-thiadiazol-2-yl)-3-(thiophen-2-yl)isoxazole-5-carboxamide 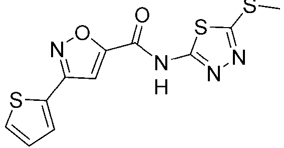 | 214 | 3-(3-cyanophenyl)-*N*-[5-(methylsulfanyl)-1,3,4-thiadiazol-2-yl]-1,2-oxazole-5-carboxamide 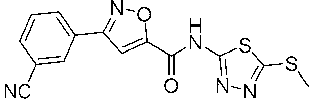 |
| 76 | *N*-(5-(methylthio)-1,3,4-thiadiazol-2-yl)-2-(thiophen-2-yl)thiazole-5-carboxamide 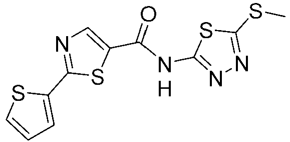 | 215 | 3-(4-methylphenyl)-*N*-[5-(methylsulfanyl)-1,3,4-thiadiazol-2-yl]-1,2-oxazole-5-carboxamide 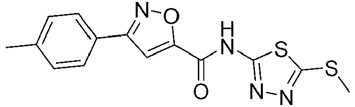 |
| 77 | 5-methyl-*N*-(5-(methylthio)-1,3,4-thiadiazol-2-yl)-2-(thiophen-2-yl)oxazole-4-carboxamide 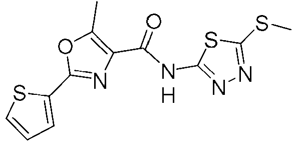 | 216 | 3-(2-chlorophenyl)-*N*-[5-(methylsulfanyl)-1,3,4-thiadiazol-2-yl]-1,2-oxazole-5-carboxamide 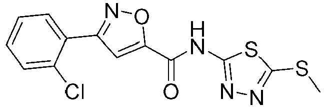 |
| 78 | 6-fluoro-4-methyl-*N*-(5-(methylthio)-1,3,4-thiadiazol-2-yl)-1H-indole-2-carboxamide 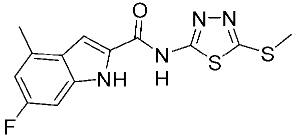 | 217 | 3-(2-fluorophenyl)-*N*-(5-(methylthio)-1,3,4-thiadiazol-2-yl)isoxazole-5-carboxamide 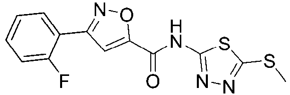 |
| 79 | *N*-(5-(methylthio)-1,3,4-thiadiazol-2-yl)benzo[c]isoxazole-3-carboxamide 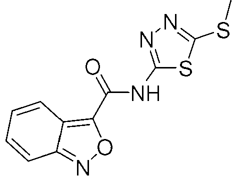 | 218 | 3-(2-methylphenyl)-*N*-[5-(methylsulfanyl)-1,3,4-thiadiazol-2-yl]-1,2-oxazole-5-carboxamide 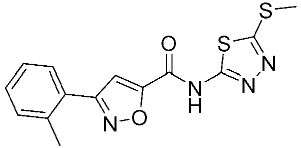 |
| 80 | *N*-(5-(methylthio)-1,3,4-thiadiazol-2-yl)-3-(tetrahydro-2*H*-pyran-4-yl)isoxazole-5-carboxamide 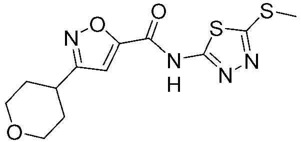 | 219 | 3-(3-methylphenyl)-*N*-[5-(methylsulfanyl)-1,3,4-thiadiazol-2-yl]-1,2-oxazole-5-carboxamide 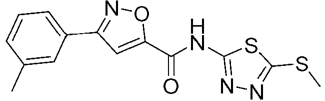 |
| 81 | *N*-(5-methyl-1,3,4-thiadiazol-2-yl)benzo[c]isoxazole-3-carboxamide 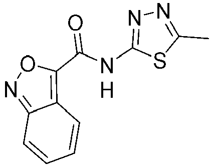 | 220 | *N*-[5-(methylsulfanyl)-1,3,4-thiadiazol-2-yl]naphtho[1,2-c][1,2]oxazole-3-carboxamide 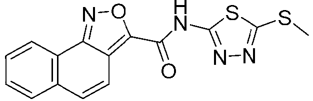 |
| 82 | 3-isopropyl-*N*-(5-(methylthio)-1,3,4-thiadiazol-2-yl)isoxazole-5-carboxamide 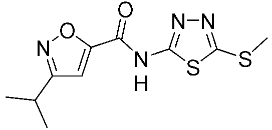 | 221 | 6-methyl-*N*-[5-(methylsulfanyl)-1,3,4-thiadiazol-2-yl]-2,1-benzoxazole-3-carboxamide 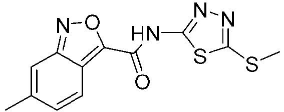 |
| 83 | *N*-(5-(methylthio)-1,3,4-thiadiazol-2-yl)-3-(3-(trifluoromethoxy)phenyl)isoxazole-5-carboxamide 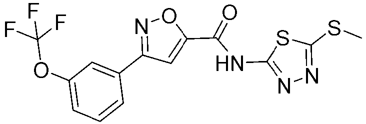 | 222 | 6-fluoro-*N*-[5-(methylsulfanyl)-1,3,4-thiadiazol-2-yl]-2,1-benzoxazole-3-carboxamide 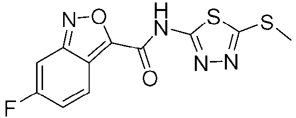 |
| 84 | 3-cyclohexyl-*N*-(5-(methylthio)-1,3,4-thiadiazol-2-yl)isoxazole-5-carboxamide 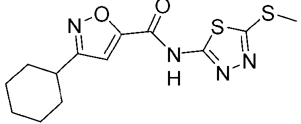 | 223 | 5-fluoro-*N*-[5-(methylsulfanyl)-1,3,4-thiadiazol-2-yl]-2,1-benzoxazole-3-carboxamide 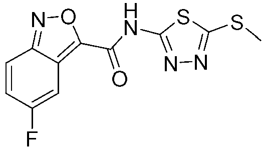 |
| 85 | 3-cyclopentyl-*N*-(5-(methylthio)-1,3,4-thiadiazol-2-yl)isoxazole-5-carboxamide 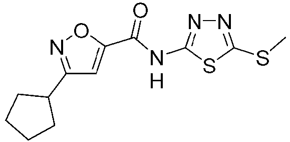 | 224 | 5-methoxy-*N*-[5-(methylsulfanyl)-1,3,4-thiadiazol-2-yl]-2,1-benzoxazole-3-carboxamide 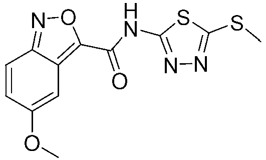 |
| 86 | 3-cyclobutyl-*N*-(5-(methylthio)-1,3,4-thiadiazol-2-yl)isoxazole-5-carboxamide 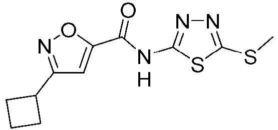 | 225 | *N*-[5-(methylsulfanyl)-1,3,4-thiadiazol-2-yl]-6-oxo-5-phenylpyran-2-carboxamide 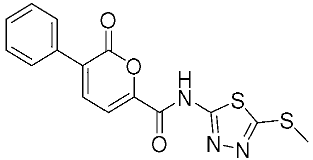 |
| 87 | *N*5-(5-(2-chlorophenyl)-1,3,4-thiadiazol-2-yl)-*N*3,*N*3-dimethylisoxazole-3,5-dicarboxamide 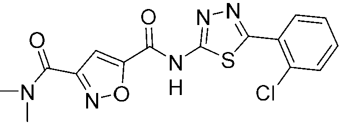 | 226 | 7-methyl-*N*-[5-(methylsulfanyl)-1,3,4-thiadiazol-2-yl]-2,1-benzoxazole-3-carboxamide 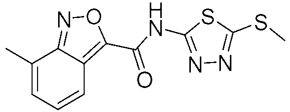 |
| 88 | *N*5-(5-(2-chlorophenyl)-1,3,4-thiadiazol-2-yl)-*N*3-(2-methoxyethyl)isoxazole-3,5-dicarboxamide 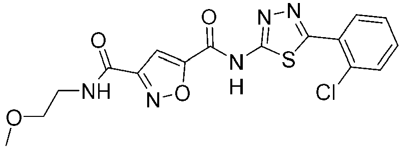 | 227 | 5-methyl-*N*-[5-(methylsulfanyl)-1,3,4-thiadiazol-2-yl]-2,1-benzoxazole-3-carboxamide 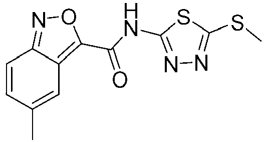 |
| 89 | *N*5-(5-(2-chlorophenyl)-1,3,4-thiadiazol-2-yl)-*N*3-methylisoxazole-3,5-dicarboxamide 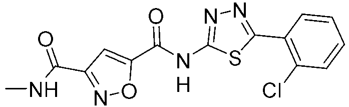 | 228 | 7-chloro-*N*-[5-(methylsulfanyl)-1,3,4-thiadiazol-2-yl]-2,1-benzoxazole-3-carboxamide 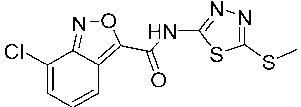 |
| 90 | *N*5-(5-(2-chlorophenyl)-1,3,4-thiadiazol-2-yl)-*N*3-hydroxyisoxazole-3,5-dicarboxamide 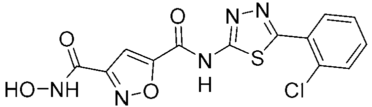 | 229 | 5-chloro-*N*-[5-(methylsulfanyl)-1,3,4-thiadiazol-2-yl]-2,1-benzoxazole-3-carboxamide 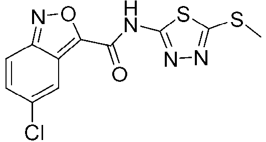 |
| 91 | *N*5-(5-(2-chlorophenyl)-1,3,4-thiadiazol-2-yl)-*N*3-methoxyisoxazole-3,5-dicarboxamide 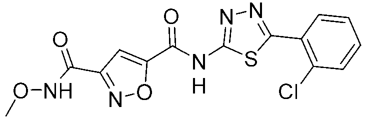 | 230 | 7-methoxy-*N*-[5-(methylsulfanyl)-1,3,4-thiadiazol-2-yl]-2,1-benzoxazole-3-carboxamide 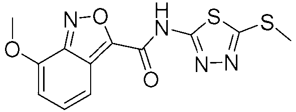 |
| 92 | *N*5-(5-(2-chlorophenyl)-1,3,4-thiadiazol-2-yl)-*N*3-phenylisoxazole-3,5-dicarboxamide 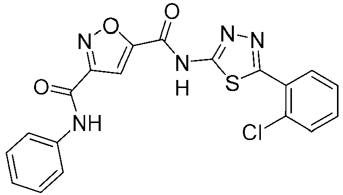 | 231 | 6-methoxy-*N*-[5-(methylsulfanyl)-1,3,4-thiadiazol-2-yl]-2,1-benzoxazole-3-carboxamide 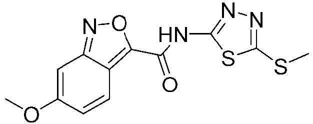 |
| 93 | *N*-(5-methyl-1,3,4-thiadiazol-2-yl)isoxazolo[4,3-h]quinoline-3-carboxamide 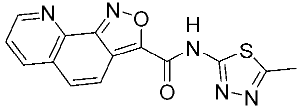 | 232 | *N*-[5-(methylsulfanyl)-1,3,4-thiadiazol-2-yl]-4-phenyl-1,2-oxazole-5-carboxamide 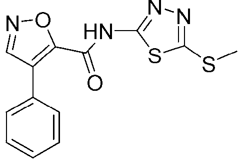 |
| 94 | *N*-(5-(methylthio)-1,3,4-thiadiazol-2-yl)isoxazolo[4,3-h]quinoline-3-carboxamide 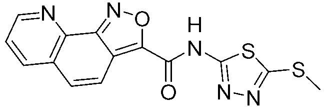 | 233 | 3-benzyl-*N*-[5-(methylsulfanyl)-1,3,4-thiadiazol-2-yl]-1,2-oxazole-5-carboxamide 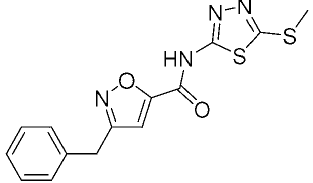 |
| 95 | 5-(5-chlorothiophen-2-yl)*-N*-[5-(methylsulfanyl)-1,3,4-thiadiazol-2-yl]-1,2-oxazole-3-carboxamide 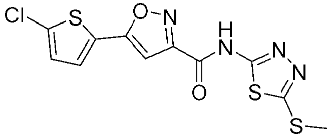 | 234 | *N*-[5-(methylsulfanyl)-1,3,4-thiadiazol-2-yl]-3-phenyl-1,2,4-oxadiazole-5-carboxamide 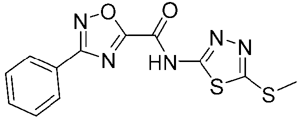 |
| 96 | *N*-(5-(methylthio)-1,3,4-thiadiazol-2-yl)-5-(thiophen-2-yl)-nicotinamide 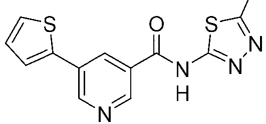 | 235 | *N*-[5-(methylsulfanyl)-1,3,4-thiadiazol-2-yl]-5-phenyl-1,3,4-oxadiazole-2-carboxamide 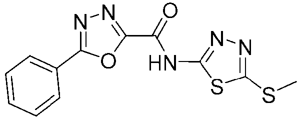 |
| 98 | *tert-*butyl 3-{[5-(methylsulfanyl)-1,3,4-thiadiazol-2-yl]carbamoyl}-5*H,*6*H,*8*H-*[1,2,4] triazolo[4,3-a]pyrazine-7-carboxylate 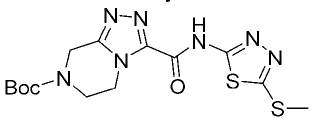 | 236 | *N*-(5-methylsulfanyl-1,3,4-thiadiazol-2-yl)-5-phenyl-1,2,4-oxadiazole-3-carboxamide 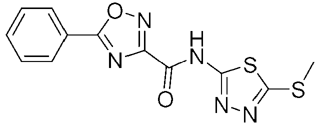 |
| 99 | *N*-(5-(methylthio)-1,3,4-thiadiazol-2-yl)-5-(2-phenylpyrrolidin-1-yl)-1,2,4-oxadiazole-3-carboxamide 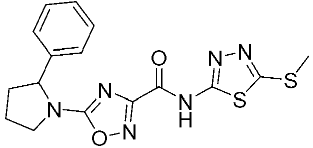 | 237 | 4-(2-fluorophenyl)-*N*-(5-methylsulfanyl-1,3,4-thiadiazol-2-yl)-6-oxo-pyran-2-carboxamide 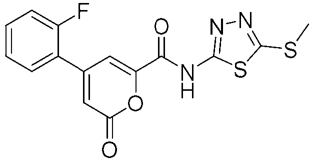 |
| 100 | 5-(3,3-dimethylpiperidin-1-yl)-*N*-(5-(methylthio)-1,3,4-thiadiazol-2-yl)-1,2,4-oxadiazole-3-carboxamide 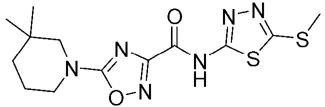 | 238 | 4-(3-fluorophenyl)-*N*-(5-methylsulfanyl-1,3,4-thiadiazol-2-yl)-6-oxo-pyran-2-carboxamide 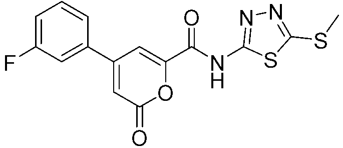 |
| 101 | *N*-[5-(methylthio)-1,3,4 -thiadiazol-2-yl]-5-(piperidin-1-yl)-1,2,4-oxadiazole-3-carboxamide 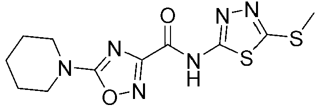 | 239 | 4-(4-fluorophenyl)-*N*-(5-methylsulfanyl-1,3,4-thiadiazol-2-yl)-6-oxo-pyran-2-carboxamide 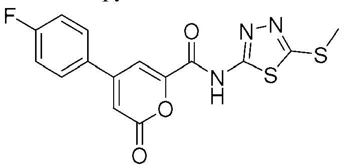 |
| 102 | 5-(4,4-dimethylpiperidin-1-yl)-*N*-(5-(methylthio)-1,3,4-thiadiazol-2-yl)-1,2,4-oxadiazole-3-carboxamide 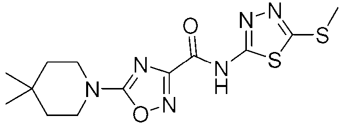 | 240 | 4-(3-chlorophenyl)-*N*-(5-methylsulfanyl-1,3,4-thiadiazol-2-yl)-6-oxo-pyran-2-carboxamide 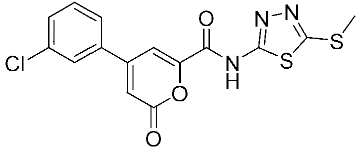 |
| 103 | *N*-(5-(methylthio)-1,3,4-thiadiazol-2-yl)-5-(2-phenylpiperidin-1-yl)-1,2,4-oxadiazole-3-carboxamide 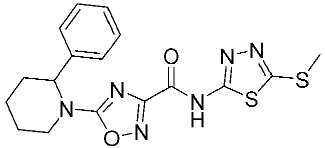 | 241 | 4-(3-methoxyphenyl)-*N*-(5-methylsulfanyl-1,3,4-thiadiazol-2-yl)-6-oxo-pyran-2-carboxamide 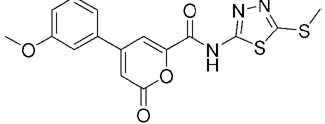 |
| 104 | *N*-[5-(methylsulfanyl)-1,3,4-thiadiazol-2-yl]-5-(pyridin-2-yl)-1,2,4-oxadiazole-3-carboxamide 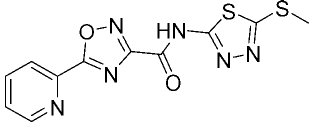 | 242 | 3,4-dimethyl-*N*-(5-methylsulfanyl-1,3,4-thiadiazol-2-yl)isoxazole-5-carboxamide 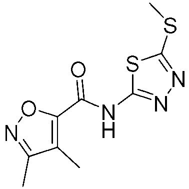 |
| *105* | *N*-[5-(methylsulfanyl)-1,3,4-thiadiazol-2-yl]-5-(pyridin-3-yl)-1,2,4-oxadiazole-3-carboxamide 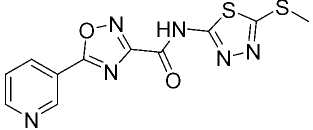 | 243 | 4-(4-chlorophenyl)-*N*-(5-methylsulfanyl-1,3,4-thiadiazol-2-yl)-6-oxo-pyran-2-carboxamide 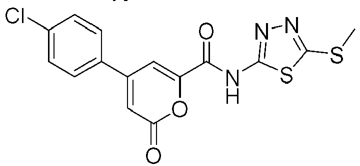 |
| 106 | *N*-(5-(methylthio)-1,3,4-thiadiazol-2-yl)-5-(pyridin-2-yl)-1,3,4-oxadiazole-2-carboxamide 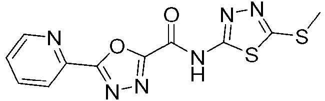 | 244 | 4-(2-methoxyphenyl)-*N*-(5-methylsulfanyl-1,3,4-thiadiazol-2-yl)-6-oxo-pyran-2-carboxamide 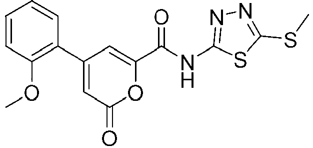 |
| 107 | *N*-[5-(methylsulfanyl)-1,3,4-thiadiazol-2-yl]-5-(pyridin-3-yl)-1,3,4-oxadiazole-2-carboxamide 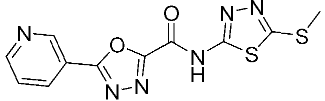 | 245 | *N*-(5-methylsulfanyl-1,3,4-thiadiazol-2-yl)-4-(2-naphthyl)-6-oxo-pyran-2-carboxamide 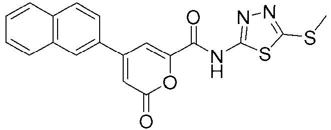 |
| 108 | *N*-(5-(methylthio)-1,3,4-thiadiazol-2-yl)-5-(piperidin-1-yl)-1,3,4-oxadiazole-2-carboxamide 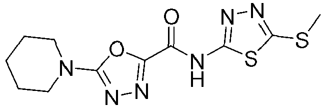 | 246 | *N*-(5-methylsulfanyl-1,3,4-thiadiazol-2-yl)-6-oxo-4-(8-quinolyl)pyran-2-carboxamide 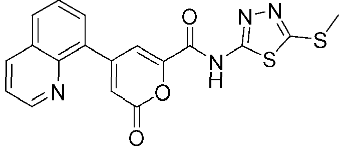 |
| 109 | 5-(3-hydroxypiperidin-1-yl)-*N*-(5-(methylthio)-1,3,4-thiadiazol-2-yl)-1,3,4-oxadiazole-2-carboxamide 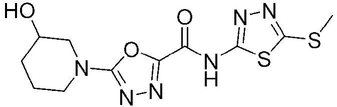 | 247 | *N*-(5-methylsulfanyl-1,3,4-thiadiazol-2-yl)-6-oxo-4-(4-quinolyl)pyran-2-carboxamide 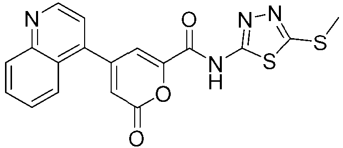 |
| 110 | 1-(5-{[5-(methylsulfanyl)-1,3,4-thiadiazol-2-yl] carbamoyl}-1,3,4-oxadiazol-2-yl) piperidine-3-carboxylic acid 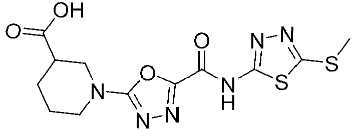 | 248 | *N*-(5-methylsulfanyl-1,3,4-thiadiazol-2-yl)-4-(o-tolyl)-6-oxo-pyran-2-carboxamide 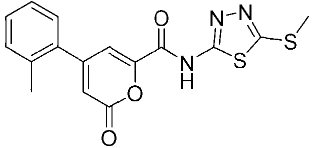 |
| 111 | 1-(5-{[5-(methylsulfanyl)-1,3,4-thiadiazol-2-yl]carbamoyl}-1,3,4-oxadiazol-2-yl)pyrrolidine-2-carboxylic acid 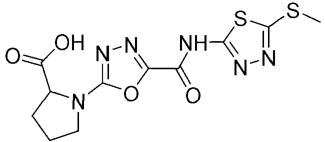 | 249 | *N*-(5-methylsulfanyl-1,3,4-thiadiazol-2-yl)-4-(m-tolyl)-6-oxo-pyran-2-carboxamide 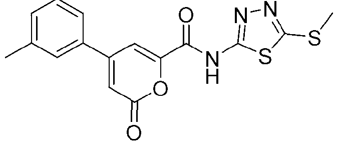 |
| 112 | 1-(5-{[5-(methylsulfanyl)-1,3,4-thiadiazol-2-yl]carbamoyl}-1,3,4-oxadiazol-2-yl)pyrrolidine-3-carboxylic acid 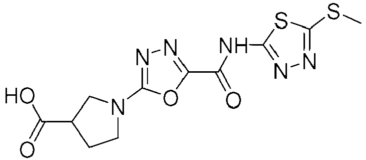 | 250 | *N*-(5-methylsulfanyl-1,3,4-thiadiazol-2-yl)-6-oxo-4-(p-tolyl)pyran-2-carboxamide 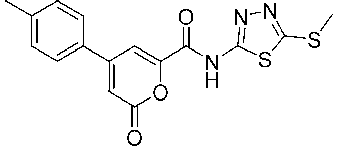 |
| 113 | 5-[benzyl(methyl)amino]-*N*-[5-(methylsulfanyl)-1,3,4-thiadiazol-2-yl]-1,3,4-oxadiazole-2-carboxamide 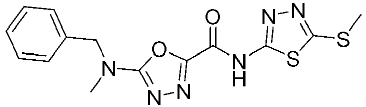 | 251 | 4-(2-chlorophenyl)-*N*-(5-methylsulfanyl-1,3,4-thiadiazol-2-yl)-6-oxo-pyran-2-carboxamide 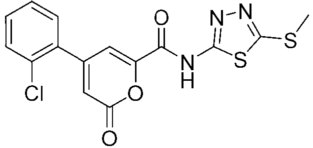 |
| 114 | 5-[methyl(1-phenylethyl)amino]-*N*-[5-(methylsulfanyl)-1,3,4-thiadiazol-2-yl]-1,3,4-oxadiazole-2-carboxamide 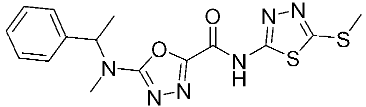 | 252 | 4-(4-methoxyphenyl)-*N*-(5-methylsulfanyl-1,3,4-thiadiazol-2-yl)-6-oxo-pyran-2-carboxamide 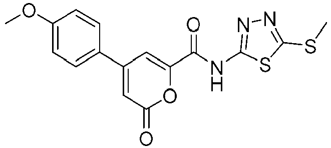 |
| *115* | 5-[2-(hydroxymethyl)pyrrolidin-1-yl]-*N-*[5-(methylsulfanyl)-1,3,4-thiadiazol-2-yl]-1,3,4-oxadiazole-2-carboxamide 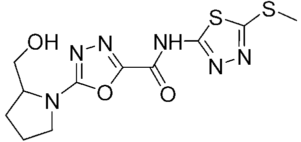 | 253 | *N*-(5-methylsulfanyl-1,3,4-thiadiazol-2-yl)-4-(1-naphthyl)-6-oxo-pyran-2-carboxamide 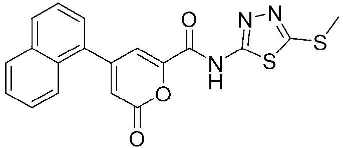 |
| 116 | 5-(3-hydroxypyrrolidin-1-yl)-*N*-[5-(methylsulfanyl)-1,3,4-thiadiazol-2-yl]-1,3,4-oxadiazole-2-carboxamide 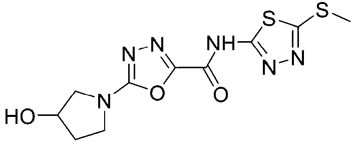 | 254 | *N*-(5-methylsulfanyl-1,3,4-thiadiazol-2-yl)-6-oxo-4-(2-quinolyl)pyran-2-carboxamide 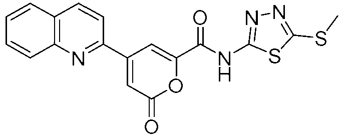 |
| 117 | (*R*)-*N*-(5-(2-chlorophenyl)-1,3,4-thiadiazol-2-yl)-5-(2-(pyridin-2-yl)pyrrolidin-1-yl)-1,3,4-oxadiazole-2-carboxamide 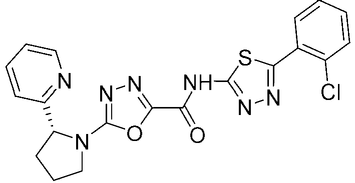 | 255 | *N*-(5-methylsulfanyl-1,3,4-thiadiazol-2-yl)-6-oxo-4-(3-quinolyl)pyran-2-carboxamide 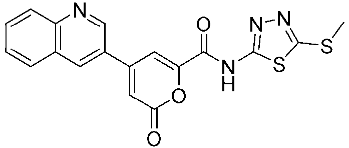 |
| 118 | (*R*)-*N*-(5-(2-chlorophenyl)-1,3,4-thiadiazol-2-yl)-5-(2-phenylpyrrolidin-1-yl)-1,3,4-oxadiazole-2-carboxamide 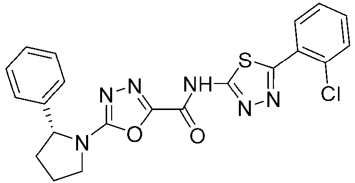 | 256 | 4-(4-isoquinolyl)-*N*-(5-methylsulfanyl-1,3,4-thiadiazol-2-yl)-6-oxo-pyran-2-carboxamide 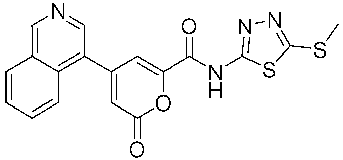 |
| 119 | 5-(3,3-dimethylpiperidin-1-yl)-*N*-(5-(methylthio)-1,3,4-thiadiazol-2-yl)-1,3,4-oxadiazole-2-carboxamide 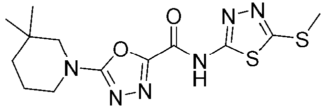 | 257 | 5-anilino-*N*-(5-methylsulfanyl-1,3,4-thiadiazol-2-yl)-1,3,4-oxadiazole-2-carboxamide 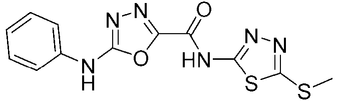 |
| 120 | 5-(4,4-dimethylpiperidin-1-yl)-N-(5-(methylthio)-1,3,4-thiadiazol-2-yl)-1,3,4-oxadiazole-2-carboxamide 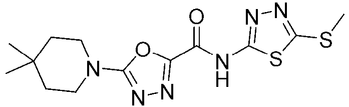 | 258 | 5-cyclohexyl*-N*-(5-methylsulfanyl-1,3,4-thiadiazol-2-yl)-1,3,4-oxadiazole-2-carboxamide 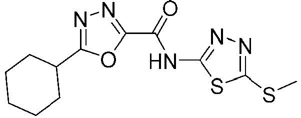 |
| 121 * | (*R*)-*N*-(5-(methylthio)-1,3,4-thiadiazol-2-yl)-5-(2-phenylpiperidin-1-yl)-1,3,4-oxadiazole-2-carboxamide 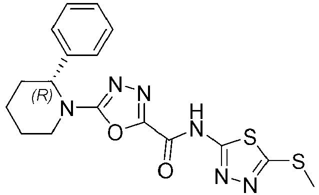 | 259 | 5-benzyl-*N*-(5-methylsulfanyl-1,3,4-thiadiazol-2-yl)-1,3,4-oxadiazole-2-carboxamide 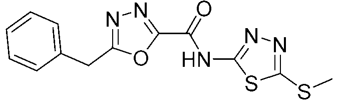 |
| 122 * | (*S*)-*N*-(5-(methylthio)-1,3,4-thiadiazol-2-yl)-5-(2-phenylpiperidin-1-yl)-1,3,4-oxadiazole-2-carboxamide 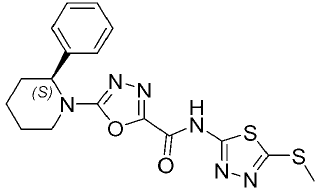 | 260 | *N*-(5-methylsulfanyl-1,3,4-thiadiazol-2-yl)-[1,2,4]triazolo[4,3-a]pyridine-3-carboxamide 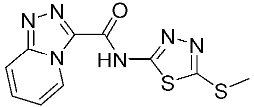 |
| 123 | *N*-(5-(methylthio)-1,3,4-thiadiazol-2-yl)-5-(2-phenylpyrrolidin-1-yl)-1,3,4-oxadiazole-2-carboxamide 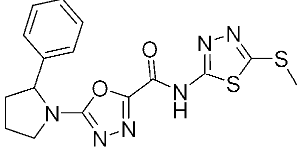 | 261 | 3-benzyl-*N*-(5-methylsulfanyl-1,3,4-thiadiazol-2-yl)-1,2,4-oxadiazole-5-carboxamide 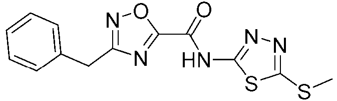 |
| 124 | *N*-(5-(methylthio)-1,3,4-thiadiazol-2-yl)-5-(3-oxopiperazin-1-yl)-1,3,4-oxadiazole-2-carboxamide 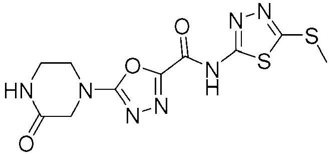 | 262 | 3-cyclohexyl-*N*-(5-(methylthio)-1,3,4-thiadiazol-2-yl)-1,2,4-oxadiazole-5-carboxamide 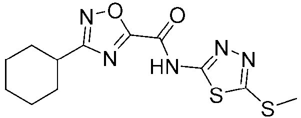 |
| 125 | (*R*)-*N*-(5-(methylthio)-1,3,4-thiadiazol-2-yl)-5-(2-phenylpyrrolidin-1-yl)-1,3,4-oxadiazole-2-carboxamide 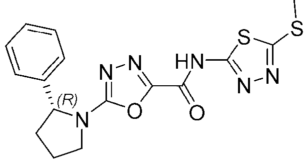 | 263 | 5-cyclohexyl-*N*-(5-(methylthio)-1,3,4-thiadiazol-2-yl)-1,2,4-oxadiazole-3-carboxamide 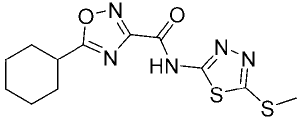 |
| 126 | (*S*)-*N*-(5-(methylthio)-1,3,4-thiadiazol-2-yl)-5-(2-phenylpyrrolidin-1-yl)-1,3,4-oxadiazole-2-carboxamide 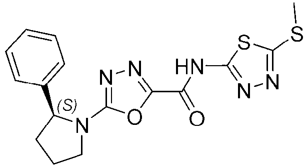 | 264 | 4-(isoquinolin-7-yl)-*N*-(5-(methylthio)-1,3,4-thiadiazol-2-yl)-2-oxo-2*H*-pyran-6-carboxamide 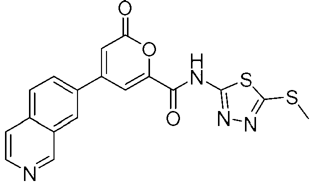 |
| 127 | *N*-(5-(thiazol-4-yl)-1,3,4-thiadiazol-2-yl)benzo[c]isoxazole-3-carboxamide 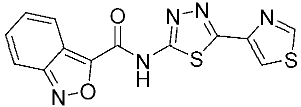 | 265 | *N*-(5-(methylthio)-1,3,4-thiadiazol-2-yl)-3-(phenylamino)-1,2,4-oxadiazole-5-carboxamide 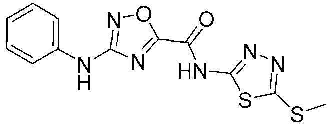 |
| 128 | *N*-(5-(thiazol-5-yl)-1,3,4-thiadiazol-2-yl)benzo[c]isoxazole-3-carboxamide 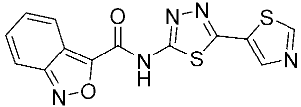 | 266 | *N*-(5-(methylthio)-1,3,4-thiadiazol-2-yl)-2-phenylisonicotinamide 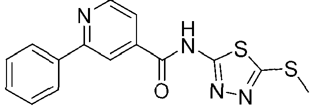 |
| 129 | *N*-[5-(1*H*-imidazol-4-yl)-1,3,4-thiadiazol-2-yl]-2,1-benzoxazole-3-carboxamide 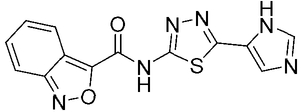 | 267 | 4-(4-methylquinolin-8-yl)-*N*-(5-(methylthio)-1,3,4-thiadiazol-2-yl)-2-oxo-2*H*-pyran-6-carboxamide 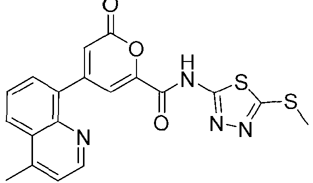 |
| 130 | *N*-[5-(pyrazin-2-yl)-1,3,4-thiadiazol-2-yl]-2,1-benzoxazole-3-carboxamide 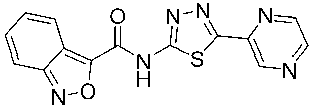 | 268 | *N*-(5-(methylthio)-1,3,4-thiadiazol-2-yl)-2-oxo-4-(phenylamino)-2*H*-pyran-6-carboxamide 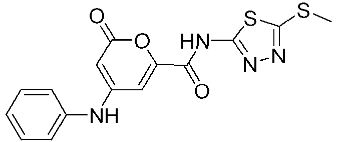 |
| 131 | *N*-(5-(thiazol-2-yl)-1,3,4-thiadiazol-2-yl)benzo[c]isoxazole-3-carboxamide 2,2,2-trifluoroacetate 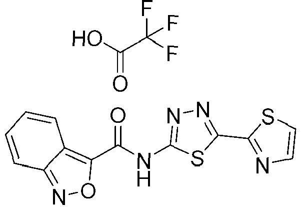 | 269 | *4*-(1-methyl-1*H*-indazol-4-yl)-*N*-(5-(methylthio)-1,3,4-thiadiazol-2-yl)-2-oxo-2*H*-pyran-6-carboxamide 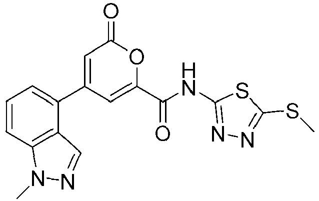 |
| 132 | [5-(2,1-benzoxazole-3-amido)-1,3,4-thiadiazol-2-yl]acetic acid 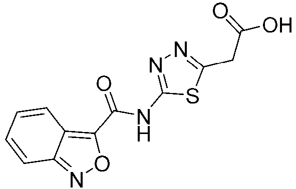 | 270 | *N*-(5-(methylthio)-1,3,4-thiadiazol-2-yl)-2-oxo-4-(quinolin-7-yl)-2*H*-pyran-6-carboxamide 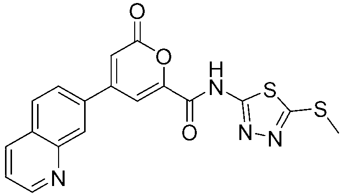 |
| 133 | *N*-[5-(methylthio)-1,3,4-thiadiazol-2-yl]-2-(pyridin-2-ylmethyl)-1,2,3,4-tetrazole-5-carboxamide 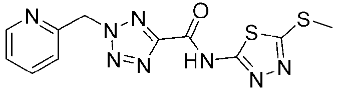 | 271 | *N*-(5-(methylthio)-1,3,4-thiadiazol-2-yl)isoxazolo[3,4-flquinoline-3-carboxamide 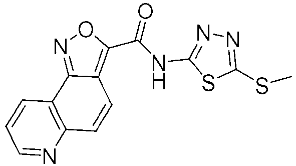 |
| 134 | 2-benzyl-*N*-(5-(methylthio)-1,3,4-thiadiazol-2-yl)-2*H*-tetrazole-5-carboxamide 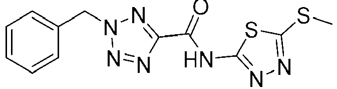 | 272 | *N*-(5-(methylthio)-1,3,4-thiadiazol-2-yl)-2-oxo-4-(6-oxo-1,6-dihydropyridin-3-yl)-2*H*-pyran-6-carboxamide 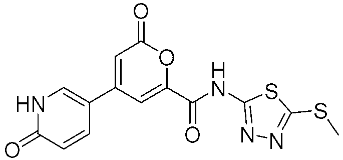 |
| 135 | *N*-(5-(methylthio)-1,3,4-thiadiazol-2-yl)-2-(pyridin-3-ylmethyl)-2H-tetrazole-5-carboxamide 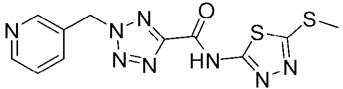 | 273 | *N*-(5-(methylthio)-1,3,4-thiadiazol-2-yl)isoxazolo[3,4-flisoquinoline-3-carboxamide 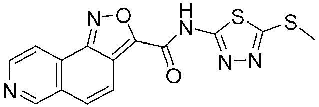 |
| 136 | *N*-(5-(methylthio)-1,3,4-thiadiazol-2-yl)-2-(pyridin-2-yl)-2H-tetrazole-5-carboxamide 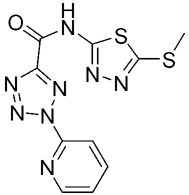 | 274 | 4-(1-methyl-1*H*-benzo[d]imidazol-4-yl)-*N*-(5-(methylthio)-1,3,4-thiadiazol-2-yl)-2-oxo-2*H*-pyran-6-carboxamide 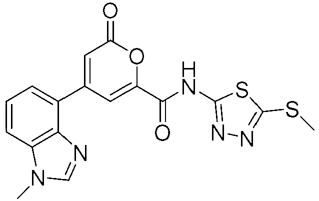 |
| 137 | *N*-(5-(methylthio)-1,3,4-thiadiazol-2-yl)-2-(pyridin-3-yl)-2*H*-tetrazole-5-carboxamide 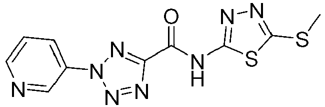 | 275 | 4-(3,4-dihydro-2*H-*benzo[b][1,4]oxazin-8-yl)-*N*-(5-(methylthio)-1,3,4-thiadiazol-2-yl)-2-oxo-2*H*-pyran-6-carboxamide 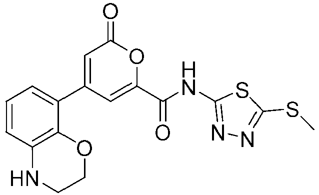 |
| 138 | 2-(2-fluorophenyl)-*N*-[5-(methylsulfanyl)-1,3,4-thiadiazol-2-yl]-1,2,3,4-tetrazole-5-carboxamide 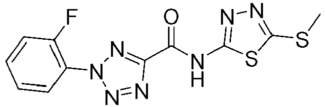 | 276 | *N*-(5-(methylthio)-1,3,4-thiadiazol-2-yl)-2-oxo-4-(6-oxo-1,6-dihydropyridin-2-yl)-2*H*-pyran-6-carboxamide 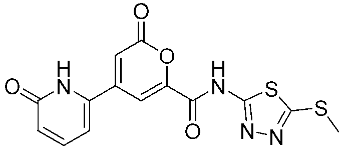 |
| 139 | *N*-[5-(methylsulfanyl)-1,3,4-thiadiazol-2-yl]-3-(thiophen-3-yl)-1,2-oxazole-5-carboxamide 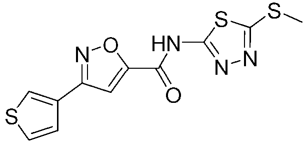 | 277 | 4-(isoquinolin-8-yl)*-N*-(5-(methylthio)-1,3,4-thiadiazol-2-yl)-2-oxo-2*H*-pyran-6-carboxamide 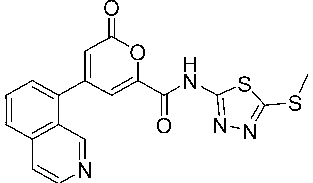 |
| 140 | *N*-[5-(methylsulfanyl)-1,3,4-thiadiazol-2-yl]-3-(1,2-thiazol-5-yl)-1,2-oxazole-5-carboxamide 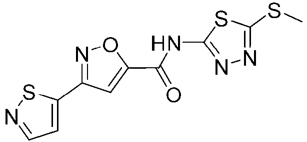 | 278 | methyl 3-((5-(methylthio)-1,3,4-thiadiazol-2-yl)carbamoyl)benzo[c]isoxazole-6-carboxylate 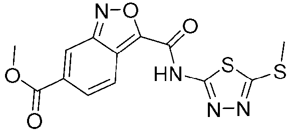 |
| 141 | *N*-(5-(methylthio)-1,3,4-thiadiazol-2-yl)-3-(thiazol-5-yl)isoxazole-5-carboxamide 2,2,2-trifluoroacetate 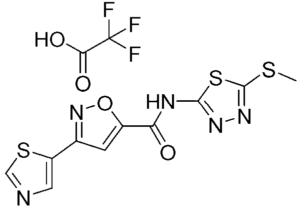 | 279 | *N*-(5-(methylthio)-1,3,4-thiadiazol-2-yl)isoxazolo[4,3-h]isoquinoline-3-carboxamide 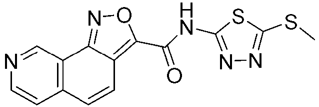 |
| 142 | *N*-(5-(methylthio)-1,3,4-thiadiazol-2-yl)-3-(thiazol-2-yl)isoxazole-5-carboxamide 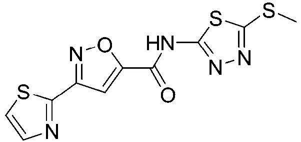 | 280 | *N*-(5-(methylamino)-1,3,4-thiadiazol-2-yl)isoxazolo[4,3-h]quinoline-3-carboxamide 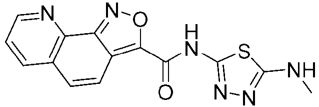 |

In some embodiments, the compound of Formula (I) is a compound selected from **Table 2.**

In some embodiments, the compound of Formula (I) is a compound selected from **Table 2,** or a pharmaceutically acceptable salt, hydrate, solvate, prodrug, stereoisomer, labeled isotope, or tautomer thereof.

In some embodiments, the compound of Formula (I) is a compound selected from **Table 2,** or a pharmaceutically acceptable salt thereof.

In some embodiments, the compound of Formula (I) is a compound selected from **Table 2,** or a hydrate thereof.

In some embodiments, the compound of Formula (I) is a compound selected from **Table 2,** or a solvate thereof.

In some embodiments, the compound of Formula (I) is a compound selected from **Table 2,** or prodrug thereof.

In some embodiments, the compound of Formula (I) is a compound selected from **Table 2,** or a stereoisomer thereof.

In some embodiments, the compound of Formula (I) is a compound selected from **Table 2,** or a labeled isotope thereof.

In some embodiments, the compound of Formula (I) is a compound selected from **Table 2,** or a tautomer thereof.

| **Table 2.** | |
|---|---|
| **#** | **Compound Name/Structure** |
| 11 | *N*-(3-(methylthio)-1,2,4-thiadiazol-5-yl)isoxazolo[4,3-h]quinoline-3-carboxamide |
| | 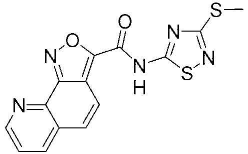 |
| 97 | *N*-(2-chlorophenyl)-5-oxo-4,5-dihydro-[1,2,3]triazolo[1,5-a]pyrimidine-3-carboxamide 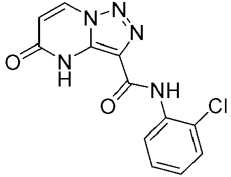 |

In some embodiments, the compound of Formula (I) is Compound 7, 22, 55, 64, 90, 93, 94, 169, 205, or 206, or a pharmaceutically acceptable salt, hydrate, solvate, prodrug, stereoisomer, labeled isotope, or tautomer thereof.

In some embodiments, the compound is a pharmaceutically acceptable salt. In some embodiments, the compound is a hydrochloride salt.

It should be understood that all isomeric forms are included within the present disclosure, including mixtures thereof. If the compound contains a double bond, the substituent may be in the E or Z configuration. If the compound contains a disubstituted cycloalkyl, the cycloalkyl substituent may have a cis or trans configuration. All tautomeric forms are also intended to be included.

Compounds of the disclosure, and pharmaceutically acceptable salts, hydrates, solvates, stereoisomers and prodrugs thereof may exist in their tautomeric form (for example, as an amide or imino ether). All such tautomeric forms are contemplated herein as part of the present disclosure.

The compounds of the disclosure may contain asymmetric or chiral centers, and, therefore, exist in different stereoisomeric forms. It is intended that all stereoisomeric forms of the compounds of the disclosure as well as mixtures thereof, including racemic mixtures, form part of the present disclosure. In addition, the present disclosure embraces all geometric and positional isomers. For example, if a compound of the disclosure incorporates a double bond or a fused ring, both the cis- and trans-forms, as well as mixtures, are embraced within the scope of the disclosure. each compound herein disclosed includes all the enantiomers that conform to the general structure of the compound. The compounds may be in a racemic or enantiomerically pure form, or any other form in terms of stereochemistry. The assay results may reflect the data collected for the racemic form, the enantiomerically pure form, or any other form in terms of stereochemistry.

Diastereomeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diastereomeric mixture by reaction with an appropriate optically active compound (*e.g*., chiral auxiliary such as a chiral alcohol or Mosher's acid chloride), separating the diastereomers and converting (*e.g*., hydrolyzing) the individual diastereomers to the corresponding pure enantiomers. Also, some of the compounds of the disclosure may be atropisomers (*e.g*., substituted biaryls) and are considered as part of this disclosure. Enantiomers can also be separated by use of a chiral HPLC column.

It is also possible that the compounds of the disclosure may exist in different tautomeric forms, and all such forms are embraced within the scope of the disclosure. Also, for example, all keto-enol and imine-enamine forms of the compounds are included in the disclosure.

All stereoisomers (for example, geometric isomers, optical isomers and the like) of the present compounds (including those of the salts, solvates, esters and prodrugs of the compounds as well as the salts, solvates and esters of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this disclosure, as are positional isomers (such as, for example, 4-pyridyl and 3-pyridyl). (For example, if a compound of Formula (I) incorporates a double bond or a fused ring, both the cis- and trans-forms, as well as mixtures, are embraced within the scope of the disclosure. Also, for example, all keto-enol and imine-enamine forms of the compounds are included in the disclosure.) Individual stereoisomers of the compounds of the disclosure may, for example, be substantially free of other isomers, or may be admixed, for example, as racemates or with all other, or other selected, stereoisomers. The chiral centers of the present disclosure can have the S or R configuration as defined by the IUPAC 1974 Recommendations. The use of the terms "salt", "solvate", "ester," "prodrug" and the like, is intended to equally apply to the salt, solvate, ester and prodrug of enantiomers, stereoisomers, rotamers, tautomers, positional isomers, racemates or prodrugs of the inventive compounds.

The compounds of Formula (I) may form salts which are also within the scope of this disclosure. Reference to a compound of the Formula herein is understood to include reference to salts thereof, unless otherwise indicated.

The present disclosure relates to compounds which are modulators of cGAS. In one embodiment, the compounds of the present disclosure are inhibitors of cGAS. In another embodiment, the cGAS is Isoform 1. In another embodiment, the cGAS is Isoform 2.

In some embodiments, the compounds of Formula (I) are selective inhibitors of cGAS.

The disclosure is directed to compounds as described herein and pharmaceutically acceptable salts, hydrates, solvates, prodrugs, stereoisomers, or tautomers thereof, and pharmaceutical compositions comprising one or more compounds as described herein, or pharmaceutically acceptable salts, hydrates, solvates, prodrugs, stereoisomers, labeled isotopes, or tautomers thereof.

### Methods of Synthesis

In some aspects, the present disclosure provides a method of preparing a compound of the present disclosure.

In some aspects, the present disclosure provides a method of a compound, comprising one or more steps as described herein.

**In** some aspects, the present disclosure provides a compound obtainable by, or obtained by, or directly obtained by a method for preparing a compound as described herein.

**In** some aspects, the present disclosure provides an intermediate as described herein, being suitable for use in a method for preparing a compound as described herein.

The compounds of the present disclosure may be made by a variety of methods, including standard chemistry. Suitable synthetic routes are depicted in the Schemes given below.

The compounds of Formula (I) may be prepared by methods known in the art of organic synthesis as set forth in part by the following synthetic schemes. In the schemes described below, it is well understood that protecting groups for sensitive or reactive groups are employed where necessary in accordance with general principles or chemistry. Protecting groups are manipulated according to standard methods of organic synthesis (T. W. Greene and P. G. M. Wuts, "Protective Groups in Organic Synthesis", Third edition, Wiley, New York 1999). These groups are removed at a convenient stage of the compound synthesis using methods that are readily apparent to those skilled in the art. The selection processes, as well as the reaction conditions and order of those skilled in the art will recognize if a stereocenter exists in the compounds of Formula (I). Accordingly, the present disclosure includes both possible stereoisomers (unless specified in the synthesis) and includes not only racemic compounds but the individual enantiomers and/or diastereomers as well. When a compound is desired as a single enantiomer or diastereomer, it may be obtained by stereospecific synthesis or by resolution of the final product or any convenient intermediate. Resolution of the final product, an intermediate, or a starting material may be affected by any suitable method known in the art. See, for example, "Stereochemistry of Organic Compounds" by E. L. Eliel, S. H. Wilen, and L. N. Mander (Wiley-Interscience, 1994).

The compounds described herein may be made from commercially available starting materials or synthesized using known organic, inorganic, and/or enzymatic processes.

### Preparation of Compounds

The compounds of the present disclosure can be prepared in a number of ways well known to those skilled in the art of organic synthesis. By way of example, compounds of the present disclosure can be synthesized using the methods described below, together with synthetic methods known in the art of synthetic organic chemistry, or variations thereon as appreciated by those skilled in the art. Preferred methods include but are not limited to those methods described below. Compounds of the present disclosure can be synthesized by following the steps outlined in General Procedures A-G which comprise different sequences of assembling intermediates or compounds. Starting materials are either commercially available or made by known procedures in the reported literature or as illustrated below.

### General Procedure A

Following this procedure, the compounds of Formula (I) were prepared in a one-step process through the amide coupling between an amine or aniline of Formula (II) and a carboxylic acid of Formula (III), metal carboxylate salt of Formula (IV), or ester of Formula (V), wherein the amines or anilines of Formula (II), carboxylic acids of Formula (III), metal carboxylate salts of Formula (IV), or esters of Formula (V) are either commercially available or known in the chemical literature, unless otherwise indicated.

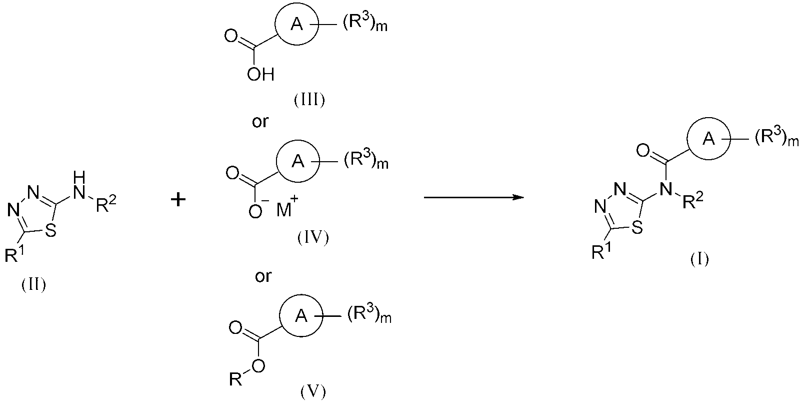

### General Procedure B

Following this procedure, the compounds of Formula (I) were prepared in a one-step process through the transition-metal catalyzed cross-coupling of an Ar-Br containing compound of Formula (VI) and a boronic acid or boronic ester compound of Formula (VII) or an organostannane compound of Formula (VIII), wherein Ar-Br containing compounds of Formula (VI) were prepared using General Procedure A and boronic acid or boronic ester compounds of Formula (VII) or organostannane compounds of Formula (VIII) are either commercially available or known in the chemical literature.

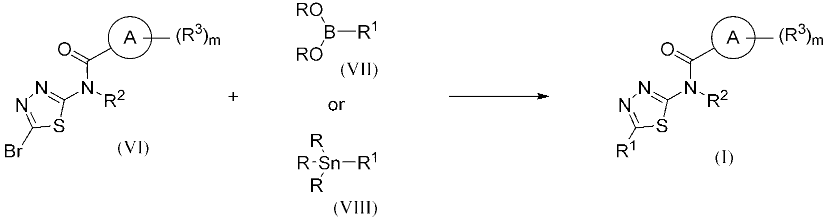

### General Procedure C

Following this procedure, the compounds of Formula (I) were prepared in a one-step process through the deprotection of a compound containing a protecting group (PG) of Formula (IX) or Formula (X), wherein compounds containing PG of Formula (IX) or Formula (X) were prepared using General Procedures A or B.

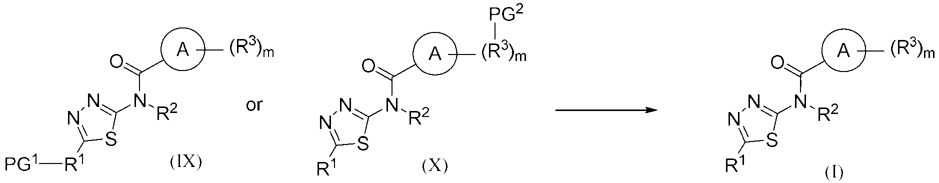

### Biological Assays

Compounds designed, selected and/or optimized by methods described above, once produced, can be characterized using a variety of assays known to those skilled in the art to determine whether the compounds have biological activity. For example, the molecules can be characterized by conventional assays, including but not limited to those assays described below, to determine whether they have a predicted activity, binding activity and/or binding specificity.

Furthermore, high-throughput screening can be used to speed up analysis using such assays. As a result, it can be possible to rapidly screen the molecules described herein for activity, using techniques known in the art. General methodologies for performing high-throughput screening are described, for example, in Devlin (1998) High Throughput Screening, Marcel Dekker; and U.S. Patent No. 5,763,263. High-throughput assays can use one or more different assay techniques including, but not limited to, those described below.

Various *in vitro* or *in vivo* biological assays are may be suitable for detecting the effect of the compounds of the present disclosure. These *in vitro* or *in vivo* biological assays can include, but are not limited to, enzymatic activity assays, electrophoretic mobility shift assays, reporter gene assays, *in vitro* cell viability assays, and the assays described herein.

In some embodiments, the compounds of the instant disclosure may be tested for their human-cGAS (h-cGAS) inhibition activity using the methodology reported in Lama et al., "Development of human cGAS-specific small molecule inhibitors for repression of double stranded DNA (dsDNA)-triggered interferon expression", Nature Communications 10, Article number: 2261 (2019), incorporated herein by reference.

### Methods of Use

In some aspects, the present disclosure provides a method of modulating cGAS activity (*e.g., in vitro* or *in vivo*)*,* comprising contacting a cell with an effective amount of a compound of the present disclosure or a pharmaceutically acceptable salt, hydrate, solvate, prodrug, stereoisomer, labeled isotope, or tautomer thereof.

In some aspects, the present disclosure provides a method of modulating cGAS activity (*e.g*., *in vitro* or *in vivo*), comprising contacting a cell with a compound of the present disclosure or a pharmaceutically acceptable salt, hydrate, solvate, prodrug, stereoisomer, labeled isotope, or tautomer thereof.

In some aspects, the present disclosure provides a method of treating or preventing a disease or disorder disclosed herein in a subject in need thereof, comprising administering to the subject a therapeutically effective amount of a compound of the present disclosure or a pharmaceutically acceptable salt, hydrate, solvate, prodrug, stereoisomer, labeled isotope, or tautomer thereof.

In some aspects, the present disclosure provides a method of treating a disease or disorder disclosed herein in a subject in need thereof, comprising administering to the subject a therapeutically effective amount of a compound of the present disclosure, or a pharmaceutically acceptable salt, hydrate, solvate, prodrug, stereoisomer, labeled isotope, or tautomer thereof, or a pharmaceutical composition of the present disclosure.

In some aspects, the present disclosure provides a method of treating or preventing a disease or disorder disclosed herein in a subject in need thereof, comprising administering to the subject a compound of the present disclosure or a pharmaceutically acceptable salt, hydrate, solvate, prodrug, stereoisomer, labeled isotope, or tautomer thereof.

In some aspects, the present disclosure provides a method of treating a disease or disorder disclosed herein in a subject in need thereof, comprising administering to the subject a compound of the present disclosure, or a pharmaceutically acceptable salt, hydrate, solvate, prodrug, stereoisomer, labeled isotope, or tautomer thereof, or a pharmaceutical composition of the present disclosure.

In some embodiments, the disease or disorder is associated with implicated cGAS activity. In some embodiments, the disease or disorder is a disease or disorder in which cGAS activity is implicated.

In some aspects, the present disclosure provides a compound of the present disclosure, or a pharmaceutically acceptable salt, hydrate, solvate, prodrug, stereoisomer, labeled isotope, or tautomer thereof, for use in modulating cGAS activity (*e.g*., *in vitro* or *in vivo*)*.*

In some aspects, the present disclosure provides a compound of the present disclosure, or a pharmaceutically acceptable salt, hydrate, solvate, prodrug, stereoisomer, labeled isotope, or tautomer thereof, for use in treating or preventing a disease or disorder disclosed herein.

In some aspects, the present disclosure provides a compound of the present disclosure, or a pharmaceutically acceptable salt, hydrate, solvate, prodrug, stereoisomer, labeled isotope, or tautomer thereof, for use in treating a disease or disorder disclosed herein.

In some aspects, the present disclosure provides use of a compound of the present disclosure, or a pharmaceutically acceptable salt, hydrate, solvate, prodrug, stereoisomer, labeled isotope, or tautomer thereof, in the manufacture of a medicament for modulating cGAS activity (*e.g*., *in vitro* or *in vivo*)*.*

In some aspects, the present disclosure provides use of a compound of the present disclosure, or a pharmaceutically acceptable salt, hydrate, solvate, prodrug, stereoisomer, labeled isotope, or tautomer thereof, in the manufacture of a medicament for treating or preventing a disease or disorder disclosed herein.

In some aspects, the present disclosure provides use of a compound of the present disclosure, or a pharmaceutically acceptable salt, hydrate, solvate, prodrug, stereoisomer, labeled isotope, or tautomer thereof, in the manufacture of a medicament for treating a disease or disorder disclosed herein.

The present disclosure provides compounds that function as modulators of cGAS activity.

The present disclosure provides compounds that function as modulators of cGAS activity.

In some embodiments, modulation is inhibition.

In some aspects, the present disclosure provides a method of treating a disease or disorder associated with modulation of cGAS. The method comprises administering to a patient in need of a treatment for diseases or disorders associated with modulation of cGAS an effective amount of a compound of Formula (I), or a pharmaceutically acceptable salt, hydrate, solvate, prodrug, stereoisomer, labeled isotope, or tautomer thereof.

In some aspects, the present disclosure provides a method of inhibiting cGAS. The method involves administering to a patient in need thereof an effective amount of a compound of Formula (I), or a pharmaceutically acceptable salt, hydrate, solvate, prodrug, stereoisomer, labeled isotope, or tautomer thereof.

In some aspects, the present disclosure provides a method of treating, preventing, inhibiting or eliminating a disease or disorder in a patient associated with the inhibition of cGAS, comprising administering to a patient in need thereof an effective amount of a compound of Formula (I), or a pharmaceutically acceptable salt, hydrate, solvate, prodrug, stereoisomer, labeled isotope, or tautomer thereof. In some embodiments, the disease may be, but not limited to, cancer or metastasis.

In some aspects, the present disclosure provides a use of an inhibitor of cGAS for the preparation of a medicament used in the treatment, prevention, inhibition or elimination of a disease or condition mediated by cGAS, wherein the medicament comprises a compound of Formula (I), or a pharmaceutically acceptable salt, hydrate, solvate, prodrug, stereoisomer, labeled isotope, or tautomer thereof.

In some aspects, the present disclosure provides a method for the manufacture of a medicament for treating, preventing, inhibiting, or eliminating a disease or condition mediated by cGAS, wherein the medicament comprises a compound of Formula (I), or a pharmaceutically acceptable salt, hydrate, solvate, prodrug, stereoisomer, labeled isotope, or tautomer thereof.

In some aspects, the present disclosure provides a compound of Formula (I), or a pharmaceutically acceptable salt, hydrate, solvate, prodrug, stereoisomer, labeled isotope, or tautomer thereof for use in the manufacture of a medicament for treating a disease associated with inhibiting cGAS.

In some aspects, the present disclosure provides use of a compound of Formula (I), or a pharmaceutically acceptable salt, hydrate, solvate, prodrug, stereoisomer, labeled isotope, or tautomer thereof in the treatment of a disease associated with inhibiting cGAS.

In some aspects, the present disclosure provides a method of treating cancer. The method comprises administering to a patient in need thereof an effective amount of a compound of Formula (I), or a pharmaceutically acceptable salt, hydrate, solvate, prodrug, stereoisomer, labeled isotope, or tautomer thereof.

In some aspects, the present disclosure provides a method of treating or preventing cancer. The method comprises administering to a patient in need thereof an effective amount of a compound of Formula (I), or a pharmaceutically acceptable salt, hydrate, solvate, prodrug, stereoisomer, labeled isotope, or tautomer thereof.

In some aspects, the present disclosure provides use of an inhibitor of cGAS for the preparation of a medicament used in treatment, prevention, inhibition or elimination of a disease or disorder associated with cancer.

In some embodiments, the disease or disorder is cancer.

In some aspects, the present disclosure provides a compound of Formula (I), or a pharmaceutically acceptable salt, hydrate, solvate, prodrug, stereoisomer, labeled isotope, or tautomer thereof, or a pharmaceutical composition comprising a compound of the present disclosure and a pharmaceutically acceptable carrier used for the treatment of diseases or disorders including, but not limited to, systemic lupus erythematosus (SLE), cutaneous lupus erythematosus (CLE), scleroderma, psoriasis, Aicardi Goutières syndrome, Sjogren's syndrome, rheumatoid arthritis, inflammatory bowel diseases, multiple sclerosis, diabetes, cardiovascular, and neurodegenerative diseases.

In some embodiments, the compounds of the instant disclosure are used for the treatment of cancer including, but not limited to, bladder cancer, bone cancer, brain cancer, breast cancer, cardiac cancer, cervical cancer, colon cancer, colorectal cancer, esophageal cancer, fibrosarcoma, gastric cancer, gastrointestinal cancer, head, spine and neck cancer, Kaposi's sarcoma, kidney cancer, pancreatic cancer, penile cancer, testicular germ cell cancer, thymoma carcinoma, thymic carcinoma, lung cancer, ovarian cancer, or prostate cancer,.

In some embodiments, the disease or condition is an inflammatory, allergic or autoimmune diseases such as systemic lupus erythematosus (SLE), cutaneous lupus erythematosus (CLE), psoriasis, insulin-dependent diabetes mellitus (IDDM), scleroderma, Aicardi Goutières syndrome, dermatomyositis, inflammatory bowel diseases, multiple sclerosis, rheumatoid arthritis and Sjogren's syndrome (SS).

In some embodiments, the compounds of the disclosure may be used to treat inflammation of any tissue and organs of the body, including musculoskeletal inflammation, vascular inflammation, neural inflammation, digestive system inflammation, ocular inflammation, inflammation of the reproductive system, and other inflammation, as exemplified below.

In some embodiments, musculoskeletal inflammation refers to any inflammatory condition of the musculoskeletal system, particularly those conditions affecting skeletal joints, including joints of the hand, wrist, elbow, shoulder, jaw, spine, neck, hip, knew, ankle, and foot, and conditions affecting tissues connecting muscles to bones such as tendons. Examples of musculoskeletal inflammation which may be treated with compounds of the disclosure include arthritis (including, for example, osteoarthritis, rheumatoid arthritis, psoriatic arthritis, ankylosing spondylitis, acute and chronic infectious arthritis, arthritis associated with gout and pseudogout, and juvenile idiopathic arthritis), tendonitis, synovitis, tenosynovitis, bursitis, fibrositis (fibromyalgia), epicondylitis, myositis, and osteitis (including, for example, Paget's disease, osteitis pubis, and osteitis fibrosa cystic). Ocular inflammation refers to inflammation of any structure of the eye, including the eye lids. Examples of ocular inflammation which may be treated with the compounds of the disclosure include blepharitis, blepharochalasis, conjunctivitis, dacryoadenitis, keratitis, keratoconjunctivitis sicca (dry eye), scleritis, trichiasis, and uveitis. Examples of inflammation of the nervous system which may be treated with the compounds of the disclosure include encephalitis, Guillain-Barre syndrome, meningitis, neuromyotonia, narcolepsy, multiple sclerosis, myelitis and schizophrenia.

Examples of inflammation of the vasculature or lymphatic system which may be treated with the compounds of the disclosure include arthrosclerosis, arthritis, phlebitis, vasculitis, and lymphangitis.

Examples of inflammatory conditions of the digestive system which may be treated with the compounds of the disclosure include cholangitis, cholecystitis, enteritis, enterocolitis. gastritis, gastroenteritis, inflammatory bowel disease (such as Crohn's disease and ulcerative colitis), ileitis, and proctitis.

Examples of inflammatory conditions of the reproductive system which may be treated with the compounds of the disclosure include cervicitis, chorioamnionitis, endometritis, epididymitis, omphalitis, oophoritis, orchitis, salpingitis, tubo-ovarian abscess, urethritis, vaginitis, vulvitis, and vulvodynia.

In some embodiments, a compound of the present disclosure may be used to treat autoimmune conditions having an inflammatory component. Such conditions include systemic lupus erythematosus, cutaneous lupus erythematosus, acute disseminated alopecia universalise, Bechet's disease, Chagas' disease, chronic fatigue syndrome, dysautonomia, encephalomyelitis, ankylosing spondylitis, aplastic anemia, hidradenitis suppurativa, autoimmune hepatitis, autoimmune oophoritis, celiac disease, Crohn's disease, diabetes mellitus type 1, giant cell arteritis, Goodpasture's syndrome. Grave's disease, Guillain-Barre syndrome, Hashimoto's disease, Henoch-Schonlein purpura, Kawasaki's disease, microscopic colitis, microscopic polyarteritis, mixed connective tissue disease, multiple sclerosis, myasthenia gravis, opsoclonus myoclonus syndrome, optic neuritis, Ord's thyroiditis, pemphigus, polyarteritis nodosa, polymyalgia, rheumatoid arthritis, Reiter's syndrome, Sjogren's syndrome, Aicardi Goutières syndrome, temporal arteritis, Wegener's granulomatosis, warm autoimmune haemolytic anemia, interstitial cystitis, Lyme disease, morphea, psoriasis, sarcoidosis, scleroderma, ulcerative colitis, and vitiligo.

In some embodiments, the compounds of the present disclosure may be used to treat T-cell mediated hypersensitivity diseases having an inflammatory component. Such conditions include contact hypersensitivity, contact dermatitis (including that due to poison ivy), urticaria, skin allergies, respiratory allergies (hay fever, allergic rhinitis) and gluten-sensitive enteropathy (Celiac disease).

In some embodiments, other inflammatory conditions which may be treated with the compounds of the present disclosure include, for example, appendicitis, dermatitis, dermatomyositis, endocarditis, fibrositis, gingivitis, glossitis, hepatitis, hidradenitis suppurativa, iritis, laryngitis, mastitis, myocarditis, nephritis, otitis, pancreatitis, parotitis, pericarditis, peritonitis, pharyngitis, pleuritis, pneumonitis, prostatitis, pyelonephritis, and stomatisi, transplant rejection (involving organs such as kidney, liver, heart, lung, pancreas (*e.g*., islet cells), bone marrow, cornea, small bowel, skin allografts, skin homografts, and heart valve xenografts, serum sickness, and graft vs host disease), acute pancreatitis, chronic pancreatitis, acute respiratory distress syndrome. Sezary's syndrome, congenital adrenal hyperplasis, nonsuppurative thyroiditis, hypercalcemia associated with cancer, pemphigus, bullous dermatitis herpetiformis, severe erythema multiforme, exfoliative dermatitis, seborrheic dermatitis, seasonal or perennial allergic rhinitis, bronchial asthma, contact dermatitis, atopic dermatitis, drug hypersensitivity reactions, allergic conjunctivitis, keratitis, herpes zoster ophthalmicus, iritis and oiridocyclitis, chorioretinitis, optic neuritis, symptomatic sarcoidosis, fulminating or disseminated pulmonary tuberculosis chemotherapy, idiopathic thrombocytopenic purpura in adults, secondary thrombocytopenia in adults, acquired (autoimmune) haemolytic anemia, leukemia and lymphomas in adults, acute leukemia of childhood, regional enteritis, autoimmune vasculitis, multiple sclerosis, chronic obstructive pulmonary disease, solid organ transplant rejection, sepsis. Preferred treatments include treatment of transplant rejection, rheumatoid arthritis, psoriatic arthritis, multiple sclerosis. Type 1 diabetes, asthma, inflammatory bowel disease, systemic lupus erythematosis, psoriasis, chronic pulmonary disease, and inflammation accompanying infectious conditions (*e.g*., sepsis).

In some embodiments, the compounds of the present disclosure and pharmaceutically acceptable salts thereof may also be used in combination with one or more other agents in the prevention or treatment of an allergic inflammatory autoimmune disease, wherein such other agents can include: antigen immunotherapy agents; anti-histamines; steroids; NSAIDs; bronchodilators (*e.g.* beta 2 agonists, adrenergic agonists, anticholinergic agents, theophylline); methotrexate; leukotriene modulators; monoclonal antibody agents such as anti-lgE, anti-TNF, anti-IL-5, anti-IL-6, anti-IL-12, anti-IL-1 and similar agents; receptor therapies agents such as entanercept; and antigen non-specific immunotherapeutic agents such interferon or other cytokines/chemokines, cytokine/chemokine receptor modulators, cytokine agonists or antagonists, and TLR antagonist.

In some aspects, the present disclosure provides a pharmaceutical composition comprising a compound of Formula (I), or a pharmaceutically acceptable salt, hydrate, solvate, prodrug, stereoisomer, labeled isotope, or tautomer thereof and a pharmaceutically acceptable carrier. The pharmaceutical acceptable carrier may further include an excipient, diluent, or surfactant.

In some aspects, the present disclosure provides a method of treating a disease or disorder associated with modulation of cGAS including, cancer or cell proliferative disorder, comprising administering to a patient suffering from at least one of said diseases or disorder a compound of Formula (I), or a pharmaceutically acceptable salt, hydrate, solvate, prodrug, stereoisomer, labeled isotope, or tautomer thereof.

In some embodiments, the therapeutic use of the compounds or compositions of the present disclosure which inhibit cGAS is to provide treatment to patients or subjects suffering from a cancer or cell proliferative disorder.

The disclosed compounds of the present disclosure can be administered in effective amounts to treat or prevent a disorder and/or prevent the development thereof in subjects.

Administration of the disclosed compounds can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the disclosed compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, and all using forms well known to those skilled in the pharmaceutical arts.

Illustrative pharmaceutical compositions are tablets and gelatin capsules comprising a compound of the present disclosure and a pharmaceutically acceptable carrier, such as a) a diluent, *e.g.,* purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, algic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, or PEG200.

Liquid, particularly injectable, compositions can, for example, be prepared by dissolution, dispersion, etc. For example, the disclosed compound is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the disclosed compounds.

The disclosed compounds can be also formulated as a suppository that can be prepared from fatty emulsions or suspensions; using polyalkylene glycols such as propylene glycol, as the carrier.

The disclosed compounds can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in U.S. Pat. No. 5,262,564 which is hereby incorporated by reference in its entirety.

Disclosed compounds can also be delivered by the use of monoclonal antibodies as individual carriers to which the disclosed compounds are coupled. The disclosed compounds can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the disclosed compounds can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels. In one embodiment, disclosed compounds are not covalently bound to a polymer, *e.g*., a polycarboxylic acid polymer, or a polyacrylate.

Parenteral injectable administration is generally used for subcutaneous, intramuscular or intravenous injections and infusions. Injectables can be prepared in conventional forms, either as liquid solutions or suspensions or solid forms suitable for dissolving in liquid prior to injection.

In some aspects, the present disclosure provides a pharmaceutical composition comprising a compound of Formula (I), or a pharmaceutically acceptable salt, hydrate, solvate, prodrug, stereoisomer, labeled isotope, or tautomer thereof and a pharmaceutically acceptable carrier. The pharmaceutical acceptable carrier may further include an excipient, diluent, or surfactant. In some embodiments, the pharmaceutical composition can further comprise an additional pharmaceutically active agent.

Compositions can be prepared according to conventional mixing, granulating or coating methods, respectively, and the present pharmaceutical compositions can contain from about 0.1% to about 99%, from about 5% to about 90%, or from about 1% to about 20% of the disclosed compound by weight or volume.

The dosage regimen utilizing the disclosed compound is selected in accordance with a variety of factors including type, species, age, weight, sex and medical condition of the patient; the severity of the condition to be treated; the route of administration; the renal or hepatic function of the patient; and the particular disclosed compound employed. A physician or veterinarian of ordinary skill in the art can readily determine and prescribe the effective amount of the drug required to prevent, counter or arrest the progress of the condition.

Effective dosage amounts of the disclosed compounds, when used for the indicated effects, range from about 0.5 mg to about 5000 mg of the disclosed compound as needed to treat the condition. Compositions for in vivo or in vitro use can contain about 0.5, 5, 20, 50, 75, 100, 150, 250, 500, 750, 1000, 1250, 2500, 3500, or 5000 mg of the disclosed compound, or, in a range of from one amount to another amount in the list of doses. In one embodiment, the compositions are in the form of a tablet that can be scored.

### Exemplary Embodiments

Embodiment 1. In some aspects, the present disclosure provides, a compound of Formula (I): 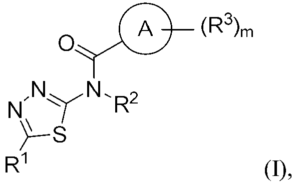 or a pharmaceutically acceptable salt, hydrate, solvate, prodrug, stereoisomer, and tautomer thereof, wherein:
Ring A is a heteroaryl or heterocyclyl;
R¹ is H, -SR⁴, -S(O)R⁴, -S(O)₂R⁴, -N(R⁴)₂, -CN, C₁-C₆ alkyl, aryl, or heteroaryl, wherein the alkyl, aryl or heteroaryl is optionally substituted with one or more R⁵, provided R¹ is not pyridinyl when A is 1-isoquinolinyl;
R² is H or C₁-C₆ alkyl;
each R³ is independently halogen, -CN, OR⁸, -NH₂, NH(R⁶), -N(R⁶)(R⁷), -NHC(O)R⁸, -CO(OR⁸), - C(O)R⁸, -C(O)N(R⁸)₂, C₁-C₆ alkyl, C₃-C₈ cycloalkyl, heteroaryl or heterocyclyl; wherein the
alkyl, cycloalkyl, heterocyclyl or heteroaryl is optionally substituted with one or more R⁶; or two R³, together with the intervening atoms, form a C₃-C₈ cycloalkyl, aryl, heterocyclyl, or heteroaryl, wherein the cycloalkyl, aryl, heterocyclyl, or heteroaryl is optionally substituted with one or more R⁷; or
two geminal R³, together with the carbon atom to which they attached, form an oxo;
R⁴ is H, C₁-C₆ alkyl, C₁-C₆ alkoxy, C₂-C₆ alkenyl, C₂-C₆ alkynyl, C₃-C₃ cycloalkyl, heterocyclyl, heteroaryl, or aryl;
each R⁵ is halogen, -C(O)OH, C₁-C₆ alkyl, C₁-C₆ alkoxy, C₂-C₆ alkenyl, C₂-C₆ alkynyl, wherein the alkyl, alkoxy, alkenyl, or alkynyl is optionally substituted with halogen, OH, NH₂,
each R⁶ is independently halogen, OH, oxo, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, OH, NH₂, -NHC(O)OR⁸, -(CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, heteroaryl, heterocyclyl, or alkylaryl; or
two R⁶, together with the intervening atoms, form a C₃-C₈ cycloalkyl, aryl, heterocyclyl, or heteroaryl; or
two R⁶, together with the carbon atom to which they are attached, form an oxo;
each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, heterocyclyl, heteroaryl, or aryl, or
two R⁷, together with the intervening atoms, form a C₃-C₈ cycloalkyl, aryl, heterocyclyl, or heteroaryl, or
two R⁷, together with carbon atom to which they are attached, form oxo;
R³ is H, -OH, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, or heteroaryl is optionally substituted with one or more R⁹;
R⁹ is -(CH₂)ₙ-NHC(O)-(CH₂)ₚ-OR¹⁰, CN, C₁-C₆ alkyl, C₁-C₆ alkoxy, C₂-C₆ alkenyl, C₂-C₆ alkynyl, C₃-C₃ cycloalkyl, heterocyclyl, heteroaryl, or aryl, wherein the alkyl, alkoxy, alkenyl, alkynyl, cycloalkyl, aryl, heterocyclyl, or heteroaryl is optionally substituted with halogen, OH, NH₂, - NHC(O)OR¹⁰, -(CH₂)ₙ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, -(CH₂)ₙ-C(O)OR¹⁰, C₁-C₆ alkyl, C₃-C₈ cycloalkyl, heteroaryl, heterocyclyl, or alkylaryl;
R¹⁰ is C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl, wherein the alkyl, alkoxy, alkenyl, alkynyl, cycloalkyl, heterocyclyl, heteroaryl, alkylaryl, or alkyl heteroaryl is optionally substituted with one or more halogen, OH, -CO(OH), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, heterocyclyl, heteroaryl, or aryl;
m is an integer from 0 to 4;
n is an integer from 0 to 6; and
p is an integer from 0 to 6.

Embodiment 2. In some aspects, the present disclosure provides the compound of Embodiment 1, wherein the compound is of Formula **(I-a):** 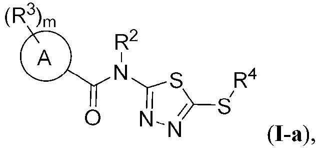 or a pharmaceutically acceptable salt, isomer, solvate, prodrug, or tautomer thereof.

Embodiment 3. In some aspects, the present disclosure provides the compound of Embodiment 1, wherein Formula **(I-a)** is Formula **(I-a-1)** 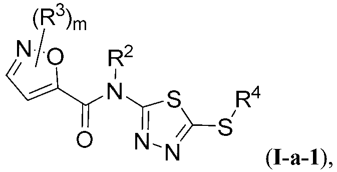 or a pharmaceutically acceptable salt, isomer, solvate, prodrug, or tautomer thereof.

Embodiment 4. In some aspects, the present disclosure provides the compound of Embodiment 1, wherein Formula **(I-a)** is Formula **(I-a-1-i)** 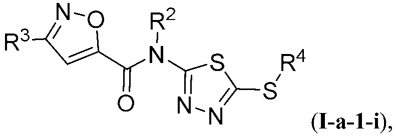 or a pharmaceutically acceptable salt, isomer, solvate, prodrug, or tautomer thereof.

Embodiment 5. In some aspects, the present disclosure provides the compound of Embodiment 1, wherein Formula **(I-a)** is Formula **(I-a-1-ii)** 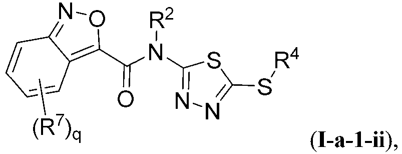 or a pharmaceutically acceptable salt, isomer, solvate, prodrug, or tautomer thereof.

Embodiment 6. In some aspects, the present disclosure provides the compound of Embodiment 1, wherein Formula **(I-a)** is Formula **(I-a-2)** 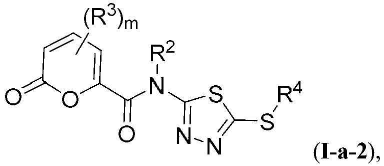 or a pharmaceutically acceptable salt, isomer, solvate, prodrug, or tautomer thereof.

Embodiment 7. In some aspects, the present disclosure provides the compound of Embodiment 1, wherein Formula **(I-a)** is Formula **(I-a-2-i)**: 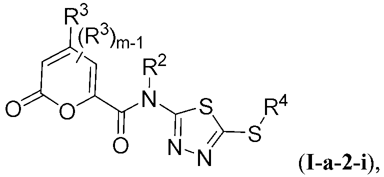 or a pharmaceutically acceptable salt, isomer, solvate, prodrug, or tautomer thereof.

Embodiment 8. In some aspects, the present disclosure provides the compound of Embodiment 1, wherein Formula **(I-a)** is Formula **(I-a-2-ii)** 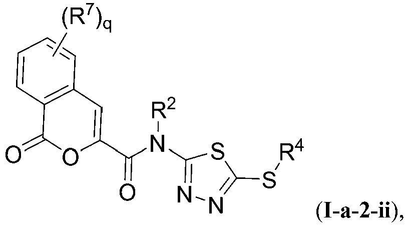 or a pharmaceutically acceptable salt, isomer, solvate, prodrug, or tautomer thereof.

Embodiment 9. In some aspects, the present disclosure provides the compound of Embodiment 1, wherein Formula **(I-a)** is Formula **(I-a-5)** to Formula **(I-a-13):** 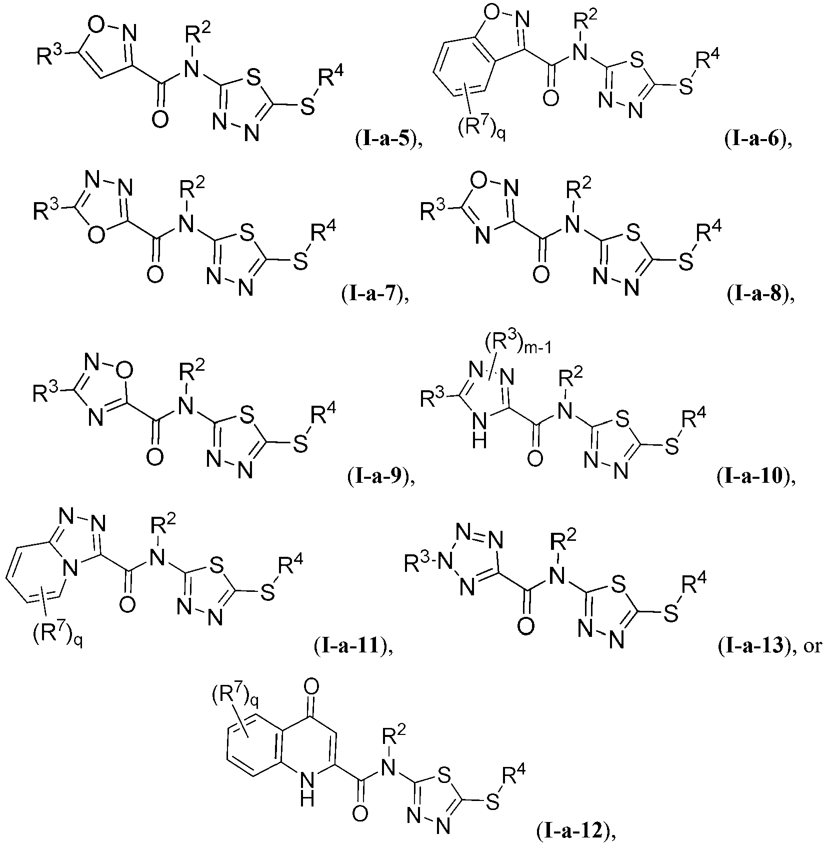 or a pharmaceutically acceptable salt, isomer, solvate, prodrug, or tautomer thereof.

Embodiment 10. In some aspects, the present disclosure provides the compound of Embodiment 1, wherein R¹ is hydrogen, -SCH₃, -SCH₂CH₃, -CH₃, -CHF₂, -CF₃, -CH₂CH₃, - (CH₂)₂CH₃, -(CH₂)₄CH₃, -CN, -CH₂CH₂OCH₃, -NH₂, -NH(CH₃), -N(CH₃)₂, -CH(CH₂CH₃), - CH(C₆H₆)(CH₂CH₃), 3-chlorophenyl, 3-fluorophenyl, 3-methoxyphenyl, 2-fluoropyridinyl.

Embodiment 11. In some aspects, the present disclosure provides the compound of Embodiment 11, wherein R¹ is -SCH₃.

Embodiment 12. In some aspects, the present disclosure provides the compound of any one of the preceding Embodiments, wherein R₂ is H.

Embodiment 13. In some aspects, the present disclosure provides the compound of any one of the preceding Embodiments, wherein R₂ is methyl.

Embodiment 14. In some aspects, the present disclosure provides the compound of any one of the preceding Embodiments, wherein A is 5- or 6-membered heterocyclyl or heteroaryl.

Embodiment 15. In some aspects, the present disclosure provides the compound of any one of the preceding Embodiments, wherein A is 6-membered heterocyclyl.

Embodiment 16. In some aspects, the present disclosure provides the compound of any one of the preceding Embodiments, wherein A is

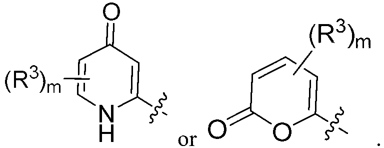

Embodiment 17. In some aspects, the present disclosure provides the compound of any one of the preceding Embodiments, wherein A is 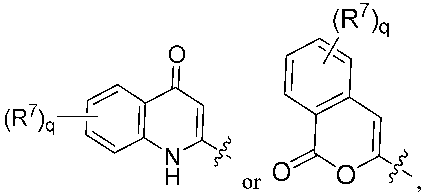 wherein q is an integer selected from 0 to 4.

Embodiment 18. In some aspects, the present disclosure provides the compound of any one of the preceding Embodiments, wherein A is a 5-membered heteroaryl.

Embodiment 19. In some aspects, the present disclosure provides the compound of any one of the preceding Embodiments, wherein A is 5-membered heteroaryl comprising at least two heteroatoms independently selected from N, O, and S.

Embodiment 20. In some aspects, the present disclosure provides the compound of Embodiment 19, wherein A is 5-membered heteroaryl comprising two heteroatoms independently selected from N, O, and S.

Embodiment 21. In some aspects, the present disclosure provides the compound of Embodiment 20, wherein A is

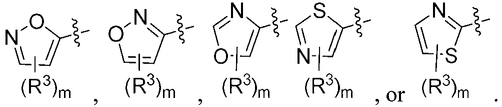

Embodiment 22. In some aspects, the present disclosure provides the compound of Embodiment 20, wherein A is 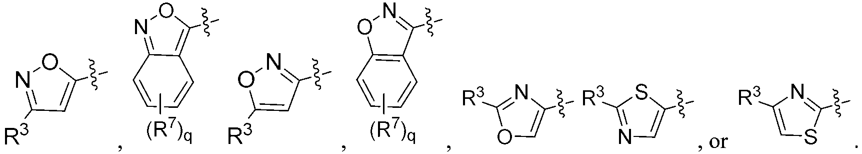 wherein q is an integer selected from 0 to 4.

Embodiment 23. In some aspects, the present disclosure provides the compound of Embodiment 19, wherein A is 5-membered heteroaryl comprising three heteroatoms independently selected from N, O, and S.

Embodiment 24. In some aspects, the present disclosure provides the compound of Embodiment 23, wherein A is

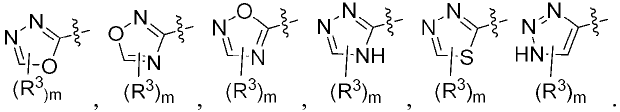

Embodiment 25. In some aspects, the present disclosure provides the compound of Embodiment 24, wherein A is 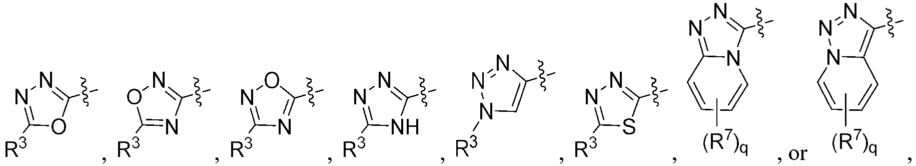 wherein q is an integer selected from 0 to 4.

Embodiment 26. In some aspects, the present disclosure provides the compound of Embodiment 19, wherein A is 5-membered heteroaryl comprising three heteroatoms independently selected from N and O.

Embodiment 27. In some aspects, the present disclosure provides the compound of Embodiment 26, wherein A is

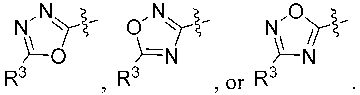

Embodiment 28. In some aspects, the present disclosure provides the compound of Embodiment 18, wherein A is 5- to 6-membered heteroaryl comprising one or four heteroatoms independently selected from N, O, and S.

Embodiment 29. In some aspects, the present disclosure provides the compound of Embodiment 28, wherein A is

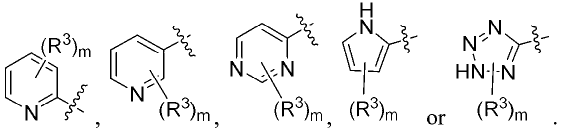

Embodiment 30. In some aspects, the present disclosure provides the compound of any one of the preceding Embodiments, wherein each R³ is independently halogen, -CN, OR⁸, -NH₂, -NH(R⁶), - N(R⁶)(R⁷), -NHC(O)R⁸, -CO(OR⁸), -C(O)R⁸, -C(O)N(R⁸)₂, C₁-C₆ alkyl, C₃-C₈ cycloalkyl, heteroaryl or heterocyclyl; wherein the alkyl, cycloalkyl, heterocyclyl or heteroaryl is optionally substituted with one or more R⁶.

Embodiment 31. In some aspects, the present disclosure provides the compound of Embodiment 30, wherein two R³, together with the intervening atoms, form a C₃-C₈ cycloalkyl, aryl, heterocyclyl, or heteroaryl, wherein the cycloalkyl, aryl, heterocyclyl, or heteroaryl is optionally substituted with one or more R⁷.

Embodiment 32. In some aspects, the present disclosure provides the compound of Embodiment 30, wherein two geminal R³, together with the carbon atom to which they attached, form an oxo.

Embodiment 33. In some aspects, the present disclosure provides the compound of Embodiment 31, wherein two R³, together with the intervening atoms, forms 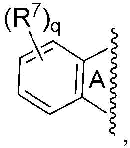 wherein q is an integer selected from 0 to 4.

Embodiment 34. In some aspects, the present disclosure provides the compound of any one of the preceding Embodiments, wherein R⁴ is C₁-C₆ alkyl.

Embodiment 35. In some aspects, the present disclosure provides the compound of any one of the preceding Embodiments, wherein R⁴ is methyl or ethyl.

Embodiment 36. In some aspects, the present disclosure provides a compound selected from

Table 1 or a pharmaceutically acceptable salt thereof.

Embodiment 37. In some aspects, the present disclosure provides the compound of any of the preceding Embodiments, or a pharmaceutically acceptable salt or stereoisomer thereof.

Embodiment 38. In some aspects, the present disclosure provides the compound of any of the preceding Embodiments, or a pharmaceutically acceptable salt thereof.

Embodiment 39. In some aspects, the present disclosure provides an isotopic derivative of the compound of any one of the preceding Embodiments.

Embodiment 40. In some aspects, the present disclosure provides a pharmaceutical composition comprising the compound of any one of the preceding Embodiments and one or more pharmaceutically acceptable carriers.

Embodiment 41. In some aspects, the present disclosure provides a method of treating or preventing an cGAS-related disease or disorder, the method comprising administering to the subject at least one therapeutically effective amount of the compound of any one of the preceding Embodiments.

Embodiment 42. In some aspects, the present disclosure provides a method of inhibiting cGAS, the method comprising administering to the subject at least one therapeutically effective amount of the compound of any one of the preceding Embodiments.

Embodiment 43. In some aspects, the present disclosure provides the compound of any one of the preceding Embodiments for use in treating or preventing an cGAS-related disease or disorder.

Embodiment 44. In some aspects, the present disclosure provides use of the compound of any one of the preceding Embodiments, in the manufacture of a medicament, for treating or preventing and cGAS-related disease or disorder.

Embodiment 45. In some aspects, the present disclosure provides the method, compound, or use of any one of the preceding Embodiments, wherein the subject is a human.

Embodiment 46. In some aspects, the present disclosure provides the method, compound, or use of any one of the preceding Embodiments, wherein the cGAS-related disease or disorder is inflammation, an auto-immune disease, a cancer, an infection, a disease or disorder of the central nervous system, a metabolic disease, a cardiovascular disease, a respiratory disease, a kidney disease, a liver disease, an ocular disease, a skin disease, a lymphatic disease, a rheumatic disease, a psychological disease, graft versus host disease, allodynia, or an cGAS-related disease in a subject that has been determined to carry a germline or somatic non-silent mutation in cGAS.

Embodiment 47. In some aspects, the present disclosure provides the method, compound, or use of any one of the preceding Embodiments, wherein the disease or disorder of the central nervous system is Parkinson's disease, Alzheimer's disease, traumatic brain injury, spinal cord injury, amyotrophic lateral sclerosis, or multiple sclerosis.

Embodiment 48. In some aspects, the present disclosure provides the method, compound, or use of any one of the preceding Embodiments, wherein the kidney disease is an acute kidney disease, a chronic kidney disease, or a rare kidney disease.

Embodiment 49. In some aspects, the present disclosure provides the method, compound, or use of any one of the preceding Embodiments, wherein the skin disease is psoriasis, hidradenitis suppurativa (HS), or atopic dermatitis.

Embodiment 50. In some aspects, the present disclosure provides the method, compound, or use of any one of the preceding claims, wherein the rheumatic disease is dermatomyositis, Still's disease, or juvenile idiopathic arthritis.

Embodiment 51. In some aspects, the present disclosure provides the method, compound, or use of any one of the preceding Embodiments, wherein the cGAS-related disease in a subject that has been determined to carry a germline or somatic non-silent mutation in cGAS is cryopyrin-associated autoinflammatory syndrome.

Embodiment 52. In some aspects, the present disclosure provides the method, compound, or use of any one of the preceding Embodiments, wherein the cryopyrin-associated auto inflammatory syndrome is familial cold autoinflammatory syndrome, Muckle-Wells syndrome, or neonatal onset multisystem inflammatory disease.

### EXAMPLES

The disclosure is further illustrated by the following examples and synthesis schemes, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

Nuclear magnetic resonance (NMR) spectra were recorded at 400 MHz as stated and at 300.3 K unless otherwise stated; the chemical shifts (δ) are reported in parts per million (ppm). Spectra were recorded using a Bruker Avance 400instrument with 8, 16 or 32 scans. DMSO-*d*₆ = deuterated dimethylsulfoxide. Methanol-*d*₄ = deuterated methanol. CHCl₃-*d* = deuterated chloroform.

LC-MS chromatograms and spectra were recorded using a Shimadzu LCMS-2020. Injection volumes were 0.7 - 8.0 µl and the flow rates were typically 0.8 or 1.2 ml/min. Detection methods were diode array (DAD) or evaporative light scattering (ELSD) as well as positive ion electrospray ionization. MS range was 100 - 1000 Da. Solvents were gradients of water and acetonitrile both containing a modifier (typically 0.01 - 0.04 %) such as trifluoroacetic acid (TFA) or ammonium carbonate.

The Examples set forth herein below provide syntheses and experimental results obtained for certain exemplary compounds. Unless otherwise indicated, all numbers expressing quantities of ingredients, reaction conditions, concentrations, properties, stabilities, and so forth used in the specification and claims are to be understood as being modified in all instances by the term "about." At the very least, each numerical parameter should at least be construed in light of the number of reported significant digits and by applying ordinary rounding techniques. Accordingly, unless indicated to the contrary, the numerical parameters set forth in the present specification and attached claims are approximations that may vary depending upon the properties sought to be obtained. Notwithstanding that the numerical ranges and parameters setting forth the broad scope of the embodiments are approximations, the numerical values set forth in the specific examples are reported as precisely as possible. Any numerical value, however, inherently contain certain errors resulting from variations in experiments, testing measurements, statistical analyses and such. The following is to be construed as merely illustrative, and not limitations of the preceding disclosure in any way whatsoever. Those skilled in the art will promptly recognize appropriate variations from the procedures both as to reactants and as to reaction and purification conditions and techniques. Unless otherwise indicated, starting materials or intermediates are commercially available or are known in the chemical literature.

Purification/separation methods include Preparative High-performance liquid chromatography (Prep HPLC).

### Example 1: Methyl 5-((5-(2-chlorophenyl)-1,3,4-thiadiazol-2-yl)carbamoyl)isoxazole-3-carboxylate (Compound 1)

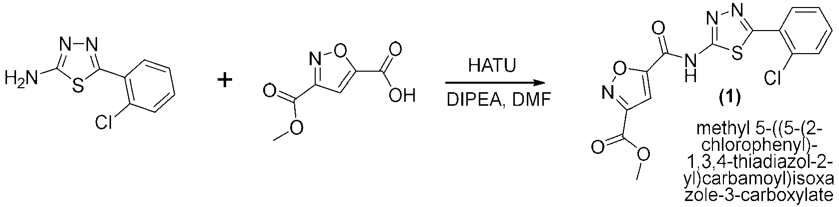

To a solution of 3-methoxycarbonylisoxazole-5-carboxylic acid (1.00 eq, 100 mg, 0.584 mmol) and 1-[bis(dimethylamino)methylene]-1H-1,2,3-triazolo[4,5-b]pyridinium 3- oxid hexafluorophosphate (HATU) (1.20 eq, 267 mg, 0.701 mmol) in N,N-dimethylformamide (DMF) (1 mL) was added diisopropylethylamine (DIPEA) (1.50 eq, 0.15 mL, 0.877 mmol) and the mixture was stirred at rt for 5 min before addition of 5-(2-chlorophenyl)-1,3,4-thiadiazol-2-amine (1.20 eq, 148 mg, 0.701 mmol) and the mixture stirred at room temperature (rt). After 16 h, water was added (5 mL) and solids collected, washed with water (3 x 3 mL), then with diethyl ether (Et₂O) (3 x 3 mL), then with ethanol (EtOH) (3 x 3 mL) and dried to provide methyl 5-((5-(2-chlorophenyl)-1,3,4-thiadiazol-2-yl)carbamoyl)isoxazole-3-carboxylate (98 mg, 43 % yield) as a cream solid. LCMS (ES, m/z) = 365.28 [M+1]+. ¹H NMR (400 MHz, DMSO-*d*₆) δ 14.09 (s, 1H), 8.15 (dd,J = 7.6, 2.0Hz, 1H), 7.86 (s, 1H), 7.71 (d,J = 7.8Hz, 1H), 7.65 - 7.41 (m, 2H).

### Example 2: 5-((5-(2-chlorophenyl)-1,3,4-thiadiazol-2-yl)carbamoyl)isoxazole-3-carboxylic acid (Compound 2)

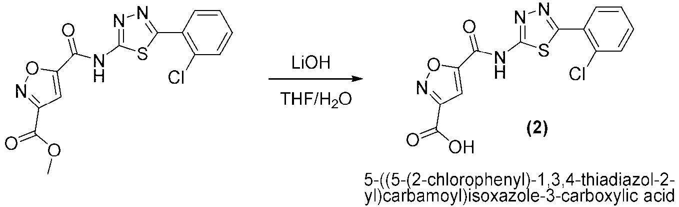

Methyl 5-[[5-(2-chlorophenyl)-1,3,4-thiadiazol-2-yl]carbamoyl]isoxazole-3-carboxylate (1.00 eq, 90 mg, 0.247 mmol) was suspended in a mixture of tetrahydrofuran (THF) (4 mL) and H₂O (2 mL). LiOH-monohydrate (2.50 eq, 26 mg, 0.617 mmol) added and stirred at rt. After 3 h, LCMS shows complete hydrolysis. 6N HCl added until pH ~1 and resulting solids collected by filtration, washed with H₂O (3 x 3 mL) and dried to provide 5-[[5-(2-chlorophenyl)-1,3,4-thiadiazol-2-yl]carbamoyl]isoxazole-3-carboxylic acid (62 mg, 71 % yield) as a light yellow solid. LCMS (ES, m/z) = 351.14 [M+1]+. ¹H NMR (400 MHz, DMSO-*d*₆) δ 14.76 - 13.58 (m, 2H), 8.16 (dd,J = 7.6, 2.0Hz, 1H), 7.82 (s, 1H), 7.72 (dd,J = 7.9, 1.5Hz, 1H), 7.58 (dtd,J = 17.9, 7.4, 1.7Hz, 2H).

### Example 3: N5-(5-(2-chlorophenyl)-1,3,4-thiadiazol-2-yl)-N3-methoxyisoxazole-3,5-dicarboxamide (Compound 91)

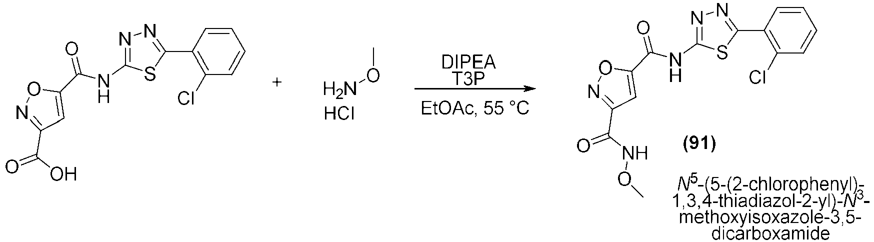

5-[[5-(2-Chlorophenyl)-1,3,4-thiadiazol-2-yl]carbamoyl]isoxazole-3-carboxylic acid (1.0 eq, 30 mg, 0.086 mmol) and O-methylhydroxylamine hydrochloride (1.2 eq, 8.8 mg, 0.10 mmol) were combined in ethyl acetate (EtOAc) (0.5 mL). Propylphosphonic acid (T3P) (50% in EtOAc) (2.00 eq, 0.10 mL, 0.171 mmol) was added followed by diisopropylethylamine (DIPEA) (3.00 eq, 0.045 mL, 0.257 mmol) and the mixture placed in a 55 °C heating block. After 90 min, LCMS shows high conversion. Water (5 mL) was added and the solids were collected, washed with water (2 x 3 mL), then with diethyl ether (Et₂O) (3 x 2 mL) then with ethanol (EtOH) (3 x 2 mL) and dried to provide N5-[5-(2-chlorophenyl)-1,3,4-thiadiazol-2-yl]-N3-methoxy-isoxazole-3,5-dicarboxamide (11 mg, 32% yield) as a tan solid. LCMS (ES, m/z) = 380.22 [M+1]+. ¹H NMR (400 MHz, DMSO-*d*₆) δ 14.01 (s, 1H), 12.42 (s, 1H), 8.15 (dd,J = 7.6, 2.0Hz, 1H), 7.78 (s, 1H), 7.72 (dd,J = 7.8, 1.5Hz, 1H), 7.63 - 7.50 (m, 2H), 3.74 (s, 3H).

### Example 4: N-(5-(2-chlorophenyl)-1,3,4-thiadiazol-2-yl)isoxazolo[4,3-h]quinoline-3-carboxamide (Compound 7)

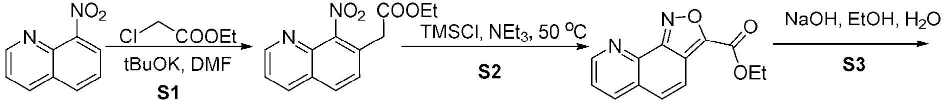

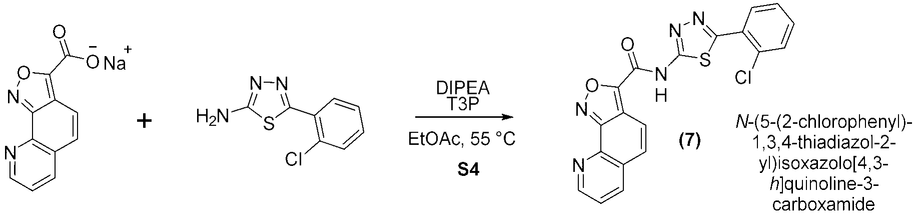

**Step-1:** Into a solution of potassium tert-butoxide (KOtBu) in tetrahydrofuran (THF) (1 M) (4.6 L, 46 mol, 3 equiv) and N,N-dimethylformamide (DMF) (5 L) was added 8-nitroquinoline (250.00 g, 1.435 mol, 1.00 equiv). Ethyl chloroacetate (211.10 g, 1.722 mol, 1.20 equiv) in DMF (5 L) was added dropwise into above solution at 0 °C. The mixture was stirred at 0 °C for 1h. The reaction was quenched with sat. ammonium chloride (2 L) at 0 °C and diluted with ethyl acetate (EtOAc) (1L). The resulting solution was extracted with ethyl acetate (5 x 500 mL) and the organic layers were combined. The solution was washed with saturated sodium chloride (3 x 500 mL). The organic layer was dried over anhydrous sodium sulfate and concentrated under vacuum. The solid was stirred over 1500 ml (ethyl acetate and petroleum ether 1:9). The solution was filtered and the filter cake was recrystallized from ethyl acetate/petroleum ether/dichloromethane (4:4:1) twice to afford ethyl 2-(8-nitroquinolin-7-yl)acetate (130 g, 34 % yield) as an off-white solid. LCMS (ES, m/z) = 261 [M+1]+.

**Step-2:** To a stirred solution of ethyl 2-(8-nitroquinolin-7-yl)acetate (100 g, 384.247 mmol, 1.00 equiv) in triethylamine (1000 mL) was added chlorotrimethylsilane (1000 mL) dropwise 0 °C under nitrogen. The mixture was then stirred at 50 °C for 3 days under nitrogen. The resulting mixture were concentrated under vacuum to afford crude product ethyl [1,2]oxazolo[4,3-h]quinoline-3-carboxylate (369 g) as an off-white solid. LCMS (ES, m/z) = 243 [M+1]+.

**Step-3:** To a stirred solution of ethyl [1,2]oxazolo[4,3-h]quinoline-3-carboxylate (240 g, 991 mmol, 1.00 equiv, crude product, including NEt₃-HCl salts) in ethanol (2.4 L) and water (1.2 L) was added sodium hydroxide (60 g, 1500 mmol, 1.51 equiv) at room temperature. The mixture was stirred at rt for 30 min. The resulting mixture was filtered and the filter cake was washed with ethanol (3 x 200 mL) to afford the product as sodium salt, [1,2]oxazolo[4,3-h]quinoline-3-carboxylate (105.8 g, 44.76%, from 4 batches) as an off-white solid. LCMS (ES, m/z) = 215 [M+1]+. ¹H NMR (400 MHz, DMSO-*d*₆) δ 8.90 (d, *J =* 4.0 Hz, 1H), 8.35 (d, *J =* 8.0 Hz, 1H), 7.94 (d, *J =* 8.8 Hz, 1H), 7.76-7.73 (m, 1H), 7.43 (d, *J* = 9.2 Hz, 1H).

**Step-4:** Sodium isoxazolo[4,3-h]quinoline-3-carboxylate (1.00 eq, 80 mg, 0.339 mmol) and 5-(2-chlorophenyl)-1,3,4-thiadiazol-2-amine (1.21 eq, 87 mg, 0.410 mmol) were combined in ethyl acetate (EtOAc) (0.5 mL). Propylphosphonic acid (T3P) (50% solution in EtOAc) (2.00 eq, 0.40 mL, 0.678 mmol) was added followed by diisopropylethylamine (3.00 eq, 0.18 mL, 1.02 mmol) and the mixture placed in a 55 °C heating block. After 5 h, LCMS shows high conversion. Water (5 mL) was added and the solids were collected, washed with water (2 x 3 mL) then with diethyl ether (Et₂O) (3 x 2 mL) then with ethanol (EtOH) (3 x 2 mL) and dried to provide *N*-[5-(2-chlorophenyl)-1,3,4-thiadiazol-2-yl]isoxazolo[4,3-h]quinoline-3-carboxamide (88 mg, 62 % yield) as a yellow solid. LCMS (ES, m/z) = 408.24 [M+1]+. ¹H NMR (400 MHz, DMSO-*d*₆) δ 14.55 (s, 1H), 8.98 (d, J = 4.5Hz, 1H), 8.44 (dd, J = 8.2, 1.7Hz, 1H), 8.20 - 7.99 (m, 2H), 7.83 (dd, J = 8.1, 4.5Hz, 1H), 7.76 - 7.67 (m, 2H), 7.65 - 7.51 (m, 2H).

### Example 5: N-(5-methyl-1,3,4-thiadiazol-2-yl)isoxazolo[4,3-h]quinoline-3-carboxamide (Compound 93)

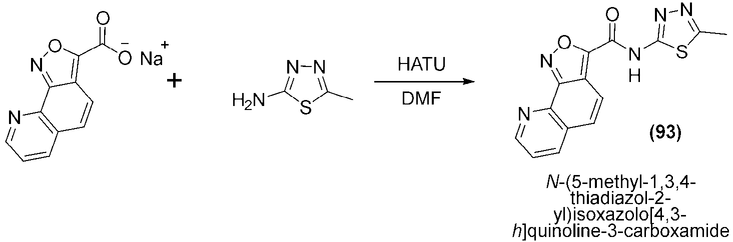

Sodium isoxazolo[4,3-h]quinoline-3-carboxylate (1.00 eq, 11.00 g, 46.6 mmol) was suspended in N,N-dimethylformamide (DMF) (200mL). 5-Methyl-1,3,4-thiadiazol-2-amine (1.20 eq, 6.42 g, 55.8 mmol) was added followed by addition of 1-[bis(dimethylamino)methylene]-1H-1,2,3-triazolo[4,5-b]pyridinium 3- oxid hexafluorophosphate (HATU) (1.20 eq, 21.27 g, 55.9 mmol) and the reaction mixture was stirred at rt for 5 days. Mixture never becomes homogeneous. LCMS shows near complete conversion. Water (~200 mL) was added and stirred 1 h at rt and solids collected by filtration and washed with water (3 x 100 mL). The solids were air dried at rt overnight then vacuum line dried overnight. Solids were converted to a fine powder using a mortar and pestle and dried again for 24 h to provide *N*-(5-methyl-1,3,4-thiadiazol-2-yl)isoxazolo[4,3-h]quinoline-3-carboxamide (11.00 g, 74 % yield) as a tan powder. LCMS (ES, m/z) = 312.09 [M+1]+. ¹H NMR (400 MHz, DMSO-*d*₆) δ 14.51 (s, 1H), 8.96 (dd,J = 4.5, 1.7Hz, 1H), 8.40 (dd,J = 8.2, 1.7 Hz, 1H), 8.01 (d,J = 9.2Hz, 1H), 7.80 (dd,J = 8.1, 4.5Hz, 1H), 7.67 (d,J = 9.2Hz, 1H), 2.63 (s, 3H).

### Example 6: N-(5-(methylthio)-1,3,4-thiadiazol-2-yl)isoxazolo[4,3-h]quinoline-3-carboxamide (Compound 94)

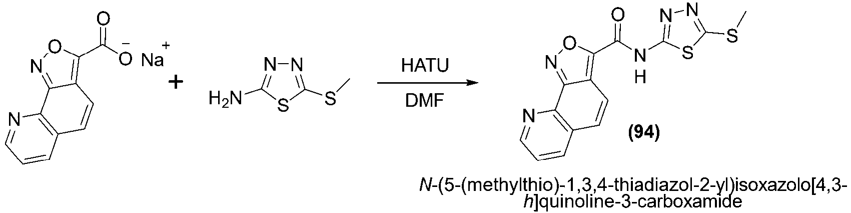

1-[Bis(dimethylamino)methylene]-1H-1,2,3-triazolo[4,5-b]pyridinium 3-oxide hexafluorophosphate (HATU) (1.20 eq, 171 mg, 0.450 mmol) was dissolved in N,N-dimethylformamide (DMF) (1mL) and sodium isoxazolo[4,3-h]quinoline-3-carboxylate (1.00 eq, 90 mg, 0.374 mmol) followed by diisopropylethylamine (DIPEA) (4.02 eq, 0.26 mL, 1.50 mmol) were successively added. After stirring for 2 minutes, 5-(methylsulfanyl)-1,3,4-thiadiazol-2-amine (1.21 eq, 70 mg, 0.452 mmol) was added and the reaction was stirred for 90 minutes at rt. Water was slowly added and a solid crashed out. It was stirred as such for 30 minutes. The solution was then filtered, solids washed with water and set aside. To the filtrate was added ethyl acetate (EtOAc) and the phases were separated. Both phases contained the final product. Formic acid (FA) was added to the aqueous phase until pH was acidic and the aqueous phase was extracted with EtOAc (2x). The EtOAc extracts were all combined, dried over MgSO₄, filtered and evaporated under reduced pressure. Crude product was purified by C18 reverse phase chromatography (0 - 100% MeCN/0.1% aqueous formic acid) to afford N-(5-methylsulfanyl-1,3,4-thiadiazol-2-yl)isoxazolo[4,3-h]quinoline-3-carboxamide (45 mg, 35% yield) as a beige solid. LCMS (ES, m/z) = 344.10 [M+1]+. ¹H NMR (400 MHz, DMSO-*d*₆) δ 8.98 (dd,J = 4.5, 1.7 Hz, 1H), 8.44 (dd,J = 8.1, 1.6 Hz, 1H), 8.00 (d,J = 9.1 Hz, 1H), 7.83 (dd,J = 8.1, 4.5 Hz, 1H), 7.72 (d,J = 9.2 Hz, 1H), 2.76 (s, 3H) [NH amide signal not observed].

### Example 7: N-[5-(pyrazin-2-yl)-1,3,4-thiadiazol-2-yl]-2,1-benzoxazole-3-carboxamide (Compound 130)

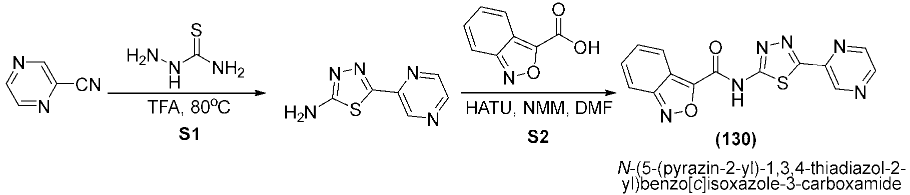

**Step 1:** 5-(Pyrazin-2-yl)-1,3,4-thiadiazol-2-amine was prepared according to the procedure in **Example 36 Step 1** from pyrazine-2-carbonitrile instead of thiazole-4-carbonitrile. LCMS (ES, m/z): 180.0 [M+H]⁺.

**Step 2:** Into a solution of benzo[c]isoxazole-3-carboxylic acid (501 mg, 3.071 mmol, 1.00 equiv) in dimethylformamide (8 mL) was added 1-[bis(dimethylamino)methylene]-1H-1,2,3-triazolo[4,5-b]pyridinium 3- oxid hexafluorophosphate (HATU) (1522 mg, 4.003 mmol, 1.30 equiv) and N-methylmorpholine (NMM) (1080 mg, 10.678 mmol, 3.48 equiv) at room temperature. The resulting mixture was stirred for 30 min at room temperature. To the above mixture was added 5-(pyrazin-2-yl)-1,3,4-thiadiazol-2-amine (550 mg, 3.069 mmol, 1 equiv) at room temperature. The resulting mixture was stirred for additional 2 h at room temperature. The precipitated solids were collected by filtration and washed with acetonitrile (5 x 2 mL). This resulted in N-[5-(pyrazin-2-yl)-1,3,4-thiadiazol-2-yl]-2,1-benzoxazole-3-carboxamide (27 mg, 2.65%) as a white solid. LCMS (ES, m/z): 325.1 [M+H]⁺. ¹H NMR (400 MHz, DMSO-*d*₆) δ 9.44 (s, 1H), 8.83 (s, 2H), 8.12 (d, *J* = 8.8 Hz, 1H), 7.85 (d, *J* = 9.2 Hz, 1H), 7.67 - 7.48 (m, 1H), 7.40 (dd, *J* = 8.8, 6.4 Hz, 1H).

### Example 8: 4-([1,1'-biphenyl]-2-yl)-N-(5-(methylthio)-1,3,4-thiadiazol-2-yl)-2-oxo-2H-pyran-6-carboxamide (Compound 182)

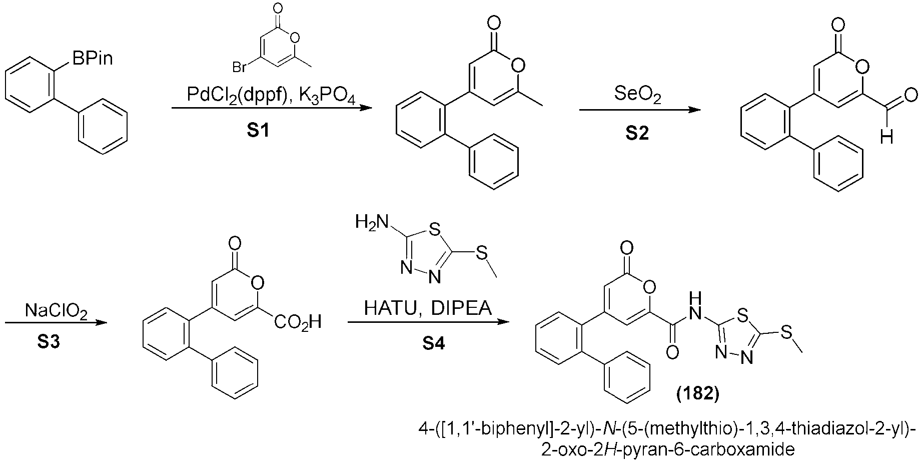

**Step 1:** A flask was loaded with 2-biphenylboronic acid (1.1 eq, 115 mg, 582 umol), 4-bromo-6-methyl-2H-pyran-2-one (1.00 eq, 100 mg, 529 umol), 1,1'-bis(diphenylphosphino)ferrocene dichloropalladium (II) (5 mol%, 19.4 mg, 26.5 umol), potassium phosphate tribasic (3.00 eq, 337 mg, 1.59 mmol), degassed 1,4-dioxane (5.0 mL) and degassed water (1.0 mL) under an argon atmosphere. The reaction mixture was stirred at 80 °C for 5 h. The crude of the reaction mixture was supported on silica gel and the solvent was removed by rotary evaporation. It was then purified by flash chromatography on silica gel (0→30% ethyl acetate (EtOAc) in hexanes) to afford 4-([1,1'-biphenyl]-2-yl)-6-methyl-2H-pyran-2-one (131 mg, 94% yield) as an off-white solid. LCMS (ES, m/z) = 263.1 [M+H]⁺. ¹H NMR (CHCl₃-*d*, 300 MHz): *δ*_{H} 7.51-7.40 (4H, m), 7.38-7.32 (3H, m), 7.26-7.24 (1H, m), 6.14 (1H, s), 5.57 (1H, s), 2.06 (3H, s).

**Step 2:** A suspension of 4-([1,1'-biphenyl]-2-yl)-6-methyl-2*H*-pyran-2-one (1.00 eq, 131 mg, 499 umol) and selenium dioxide (10.00 eq, 554 mg, 4.99 mmol) in anhydrous 1,4-dioxane (5.0 mL) under an argon atmosphere was stirred at 100 °C for 22 h. The reaction mixture was filtered and the filter cake was rinsed with dichloromethane (DCM). The filtrate was concentrated by rotary evaporation to afford a mixture of 4-([1,1'-biphenyl]-2-yl)-2-oxo-2*H*-pyran-6-carbaldehyde (131 mg, 95% yield) and 4-([1,1'-biphenyl]-2-yl)-6-(hydroxymethyl)-2*H*-pyran-2-one as a light-yellow solid, which was used without further purification. LCMS (ES, m/z) = 277.0 [M+H]⁺.

**Step 3:** To a solution of 4-([1,1'-biphenyl]-2-yl)-2-oxo-2*H*-pyran-6-carbaldehyde (1.00 eq, 131 mg, 474 umol) in the mixture of solvents t-butanol (3.35 mL), water (1.67 mL), tetrahydrofuran (THF) (3.35 mL), and 2-methyl-2-butene (1.12 mL) at 0 °C was added sodium phosphate monobasic (6.00 eq, 341 mg, 2.84 mmol) followed by sodium chlorite (3.00 eq, 129 mg, 1.42 mmol) in one portion. The reaction mixture was stirred at rt for 2.5 h. The reaction mixture was diluted with water (25 mL) and it was acidified with 2 M HCl_{(aq)} to pH 1. The aqueous phase was extracted with ethyl acetate (EtOAc) (3 x 15 mL) and the combined organic extracts were dried over MgSO₄ and concentrated to afford crude 4-([1,1'-biphenyl]-2-yl)-2-oxo-2*H*-pyran-6-carboxylic acid (152 mg, 100% yield) as a yellow solid, which was used without further purification. LCMS (ES, m/z) = 291.0 [M-H]⁻.

**Step 4:** To a solution of 4-([1,1'-biphenyl]-2-yl)-2-oxo-2*H*-pyran-6-carboxylic acid (1.00 eq, 50.0 mg, 164 umol), 2-amino-5-(methylthio)-1,3,4-thiadiazole (1.20 eq, 29.6 mg, 197 umol) and *N,N-*diisopropylethylamine (3.00 eq, 86.7 uL, 493 umol) in anhydrous N,N-dimethylformamide (DMF) (1.6 mL) under an argon atmosphere, was added 1-[Bis(dimethylamino)methylene]-1H-1,2,3-triazolo[4,5-b]pyridinium 3- oxid hexafluorophosphate (HATU) (1.20 eq, 74.9 mg, 197 umol) in one portion. The reaction mixture was stirred at r.t. for 15 min. The crude was purified by reversed-phase chromatography (30-70% MeCN in 0.1% aqueous formic acid) to afford the desired product. The material was re-purified by flash chromatography on silica gel (0-50% 0.1% formic acid in MeCN in hexanes) to afford 4-([1,1'-biphenyl]-2-yl)-*N*-(5-(methylthio)-1,3,4-thiadiazol-2-yl)-2-oxo-2*H*-pyran-6-carboxamide (12.6 mg, 18% yield) as a yellow solid. LCMS (ES, m/z) = 422.0 [M+H]⁺. ¹H NMR (DMSO-*d*₆, 300 MHz): *δ*_{H} 2.72 (3H, s), 6.48 (1H, d, *J =* 1.5 Hz), 6.93 (1H, d, *J =* 1.5 Hz), 7.42-7.30 (5H, m), 7.66-7.51 (4H, m).

### Example 9: 5-((3,4-rac-trans-dihydroxypiperidin-1-yl)methyl)-N-(5-(methylthio)-1,3,4-thiadiazol-2-yl)benzo[c]isoxazole-3-carboxamide (Compound 183)

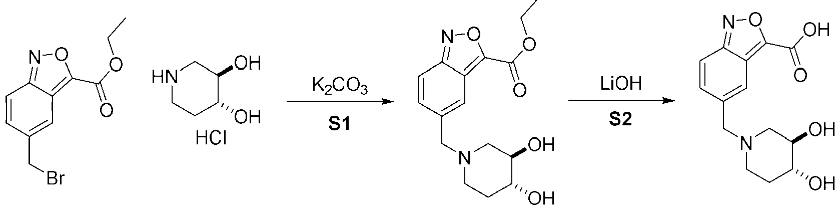

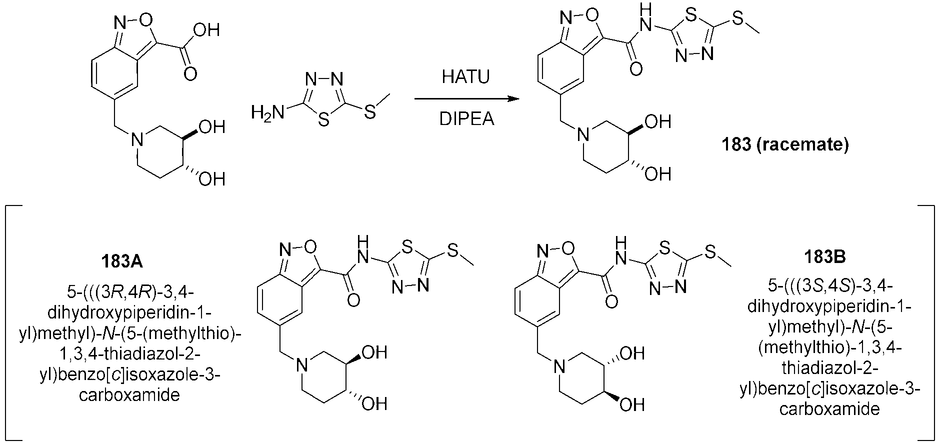

**Step 1:** A suspension of ethyl 5-(bromomethyl)benzo[c]isoxazole-3-carboxylate (1.0 eq, 75.0 mg, 264 umol), *trans*-3,4-dihydroxypiperidine hydrochloride (1.1 eq, 46.0 mg, 290 umol) and potassium carbonate (1.1 eq, 40.5 mg, 290 umol) in anhydrous N,N-dimethylformamide (DMF) (2 mL) was stirred at rt for 16 h. The crude was purified by reversed-phase chromatography (2-30% MeCN in 0.1% aqueous formic acid) to afford ethyl 5-((*trans*-3,4-dihydroxypiperidin-1-yl)methyl)benzo[c]isoxazole-3-carboxylate (73.4 mg, 87% yield) as a yellow oil. LCMS (ES, m/z) = 321.2 [M+H]⁺. ¹H NMR (CHCl₃-*d*, 300 MHz): *δ*_{H} 7.92 (1H, s), 7.73 (1H, d, *J =* 8.9 Hz), 7.50 (1H, d, *J =* 9.2 Hz), 6.75 (2H, br. s), 4.55 (1H, q, *J =* 7.1 Hz), 3.99-3.88 (2H, m), 3.76 (1H, s), 3.63 (1H, s), 3.21 (1H, *d, J =* 11.7 Hz), 3.12-3.04 (1H, m), 2.68-2.56 (2H, m), 2.17-2.10 (1H, m), 1.79-1.72 (1H, m), 1.49 (3H, *t, J* = 7.1 Hz).

**Step 2:** A solution of ethyl 5-((*trans*-3,4-dihydroxypiperidin-1-yl)methyl)benzo[*c*]isoxazole-3-carboxylate (1.0 eq, 73.4 mg, 229 umol) and lithium hydroxide monohydrate (1.2 eq, 11.8 mg, 275 umol) in water (2 mL) and tetrahydrofuran (THF) (2 mL) was stirred at rt for 6 h. The volatile solvent was removed by rotary evaporation and the aqueous phase was acidified to pH 6 with 2 M HCl. The aqueous phase was freeze-dried to afford 5-((*trans*-3,4-dihydroxypiperidin-1-yl)methyl)benzo[*c*]isoxazole-3-carboxylic acid (67.0 mg, 100% yield) as an off-white solid. The crude, which contained lithium chloride, was used without further purification. LCMS (ES, m/z) = 293.1 [M+H]⁺.

**Step 3:** A solution of 5-((*trans*-3,4-dihydroxypiperidin-1-yl)methyl)benzo[*c*]isoxazole-3-carboxylic acid (1.0 eq, 67.0 mg, 229 umol), 2-amino-5-(methylthio)-1,3,4-thiadiazole (1.2 eq, 41.3 mg, 275 umol) and *N*,*N*-diisopropylethylamine (3.0 eq, 121 uL, 688 umol) in anhydrous N,N-dimethylformamide (DMF) (2 mL) under an Ar atmosphere was treated with 1-[Bis(dimethylamino)methylene]-1H-1,2,3-triazolo[4,5-b]pyridinium 3- oxid hexafluorophosphate (HATU) (1.5 eq, 131 mg, 344 umol). The reaction mixture was stirred at rt for 30 min. The reaction mixture was directly purified by reversed-phase chromatography (5-40% MeCN in 0.1% aqueous formic acid) followed by additional PREP HPLC purification (pH10 buffer/MeOH) to afford 5-((*trans*-3,4-dihydroxypiperidin-1-yl)methyl)-*N*-(5-(methylthio)-1,3,4-thiadiazol-2-yl)benzo[c]isoxazole-3-carboxamide (4.60 mg, 4.8% yield) as a bright yellow solid. LCMS (ES, m/z) = 422.1 [M+H]⁺. ¹H NMR (DMSO-*d*₆, 400 MHz): *δ*_{H} 8.86 (1H, br. s), 8.34 (1H, s), 7.68 (1H, d, *J* = 8.9 Hz), 7.45 (1H, d, *J* = 8.9 Hz), 4.83 (1H, br. s), 3.69 (1H, br. s), 3.31-3.29 (4H, m), 2.96 (2H, br. s), 2.65 (3H, s), 1.89 (1H, br. s), 1.49 (1H, br. s).

### Example 10: 3-(Isoquinolin-1-yl)-N-(5-(methylthio)-1,3,4-thiadiazol-2-yl)isoxazole-5-carboxamide (Compound 150)

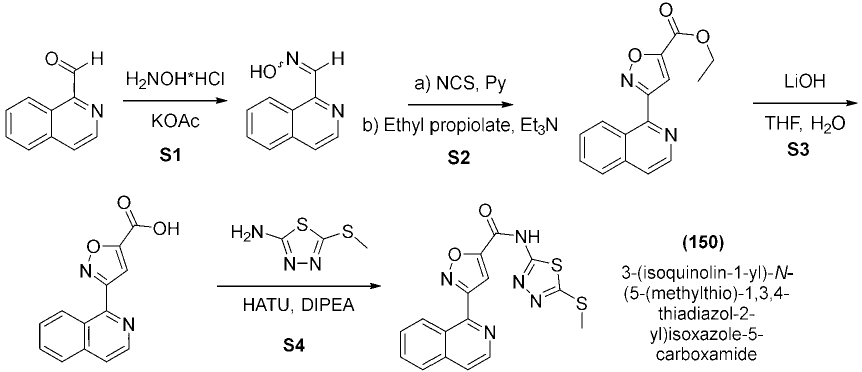

**Step 1:** A suspension of hydroxylamine hydrochloride (3.20 eq, 354 mg, 5.09 mmol), and potassium acetate (KOAc) (4.00 eq, 631 mg, 6.36 mmol) in 80% aqueous ethanol (6.4 mL) was stirred at rt for 30 min. Isoquinoline-1-carbaldehyde (1.00 eq, 250 mg, 1.59 mmol) was added and the reaction mixture was refluxed for 1 h. The reaction mixture was cooled to rt, the solvent was evaporated under vacuum and the residue was taken up in water (25 mL) and it was extracted with dichloromethane (DCM) (3 x 15 mL) then with ethyl acetate (EtOAc) (3 x 15 mL). The combined organic extracts were dried over Na₂SO₄ and the solvent was removed by rotary evaporation to afford isoquinoline-1-carbaldehyde oxime (274 mg, 100% yield) as a pink solid, which was used without further purification. LCMS (ES, m/z) = 173.1 [M+H]⁺. ¹H NMR (CHCl₃-*d*, 300 MHz): *δ*_{H} 11.05 (1H, br s), 8.98 (1H, d, *J* = 8.5 Hz), 8.58 (1H, s), 8.47 (1H, d, *J =* 5.6 Hz), 7.76 (1H, d, *J =* 8.1 Hz), 7.62 (1H, td, *J =* 7.6, 1.3 Hz), 7.50-7.55 (2H, m).

**Step 2:** A solution of isoquinoline-1-carbaldehyde oxime (1.00 eq, 100 mg, 581 µmol) in anhydrous tetrahydrofuran (THF) (11.6 mL) was treated with *N*-chlorosuccinimide (NCS) (1.11 eq, 86.1 mg, 645 µmol) and pyridine (Py) (0.50 eq, 23.5 µL, 290 µmol). The reaction mixture was heated for 30 minutes at 55 °C. The reaction mixture was cooled to 0 °C and it was treated with ethyl propiolate (3.00 eq, 177 µL, 1.74 mmol) (added dropwise) and triethylamine (NEt₃) (1.00 eq, 81.4 µL, 581 µmol) (added dropwise), maintaining the temperature at 5 °C and the reaction mixture was stirred for 15 min. The reaction mixture was filtered and the solids were washed with tetrahydrofuran (10 mL). The filtrate was concentrated *in vacuo* and the residue was purified by flash chromatography on silica gel (0-30% ethyl acetate (EtOAc) /hexanes) to afford ethyl 3-(isoquinolin-1-yl)isoxazole-5-carboxylate (109 mg, 70% yield) as a yellow solid. LCMS (ES, m/z) = 269.1 [M+H]⁺. ¹H NMR (CHCl₃-*d*, 400 MHz): *δ*_{H} 9.13 (1H, d, *J =* 8.3 Hz), 8.65 (1H, d, *J =* 5.6 Hz), 7.92 (1H, d, *J* = 8.0 Hz), 7.74-7.80 (3H, m), 7.71 (1H, s), 4.50 (2H, *q, J =* 7.1 Hz), 1.49 (3H, *t, J =* 7.1 Hz).

**Step 3:** A solution of ethyl 3-(isoquinolin-1-yl)isoxazole-5-carboxylate (1.00 eq, 100 mg, 373 µmol) and lithium hydroxide monohydrate (1.10 eq, 17.6 mg, 410 µmol) in water (3.7 mL) and tetrahydrofuran (THF) (3.7 mL) was stirred at rt for 2 h. The reaction mixture was cooled to 0 °C and it was acidified to pH 4 with 2 M HCl and diluted with water (25 mL). The aqueous phase was lyophilized to afford 3-(isoquinolin-1-yl)isoxazole-5-carboxylic acid (90.0 mg, 100% yield) as an off-white solid, which was used without further purification. LCMS (ES, m/z) = 241.1 [M+H]⁺.

**Step 4:** To a solution of 3-(isoquinolin-1-yl)isoxazole-5-carboxylic acid (1.00 eq,87.3 mg, 363 µmol), 2-amino-5-(methylthio)-1,3,4-thiadiazole (1.20 eq, 65.5 mg, 436 µmol), *N,N-*diisopropylethylamine (3.00 eq, 191 µL, 1.09 mmol) and anhydrous N,N-dimethylformamide (DMF) (3.6 mL) was added 1-[bis(dimethylamino)methylene]-1H-1,2,3-triazolo[4,5-b]pyridinium 3- oxid hexafluorophosphate (HATU) (1.50 eq, 207 mg, 545 µmol) in one portion and the reaction mixture was stirred at rt for 30 min. The reaction mixture was treated with water (25 mL); a colloid was observed. The aqueous phase was extracted with dichloromethane (DCM) (3 x 15 mL). The combined organic extracts were concentrated by rotary evaporation and the resulting yellow solid was then triturated with DCM overnight to afford 3-(isoquinolin-1-yl)-*N*-(5-(methylthio)-1,3,4-thiadiazol-2-yl)isoxazole-5-carboxamide (95.0 mg, 68% yield) as an off-white solid. LCMS (ES, m/z) = 370.0 [M+H]⁺. ¹H NMR (DMSO-*d*₆, 300 MHz): *δ*_{H} 8.95 (1H, dd, *J =* 8.4, 1.3 Hz), 8.73 (1H, d, *J* = 5.6 Hz), 8.13-8.16 (2H, m), 8.07 (1H, d, *J* = 5.6 Hz), 7.81-7.93 (2H, m), 2.76 (3H, s).

### Example 11: 5-(Benzo[d][1,3]dioxol-5-ylmethyl)-N-(5-(methylthio)-1,3,4-thiadiazol-2-yl)benzo[c]isoxazole-3-carboxamide (Compound 185)

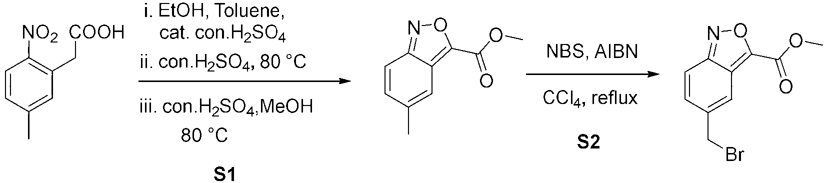

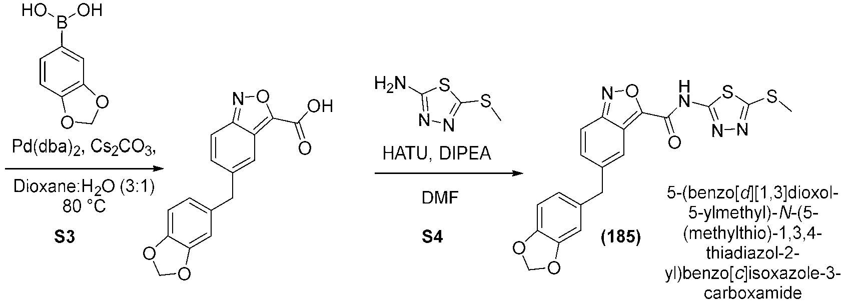

**Step 1:** A flame-dried flask was charged with 2-(5-methyl-2-nitrophenyl)acetic acid (3.00 g, 15.4 mmol), ethanol (EtOH) (25.0 mL), sulfuric acid (82 µL, 1.54 mmol), and toluene (85.0 mL). The flask was fitted with a condenser, and the solution was refluxed for 14 h. The solvent was removed under reduced pressure and sulfuric acid (25.0 mL) was added. After addition, the reaction was heated to 90 °C and stirred for 90 min. The solution was then poured onto ice (300 g), and the mixture was extracted with ether (2 × 100 mL) and ethyl acetate (100 ml). The combined organic layers were dried over MgSO4 and concentrated in vacuo. The crude product was dissolved in MeOH (30 mL) and sulfuric acid (800 µL, 12.3 mmol) was added. The reaction mixture stirred under reflux for 14 h. Reaction mixture was evaporated to dryness and purified by flash chromatography through Si gel (0-40% ethyl acetate/hexanes) to provide methyl 5-methylbenzo[c]isoxazole-3-carboxylate (606 mg, 21 %) as an off-white solid. LCMS (ES, m/z) = 192.1 [M+1]⁺. ¹H NMR (400 MHz, DMSO-*d*₆): *δ*_{H} 7.75-7.73 (m, 1H), 7.60-7.58 (m, 1H), 7.38-7.36 (m, 1H), 4.00 (s, 3H), 2.39 (m, 3H).

**Step 2:** To a solution of methyl 5-methylbenzo[c]isoxazole-3-carboxylate (989 mg, 4.66 mmol) in carbon tetrachloride (CCl₄) (16.2 mL) was added N-bromosuccinimide (NBS) (829 mg, 4.66 mmol) and 2,2'-azobis(2-methylpropionitrile) (AIBN) (76.5 mg, 466 µmol) at room temperature. The resulting mixture was stirred for 16 h at 80 °C. After filtration, the filtrate was concentrated under reduced pressure. The residue was purified by silica gel column chromatography, eluted with hexanes/ethyl acetate (EtOAc) (4:1) to provide methyl 5-(bromomethyl)benzo[c]isoxazole-3-carboxylate (760 mg, 60 %) as a light yellow solid. LCMS (ES, m/z) = 271.9 [M+1]⁺. ¹H NMR (400 MHz, Methanol-*d*₄) *δ* 7.98-7.96 (m, 1H), 7.76 (d, *J =* 8.4, 1H), 7.54 (d, *J =* 8.4 Hz, 1H), 4.86 (s, 2H), 4.10 (s, 3H).

**Step 3:** Methyl 5-(bromomethyl)benzo[c]isoxazole-3-carboxylate (200 mg, 741 umol) and 3,4-methylenedioxyphenylboronic acid, pinacol ester (184 mg, 741 µmol) were taken up in dioxane (6.00 mL)-H₂O (2.00 mL), and cesium carbonate (724 mg, 2.22 mmol) was added. The suspension was degassed and purged with nitrogen (3x). 1,1'-Bis(diphenylphosphino)ferrocene dichloropalladium (II) (10.8 mg, 14.8 umol) was added and the suspension was degassed with nitrogen (3x). The reaction mixture was heated to 80 °C and was monitored by LC-MS. After overnight, the black reaction mixture filtered through celite, washed with ethyl acetate/methanol. The filtrate was acidified with resin (Amberlist IR-120), filtered and the filtrate was concentrated. The crude product was purified by normal phase chromatography eluting from 100% dichloromethane (DCM) to 10% MeOH in DCM over 25 min to give 5-(benzo[*d*][1,3]dioxol-5-ylmethyl)benzo[c]isoxazole-3-carboxylic acid (115 mg, 52 %). LCMS (ES, m/z) = 298.0 [M+1]⁺. ¹H NMR (400 MHz, Methanol-*d*₄): *δ*_{H} 7.68 (1H, s), 7.59 (1H, d, *J* = 9.3 Hz), 7.28 (1H, d, *J =* 9.3 Hz), 6.69-6.74 (3H, m), 5.88 (2H, s), 3.93 (2H, s).

**Step 4:** *N*,*N*-Diisopropylethylamine (160 uL, 920 µmol) and 1-[bis(dimethylamino)methylene]-1H-1,2,3-triazolo[4,5-b]pyridinium 3- oxid hexafluorophosphate (HATU) (160 mg, 422 µmol) were added sequentially to a solution of 5-(benzo[*d*][1,3]dioxol-5-ylmethyl)benzo[c]isoxazole-3-carboxylic acid (114 mg, 383 µmol) in N,N-dimethylformamide (DMF) (4 mL) at 0 °C and stirred for 10 min. To the resultant solution 2-amino-5-(methylthio)-1,3,4-thiadiazole (56.5 mg, 383 µmol) was added, stirred at 0 °C for 1 h and then at rt for 15h. After completion, the reaction was diluted with water and extracted with ethyl acetate (3x) and combined organic layers were washed with brine and concentrated. The crude product was subjected to column chromatography through silica gel and eluted with dichloromethane/methanol (DCM/MeOH) from 0 to 10% MeOH to provide desired product, 5-(benzo[*d*][1,3]dioxol-5-ylmethyl)-*N*-(5-(methylthio)-1,3,4-thiadiazol-2-yl)benzo[*c*]isoxazole-3-carboxamide as yellow solid (67 mg, 41% yield). LCMS (ES, m/z) = 427.0 [M+1]⁺. ¹H NMR (400 MHz, DMSO-*d*₆): *δ*_{H} 7.83 (1H, s), 7.74 (1H, d, *J =* 9.3 Hz), 7.38 (1H, d*, J =* 9.3 Hz), 6.83-6.86 (2H, m), 6.76 (1H, d, *J =* 8.0 Hz), 5.96 (2H, s), 3.95 (2H, s), 2.74 (3H, s).

### Example 12: N-(5-(methylthio)-1,3,4-thiadiazol-2-yl)-[1,2,3]triazolo[1,5-a]quinoline-3-carboxamide (Compound 190)

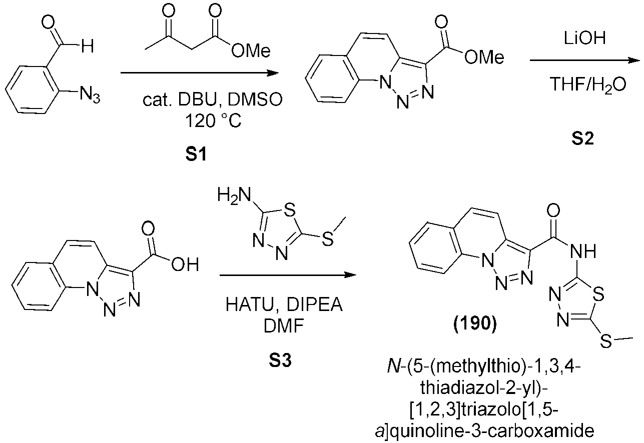

**Step 1:** 2-Azidobenzaldehyde (200 mg, 1.36 mmol) was dissolved in dimethylsulfoxide (DMSO) (2.0 mL) and methyl acetoacetate, 99% (147 µL, 1.36 mmol) was added followed by 1,8-diazabicyclo[5.4.0]undec-7-ene (DBU) (41 µL, 272 µmol). The resulting reaction mixture was stirred for 24 h at 120 °C. The reaction mixture was allowed to cool to rt, water was added and the mixture was extracted with ethyl acetate (EtOAc). The organic was washed with brine, dried over sodium sulfate and concentrated. The crude product was subjected to column chromatography on silica gel (0 - 30% EtOAc/hexanes) to give methyl [1,2,3]triazolo[1,5-*a*]quinoline-3-carboxylate (160 mg, 26 %) as an off-white solid. LCMS (ES, m/z) = 228.1 [M+1]⁺. ¹H NMR (400 MHz, CHCl₃-*d*): *δ*_{H} 8.87 (1H, d, *J =* 8.4 Hz), 8.12 (1H, d*, J =* 9.3 Hz), 7.94 (1H, d*, J =* 8.0 Hz), 7.80-7.87 (2H, m), 7.69 (1H, t, *J =* 7.6 Hz), 4.08 (3H, s).

**Step 2:** Methyl [1,2,3]triazolo[1,5-*a*]quinoline-3-carboxylate (110 mg, 483 umol) was dissolved in a mixture of tetrahydrofuran (THF) (6.6 mL) and H₂O (3.3 mL). The reaction mixture was cooled to 0 °C, followed by addition of lithium hydroxide monohydrate (61 mg, 1.45 mmol). The reaction was stirred at 0 °C-10 °C for 90 min. The reaction was neutralized using an acidic resin (Amberlist IR-120) and filtered to remove resin. The filtrate was concentrated and the solid obtained was used for the next step without further purification. LCMS (ES, m/z) = 214.1 [M+1]⁺.

**Step 3:** *N*,*N*-Diisopropylethylamine (DIPEA) (196 uL, 1.13 mmol) and 1-[Bis(dimethylamino)methylene]-1H-1,2,3-triazolo[4,5-b]pyridinium 3- oxid hexafluorophosphate (HATU) (196 mg, 516 µmol) were added sequentially to a solution of [1,2,3]triazolo[1,5-*a*]quinoline-3-carboxylic acid (100 mg, 469 µmol) in N,N-dimethylformamide (DMF) (4.1 mL) at 0 °C and stirred for 15 min. To the resultant solution 2-amino-5-(methylthio)-1,3,4-thiadiazole (69 mg, 469 µmol) was added and stirring was continued at 0 °C for 1 h and then at rt for 15h. Incomplete conversion of acid after 15h, additional N,N-Diisopropylethylamine (DIPEA) (98 uL, 563 µmol), HATU (98 mg, 258 µmol) and 2-amino-5-(methylthio)-1,3,4-thiadiazole (34.5 mg, 234 µmol) were added and stirred for 3h. After completion, the reaction was diluted with water. Precipitate formed was collected by filtration, the precipitate was rinsed with ethyl acetate (EtOAc), MeOH and acetonitrile (MeCN) to give 35 mg of product (99% purity, LC-MS 254 nm). Whereas, the filtrate was extracted with ethyl acetate (3x). The combined organic layers were washed with brine and concentrated to give a crude solid, which was rinsed with EtOAc, MeOH and MeCN to give an additional 24 mg of product. Total amount of *N-*(5-(methylthio)-1,3,4-thiadiazol-2-yl)-[1,2,3]triazolo[1,5-*a*]quinoline-3-carboxamide (59.0 mg, 37 %) was obtained as an off-white solid. LCMS (ES, m/z) = 343.0 [M+1]⁺. ¹H NMR (400 MHz, DMSO-*d*₆): *δ*_{H} 13.35 (1H, s), 8.78 (1H, d, *J =* 8.4 Hz), 8.17-8.23 (3H, m), 7.98 (1H, t, *J =* 7.8 Hz), 7.82 (1H, t, *J =* 7.6 Hz), 2.75 (3H, s).

### Example 13: N⁶,N⁶-dimethyl-N³-(5-(methylthio)-1,3,4-thiadiazol-2-yl)benzo[c]isoxazole-3,6-dicarboxamide (Compound 192)

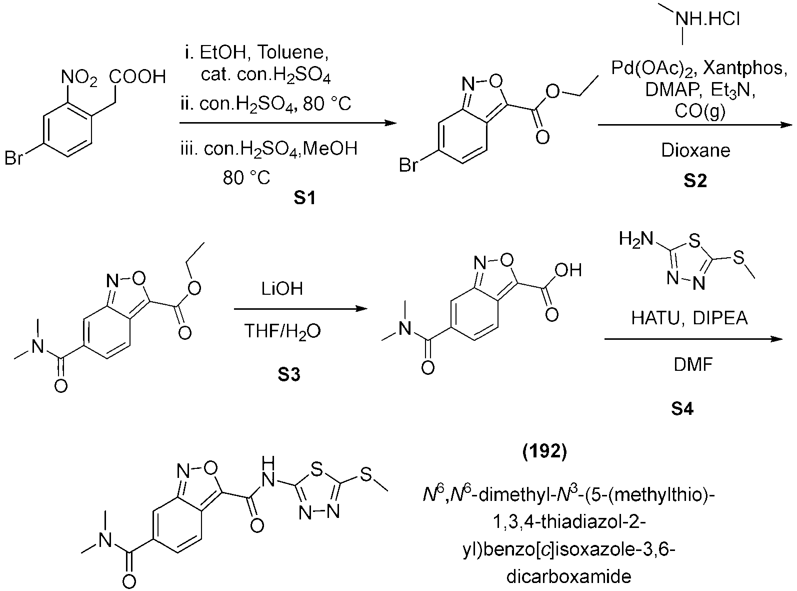

**Step 1:** Following the procedure from step **1** of **Example 11** ethyl 6-bromobenzo[c]isoxazole-3-carboxylate (5.20 g, 51 %) was obtained as a white solid LCMS (ES, m/z) = 272.0 [M+1]⁺. ¹H NMR (400 MHz, CHCl₃-*d*) *δ*_{H} 7.94 (1H, s), 7.82 (1H, d, *J* = 9.2 Hz), 7.29 (1H, d, *J =* 9.2 Hz), 4.55 (2H, q, *J =* 7.1 Hz), 1.49 (3H, t, *J =* 7.1 Hz).

**Step 2:** A dry vial was charged with ethyl 6-bromobenzo[c]isoxazole-3-carboxylate (100 mg, 370 µmol), palladium (II) acetate (Pd(OAc)₂) (6.65 mg, 29.6 µmol), xantphos (17.1 mg, 29.6 µmol), 4-dimethylaminopyridine (DMAP) (13.8 mg, 111 µmol) and dioxane (3.2 mL). The flask was evacuated and backfilled with nitrogen (3 times). Triethylamine (516 µL, 3.70 mmol) and dimethylamine hydrochloride (45.3 mg, 555 µmol) were added, and the flask was then evacuated once more and fitted with a CO balloon and gas was bubbled for 1 min. The vial was sealed and heated at 80 °C for 17 h under a CO atmosphere. After overnight, the reaction mixture was filtered through celite, washed with ethyl acetate (EtOAc) and methanol (MeOH), and the filtrate was concentrated. The crude product was purified by silica gel column chromatography (0 - 100% EtOAc/hexanes) to give ethyl 6-(dimethylcarbamoyl)benzo[c]isoxazole-3-carboxylate (47.0 mg, 48 %) as an off-white solid. LCMS (ES, m/z) = 263.1 [M+1]⁺. ¹H NMR (400 MHz, CHCl₃-*d*): *δ*_{H} 7.99 (1H, dd, *J =* 8.9, 1.1 Hz), 7.73 (1H, s), 7.28 (1H, d, *J =* 1.3 Hz), 4.56 (2H, q, *J =* 7.1 Hz), 3.16 (3H, s), 3.04 (3H, s), 1.50 (3H, t, *J =* 7.1 Hz).

**Step 3:** To a solution of ethyl 6-(dimethylcarbamoyl)benzo[c]isoxazole-3-carboxylate (47.0 mg, 179 µmol) in tetrahydrofuran (THF) (2.4 mL)-H₂O (1.2 mL) at 0 °C, was added lithium hydroxide (LiOH) monohydrate (22.6 mg, 538 µmol) and the resulting mixture was stirred for 1 h at 0-10 °C. The reaction mixture was acidified by addition of a resin (Amberlist IR-120, strongly acidic) followed by filtration to remove resin. The resin was further rinsed with ethyl acetate/methanol (EtOAc/MeOH) and filtrate concentrated to provide 6-(dimethylcarbamoyl)benzo[c]isoxazole-3-carboxylic acid (42 mg, 100% yield) as a yellow solid which was used directly in the next step without further purification. LCMS (ES, m/z) = 235.1 [M+1]⁺.

**Step 4:** *N*,*N*-Diisopropylethylamine (78.1 uL, 448 µmol) and 1-[bis(dimethylamino)methylene]-1H-1,2,3-triazolo[4,5-b]pyridinium 3- oxid hexafluorophosphate (HATU) (81.8 mg, 215 µmol) were added sequentially to a solution of 6-(dimethylcarbamoyl)benzo[c]isoxazole-3-carboxylic acid (42.0 mg, 179 µmol) in N,N-dimethylformamide (DMF) (1.55 mL) at 0 °C and stirred for 10 min. To the resultant solution 2-amino-5-(methylthio)-1,3,4-thiadiazole (26.4 mg, 179 µmol) was added, stirred at 0 °C for 10 min and then at rt for 3 h. After completion, the reaction was diluted with water and lyophilized. The solid thus obtained was treated with EtOH/water to give a precipitate which was collected by filtration. The EtOH/water filtrate contained some product which was recovered by C18 reverse phase chromatography (10 - 100% MeCN/water). The precipitate and product from reverse phase were combined and lyophilized. This product was further washed with ether to provide *N*⁶,*N*⁶-dimethyl-*N*³-(5-(methylthio)-1,3,4-thiadiazol-2-yl)benzo[c]isoxazole-3,6-dicarboxamide (17.0 mg, 26 % yield) as a light yellow solid. LCMS (ES, m/z) = 364.0 [M+1]⁺. ¹H NMR (400 MHz, DMSO-*d*₆): *δ*_{H} 8.13 (1H, d, *J* = 8.9 Hz), 7.86 (1H, s), 7.30 (1H, d, *J =* 9.0 Hz), 3.01 (3H, s), 2.94 (3H, s), 2.73 (3H, s).

### Example 14: 6-(hydroxymethyl)-N-(5-(methylthio)-1,3,4-thiadiazol-2-yl)benzoic]isoxazole-3-carboxamide (Compound 194)

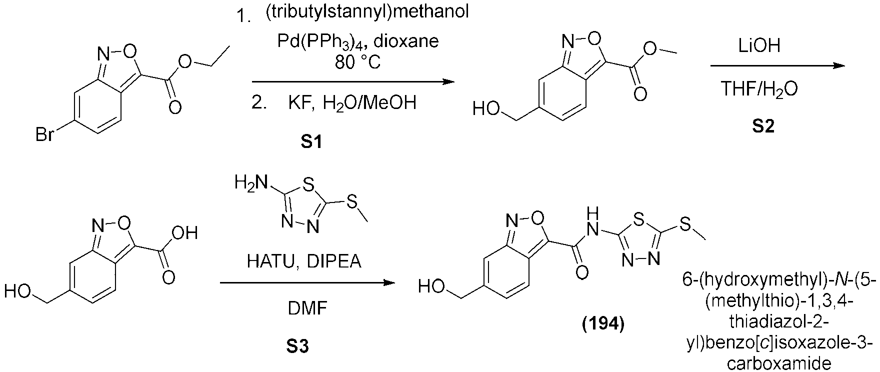

Step 1: Anhydrous dioxane (10.0 mL) and ethyl 6-bromobenzo[c]isoxazole-3-carboxylate (400 mg, 1.48 mmol) were added to (tributylstannyl)methanol (740 mg, 2.07 mmol) and tetrakis(triphenylphosphine)palladium (Pd(PPh₃)₄) (342 mg, 296 µmol) under nitrogen at room temperature. The mixture was stirred at 80 °C for 24 h. After cooling to room temperature, potassium fluoride (KF) (258 mg, 4.44 mmol) and water/methanol (MeOH) (1:1, 10 mL) were added and stirred overnight. The reaction mixture was filtered through celite and the filtrate was concentrated and purified by flash chromatography through silica gel (0 - 10% MeOH/ dichloromethane (DCM)) to provide methyl 6-(hydroxymethyl)benzo[c]isoxazole-3-carboxylate (150 mg, 24 %). LCMS (ES, m/z) = 208.1 [M+1]⁺.

**Step 2:** To a solution of methyl 6-(hydroxymethyl)benzo[c]isoxazole-3-carboxylate (150 mg, 339 µmol) in tetrahydrofuran (THF) (4.6 mL)-H₂O (2.3 mL) at 0 °C, was added lithium hydroxide (LiOH) monohydrate (43 mg, 1.02 mmol) and the resulting mixture was stirred for 1 h at 0 °C. A resin (Amberlist IR-120, strongly acidic) was added followed by filtration to remove the resin. The resin was further rinsed with ethyl acetate (EtOAc) /MeOH and filtrate was concentrated to provide 6-(hydroxymethyl)benzo[c]isoxazole-3-carboxylic acid (65 mg, 100% yield), which was used directly in the next step without further purification. LCMS (ES, m/z) = 194.0 [M+1]⁺.

**Step 3:** *N*,*N*-Diisopropylethylamine (147 µL, 841 umol) and 1-[bis(dimethylamino)methylene]-1H-1,2,3-triazolo[4,5-b]pyridinium 3- oxid hexafluorophosphate (HATU) (154 mg, 404 µmol) were added sequentially to a solution of 6-(hydroxymethyl)benzo[c]isoxazole-3-carboxylic acid (65.0 mg, 337 µmol) in N,N-dimethylformamide (DMF) (2.91 mL) at 0 °C and stirred for 10 min. To the resultant solution 2-amino-5-(methylthio)-1,3,4-thiadiazole (49.5 mg, 337 µmol) was added, stirred at 0 °C for 10 min and then at rt for 90 min. After completion, the reaction mixture was diluted with water and extracted with ethyl acetate (3x). The combined organic layers were washed with brine and concentrated. The crude product purified by flash chromatography through silica gel (0 - 10% MeOH/ dichloromethane (DCM)) to provide 6-(hydroxymethyl)-*N*-(5-(methylthio)-1,3,4-thiadiazol-2-yl)benzo[c]isoxazole-3-carboxamide (28.0 mg, 26 % yield) as a light yellow solid. LCMS (ES, m/z) = 323.0 [M+1]⁺.¹H NMR (400 MHz, DMSO-*d*₆): *δ*_{H} 8.01 (1H, d, *J =* 8.9 Hz), 7.62 (1H, s), 7.23 (1H, d, *J* = 9.0 Hz), 5.53 (1H, s), 4.58 (2H, s), 2.72 (3H, s).

### Example 15: 3-methyl-N-(5-(methylthio)-1,3,4-thiadiazol-2-yl)-2-oxo-4-phenyl-2H-pyran-6-carboxamide (Compound 203)

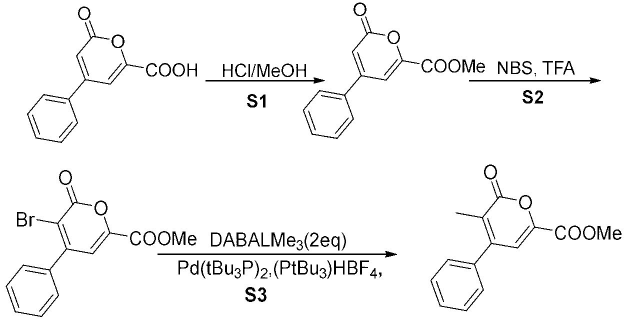

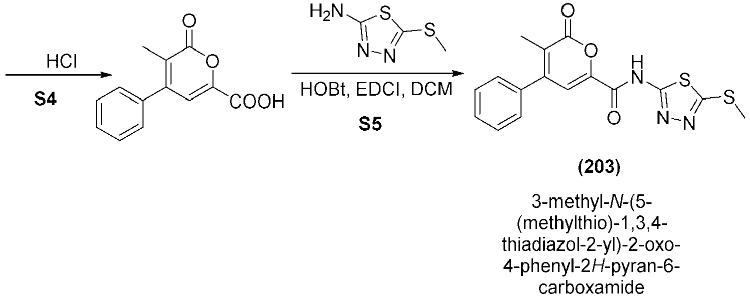

**Step 1:** To a solution of 2-oxo-4-phenyl-2H-pyran-6-carboxylic acid (350.00 mg, 1.619 mmol, 1.00 equiv) in methanol (MeOH) (4.00 mL) was added HCl (4 M) in 1,4-dioxane (1.50 mL). The resulting mixture was stirred for 3h at room temperature and the resulting solution was concentrated under vacuum. The residue was diluted with water (30 mL). The mixture was basified to pH 9 with saturated sodium bicarbonate (aq.). The resulting mixture was extracted with ethyl acetate (3 x 30 mL). The combined organic layers were washed with brine (2 x 10 mL), dried over anhydrous sodium sulfate. After filtration, the filtrate was concentrated under reduced pressure to afford methyl 2-oxo-4-phenyl-2H-pyran-6-carboxylate (280 mg, 75% yield) as a brown solid. LCMS (ES, m/z) = 231 [M+H]⁺.

**Step 2:** To a solution of methyl 2-oxo-4-phenyl-2H-pyran-6-carboxylate (400.00 mg, 1.737 mmol, 1.00 equiv) in trifluoroacetic acid (TFA) (6.00 mL) was added N-bromosuccinimide (NBS) (371.09 mg, 2.084 mmol, 1.20 equiv) at room temperature. The resulting solution was stirred for 3 h at 70 °C. The resulting mixture was concentrated under reduced pressure. The residue was purified by silica gel column chromatography (5:4 petroleum ether/ ethyl acetate (EtOAc)) to afford methyl 3-bromo-2-oxo-4-phenyl-2H-pyran-6-carboxylate (500 mg, 93% yield) as a yellow oil. LCMS (ES, m/z) = 309 [M+H]+.

**Step 3:** Under nitrogen, to a mixture solution of methyl 3-bromo-2-oxo-4-phenyl-2H-pyran-6-carboxylate (300 mg, 0.971 mmol, 1.00 equiv), bis(tri-t-butylphosphine)palladium (Pd(t-Bu₃P)₂) (49.60 mg, 0.097 mmol, 0.10 equiv) and tri-tert-butylphosphonium tetrafluoroborate (t-Bu₃P)HBF₄) (28.14 mg, 0.097 mmol, 0.10 equiv) in tetrahydrofuran (THF) (5.00 mL) was added (µ-1,4-diazabicyclo[2.2.2]octane-kN¹:kN⁴)(hexamethyl)dialuminium (DABAL-Me₃) (497.57 mg, 1.942 mmol, 2.00 equiv) dropwise at room temperature. The resulting solution was stirred for 2 h at 70 °C. The resulting mixture was concentrated under vacuum. The residue was purified by silica gel column chromatography (5:4 petroleum ether/ ethyl acetate (EtOAc)) to afford methyl 3-methyl-2-oxo-4-phenyl-2H-pyran-6-carboxylate (100 mg, 42 % yield) as a yellow oil. LCMS (ES, m/z) = 245 [M+H]+.

**Step 4:** A solution of methyl 3-methyl-2-oxo-4-phenyl-2H-pyran-6-carboxylate (100 mg, 0.409 mmol, 1.00 equiv) in HCl (6M) (1.00 mL) was stirred for 4 h at 100 °C. The resulting mixture was concentrated under reduced pressure, and then the residue was diluted with water (10 mL). The mixture was basified to pH 8 with saturated sodium bicarbonate (aq.). The aqueous layer was extracted with ethyl acetate (2 x 10 mL). The aqueous layer was acidified to pH 3 with HCl (aq., 1M). The resulting mixture was extracted with ethyl acetate (2 x 50 mL). The combined organic layers were washed with brine (2 x 5 mL), dried over anhydrous sodium sulfate. After filtration, the filtrate was concentrated under reduced pressure to afford 3-methyl-2-oxo-4-phenyl-2H-pyran-6-carboxylic acid (50 mg, 53% yield) as a yellow oil. LCMS (ES, m/z) = 231 [M+H]+.

**Step 5:** To a solution of 3-methyl-2-oxo-4-phenyl-2H-pyran-6-carboxylic acid (50.0 mg, 0.217 mmol, 1.00 equiv) in dichloromethane (0.50 mL) was added 5-(methylthio)-1,3,4-thiadiazol-2-amine (38.4 mg, 0.260 mmol, 1.20 equiv) and hydroxybenzotriazole (HOBt) (44.02 mg, 0.326 mmol, 1.50 equiv) and 1-ethyl-3-(3-dimethylaminopropyl)carbodiimide (EDCI) (124.90 mg, 0.651 mmol, 3.00 equiv) at room temperature. The resulting solution was stirred for 1 h at room temperature .The resulting mixture was diluted with water (10 mL). The resulting mixture was extracted with dichloromethane (3 x 50 mL). The combined organic layers were washed with brine (2 x 5 mL), dried over anhydrous sodium sulfate. After filtration, the filtrate was concentrated under reduced pressure, and then the residue was purified by Prep-HPLC (conditions: Column, XBridge Prep OBD C18 Column, 30*150mm 5um; Mobile phase: Water (Phase A: 10mmol/L NH₄HCO₃) and acetonitrile (MeCN) (Phase B), Gradient: 20% Phase B up to 35% Phase B in 7 min; Detector, UV 254) to afford 3-methyl-N-(5-(methylthio)-1,3,4-thiadiazol-2-yl)-2-oxo-4-phenyl-2H-pyran-6-carboxamide (8.1 mg, 10 % yield) as a yellow solid. LCMS (ES, m/z) = 360.15 [M+H]+. ¹H NMR (400 MHz, DMSO-d6) δ 7.61-7.49 (m, 5H), 7.30 (s, 1H), 2.69 (s, 3H), 2.07 (s, 3H).

### Example 16: N-[5-(2-chlorophenyl)-1,3,4-thiadiazol-2-yl]-6-oxo-4-phenylpyran-2-carboxamide (Compound 205)

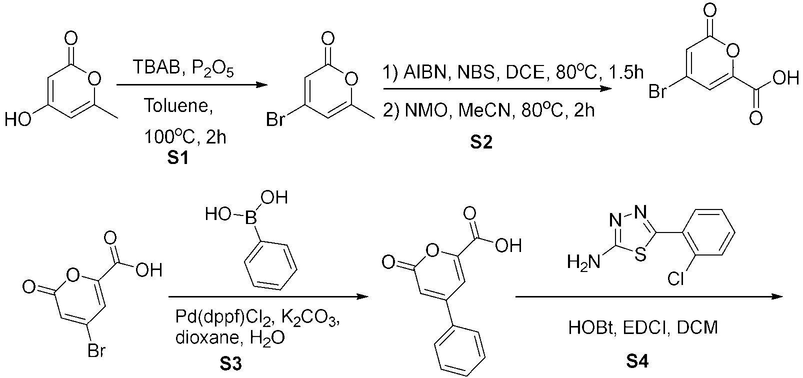

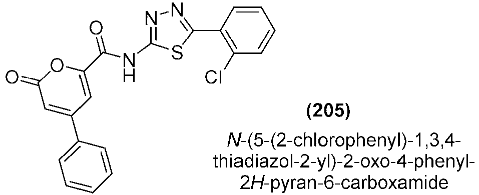

**Step 1:** To a stirred mixture of 4-hydroxy-6-methyl-2H-pyran-2-one (300.0 g, 2.37 mol) and tetra-*n*-butylammonioum bromide (TBAB) (1150.3 g, 3.56 mol) in toluene (1000 mL) was slowly added phosphorus pentoxide (P₂O₅) (847.54 g, 5.97 mmol) at room temperature. The mixture was for 2 h at 100 °C then cooled to room temperature and quenched by the addition of 1.5 L of water. The layers were separated and the aqueous phase was extracted with 3 x 500 mL of ethyl acetate. The combined organic layers were washed with 2 x 200 mL of brine, dried over anhydrous sodium sulfate and then concentrated under vacuum to afford 413 g of crude product, which was suspended in 2 L of N,N-dimethylformamide (DMF) /water (9:1) and stirred at room temperature for 1h. The solid was collected by filtration to afford 4-bromo-6-methyl-2H-pyran-2-one (310 g, 68 % yield) as a reddish brown solid. LCMS (ES, m/z) = 189/191 [M+H]+.

**Step 2:** To a stirred mixture of 4-bromo-6-methyl-2H-pyran-2-one (50.0 g, 264.54 mmol) and N-bromosuccinimide (NBS) (56.50 g, 317.44 mmol) in dichloroethane (DCE) (500 mL) was slowly added azobisisobutyronitrile (AIBN) (4.34 g, 26.454 mmol) at room temperature. The mixture was stirred for 2 h at 80 °C and then concentrated under vacuum. The residue was dissolved in acetonitrile (MeCN) (500 mL). N-methylmorpholine-N-oxide (NMO) (61.98 g, 529.07 mmol) was slowly added at room temperature. The resulting mixture was stirred for 1.5 h at 80 °C and then quenched by the addition of 1 L of water at room temperature. The solid was filtered off and the filtrate was extracted with 3 x 500 mL of ethyl acetate. The aqueous layer was acidified with concentrated hydrochloric acid (12 N) to pH ~ 3 and extracted with 3 x 500 mL of ethyl acetate. The combined organic layers were dried over anhydrous sodium sulfate and then concentrated under vacuum to afford 4-bromo-2-oxo-2H-pyran-6-carboxylic acid (4.5 g, 7.9 % yield) as a yellow solid as a ~3:1 mixture of 4-bromo-2-oxo-2H-pyran-6-carboxylic acid:pyrrolidine-2,5-dione from N-bromosuccinimide (NBS). Used directly in the next step without further purification. LCMS (ES, *m*/*z)* = 217/219 [M - 1]. ¹H NMR (300 MHz, DMSO-*d*₆) δ 7.26 (d, *J* = 1.8 Hz, 1H), 7.13 (d*, J =* 1.8 Hz, 1H).

**Step 3:** Into a solution of 4-bromo-6-oxopyran-2-carboxylic acid (200 mg, 0.913 mmol, 1 equiv) in dioxane/H₂O (2 mL) were added phenyl boronic acid (167 mg, 1.370 mmol, 1.50 equiv), [1,1'-bis(diphenylphosphino)ferrocene]dichloropalladium(II) (Pd(dppf)Cl₂) (134 mg, 0.183 mmol, 0.20 equiv) and K₂CO₃ (252 mg, 1.823 mmol, 2.00 equiv) at room temperature. The resulting mixture was stirred for 2 h at 80 °C under nitrogen atmosphere. The resulting mixture was diluted with water (20 mL). The resulting mixture was extracted with ethyl acetate (EtOAc) (3 x 50 mL). The combined organic layers were washed with water brine (2 x 5 mL), dried over anhydrous Na₂SO₄. After filtration, the filtrate was concentrated under reduced pressure. The residue was purified by silica gel column chromatography (1:1 petroleum ether/EtOAc) to afford 6-oxo-4-phenylpyran-2-carboxylic acid (80 mg, 38 % yield) as an off-white solid. LCMS (ES, m/z): 217[M+H]⁺.

Step 4: Into solution of 6-oxo-4-phenylpyran-2-carboxylic acid (50 mg, 0.231 mmol, 1 equiv) in dichloromethane (DCM) (1 mL) were added hydroxybenzotriazole (HOBt) (47 mg, 0.348 mmol, 1.50 equiv) and 1-ethyl-3-(3-dimethylaminopropyl)carbodiimide (EDCI) (133 mg, 0.694 mmol, 3.00 equiv) at room temperature. The resulting mixture was stirred for 0.5 h at room temperature. To the above stirred mixture was added 5-(2-chlorophenyl)-1,3,4-thiadiazol-2-amine (54 mg, 0.255 mmol, 1.10 equiv) at room temperature. The resulting mixture was stirred for 1 h at room temperature. The precipitated solids were collected by filtration and washed with acetonitrile (3 x 10 mL).The crude product was purified by Prep-HPLC with the following conditions (Column: XBridge Shield RP18 OBD Column, 30*150 mm, 5µm; Mobile Phase A: Water(10 mmol/L NH4HCO3), Mobile Phase B: MeCN; Flow rate: 60 mL/min; Gradient: 28% B to 43% B in 8 min, 43% B; Wave Length: 254 nm;) to afford N-[5-(2-chlorophenyl)-1,3,4-thiadiazol-2-yl]-6-oxo-4-phenylpyran-2-carboxamide (6 mg, 6 % yield) as a green solid. LCMS (ES, m/z) = 410.05 [M+H]⁺. ¹H NMR (400 MHz, DMSO-d₆) δ 8.07 (dd, J = 6.0, 3.6 Hz, 1H), 7.90 (dd, J = 6.8, 3.2 Hz, 2H), 7.69-7.60 (m, 2H), 7.59-56 (m, 3H), 7.51-7.78 (m, 2H), 6.83 (s, 1H).

### Example 17A: N-[5-(2-chlorophenyl)-1,3,4-thiadiazol-2-yl]-6-oxo-4-(phenylamino) pyran-2-carboxamide (Compound 206)

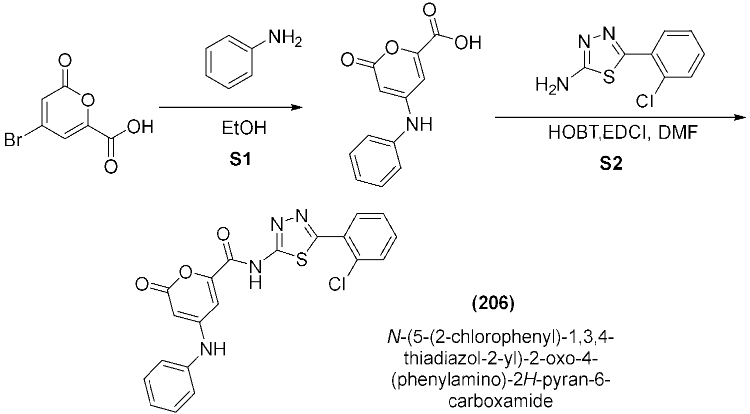

**Step 1:** Into a solution of 4-bromo-6-oxopyran-2-carboxylic acid (3 g, 13.7 mmol, 1 equiv) in ethanol (EtOH) (40 mL) was added aniline (1275 mg, 13.69 mmol, 1.00 equiv) and stirred for 12 h at room temperature. The resulting mixture was diluted with water (200 mL). The resulting mixture was extracted with ethyl acetate (EtOAc) (3 x 200 mL), dried over anhydrous Na₂SO₄. After filtration, the filtrate was concentrated under reduced pressure. The crude product was purified by Prep-HPLC with the following conditions (Column: Xselect CSH C18 OBD Column 30*150mm 5µm; Mobile Phase A: MeCN, Mobile Phase B: water (0.05% trifluoroacetic acid (TFA)); Flow rate: 60 mL/min; Gradient: 20% B to 35% B in 8 min, 35% B; Wave Length: 254/220 nm; RT1(min): 7.72; Number Of Runs: 0) to afford 6-oxo-4-(phenylamino)pyran-2-carboxylic acid (800 mg, 25 % yield) as a brown solid. LCMS (ES, m/z) = 232.05[M+H]⁺.

**Step 2:** To a solution of 6-oxo-4-(phenylamino)pyran-2-carboxylic acid (130 mg, 0.562 mmol, 1 equiv) in N,N-dimethylformamide (DMF) (2 mL) was added 1-ethyl-3-(3-dimethylaminopropyl)carbodiimide (EDCI) (323 mg, 1.685 mmol, 3.00 equiv), hydroxybenzotriazole (HOBt) (114 mg, 0.844 mmol, 1.50 equiv) and 5-(2-chlorophenyl)-1,3,4-thiadiazol-2-amine (130 mg, 0.614 mmol, 1.09 equiv) and the mixture was stirred for 1h at room temperature. The reaction was quenched by the addition of water (2mL) at room temperature. The precipitated solids were collected by filtration and washed with water (10 mL). The residue was washed with dimethylsulfoxide:acetonitrile (DMSO:MeCN) (3:1) (3 x 4 mL).This resulted in*N*-[5-(2-chlorophenyl)-1,3,4-thiadiazol-2-yl]-6-oxo-4-(phenylamino)pyran-2-carboxamide (39.6 mg, 17 % yield) as a yellow solid. LCMS (ES, m/z) = 424.9[M+H]⁺. ¹H NMR (400 MHz, DMSO-d6) δ 13.57 (br, 1H), 9.68 (s, 1H), 8.14 (dd, J = 7.6, 2.0 Hz, 1H), 7.71 (dd, J = 8.0, 1.6 Hz, 1H), 7.60-7.56 (m, 2H), 7.50-7.43 (m, 2H), 7.34 - 7.28 (m, 2H), 7.25 (dd, J = 7.2, 1.2 Hz, 1H), 7.14 (d, J = 2.0 Hz, 1H), 5.52 (d, J = 2.0 Hz, 1H).

### Example 17B: N-(5-(methylthio)-1,3,4-thiadiazol-2-yl)-2-oxo-4-phenoxy-2H-pyran-6-carboxamide (Compound 202)

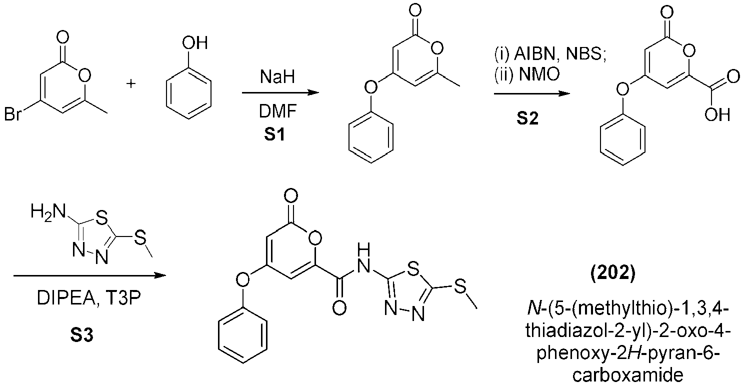

**Step 1:** Phenol (1.61 eq, 40 mg, 0.425 mmol) was dissolved in N,N-dimethylformamide (DMF) (1 mL) and then 4-bromo-6-methyl-pyran-2-one (1.00 eq, 50 mg, 0.265 mmol) was added. The solution was stirred for 30 minutes prior to addition of NaH (1.60 eq, 17 mg, 0.423 mmol). It was then stirred at rt for 15 h. Dimethylsulfoxide (DMSO) (0.5 mL) was added and reaction mixture was directly purified by C18 reverse phase chromatography (0 to 100% MeCN/Water) to afford 6-methyl-4-phenoxy-pyran-2-one (31 mg, 58 % yield) as a yellow solid. LCMS (ES, *m*/*z*) = 202.93 [M+H]⁺. ¹H NMR (CDCl₃, 300 MHz): δ 7.48 - 7.38 (m, 2H), 7.34 - 7.27 (m, 1H), 7.11 - 7.04 (m, 2H), 6.01 - 5.93 (m, 1H), 5.20 (d,J = 2.2 Hz, 1H), 2.30 - 2.22 (m, 3H).

**Step 2:** To a suspension of 6-methyl-4-phenoxy-pyran-2-one (1.00 eq., 31 mg, 0.15 mmol) in CCl₄ (0.2 mL) was added azobisisobutyronitrile (AIBN)(0.1 eq., 2.5 mg, 0.015 mmol) followed by *N-*bromosuccinimide (NBS) (1.2 eq., 33 mg, 0.18 mmol) and the mixture was stirred at 80 °C. After 2 h, volatiles were removed in vacuo and the crude material re-suspended in MeCN (0.75 mL). *N-*methylmorpholine *N*-oxide (NMO) (4.0 eq., 72 mg, 0.61 mmol) was then added and stirred a further 90 min at 80 °C. The mixture was then diluted with water and ethyl acetate and the phases were separated and the ethyl acetate extract set aside. The aqueous phase was then acidified to pH = 1 with 1N HCl and extracted with ethyl acetate (x 2). These ethyl acetate extracts were combined, dried (MgSO₄), filtered and concentrated under reduced pressure to provide crude 2-oxo-4-phenoxy-2H-pyran-6-carboxylic acid (31 mg, 87 % yield) which was used directly in the next step. LCMS (ES, *m*/*z*) = 232.94 [M+H]⁺.

**Step 3:** 5-(Methylsulfanyl)-1,3,4-thiadiazol-2-amine (1.01 eq, 21 mg, 0.136 mmol) was dissolved in tetrahydrofuran (THF) (0.5 mL) and diisopropylethylamine (DIPEA) (3.01 eq, 0.070 mL, 0.402 mmol) added followed by 6-oxo-4-phenoxy-pyran-2-carboxylic acid (1.00 eq, 31 mg, 0.134 mmol). Propylphosphonic acid (T3P) (2.32 eq, 0.18 mL, 0.309 mmol) was then added and the reaction mixture was stirred at rt for 30 minutes. Volatiles were removed *in vacuo* and the crude material dissolved in a small quantity of dimethylsulfoxide (DMSO) and purified directly by C18 reverse phase chromatography (0 to 100% MeCN/Water) to afford *N*-(5-methylsulfanyl-1,3,4-thiadiazol-2-yl)-6-oxo-4-phenoxy-pyran-2-carboxamide (5.7 mg, 12 % yield) as a brown solid. LCMS (ES, m/z) = 362.22 [M+1]+. ¹HNMR (400 MHz, CDCl₃) δ 7.52 - 7.45 (m, 2H), 7.40 - 7.32 (m, 1H), 7.23 (d,J = 2.3 Hz, 1H), 7.14 - 7.06 (m, 2H), 5.57 (d,J = 2.3 Hz, 1H), 2.78 (s, 3H).

### Example 18: N-(5-(2-chlorophenyl)-1,3,4-thiadiazol-2-yl)-3-hydroxy-2-oxo-2H-pyran-6-carboxamide (Compound 64)

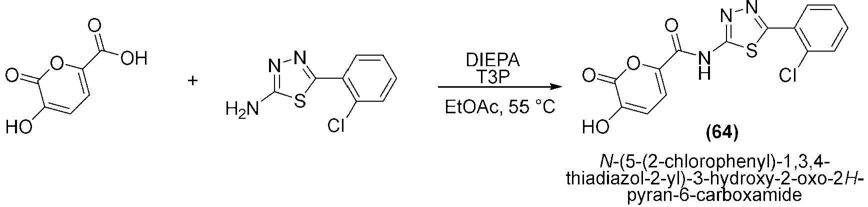

5-Hydroxy-6-oxo-pyran-2-carboxylic acid (1.00 eq, 44 mg, 0.283 mmol) and 5-(2-chlorophenyl)-1,3,4-thiadiazol-2-amine (1.21 eq, 73 mg, 0.343 mmol) were combined in ethyl acetate (EtOAc) (0.5 mL). Diisopropylethylamine (DIPEA) (3.00 eq, 0.15 mL, 0.850 mmol) was added followed by propylphosphonic acid (T3P) (50% in EtOAc) (2.00 eq, 0.34 mL, 0.567 mmol) and the mixture placed in a 55 °C heating block. After 16 h water (5 mL) was added and the solids were collected, washed with water (2 x 3 mL) then with diethyl ether (Et₂O) (3 x 3 mL) then with ethanol (EtOH) (3 x 3 mL) and dried to provide *N*-[5-(2-chlorophenyl)-1,3,4-thiadiazol-2-yl]-5-hydroxy-6-oxo-pyran-2-carboxamide (8.0 mg, 8 % yield) as a brown solid. LCMS (ES, m/z) = 350.21 [M+1]+. ¹H NMR (400 MHz, DMSO-*d*₆) δ 8.13 (dd,J = 7.7, 2.2Hz, 1H), 7.70 (dd,J = 7.7, 1.6Hz, 1H), 7.64 - 7.50 (m, 3H), 7.41 (d,J = 7.5Hz, 1H), 6.80 (d,J = 7.5Hz, 1H). (NH signal not observed).

### Example 19: [5-(2,1-benzoxazole-3-amido)-1,3,4-thiadiazol-2- yl]acetic acid (Compound 132)

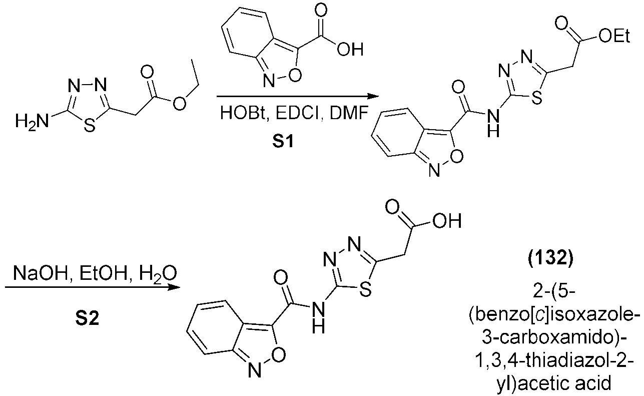

**Step 1:** To a stirred solution of 2,1-benzoxazole-3-carboxylic acid (200 mg, 1.23 mmol, 1 equiv) and ethyl 2-(5-amino-1,3,4-thiadiazol-2-yl) acetate (301 mg, 1.61 mmol, 1.31 equiv) in N,N-dimethylformamide (DMF) (2 mL) was added hydroxybenzotriazole (HOBt) (200 mg, 1.48 mmol, 1.21 equiv) and 1-ethyl-3-(3-dimethylaminopropyl)carbodiimide (EDCI) (940 mg, 4.90 mmol, 4.00 equiv). The resulting mixture was stirred for 1 hour at room temperature. The resulting mixture was diluted with water (10 mL). The resulting mixture was extracted with ethyl acetate (EtOAc) (3 x 20 mL). The combined organic layers were washed with water (2 x 5 mL), dried over anhydrous Na₂SO₄. After filtration, the filtrate was concentrated under reduced pressure. The residue was purified by silica gel column chromatography (3:1 petroleum ether/EtOAc) to afford ethyl 2-[5-(2,1-benzoxazole-3-amido)-1,3,4-thiadiazol-2-yl]acetate (67 mg, 16 % yield) as a light yellow solid. LCMS (ES, m/z) = 333.0 [M+H]⁺.

**Step 2:** To a stirred solution of ethyl 2-[5-(2,1-benzoxazole-3-amido)-1,3,4-thiadiazol-2-yl] acetate (60 mg, 0.181 mmol, 1 equiv) in ethanol (EtOH) (0.6 mL) and H₂O (0.2 mL) was added NaOH (8 mg, 0.200 mmol, 1.11 equiv). The resulting mixture was stirred for 1 hour at room temperature. The mixture was acidified to pH = 4 with HCl (6 M). The resulting mixture was extracted with ethyl acetate (EtOAc) (3 x 5 mL). The combined organic layers were washed with water (2 x 5 mL), dried over anhydrous Na₂SO₄. After filtration, the filtrate was concentrated under reduced pressure. The crude product (30 mg) was purified by Prep-HPLC (conditions: Column: Xselect CSH C18 OBD Column 30*150mm 5µm, n; Mobile Phase A: acetonitrile (MeCN), Mobile Phase B: Water (0.05% trifluoroacetic acid (TFA)); Flow rate: 60 mL/min; Gradient: 15% B to 40% B in 8 min, 40% B to 40% B in 10 min, 40% B; Wave Length: 254/220 nm; RT1(min): 9.12) to afford [5-(2,1-benzoxazole-3-amido)-1,3,4-thiadiazol-2-yl]acetic acid (2.6 mg, 5 % yield) as a light yellow solid. LCMS (ES, m/z) = 305.0 [M+1]⁺. ¹H NMR (400 MHz, DMSO-*d*₆) δ 8.13 (d, *J* = 8.0 Hz, 1H), 7.93 (d, *J* = 8.8 Hz, 1H), 7.55-7.48(m, 1H), 7.36-7.26 (m, 1H), 4.13 (s, 2H).

### Example 20: N-(5-(methylthio)-1,3,4-thiadiazol-2-yl)-5-(3-phenylpyrrolidin-1-yl)-1,3,4-oxadiazole-2-carboxamide (Compound 73)

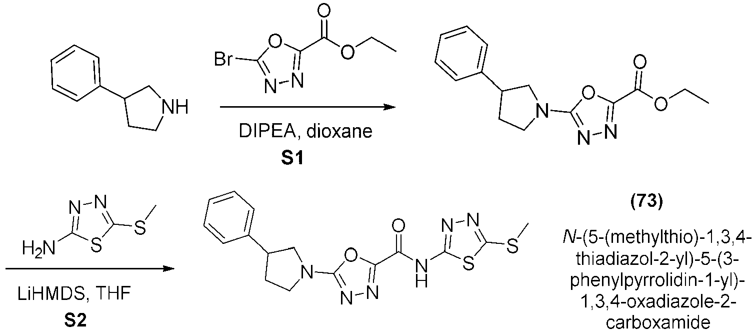

**Step 1:** Ethyl 5-bromo-1,3,4-oxadiazole-2-carboxylate (1.00 eq, 100 mg, 0.452 mmol) was dissolved in dioxane (2.5 mL). 3-Phenylpyrrolidine (1.50 eq, 0.10 mL, 0.679 mmol) was added followed by diisopropylethylamine (DIPEA) (2.00 eq, 0.16 mL, 0.905 mmol) and the mixture stirred at rt overnight. After 16 h, LCMS shows high conversion. Mixture was diluted with ethyl acetate (EtOAc) (40 mL), washed with sat'd NaHCO₃ (10 mL), dried (Na₂SO₄), filtered and concentrated to dryness. Crude material purified by flash chromatography through Si gel (25g Si, 0-100% Ethyl acetate/hexanes) to provide ethyl 5-(3-phenylpyrrolidin-1-yl)-1,3,4-oxadiazole-2-carboxylate (66 mg, 49 % yield) as a clear oil. LCMS (ES, m/z) = 288.06 [M+1]+.

**Step 2:** 5-(Methylsulfanyl)-1,3,4-thiadiazol-2-amine (1.40 eq, 23 mg, 0.146 mmol) was dissolved in tetrahydrofuran (THF) (0.5 mL) in a flask under nitrogen. Lithium bis(trimethylsilyl)amide (LiHMDS) (1M in THF) (1.50 eq, 0.16 mL, 0.157 mmol) was then added and stirred 5 min at rt then a solution of ethyl 5-(3-phenylpyrrolidin-1-yl)-1,3,4-oxadiazole-2-carboxylate (1.00 eq, 30 mg, 0.104 mmol) in THF (1 mL) was added and the mixture stirred at rt. After 3 h, LCMS shows complete conversion. Water (5 mL) was added and the resulting solids collected, washed with water (3 x 3 mL) then with diethyl ether (Et₂O) (3 x 3 mL) to provide N-(5-methylsulfanyl-1,3,4-thiadiazol-2-yl)-5-(3-phenylpyrrolidin-1-yl)-1,3,4-oxadiazole-2-carboxamide (6.0 mg, 15 % yield) as a cream solid. LCMS (ES, m/z) = 389.25 [M+1]+. ¹H NMR (400 MHz, DMSO-*d*₆) δ 7.40 - 7.31 (m, 4H), 7.25 (tt,J = 5.5, 2.7Hz, 1H), 3.99 - 3.89 (m, 1H), 3.71 (t,J = 7.9Hz, 1H), 3.63 - 3.51 (m, 2H), 3.45 (t,J = 9.2Hz, 1H), 2.61 (s, 3H), 2.47 - 2.29 (m, 1H), 2.10 (p,J = 9.4Hz, 1H). [amide NH signal not observed].

### Example 21: 1-(5-{[5-(methylsulfanyl)-1,3,4-thiadiazol-2-yl]carbamoyl}-1,3,4-oxadiazol-2-yl)piperidine-3-carboxylic acid (Compound 110)

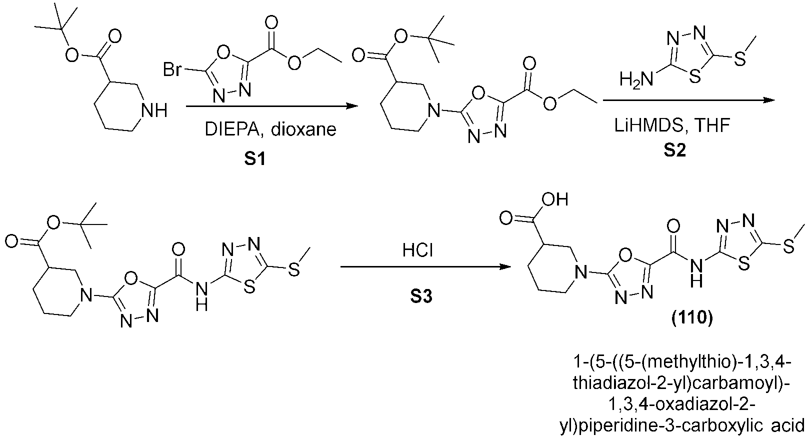

**Steps 1 and 2:** *tert*-Butyl 1-(5-((5-(methylthio)-1,3,4-thiadiazol-2-yl)carbamoyl)-1,3,4-oxadiazol-2-yl)piperidine-3-carboxylate was prepared according to **Example 23 Steps 1 - 2** from *tert*-butyl piperidine-3-carboxylate instead of piperidine. LCMS (ES, m/z) = 427.0 [M+H]⁺.

**Step 3:** A solution of *tert*-butyl 1-(5- {[5-(methylsulfanyl)-1,3,4-thiadiazol-2-yl] carbamoyl}-1,3,4-oxadiazol-2-yl) piperidine-3-carboxylate (25 mg, 0.059 mmol, 1 equiv) in HCl (4 M) in 1,4-dioxane (3 mL) was stirred for 5 h at room temperature. The resulting mixture was stirred for additional 2h at room temperature and concentrated under vacuum. The crude product was purified by Prep-HPLC (conditions: Column, Xselect CSH C18 OBD Column 30*150mm 5um, n; Mobile phase: acetonitrile (MeCN) and water (0.05% TFA), Gradient:15% water (0.05%TFA) and up to 45% water (0.05%TFA) in 8 min) to afford 1-(5-{[5-(methylsulfanyl)-1,3,4-thiadiazol-2-yl] carbamoyl}-1,3,4-oxadiazol-2-yl) piperidine-3-carboxylic acid (3.5 mg, 15.94%) as a white solid. LCMS (ES, m/z) = 371.1 [M+H]⁺. ¹H NMR (400 MHz, DMSO-d6) δ 4.05-4.01 (m, 1H), 3.85-3.66 (m, 1H), 3.51 - 3.24 (m, 2H), 2.74 (s, 3H), 2.64-2.50 (m, 1H), 2.02 - 2.00 (m, 1H), 1.99-1.98 (m, 3H).

### Example 22: tert-butyl 3-{[5-(methylsulfanyl)-1,3,4-thiadiazol-2-yl]carbamoyl}-5H,6H,8H-[1,2,4] triazolo[4,3-a]pyrazine-7-carboxylate (Compound 98)

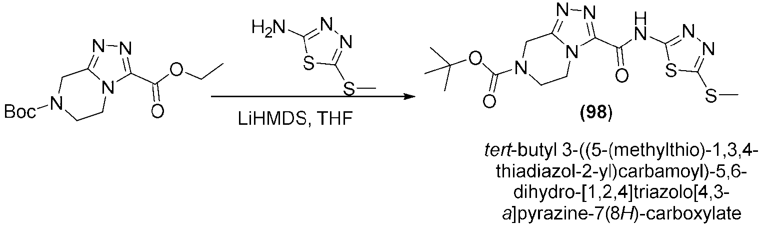

A mixture of 7-tert-butyl 3-ethyl 5*H*,6*H*,8*H*-[1,2,4]triazolo[4,3-a]pyrazine-3,7-dicarboxylate (200 mg, 0.675 mmol, 1.00 equiv) and 5-(methylsulfanyl)-1,3,4-thiadiazol-2-amine (130 mg, 0.883 mmol, 1.31 equiv) in tetrahydrofuran (THF) (2 mL) was added lithium bis(trimethylsilyl)amide (LiHMDS) (1.5 mL, 1.5 mmol, 2 equiv, 1 M in THF) dropwise at 0 °C. The resulting mixture was stirred for 1 h at room temperature under nitrogen atmosphere. The reaction was quenched with water at 0 °C. The resulting mixture was concentrated under vacuum. The crude product was purified by Prep-HPLC (conditions: Column, Xselect CSH C18 OBD Column 30*150mm 5um, n; mobile phase: acetonitrile (MeCN) and Water (0.05% trifluoroacetic acid (TFA)), Gradient: 30% water (0.05% TFA) up to 50% water (0.05%TFA) in 10 min; Detector, UV 254) to afford tert-butyl 3-{[5-(methylsulfanyl)-1,3,4-thiadiazol-2-yl]carbamoyl}-5*H*,6*H*,8*H*-[1,2,4]triazolo[4,3-a]pyrazine-7-carboxylate (29.6 mg, 11 % yield) as a white solid. LCMS (ES, m/z) = 398.0 [M+H]⁺. ¹H NMR (400 MHz, DMSO-d6) δ 13.60 (br, 1H), 4.80 (s, 2H), 4.35 (t, *J* = 5.6 Hz, 2H), 3.79 (t, *J* = 5.6 Hz, 2H), 2.75 (s, 3H), 1.45 (s, 9H).

### Example 23: N-(5-(methylthio)-1,3,4-thiadiazol-2-yl)-5-(piperidin-1-yl)-1,3,4-oxadiazole-2-carboxamide (Compound 108)

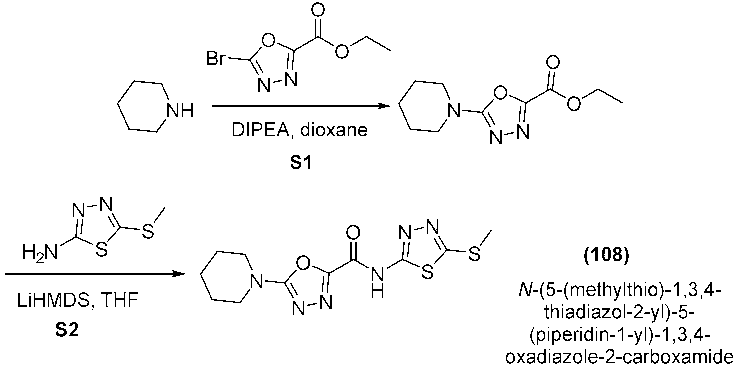

**Step 1:** Into a solution of piperidine (128 mg, 1.503 mmol, 1.00 equiv) in dioxane (3 mL) was added ethyl 5-bromo-1,3,4-oxadiazole-2-carboxylate (240 mg, 1.160 mmol, 0.77 equiv) and diisopropylethylamine (DIPEA) (340 mg, 2.365 mmol, 1.57 equiv). The resulting mixture was stirred for 2 h at room temperature under nitrogen atmosphere. The resulting mixture was diluted with water (50 mL). The resulting mixture was extracted with ethyl acetate (EtOAc) (3 x 50 mL). The combined organic layers were washed with water (1 x 20 mL), dried over anhydrous Na₂SO₄. After filtration, the filtrate was concentrated under reduced pressure. The residue was purified by silica gel column chromatography (12:1 petroleum ether / ethyl acetate) to afford ethyl 5-(piperidin-1-yl)-1,3,4-oxadiazole-2-carboxylate (50 mg, 14 % yield) as a white solid. LCMS (ES, m/z) = 226.0 [M+H]⁺.

**Step 2:** The trifluoroacetic acid (TFA) salt of *N*-(5-(Methylthio)-1,3,4-thiadiazol-2-yl)-5-(piperidin-1-yl)-1,3,4-oxadiazole-2-carboxamide was prepared from ethyl 5-(piperidin-1-yl)-1,3,4-oxadiazole-2-carboxylate (1 equiv) and 5-(methylsulfanyl)-1,3,4-thiadiazol-2-amine 2,2,2-trifluoroacetate (1.2 equiv) according to **Example 22.** LCMS (ES, m/z) = 327.0 [M+H]⁺. ¹H NMR (400 MHz, DMSO-*d*₆) δ 13.57 (br, 1H), 3.61-3.53 (m, 4H), 2.74 (s, 3H), 1.65-1.61 (m, 6H).

### Example 24: N-[5-(methylsulfanyl)-1,3,4-thiadiazol-2-yl]-3-(thiophen-3-yl)-1,2-oxazole-5-carboxamide (Compound 139)

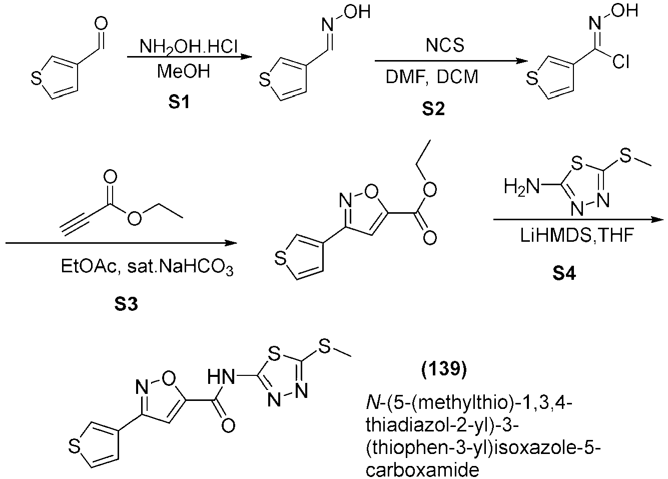

**Step 1:** Into a solution of 3-thiophenecarboxaldehyde (500 mg, 4.458 mmol, 1 equiv) in methanol (14 mL) was added hydroxylamine hydrochloride (929 mg, 13.369 mmol, 3.00 equiv). The resulting mixture was stirred overnight at room temperature. The mixture was then diluted with water (30 mL) and extracted with ethyl acetate (EtOAc) (3 x 50 mL). The combined organic layers were washed with brine (50 mL), dried over anhydrous Na₂SO₄. After filtration, the filtrate was concentrated under reduced pressure to afford (*E*)-*N*-(thiophen-3-ylmethylidene)hydroxylamine) (485 mg, 86 % yield) as a brown solid. LCMS (ES, m/z) = 128.0 [M+H]⁺.

**Step 2:** Into a solution of (*E*)-N-(thiophen-3-ylmethylidene)hydroxylamine (804 mg, 6.323 mmol, 1 equiv) in dimethylformamide (DMF) (12 mL) and dichloromethane (DCM) (4 mL) was added N-chlorosuccinimide (NCS) (1.01g, 7.564 mmol, 1.20 equiv) at room temperature. The resulting mixture was stirred for 30 min at room temperature. The mixture was then diluted with water (200 mL) and extracted with ethyl acetate (EtOAc) (3 x 200 mL). The combined organic layers were washed with water (3 x 20 mL), dried over anhydrous Na₂SO₄. After filtration, the filtrate was concentrated under reduced pressure. The crude product was used in the next step directly without further purification. LCMS (ES, m/z) = 162.0 [M+H]⁺.

**Step 3:** To the above crude product prepared in Step 2 was added ethyl acetate (EtOAc) (18 mL), ethyl propiolate (1861 mg, 18.970 mmol, 3.00 equiv) and sat. sodium bicarbonate (aq, 9 ml) at room temperature. The resulting mixture was stirred overnight at room temperature. The mixture was then diluted with water (20 mL) and extracted with EtOAc (3 x 50 mL). The combined organic layers were washed with brine (10 mL), dried over anhydrous Na₂SO₄. After filtration, the filtrate was concentrated under reduced pressure. The residue was purified by silica gel column chromatography (1:1 petroleum ether / ethyl acetate) to afford ethyl 3-(thiophen-3-yl)-1,2-oxazole-5-carboxylate (490 mg, 25 % yield) as a brown solid. LCMS (ES, m/z) = 224.00 [M+H]⁺.

**Step 4:** Into a mixture of ethyl 3-(thiophen-3-yl)-1,2-oxazole-5-carboxylate (444 mg, 1.99 mmol, 1 equiv) and 5-(methylsulfanyl)-1,3,4-thiadiazol-2-amine (352 mg, 2.39 mmol, 1.20 equiv) in tetrahydrofuran (4 mL) was added lithium bis(trimethylsilyl)amide (LiHMDS) (4 mL, 4.0 mmol, 2.00 equiv, 1 M in tetrahydrofuran (THF)) dropwise at 0 °C under nitrogen atmosphere. The resulting mixture was stirred for 4 h at room temperature. The resulting mixture was diluted with water (20 mL) and extracted with ethyl acetate (EtOAc) (3 x 30 mL). The combined organic layers were washed with brine (30 mL) and dried over anhydrous Na₂SO₄. After filtration, the filtrate was concentrated under reduced pressure. The crude product (200 mg) was purified by Prep-HPLC (conditions: XBridge Prep OBD C18 Column, 30*150 mm, 5µm; Mobile Phase A: Water (10 mmol/L NH₄HCO₃), Mobile Phase B: acetonitrile (MeCN); Flow rate: 60 mL/min; Gradient: 15% B to 25% B in 8 min, 25% B; Wave Length: 254 nm; RT1(min): 7.5) to afford *N-*[5-(methylsulfanyl)-1,3,4-thiadiazol-2-yl]-3-(thiophen-3-yl)-1,2-oxazole-5-carboxamide (16.3 mg, 2.5 % yield) as a white solid. LCMS (ES, m/z) = 325. 0 [M+H]⁺. ¹H NMR (400 MHz, DMSO-*d*₆) δ 8.24 (s, 1H), 7.75 (d, *J =* 5.2 Hz, 1H), 7.61(s, 1H), 7.59 (d, *J* = 5.2 Hz, 1H), 2.69 (s, 3H).

### Example 25: N-(5-(methylthio)-1,3,4-thiadiazol-2-yl)-5-(2-phenylpyrrolidin-1-yl)-1,2,4-oxadiazole-3-carboxamide (Compound 99)

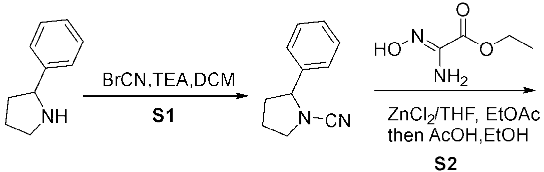

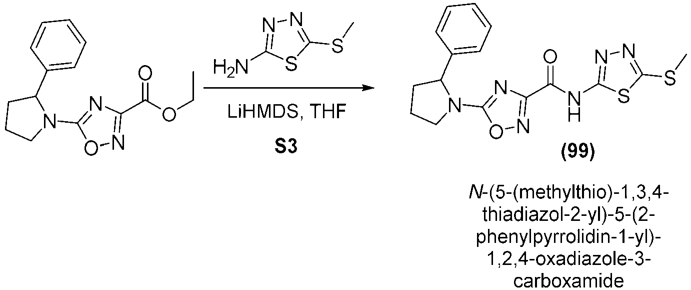

**Step 1:** To a stirred solution of 2-phenylpyrrolidine (500 mg, 3.396 mmol, 1 equiv) in dichloromethane (DCM) (8 mL) was added triethylamine (TEA) (1.03 g, 10.189 mmol, 3.00 equiv) and BrCN (431 mg, 4.069 mmol, 1.20 equiv) at room temperature. The resulting mixture was stirred for 1 h at room temperature. The resulting mixture was poured into water and extracted with ethyl acetate (3 x 50 mL). The combined organic layers were washed with water (20 mL), dried over anhydrous Na₂SO₄. After filtration, the filtrate was concentrated under reduced pressure. The residue was purified by silica gel column chromatography (1:1 petroleum ether / ethyl acetate) to afford 2-phenylpyrrolidine-1-carbonitrile (450 mg, 77 % yield) as a red oil. LCMS (ES, m/z) = 173 [M+H]⁺. ¹H NMR (400 MHz, DMSO-*d*₆) δ 7.48 - 7.25 (m, 5H), 4.68 (t, *J =* 7.2 Hz, 1H), 3.72 - 3.64 (m, 1H), 3.56 - 3.49 (m, 1H), 2.33 - 2.24 (m, 1H), 2.01 - 1.87 (m, 2H), 1.79 - 1.68 (m, 1H).

**Step 2:** To a stirred solution of 2-phenylpyrrolidine-1-carbonitrile (100 mg, 0.581 mmol, 1 equiv) and ethyl (Z)-2-amino-2-(hydroxyimino)acetate (92 mg, 0.696 mmol, 1.20 equiv) in ethyl acetate (2 mL) was added the solution of ZnCl₂ (0.5 M) in tetrahydrofuran (THF) (1.5 mL, 0.75 mmol, 1.28 equiv) at room temperature under nitrogen atmosphere. The resulting mixture was stirred for 1 h at 50 °C under nitrogen atmosphere. To the above stirred mixture was added ethanol (EtOH) (1 mL) and acetic acid (AcOH) (0.5 mL) at room temperature under nitrogen atmosphere. The resulting mixture was stirred for 1 h at 100 °C under nitrogen atmosphere. The resulting mixture was poured into water and extracted with EA (3 x 50 mL). The combined organic layers were washed with water (10 mL), dried over anhydrous Na₂SO₄. After filtration, the filtrate was concentrated under reduced pressure. The residue was purified by silica gel column chromatography (1:1 petroleum ether / ethyl acetate) to afford ethyl 5-(2-phenylpyrrolidin-1-yl)-1,2,4-oxadiazole-3-carboxylate (120 mg, 72 % yield) as a colorless oil. LCMS (ES, m/z) = 288 [M+H]⁺. ¹H NMR (400 MHz, DMSO-*d*₆) δ 7.38 - 7.32 (m, 2H), 7.30 - 7.23 (m, 3H), 5.11 (dd, *J* = 8.0, 3.6 Hz, 1H), 4.97 (q, *J* = 7.1 Hz, 2H), 3.97 - 3.88 (m, 1H), 3.78 - 3.68 (m, 1H), 2.05 - 1.81 (m, 4H), 1.27 (t, *J =* 7.1 Hz, 3H).

**Step 3:** *N*-(5-(Methylthio)-1,3,4-thiadiazol-2-yl)-5-(2-phenylpyrrolidin-1-yl)-1,2,4-oxadiazole-3-carboxamide was prepared from ethyl 5-(2-phenylpyrrolidin-1-yl)-1,2,4-oxadiazole-3-carboxylate (1 equiv) and 5-(methylthio)-1,3,4-thiadiazol-2-amine (1.2 equiv) according to **Example 22.** LCMS (ES, m/z) = 389.10 [M+H]⁺. ¹H NMR (400 MHz, DMSO-*d*₆) δ 13.40 (br, 1H), 7.39 - 7.32 (m, 2H), 7.31 - 7.25 (m, 3H), 5.16 - 5.11 (m, 1H), 3.99 - 3.92 (m, 1H), 3.83 - 3.74 (m, 1H), 2.74 (s, 3H), 2.08 - 1.96 (m, 2H), 1.92 - 1.84 (m, 2H).

### Example 26: N-[5-(methylsulfanyl)-1,3,4-thiadiazol-2-yl]-5-(127yridine-2-yl)-1,2,4-oxadiazole-3-carboxamide (Compound 104)

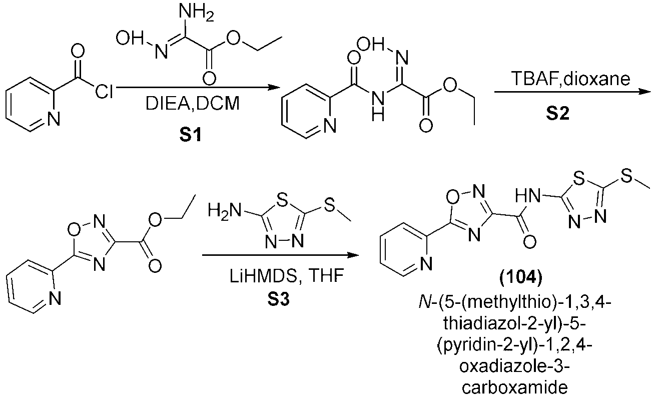

**Step 1:** To a stirred mixture of ethyl (Z)-2-amino-2-(hydroxyimino) acetate (300 mg, 2.27 mmol, 1.00 equiv) in dichloromethane (DCM) (3 mL) and diisopropylethylamine (DIPEA) (470 mg, 3.64 mmol, 1.60 equiv) was added pyridine-2-carbonyl chloride (640 mg, 4.52 mmol, 1.99 equiv) at - 15 °C. The resulting mixture was stirred for an additional 1 hour at room temperature. The mixture was then concentrated under vacuum to provide crude ethyl (Z)-2-(hydroxyimino)-2-(picolinamido) acetate (1.4 g) as a brown solid which was used directly in the next step without further purification. LCMS (ESI, *m*/*z)* = 238.0[M+H]⁺.

**Step2:** To a stirred mixture of ethyl (Z)-2-(hydroxyimino)-2-(picolinamido) acetate (800 mg, 3.37 mmol, 1.00equiv) in dioxane (8 mL) was added tetra-n-butylammonioum fluoride (TBAF) (6.7 mL, 25.6 mmol, 7.60 equiv). The resulting mixture was stirred for 2 hours at 80 °C. The resulting mixture was diluted with water (20 ml) and extracted with ethyl acetate (EtOAc) (3 x 20 mL). The combined organic layers were washed with water (2 x 3 mL) and dried over anhydrous Na₂SO₄. After filtration, the filtrate was concentrated under reduced pressure and purified by silica gel column chromatography (1:1 petroleum ether / ethyl acetate) to afford ethyl 5-(pyridin-2-yl)-1,2,4-oxadiazole-3-carboxylate (270 mg, 36 % yield) as a white solid. LCMS (ES, m/z) = 220.0 [M+H]⁺.

**Step 3:** *N*-[5-(Methylsulfanyl)-1,3,4-thiadiazol-2-yl]-5-(pyridin-2-yl)-1,2,4-oxadiazole-3-carboxamide was prepared from ethyl 5-(pyridin-2-yl)-1,2,4-oxadiazole-3-carboxylate (1 equiv) and 5-(methylthio)-1,3,4-thiadiazol-2-amine (1.2 equiv) according to **Example 22.** LCMS (ES, *m*/*z*) = 320.9 [M+H]⁺. ¹H NMR (400 MHz, DMSO-*d*₆) δ 8.88 (d, *J =* 4.0 Hz, 1H), 8.35 (d, *J =* 8.0 Hz, 1H), 8.19-8.15 (m, 1H), 7.80-7.76 (m, 1H), 2.77 (s, 3H).

### Example 27: N-(5-(methylthio)-1,3,4-thiadiazol-2-yl)-5-(pyridin-2-yl)-1,3,4-oxadiazole-2-carboxamide (Compound 106)

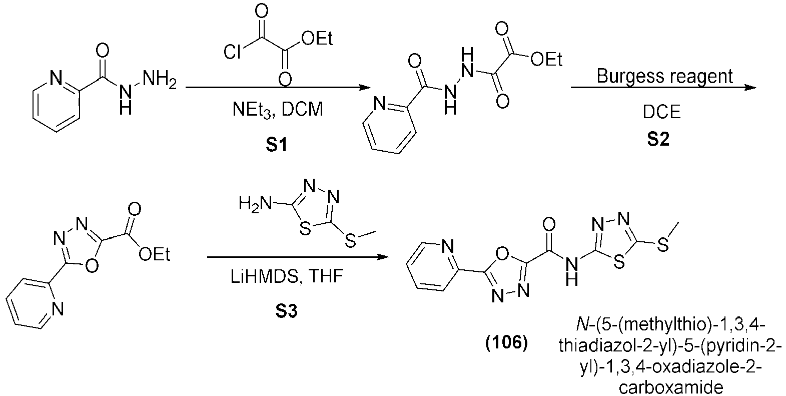

**Step 1:** Into a solution of pyridine-2-carbohydrazide (600 mg, 4.38 mmol, 1.00 equiv) in dichloromethane (DCM) (6 mL) was added ethyl chloroglyoxylate (657 mg, 4.81 mmol, 1.10 equiv) and NEt₃ (885 mg, 8.75 mmol, 2.00 equiv) at room temperature. The resulting mixture was stirred for 1 hour at room temperature. The resulting mixture was concentrated under reduced pressure. The residue was diluted with acetonitrile (MeCN) (5 ml) and stirred for 30 min at room temperature. The solid was collected by filtration and washed with MeCN (3 mL), dried under reduced pressure to afford ethyl 2-oxo-2-(pyridin-2-ylformohydrazido) acetate (1.5 g, 94 % yield) as a brown solid. LCMS (ES, *m*/*z)* = 238 [M+H]⁺.

**Step 2:** Into a solution of ethyl 2-oxo-2-(pyridin-2-ylformohydrazido) acetate (1.2 g, 5.06 mmol, 1 equiv) in dichloroethane (DCE) (12 mL) was added methyl N-(triethylammoniumsulfonyl) carbamate (Burgess reagent) (5.06 g, 21.2 mmol, 4.2 equiv) at room temperature. The resulting mixture was stirred for 3 hours at 50 °C. The resulting solution was diluted with water (10 ml) and extracted with dichloromethane (DCM) (3 x 30mL). The combined organic layers were washed with water (10 mL), dried over anhydrous Na₂SO₄. After filtration, the filtrate was concentrated under reduced pressure. The residue was purified by silica gel column chromatography (1:1 petroleum ether / ethyl acetate) to afford ethyl 5-(pyridin-2-yl)-1,3,4-oxadiazole-2-carboxylate (380 mg, 34 % yield) as a white solid. LCMS (ES, *m*/*z*) = 220 [M+H]⁺.

**Step 3:** *N*-(5-(Methylthio)-1,3,4-thiadiazol-2-yl)-5-(pyridin-2-yl)-1,3,4-oxadiazole-2-carboxamide was prepared from ethyl 5-(pyridin-2-yl)-1,3,4-oxadiazole-2-carboxylate (1 equiv) and 5-(methylsulfanyl)-1,3,4-thiadiazol-2-amine (1.2 equiv) according to **Example 22.** LCMS (ES, *m*/*z*) = 321.0 [M+H]⁺. ¹H NMR (400 MHz, DMSO-*d*₆) δ 8.84 (d, *J* = 8.8 Hz, 1H), 8.32 (d, *J =* 8.8 Hz, 1H), 8.12 - 8.11 (m, 1H), 7.73 - 7.70 (m, 1H), 2.76 (s, 3H).

### Example 28: (R)-N-(5-(methylthio)-1,3,4-thiadiazol-2-yl)-5-(2-phenylpiperidin-1-yl)-1,3,4-oxadiazole-2-carboxamide (Compound 121*) and (S)-N-(5-(methylthio)-1,3,4-thiadiazol-2-yl)-5-(2-phenylpiperidin-1-yl)-1,3,4-oxadiazole-2-carboxamide (Compound 122*)

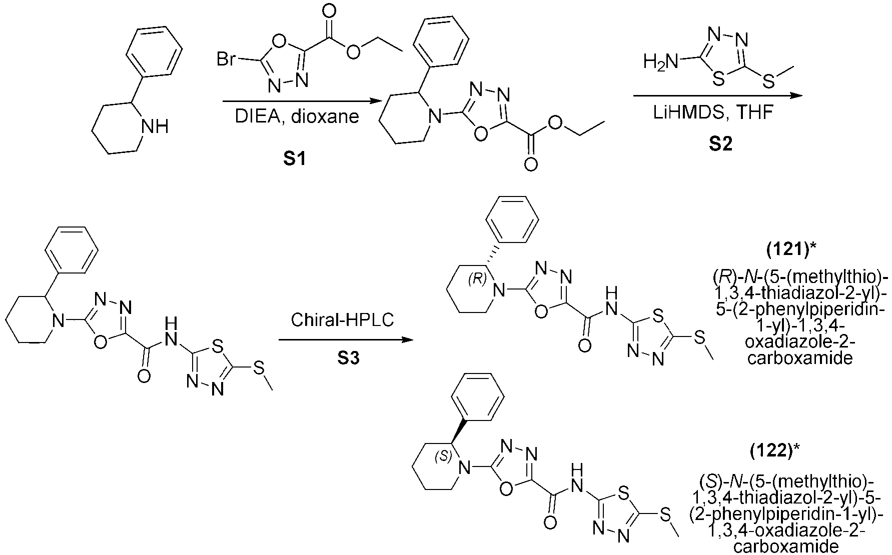

**Steps 1-2:** *N*-(5-(Methylthio)-1,3,4-thiadiazol-2-yl)-5-(2-phenylpiperidin-1-yl)-1,3,4-oxadiazole-2-carboxamide was prepared according to **Example 23 Steps 1 - 2** from 2-phenylpiperidine instead of piperidine.

**Step3:** The R and S isomers of *N*-(5-(Methylthio)-1,3,4-thiadiazol-2-yl)-5-(2-phenylpiperidin-1-yl)-1,3,4-oxadiazole-2-carboxamide were separated by chiral-HPLC with the following conditions: Column: CHIRAL ART Amylose-SA, 2*25 cm, 5 µm; Mobile Phase A: Hex(0.2% FA)--HPLC, Mobile Phase B: EtOH: dichloromethane (DCM)=1: 1--HPLC; Flow rate: 20 mL/min; Gradient: 30% B to 30% B in 15 min; Wave Length: 220/254 nm; RT1(min): 11.55; RT2(min): 13.93; Sample Solvent: MeOH: DCM=1: 1; Injection Volume: 0.5 mL; Number Of Runs: 6.

(*R*)-N-(5-(methylthio)-1,3,4-thiadiazol-2-yl)-5-(2-phenylpiperidin-1-yl)-1,3,4-oxadiazole-2-carboxamide (Compound **121*),** RT2(min): 13.93, LCMS (ES, *m*/*z*) = 403.1[M+H]⁺, ¹HNMR (400 MHz, DMSO-*d*₆) δ 13.75 (br, 1H), 7.43-7.41 (m, 2H), 7.34-7.28 (m, 3H), 5.52-5.39 (m, 1H), 4.10-4.07 (m, 1H), 3.27-3.23 (m, 1H), 2.74 (s, 3H), 2.41-2.33 (m, 1H), 2.07-2.01 (m, 1H), 1.68-1.63 (m, 3H), 1.42-1.33 (m, 1H)

(*S*)-*N*-(5-(methylthio)-1,3,4-thiadiazol-2-yl)-5-(2-phenylpiperidin-1-yl)-1,3,4-oxadiazole-2-carboxamide (Compound **122*),** RT1(min): 11.55, LCMS (ES, *m*/*z*) = 403.1 [M+H]⁺, ¹HNMR (400 MHz, DMSO-*d*₆) δ 13.75 (br, 1H), 7.43-7.41 (m, 2H), 7.34-7.28 (m, 3H), 5.52-5.39 (m, 1H), 4.10-4.07 (m, 1H), 3.27-3.23 (m, 1H), 2.74 (s, 3H), 2.41-2.33 (m, 1H), 2.07-2.01 (m, 1H), 1.68-1.63 (m, 3H), 1.42-1.33 (m, 1H).

Stereochemistry of Compounds **121*** and **122*** were arbitrarily assigned.

### Example 29: N-[5-(methylthio)-1,3,4-thiadiazol-2-yl]-2-(pyridin-2-ylmethyl)-1,2,3,4-tetrazole-5-carboxamide (Compound 133)

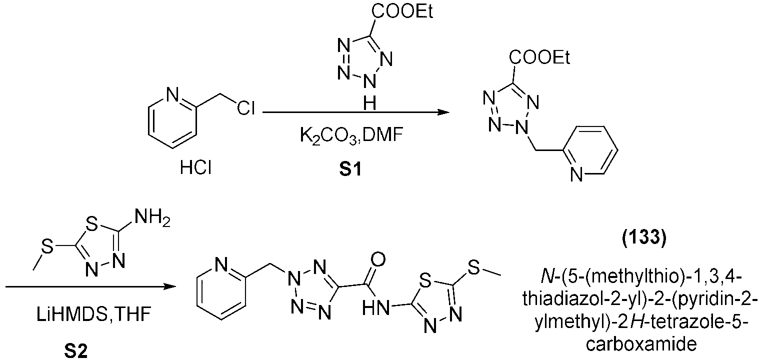

**Step 1:** Into a solution of ethyl 2*H*-1,2,3,4-tetrazole-5-carboxylate (500 mg, 3.52 mmol, 1.00 equiv) in N,N-dimethylformamide (DMF) (5 mL) was added 2-(chloromethyl)pyridine hydrochloride (1.10g, 6.79 mmol, 1.93 equiv) and K₂CO₃ (1459 mg, 10.5 mmol, 3.00 equiv) at room temperature. The resulting mixture was stirred for 5 h at 50 °C. The resulting mixture was diluted with water (30 mL) and extracted with ethyl acetate (EtOAc) (3 x 50 mL). The combined organic layers were washed with water (2 x 10 mL), dried over anhydrous Na₂SO₄. After filtration, the filtrate was concentrated under reduced pressure. The residue was purified by silica gel column chromatography (1:1 petroleum ether / ethyl acetate) to afford ethyl 2-(pyridin-2-ylmethyl) -1,2,3,4-tetrazole-5-carboxylate (340 mg, 40 % yield) as an off-white oil. LCMS (ES, *m*/*z*) = 234 [M+H]⁺.

**Step 2:** *N*-[5-(Methylthio)-1,3,4-thiadiazol-2-yl]-2-(pyridin-2-ylmethyl)-1,2,3,4-tetrazole-5-carboxamide was prepared from ethyl 2-(pyridin-2-ylmethyl)-1,2,3,4-tetrazole-5-carboxylate (1 equiv) and 5-(methylthio)-1,3,4-thiadiazol-2-amine (1.2 equiv) according to **Example 23 Step 2.** LCMS (ES, m/z) = 335.0 [M+H]⁺. ¹H NMR (400 MHz, DMSO-*d*₆) δ 8.70 (d, *J=* 2.4 Hz, 1H), 8.61 - 8.57 (m, 1H), 7.82 (dd, *J* = 8.0, 2.0 Hz, 1H), 7.43-7.40 (m, 1H), 6.03 (s, 2H), 2.63 (s, 3H).

### Example 30: N-(5-(methylthio)-1,3,4-thiadiazol-2-yl)-2-(pyridin-2-yl)-2H-tetrazole-5-carboxamide (Compound 136)

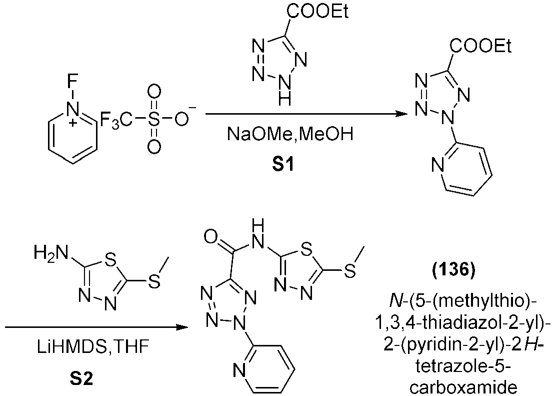

**Step 1:** Into a solution of ethyl 2*H*-1,2,3,4-tetrazole-5-carboxylate (300 mg, 2.11 mmol, 1.00 equiv) in methanol (MeOH) (15 mL) and sodium methoxide (NaOMe) (125 mg, 2.31 mmol, 1.10 equiv) at room temperature was added *N*-fluoropyridinium triflate (1043 mg, 4.22 mmol, 2.00 equiv) dropwise at -70 °C under nitrogen. The resulting mixture was stirred for overnight at room temperature under nitrogen. The reaction was quenched with sat. NH₄Cl (aq.) at 0 °C. The resulting mixture was extracted with ethyl acetate (EtOAc) (3 x 100 mL). The combined organic layers were washed with water (20 mL), dried over anhydrous Na₂SO₄. After filtration, the filtrate was concentrated under reduced pressure. The residue was purified by silica gel column chromatography (1:1 petroleum ether / EtOAc) to afford ethyl 2-(pyridin-2-yl)-1,2,3,4-tetrazole-5-carboxylate (70 mg, 15 % yield) as a white solid. LCMS (ES, *m*/*z*) = 220.0 [M+H]⁺.

**Step 2:** *N*-(5-(Methylthio)-1,3,4-thiadiazol-2-yl)-2-(pyridin-2-yl)-2*H*-tetrazole-5-carboxamide was prepared from ethyl 2-(pyridin-2-yl)-1,2,3,4-tetrazole-5-carboxylate (1 equiv) and 5-(methylthio)-1,3,4-thiadiazol-2-amine (1.2 equiv) according to **Example 23 Step 2.** LCMS (ES, *m*/*z*) = 321.0 [M+H]⁺. ¹H NMR (400 MHz, DMSO-*d*₆) δ 8.76 (d, *J* = 4.0 Hz, 1H), 8.28 - 8.22 (m, 2H), 7.78 - 7.76 (m, 1H), 2.77 (s, 3H).

### Example 31: N-(5-(methylthio)-1,3,4-thiadiazol-2-yl)-2-(pyridin-3-yl)-2H-tetrazole-5-carboxamide (Compound 137)

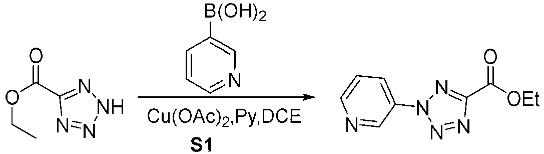

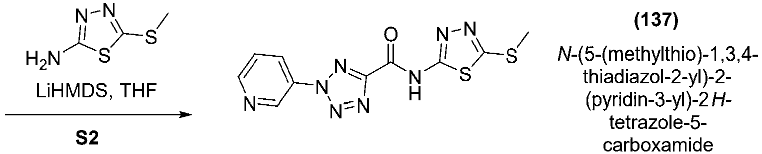

**Step 1:** To a stirred solution of ethyl 2*H*-tetrazole-5-carboxylate (300 mg, 2.11 mmol, 1.00 equiv) and pyridin-3-ylboronic acid (390 mg, 3.17 mmol, 1.50 equiv) in dichloroethane (DCE) (3 mL) were added pyridine (Py) (334 mg, 4.22 mmol, 2.00 equiv) and Cu(OAc)₂ (765 mg, 4.21 mmol, 2.00 equiv) at room temperature. The resulting mixture was stirred for 2 days at 50 °C under air atmosphere. The reaction was quenched with water (20 mL) at room temperature, extracted with ethyl acetate (EtOAc) (3 x 50 mL). The combined organic layers were washed with brine (3 x 10 mL), dried over anhydrous Na₂SO₄. After filtration, the filtrate was concentrated under reduced pressure, and then the residue was purified by C18 reverse flash chromatography to afford ethyl 2-(pyridin-3-yl)-2H-tetrazole-5-carboxylate (45 mg, 9 % yield) as a colorless oil. LCMS (ES, m/z) = 220.1 [M +H]⁺.

**Step 2:** *N*-(5-(Methylthio)-1,3,4-thiadiazol-2-yl)-2-(pyridin-3-yl)-2*H*-tetrazole-5-carboxamide was prepared from ethyl 2-(pyridin-3-yl)-1,2,3,4-tetrazole-5-carboxylate (1 equiv) and 5-(methylthio)-1,3,4-thiadiazol-2-amine (1.2 equiv) according to **Example 22.** LCMS (ES, m/z) = 321.1[M+H]⁺. ¹HNMR (400 MHz, DMSO-*d*₆) δ 9.38 (s, 1H), 8.86 (d, *J=* 4.8 Hz, 1H), 8.57 (d, J=8.4 Hz, 1H), 7.74 (dd, *J=* 8.4, 4.8 Hz, 1H), 2.63 (s, 3H).

### Example 33: 3-((5-(2-chlorophenyl)-1,3,4-thiadiazol-2-yl)carbamoyl)benzo[c]isoxazole-7-carboxylic acid (Compound 178)

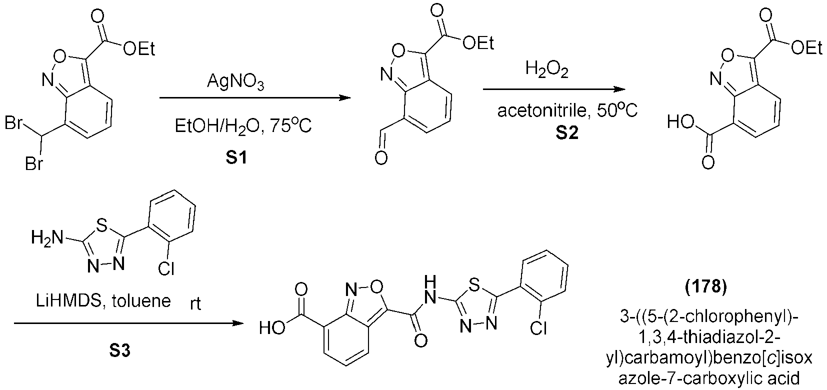

**Step 1:** Ethyl 7-(dibromomethyl)benzo[c]isoxazole-3-carboxylate (400 mg, 1.10 mmol) was dissolved in ethanol (EtOH) (8.3 mL) / water (2.75 mL) to which was added silver nitrate (416 mg, 2.42 mmol). The resulting solution was stirred at 75 °C for 2 hours. The solution was then filtered and the filtrate was concentrated to give ethyl 7-formylbenzo[*c*]isoxazole-3-carboxylate (250 mg, 100 % yield) which was used directly in the next step without further purification. LCMS (ES, *m*/*z*) = 220.1 [M+1]⁺.

**Step 2:** To a stirred solution of ethyl 7-formylbenzo[*c*]isoxazole-3-carboxylate (130 mg, 0.591 mmol) in acetonitrile (5.9 mL) was added hydrogen peroxide (2.74 mmol). The reaction mixture was slowly heated to 50 °C for 3 days until all aldehyde was consumed. Solvents were then removed under vacuum and the residue was purified by C18 reverse phase chromatography to give 3-(ethoxycarbonyl)benzo[c]isoxazole-7-carboxylic acid (70.0 mg, 50 % yield) as a yellow solid. LCMS (ES, *m*/*z*) = 236.1 [M+1]⁺.

**Step 3:** An oven-dried vial equipped with a stir bar was charged with 3-(ethoxycarbonyl)benzo[c]isoxazole-7-carboxylic acid (20.0 mg, 0.085 mmol) , 5-(2-chlorophenyl)-1,3,4-thiadiazol-2-amine (36.0 mg, 0.17 mmol) in dry toluene (850 µL) under argon. Lithium bis(trimethylsilyl)amide (LiHMDS) (340 uL, 0.34 mmol) was added slowly with vigorous stirring at room temperature, and the reaction mixture was stirred at room temperature until complete conversion. The reaction mixture was quenched with NH₄Cl and extracted with 2-methyl tetrahydrofuran (2-Me THF). The organic layer was washed with brine, dried over sodium sulfate and concentrated. The residue was purified by flash chromatography through silica gel followed by C18 reverse phase chromatography to provide 3-((5-(2-chlorophenyl)-1,3,4-thiadiazol-2-yl)carbamoyl)benzo[c]isoxazole-7-carboxylic acid (2.00 mg, 5.9 % yield). LCMS (ES, *m*/*z*) = 401.1 [M+1]⁺. ¹H NMR (400 MHz, DMSO-*d*₆): *δ*_{H} 8.56 (1H, s), 8.04 (2H, s), 7.59 (1H, s), 7.44-7.46 (2H, m), 7.27 (1H, s). [COOH and NH signals not observed].

### Example 34: N-(5-(2-chlorophenyl)-1,3,4-thiadiazol-2-yl)-3-(2-methoxyethoxy)isoxazole-5-carboxamide (Compound 175)

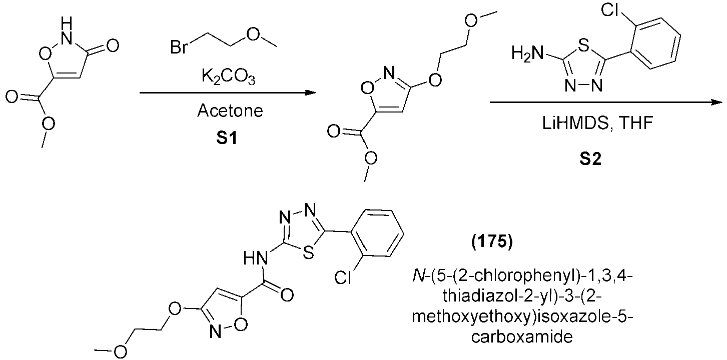

**Step 1:** Potassium carbonate (1.20 eq, 450 mg, 3.25 mmol) was added to a solution of methyl 3-hydroxyisoxazole-5-carboxylate (1.00 eq, 400 mg, 2.71 mmol) in acetone (6 mL) followed by the addition of 2-bromoethyl methyl ether (1.00 eq, 255 µL, 2.71 mmol). The reaction mixture was refluxed for 22 h. Acetone was then removed *in vacuo,* and the residue was taken up with water and extracted with ethyl acetate (20 x 2 mL). The combined organic extracts were dried and concentrated to dryness. The residue was purified by flash chromatography through silica gel (0 - 10% ethyl acetate (EtOAc) /hexanes) to give methyl 3-(2-methoxyethoxy)isoxazole-5-carboxylate (340 mg, 62 % yield) as a white solid. LCMS (ESI, m/z) = 202.1 [M+1]⁺. ¹H NMR (400 MHz, CDCl₃) δ 6.58 (s, 1H), 4.45 (t, *J =* 4.3Hz, 2H), 3.95 (s, 3H), 3.75 (t, *J =* 4.3Hz, 2H), 3.44 (s, 3H).

**Step 2:** Lithium bis(trimethylsilyl)amide (LiHMDS) was added to a suspension of methyl 3-(2-methoxyethoxy)isoxazole-5-carboxylate (1.00 eq, 50 mg, 0.249 mmol) and 5-(2-Chlorophenyl)-1,3,4-thiadiazol-2-amine (1.00 eq, 55 mg, 0.249 mmol) in tetrahydrofuran (THF) (2 mL). The reaction mixture was refluxed for 5 h. The reaction mixture was then concentrated to dryness and the residue was purified by flash chromatography through silica gel (0 - 10% MeOH/ dichloromethane (DCM)). Appropriate fractions combined and concentrated to dryness then the residue was dissolved in CH₃CN / H₂O and lyophilized to afford N-(5-(2-chlorophenyl)-1,3,4-thiadiazol-2-yl)-3-(2-methoxyethoxy)isoxazole-5-carboxamide (20 mg, 21 % yield) as a white solid. LCMS (ESI, m/z) = 381.1 [M+1]⁺. ¹H NMR (400 MHz, DMSO-*d*₆) δ 8.15 (d, *J=* 7.5Hz, 1H), 7.71 (d, *J* = 7.8Hz, 1H), 7.61 - 7.53 (m, 2H), 7.2 (s, 1H), 4.38 (t, *J*=4.1Hz, 2H), 3.69 (t, *J*=4.1Hz, 2H), 3.31 (s, 3H).

### Example 35: 5-(5-chlorothiophen-2-yl)-N-(5-(methylthio)-1,3,4-thiadiazol-2-yl)isoxazole-3-carboxamide (Compound 95)

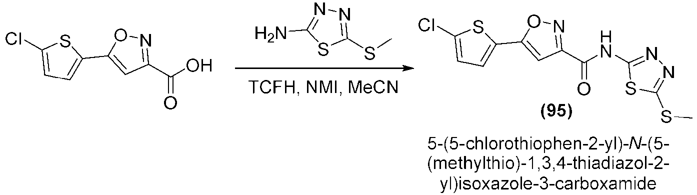

Into a solution of 5-(5-chlorothiophen-2-yl)-1,2-oxazole-3-carboxylic acid (200 mg, 0.871 mmol, 1.00 equiv) in acetonitrile (MeCN) (2 ml) was added N-methylimidazole (NMI) (143 mg, 1.742 mmol, 2.00 equiv) and chloro-N,N,N',N'-tetramethylformamidinium hexafluorophosphate (TCFH) (293 mg, 1.045 mmol, 1.20 equiv) at room temperature. The resulting mixture was stirred for 10 min at room temperature. To the above mixture was added 5-(methylsulfanyl)-1,3,4-thiadiazol-2-amine (141 mg, 0.958 mmol, 1.10 equiv). The resulting mixture was stirred for 2 hours at room temperature. The precipitated solids were collected by filtration and washed with acetonitrile (3 x 2 mL). The crude product (260 mg) was purified by Prep-HPLC (conditions: XBridge Prep OBD C18 Column, 30*150 mm, 5µm; Mobile Phase A: Water (10 mmol/L NH₄HCO₃), Mobile Phase B: acetonitrile (MeCN); Flow rate: 60 mL/min; Gradient: 15% B to 25% B in 8 min, 25% B; Wave Length: 254 nm) to afford 5-(5-chlorothiophen-2-yl)-*N*-[5-(methylsulfanyl)-1,3,4-thiadiazol-2-yl]-1,2-oxazole-3-carboxamide (154 mg, 49 % yield) as an off-white solid. LCMS (ES, m/z) = 359.0 [M+H]⁺. ¹H NMR (400 MHz, DMSO-*d*₆) δ 13.67 (br, 1H), 7.74 (d, *J* = 4.0Hz, 1H), 7.47 (s, 1H), 7.37 *(d, J=* 4.0 Hz, 1H), 2.76 (s, 3H).

### Example 36: N-(2-chlorophenyl)-5-oxo-4H-[1,2,3]triazolo[1,5-a]pyrimidine-3-carboxamide (Compound 97)

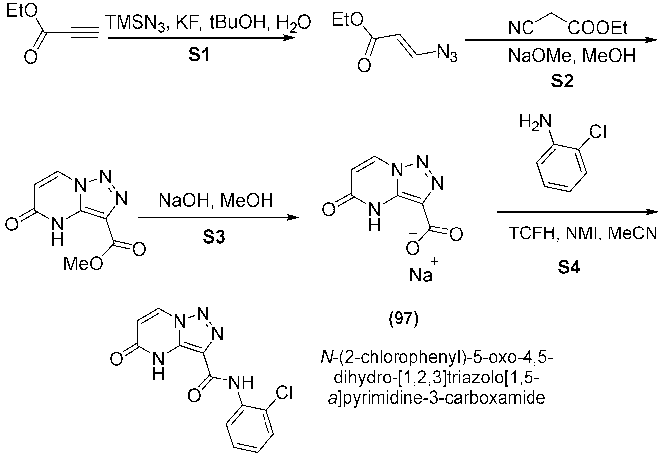

**Step 1:** Into a solution of ethyl propiolate (10 g, 102 mmol, 1.00 equiv) in *tert*-butanol (tBuOH) (200 mL) and water (20 mL) was added potassium fluoride (KF) (11.84 g, 203.8 mmol, 2.00 equiv) and azidotrimethylsilane (TMSN₃) (23.47 g, 203.7 mmol, 2.00 equiv). The mixture was stirred at room temperature for 3 hours. The reaction mixture was diluted with water (300 mL). The resulting solution was extracted with ethyl acetate (5 x 300 mL) and the organic layers were combined. The organic extract was dried over anhydrous sodium sulfate and concentrated under vacuum to afford ethyl (2*E*)-3-azidoprop-2-enoate (11.1 g, 77 % yield) as a yellow oil. LCMS (ES, m/z) = 142 [M+H]⁺.

**Step 2:** To a stirred solution of sodium methoxide (NaOMe) (30%) in methanol (MeOH) (165 mL), a solution of ethyl (2*E*)-3-azidoprop-2-enoate (11.1 g, 78.6 mmol, 1.00 equiv) and ethyl cyanoacetate (9.79 g, 86.5 mmol, 1.10 equiv) in methanol (110 mL) was added dropwise at room temperature. The reaction mixture was stirred at room temperature for 0.5 hours. The precipitated solids were then collected by filtration and washed with methanol (10 x 50 mL) and dried under vacuum to afford methyl 5-oxo-4*H* [1,2,3]triazolo[1,5-a]pyrimidine-3-carboxylate (2.0 g, 6.5 % yield) as a light yellow solid. LCMS (ES, m/z) = 195 [M+H]⁺.

**Step 3:** Into a solution of methyl 5-oxo-4*H*-[1,2,3]triazolo[1,5-a]pyrimidine-3-carboxylate (30 g, 154 mmol, 1.00 equiv) in methanol (MeOH) (500 mL) was added sodium hydroxide (NaOH) (aq, 150 mL, 10 M). The mixture was stirred at room temperature for 0.5 hours. The precipitated solids were then collected by filtration and washed with methanol (5 x 50 mL) to afford crude product. The crude product was diluted with methanol and water (20/1) (600 mL) overnight. Then the solid were collected by filtration, washed with methanol (10 x 20 mL) and dried under vacuum to afford sodium 5-oxo-4,5-dihydro-[1,2,3]triazolo[1,5-a]pyrimidine-3-carboxylate (20.8 g, 75 % yield) as a light yellow solid. LCMS (ES, m/z) = 181.0 [M+H]⁺. ¹H NMR (400 MHz, DMSO-*d*₆) δ 7.90 (d, *J* = 5.6 Hz, 1H), 5.55 (d, *J* = 5.6 Hz, 1H).

**Step 4:** *N*-(2-Chlorophenyl)-5-oxo-4*H*-[1,2,3]triazolo[1,5-a]pyrimidine-3-carboxamide was prepared from sodium 5-oxo-4,5-dihydro-[1,2,3]triazolo[1,5-a]pyrimidine-3-carboxylate (1.5 eq) and 2-chloroaniline (1 eq) according to **Example 35.** LCMS (ES, m/z) = 290.0 [M+H]⁺. ¹H NMR (400 MHz, DMSO-*d*₆) δ 10.80 (s, 1H), 8.56 (d, *J =* 8.4, 1H), 7.99 (d, *J =* 5.6 Hz, 1H), 7.53 (d, *J =* 8.0, 1H), 7.40 - 7.31 (m, 1H), 7.13-7.09 (m, 1H), 5.63 (d, *J* = 6.0 Hz, 1H).

### Example 37: N-(5-(thiazol-4-yl)-1,3,4-thiadiazol-2-yl)benzo[c]isoxazole-3-carboxamide (Compound 127)

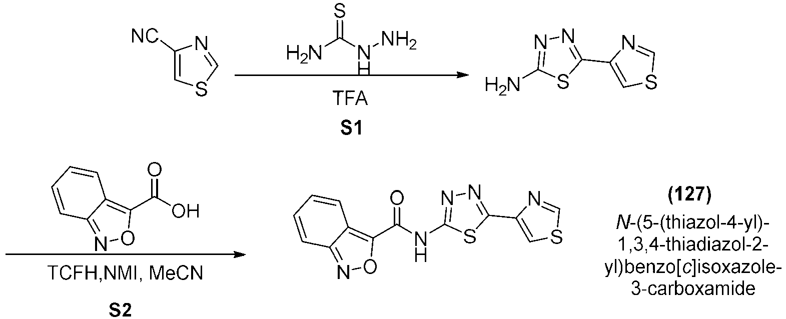

**Step 1:** To a stirred solution of thiazole-4-carbonitrile (400 mg, 3.632 mmol, 1 equiv) in trifluoroacetic acid (TFA) (4 mL) was added hydrazinecarbothioamide (364 mg, 3.99 mmol, 1.10 equiv) at room temperature. The resulting mixture was stirred for 2 h at 80 °C. The resulting solution was concentrated under vacuum, and then the residue was diluted with water (15 mL), and then pH of the mixture was adjusted to 10 with NaOH (2M). The precipitated solids were collected by filtration to afford 5-(thiazol-4-yl)-1,3,4-thiadiazol-2-amine (500 mg) as a yellow solid. LCMS (ES, m/z) = 185 [M+H]⁺.

**Step 2:** *N*-(5-(Thiazol-4-yl)-1,3,4-thiadiazol-2-yl)benzo[c]isoxazole-3-carboxamide was prepared from benzo[c]isoxazole-3-carboxylic acid (1.0 eq) and 5-(thiazol-4-yl)-1,3,4-thiadiazol-2-amine (1.0 eq) according to **Example 35.** LCMS (ES, m/z) = 330.1[M+H]⁺. ¹HNMR(400 MHz, DMSO-*d*₆) δ 14.71 (br, 1H), 9.35 (s, 1H), 8.55 (s, 1H), 8.14 (d, *J* = 8.0 Hz, 1H), 7.85 (d, *J =* 9.2 Hz, 1H), 7.58-7.54 (m, 1H), 7.41-7.37 (m, 1H).

### Example 38: N-(5-(pyrimidin-2-yl)-1,3,4-thiadiazol-2-yl)benzo[c]isoxazole-3-carboxamide (Compound 148)

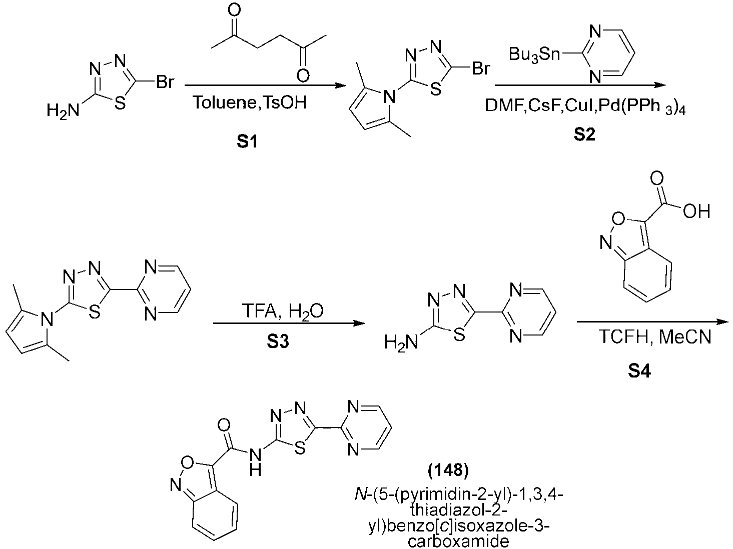

**Step 1:** To a solution of 5-bromo-1,3,4-thiadiazol-2-amine (2.00 g, 11.1 mmol, 1.0 equiv) and 2,5-hexanedione (1.27 g, 11.1 mmol, 1 equiv) in Toluene (10 mL) was added *p*-toluene sulfonic acid monohydrate (TsOH) (0.57 g, 3.33 mmol, 0.3 equiv). The mixture stirred at 110 °C for 2 h. The resulting mixture was concentrated under reduced pressure and the crude residue was purified by flash chromatography through silica gel (5:1 petroleum ether / ethyl acetate) to afford 2-bromo-5-(2,5-dimethylpyrrol-1-yl)-1,3,4-thiadiazole (1.5 g, 37 % yield) as a brown solid. LCMS (ES, m/z) = 258.0 [M+1]⁺.

**Step 2:** To a solution of 2-bromo-5-(2,5-dimethylpyrrol-1-yl)-1,3,4-thiadiazole (200 mg, 0.775 mmol, 1 equiv) in N,N-dimethylformamide (DMF) (1 mL) was added CsF (353 mg, 2.32 mmol, 3 equiv), tetrakis(triphenylphosphine)palladium (Pd(PPh₃)₄) (89.5 mg, 0.078 mmol, 0.1 equiv), CuI (14.8 mg, 0.078 mmol, 0.1 equiv) and 2-(tributylstannyl)pyrimidine(286 mg, 0.775 mmol, 1 equiv). The mixture was stirred at 80°C under nitrogen atmosphere. After 2 h, the reaction was quenched by the addition of water (10 mL) at room temperature. The aqueous layer was extracted with ethyl acetate (EtOAc) (3 x 10 mL) and concentrated under reduced pressure. The residue was purified by flash chromatography through silica gel (5:1 petroleum ether / ethyl acetate) to afford 2-[5-(2,5-dimethylpyrrol-1-yl)-1,3,4-thiadiazol-2-yl]pyrimidine (160 mg, 56 % yield) as a brown liquid. LCMS (ES, m/z) = 258.1 [M+1]⁺.

**Step 3:** To a solution of trifluoroacetic acid (TFA) (1.00 mL) and H₂O (1.00 mL) was added 2-[5-(2,5-dimethylpyrrol-1-yl)-1,3,4-thiadiazol-2-yl]pyrimidine (160 mg, 0.622 mmol, 1 equiv). The mixture was stirred at rt for 2 h. The resulting mixture was then concentrated under reduced pressure. The mixture was basified to pH 10 with saturated NaHCO₃ (aq.). The resulting mixture was extracted with ethyl acetate (EtOAc) (3 x 10 mL), and dried over anhydrous Na₂SO₄. After filtration, the filtrate was concentrated under reduced pressure. The crude product was purified by Prep-HPLC (conditions: Xselect CSH C18 OBD Column 30*150mm 5um, n; mobile phase, acetonitrile (MeCN) and water (0.05% trifluoroacetic acid (TFA)), Gradient: 3% water (0.05% TFA) up to 10% water (0.05%TFA) in 8 min; Detector, UV 254 nm) to afford 5-(pyrimidin-2-yl)-1,3,4-thiadiazol-2-amine (60 mg, 86 % yield) as an off-white solid. LCMS (ES, m/z) = 180.1 [M+1]⁺.

**Step 4:** The trifluoroacetic acid (TFA) salt of N-(5-(pyrimidin-2-yl)-1,3,4-thiadiazol-2-yl)benzo[c]isoxazole-3-carboxamide was prepared from 5-(pyrimidin-2-yl)-1,3,4-thiadiazol-2-amine (1 equiv) and benzo[c]isoxazole-3-carboxylic acid (1.2 equiv) according to **Example 35.** LCMS (ES, m/z) = 325.0[M+1]⁺. ¹H NMR (400 MHz, DMSO-*d*₆) δ 9.02 (d, *J =* 4.9 Hz, 2H), 8.15 (d, *J =* 8.8 Hz, 1H), 7.84 (d, *J =* 8.8 Hz, 1H), 7.67-7.62 (m, 1H), 7.57-7.55 (m, 1H), 7.40-7.38 (m, 1H).

### Example 39A: N-(5-(methylthio)-1,3,4-thiadiazol-2-yl)-5-(phenylamino)benzo[c]isoxazole-3-carboxamide (Compound 162)

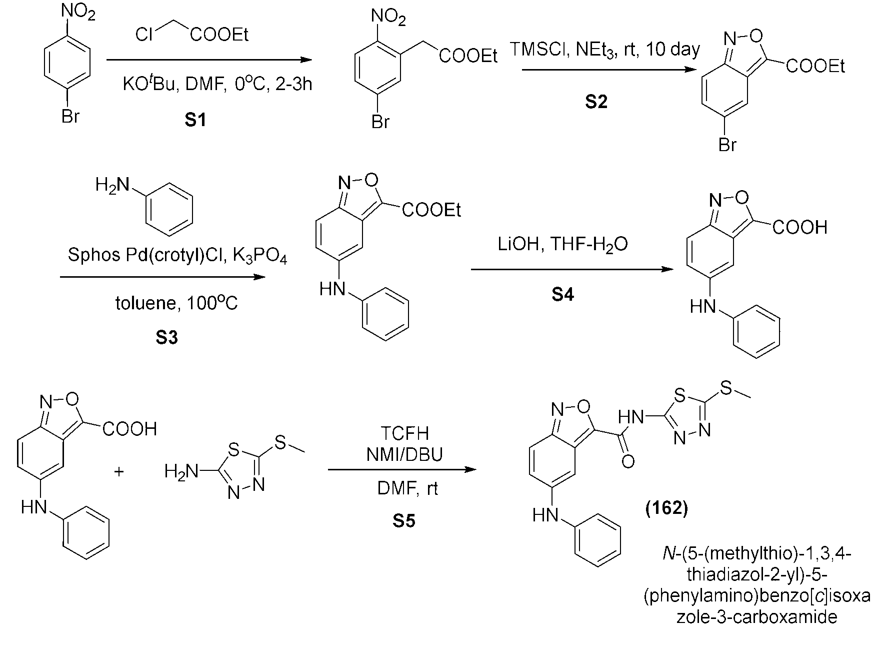

**Step 1:** Potassium t-butoxide (2.78 g, 24.8 mmol) was suspended in N,N-dimethylformamide (DMF) (20 mL) and cooled in an ice-water bath. A solution of 1-bromo-4-nitrobenzene (2.00 g, 9.90 mmol) and ethylchloroacetate (1.06 mL, 9.90 mmol) in DMF (9.9 mL) was then added dropwise under argon. The resulting purple solution was stirred at 0 °C for 2.5 h. Upon completion, 3N HCl (9.6 mL, 28.7 mmol) was added dropwise. The resulting solution was diluted with water and extracted with ethyl acetate (EtOAc). Organic extract was combined, washed with brine, dried over sodium sulfate and concentrated to give ethyl 2-(5-bromo-2-nitrophenyl)acetate (1.90 g, 67 % yield) as an oil. LCMS (ES, *m*/*z)* = 288.0/ 290.0[M+1]⁺.

**Step 2:** 2-(5-Methyl-2-nitrophenyl)acetic acid (1.50 g, 5.21 mmol) was dissolved in N,N-dimethylformamide (DMF) (26 mL) and treated with triethylamine (3.63 mL, 26.0 mmol) and chlorotrimethylsilane (TMSCl) (3.30 mL, 26.0 mmol) sequentially. The resulting mixture was stirred at rt for 5 days. The reaction mixture was then poured into ice cold diluted HCl and extracted with ethyl acetate (EtOAc). Organic phase was collected, concentrated and purified by flash chromatography through silica gel to give ethyl 5-bromobenzo[c]isoxazole-3-carboxylate (0.80 g, 57 % yield). LCMS (ES, *m*/*z*) = 269.9/271.9 [M+1]⁺.

**Step 3:** Ethyl 5-bromobenzo[c]isoxazole-3-carboxylate (60.0 mg, 0.222 mmol), aniline (24.8 mg, 0.267 mmol), potassium phosphate,tribasic (94.3 mg, 0.444 mmol) were mixed in toluene (2.2 mL). The mixture was degassed with argon before addition of chloro(crotyl)(2-dicyclohexylphosphino-2',6'-dimethoxybiphenyl)palladium(II) (13.5mg, 0.022mmol) and was heated at 110°C until complete consumption of starting material. Upon completion, the reaction mixture was cooled down and diluted with ethyl acetate (EtOAc), washed with water, brine, dried over sodium sulfate and concentrated. The residue was purified by flash chromatography through silica gel (0 - 40% EtOAc/Hexanes) to give ethyl 5-(phenylamino)benzo[c]isoxazole-3-carboxylate (30.0 mg, 48 % yield). LCMS (ES, *m*/*z*) = 283.1 [M+1]⁺.

**Step 4:** Ethyl 5-(phenylamino)benzo[c]isoxazole-3-carboxylate (50.0 mg, 0.177 mmol) was dissolved in tetrahydrofuran (THF) (886 µL)/H₂O (886 µL) and 1N lithium hydroxide (LiOH/THF) solution (213 µL, 0.213 mmol) was added. The solution was stirred at rt until complete conversion of starting material. Amberlite-120[H] was then added to acidify the reaction mixture and was then removed by filtration. The filtrate was concentrated to provide 5-(phenylamino)benzo[c]isoxazole-3-carboxylic acid (48.0 mg, 100 % yield) which was used directly in the next step without further purification. LCMS (ES, m/z) = 255.0 [M+1]⁺.

**Step 5:** 5-(Phenylamino)benzo[c]isoxazole-3-carboxylic acid (50.0 mg, 0.197 mmol), 1-methylimidazole (54.9 µL, 0.688 mmol), 2-amino-5-(methylthio)-1,3,4-thiadiazole (43.4 mg, 0.295 mmol) and 1,8-diazabicyclo[5.4.0]undec-7-ene (29.9 mg, 0.197 mmol) were dissolved in anhydrous N,N-dimethylformamide (DMF) (1.97 mL). chloro-N,N,N',N'-tetramethylformamidinium hexafluorophosphate (TCFH) (548 mg, 0.216 mmol) was added. The reaction mixture was stirred at rt until complete conversion. The reaction mixture was diluted with ethyl acetate (EtOAc), washed with water, brine, dried over sodium sulfate, and concentrated. The residue was purified by flash chromatography through silica gel to provide N-(5-(methylthio)-1,3,4-thiadiazol-2-yl)-5-(phenylamino)benzo[c]isoxazole-3-carboxamide (60.0 mg, 80 % yield) as an orange powder. LCMS (ES, *m*/*z*) = 384.1 [M+1]⁺. ¹H NMR (400 MHz, CHCl₃-*d*): *δ*_{H} 10.11 (1H, s), 7.67 (1H, d, *J =* 9.5 Hz), 7.40 (2H, t, *J =* 7.7 Hz), 7.35 (1H, s), 7.23 (2H, d, *J* = 7.9 Hz), 7.14 (2H, t, *J* = 8.2 Hz), 6.08 (1H, s), 2.79 (3H, s).

### Example 39B: N-(5-(2-chlorophenyl)-1,3,4-thiadiazol-2-yl)-3-(2-methoxyethyl)isoxazole-5-carboxamide (Compound 169)

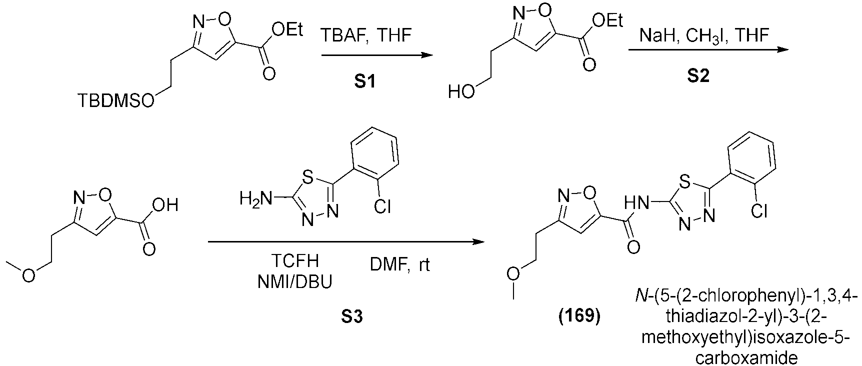

**Step 1:** To a solution of ethyl 3-(2-((*tert*-butyldimethylsilyl)oxy)ethyl)isoxazole-5-carboxylate (1.11 g, 3.67 mmol) in tetrahydrofuran (THF) (12 mL) at 10 °C was added tetra-n-butylammonioum fluoride (TBAF) (5.51 mL, 5.51 mmol) and the mixture was stirred at room temperature for 1 h. The mixture was quenched with water and subsequently extracted with ethyl acetate (EtOAc). The combined organic layers were dried over sodium sulfate and concentrated. The residue was purified by flash chromatography through silica gel (0 - 40% EtOAc/Hexane) to give ethyl 3-(2-hydroxyethyl)isoxazole-5-carboxylate (410 mg, 60 % yield) as a colorless oil. LCMS (ES, *m*/*z*) = 186.1 [M+1]⁺.

**Step 2:** Ethyl 3-(2-hydroxyethyl)isoxazole-5-carboxylate (100 mg, 0.540 mmol) was dissolved in tetrahydrofuran (THF) (5.4 mL). The solution was cooled down with an ice-water bath and sodium hydride (25.9 mg of 60% oil disp., 0.648 mmol) was added. After stirring for 10 min, methyl iodide (50.4 µL, 0.810 mmol) was added. The resulting mixture was warmed up to rt and kept stirring at rt overnight. NaH (2 eq) and CH₃I (4 eq) were supplemented and stirring at rt was continued for another 1 h. Amberlite IR-120 [H] was then added to acidify the reaction mixture. Resin was removed by filtration and the filtrate was concentrated to give a crude product of 3-(2-methoxyethyl)isoxazole-5-carboxylic acid (9 0mg, 78 % yield) which was used in the next step without further purification. LCMS (ES, *m*/*z*) = 172.1 [M+1]⁺.

**Step 3:** *N*-(5-(2-Chlorophenyl)-1,3,4-thiadiazol-2-yl)-3-(2-methoxyethyl)isoxazole-5-carboxamide was prepared using 3-(2-methoxyethyl)isoxazole-5-carboxylic acid following step 5 of **Example 39A.** LCMS (ES, *m*/*z*) = 365.1[M+1]⁺. ¹H NMR (400 MHz, DMSO-*d*₆) δ 8.12 (1H, d, *J* = 7.4 Hz), 7.69 (1H, d, *J =* 7.8 Hz), 7.51-7.59 (2H, m), 7.43 (1H, s), 3.64 (2H, t, *J* = 6.1 Hz), 3.27 (3H, s), 2.96 (2H, t, *J =* 6.1 Hz). [NH signal not observed].

### Example 40: N-(5-(2-chlorophenyl)-1,3,4-thiadiazol-2-yl)-3-(methoxymethyl)isoxazole-5-carboxamide (Compound 170)

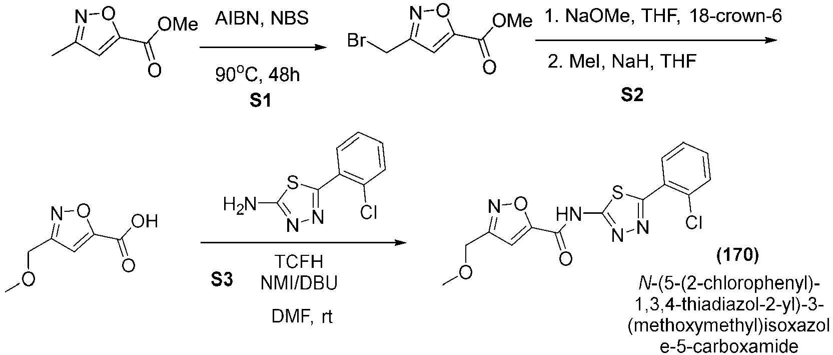

**Step 1**:To a solution of methyl 3-methylisoxazole-5-carboxylate (1.00 g, 7.09 mmol) in carbon tetrachloride (35 mL) was added N-bromosuccinimide (NBS) (1.51 g, 8.50 mmol) and 2,2'-azobis(2-methylpropionitrile) (AIBN) (175 mg, 1.06 mmol) at room temperature. The resulting mixture was stirred at 90 °C. After 2 days the reaction mixture was cooled to rt, filtered through celite and the filtrate was concentrated under reduced pressure. The residue was purified by flash chromatography through silica gel (0 - 10% ethyl acetate (EtOAc) /Hexane) to afford methyl 3-(bromomethyl)isoxazole-5-carboxylate (0.51 g, 33 % yield) as a white solid. LCMS (ES, *m*/*z)* = 219.9/221.9[M+1]⁺.

Step 2: Methyl 3-(bromomethyl)isoxazole-5-carboxylate (100 mg, 0.455 mmol) and 18-Crown-6 (36.0 mg, 0.136 mmol) were dissolved in tetrahydrofuran (THF) (1.5 mL). Sodium methoxide (NaOMe) (31.2 µL, 0.545 mmol) was then added and the reaction mixture was warmed up to rt and kept stirring overnight. NaOMe (1 eq) and 18-crown-6 (1 eq) were supplemented and the reaction was heated at 65 °C for 1 h. The reaction mixture was then cooled to rt and acidified with Amberlite IR-120 [H]. The resin was filtered off and the filtrate was concentrated. The residue was taken in anhydrous THF, cooled down with an ice-water bath. Sodium hydride (NaH) (36.4 mg, 0.909 mmol) was added. After stirring at 0 °C for 5 mins, CH₃I (141 µL, 2.27 mmol) was added and the mixture was stirred at rt overnight before acidification with Amberlite IR-120[H]. The resin was filtered off and the filtrate was concentrated and the residue was purified by C18 reverse phase chromatography to give 3-(methoxymethyl)isoxazole-5-carboxylic acid (35 mg, 49 % yield). LCMS (ES, *m*/*z*) = 158.1 [M+1]⁺.

**Step 3:** *N*-(5-(2-Chlorophenyl)-1,3,4-thiadiazol-2-yl)-3-(methoxymethyl)isoxazole-5-carboxamide was prepared using 3-(methoxymethyl)isoxazole-5-carboxylic acid following step 5 of **Example 39A.** LCMS (ES, *m*/*z*) = 351.0 [M+1]⁺. ¹H NMR (400 MHz, DMSO-*d*₆) δ 8.13 (1H, d, *J =* 7.4 Hz), 7.69 (1H, d, *J* = 7.7 Hz), 7.49-7.58 (3H, m), 4.58 (2H, s), 3.33 (3H, s). [NH signal not observed].

### Example 41: N-(5-(methylthio)-1,3,4-thiadiazol-2-yl)-4,5-dihydroisoxazolo[4,5-h]quinoline-3-carboxamide (Compound 180)

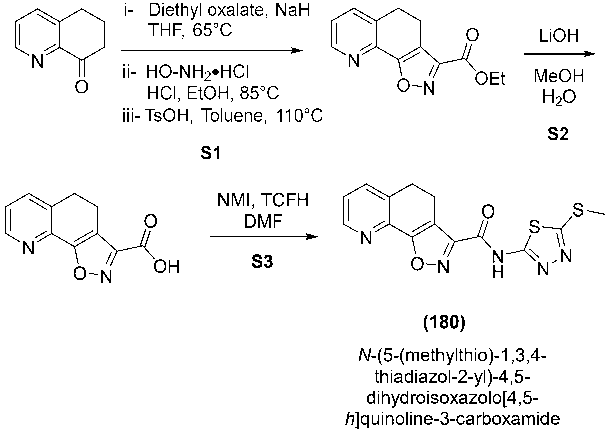

**Step 1:** Sodium hydride (544 mg, 13.6 mmol) was added portion wise to a solution of 7,8-dihydroquinolin-5(6H)-one (1.00 g, 6.79 mmol) in tetrahydrofuran (THF) (50 mL), and the resulting mixture was refluxed for 15 minutes. Diethyl oxalate (969 µL, 7.13 mmol) was added, and the reaction was refluxed for 1 hour. Upon completion of the alkylation, 30 mL of conc. HCl in ethanol (EtOH) (1:9) was added, followed by hydroxylamine hydrochloride (496 mg, 7.13 mmol). The mixture was refluxed for 1 h until full formation of the oxime. Solvents were removed *in vacuo* and the resulting residue was suspended in 100 mL toluene. *p*-Toluene sulfonic acid monohydrate (TsOH) (1.29 g, 6.79 mmol) was added, and the mixture was refluxed for 40 h. Toluene was removed *in vacuo* and the crude mixture was taken in 100 mL MeOH and stirred for 1 h. Residual solids were removed by filtration and the filtrate was concentrated. The crude material was purified by C18 reverse phase chromatography (5-55% MeOH/water + 0.1% HCl) to yield ethyl 4,5-dihydroisoxazolo[4,5-h]quinoline-3-carboxylate (102 mg, 6.1 % yield). ¹H NMR (DMSO-d₆, 400 MHz): δ 8.50 (1H, d, *J* = 4.8 Hz), 7.81 (1H, d, *J* = 7.7 Hz), 7.40 (1H, dd, *J* = 7.7*,* 4.8 Hz), 4.38 (2H, q, *J* = 7.1 Hz), 3.12 (2H, t, *J =* 7.9 Hz), 2.97 (2H, t, *J =* 7.9 Hz), 1.33 (3H, t, *J =* 7.1 Hz).

**Step 2:** Ethyl 4,5-dihydroisoxazolo[4,5-h]quinoline-3-carboxylate was solubilized in MeOH (4 mL) and water (1 mL). LiOH (100 µL, 3N in water) was added and the reaction mixture was stirred for 1 h. Upon completion, the mixture was quenched with sat. aq. NH₄Cl, treated with DOWEX-120 resin until acidic, filtered and lyophilized. The resulting compound was purified by C18 reverse phase chromatography (10-55% MeCN/water + 0.1% HCl) to give 4,5-dihydroisoxazolo[4,5-h]quinoline-3-carboxylic acid (8.0 mg, 18 % yield). ¹H NMR (DMSO-d₆, 400 MHz): δ 8.49 (1H, d, *J =* 4.8 Hz), 7.80 (1H, d*, J =* 7.7 Hz), 7.39 (1H, dd, *J* = 7.7, 4.9 Hz), 3.10 (2H, *t, J =* 8.0 Hz), 2.96 (2H, *t, J* = 7*.*9 Hz).

**Step 3:** 4,5-Dihydroisoxazolo[4,5-h]quinoline-3-carboxylic acid (8.00 mg, 37 µmol), 2-amino-5-(methylthio)-1,3,4-thiadiazole (8.34 mg, 56 µmol), chloro-N,N,N',N'-tetramethylformamidinium hexafluorophosphate (TCFH) (11.4 mg, 41 µmol) and N-methylimidazole (NMI) (10 µL, 130 umol) were stirred in N,N-dimethylformamide (DMF) (500 µL) at room temperature. After 2 h, NaOH (2N aqueous solution, 0.25 mL, 0.5 mmol) was added, and the mixture was stirred at room temperature. After 1 h, the reaction mixture was directly purified by C18 reverse phase chromatography (10-55% MeCN/water + 0.1% formic acid) to afford N-(5-(methylthio)-1,3,4-thiadiazol-2-yl)-4,5-dihydroisoxazolo[4,5-h]quinoline-3-carboxamide (2.5 mg, 20 % yield). LCMS (ES, *m*/*z*) = 346.1 [M+1]⁺. ¹H NMR (DMSO-d₆, 400 MHz): δ 8.50 (1H, d, *J =* 4.9 Hz), 7.81 (1H, *d, J =* 7.7 Hz), 7.40 (1H, dd, *J =* 7.7, 4.8 Hz), 3.12 (2H, t, *J =* 7.7 Hz), 3.02 (2H, *t, J =* 7.8 Hz), 2.71 (3H, s).

### Example 42: N-(5-(methylthio)-1,3,4-thiadiazol-2-yl)-5-(phenylthio)benzoiclisoxazole-3-carboxamide (Compound 163)

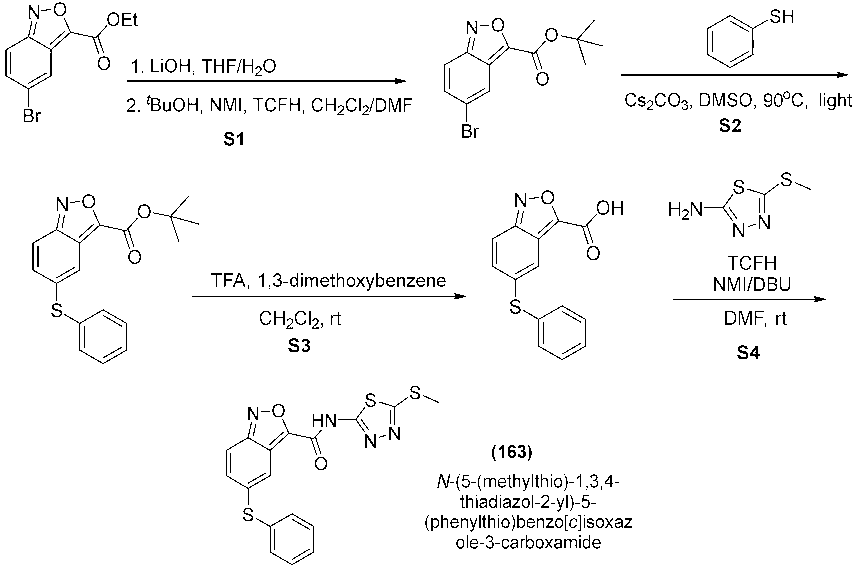

**Step 1:** Ethyl 5-bromobenzo[c]isoxazole-3-carboxylate (550 mg, 2.04 mmol) prepared in **Example 39A** was dissolved in tetrahydrofuran (THF) (10 mL)/H₂O (10 mL) and 1N lithium hydroxide (2.44 mL, 2.44 mmol) was added. The solution was kept stirring at rt until complete conversion of starting material. Amberlite-120[H] was then added to acidify the reaction mixture and was then removed by filtration. The filtrate was concentrated. The residue was taken up in anhydrous CH₂Cl₂ (17 mL), and added with *t*-butanol (296 µL, 3.10 mmol) and triethylamine (288 µL, 2.07 mmol). The solution was cooled down with an ice-water bath. 1-Methylimidazole (576 µL, 7.23 mmol) and chloro-N,N,N',N'-tetramethylformamidinium hexafluorophosphate (TCFH) (603 mg, 2.27 mmol) were added sequentially. The reaction mixture was allowed to warm up to room temperature. N,N-dimethylformamide (DMF) (3.4 mL) was added to provide a homogeneous mixture. The resulting solution was kept stirring overnight. The reaction mixture was then diluted with ethyl acetate (EtOAc), washed with water, brine, dried over sodium sulfate and concentrated. The residue was purified by flash chromatography through silica gel to provide *tert-*butyl 5-bromobenzo[c]isoxazole-3-carboxylate (440 mg, 71 % yield) as a light yellow solid. LCMS (ES, *m*/*z*) = 298.0/300.0 [M+1]⁺.

**Step 2:** *tert*-Butyl 5-bromobenzo[c]isoxazole-3-carboxylate (200 mg, 0.671 mmol), thiophenol (82.7 µL, 0.805 mmol), and cesium carbonate (262 mg, 0.805 mmol) were mixed in dimethylsulfoxide (DMSO) (6.7 mL). The mixture was stirred at rt for 10 min and then heated at 90 °C under light for 1 h. Upon completion, the reaction mixture was cooled down before being diluted with ethyl acetate (EtOAc). The organic phase was washed with water, then brine, and dried over sodium sulfate. Solvents were then removed and the residue was purified by flash chromatography through silica gel to give *tert*-butyl 5-(phenylthio)benzo[c]isoxazole-3-carboxylate (190 mg, 87 % yield). LCMS (ES, *m*/*z*) = 328.1 [M+1]⁺.

**Step 3:** *tert*-Butyl 5-(phenylthio)benzo[c]isoxazole-3-carboxylate (60.0 mg, 0.183 mmol) and 1,3-dimethoxybenzene (48.0 µL, 0.366 mmol) were dissolved in CH₂Cl₂ (1.7 mL) and trifluoroacetic acid (TFA) (167 µL) was added dropwise. The solution was kept stirring at rt until completion. Solvents were then removed and the resulting residue was purified by flash chromatography through silica gel to provide 5-(phenylthio)benzo[c]isoxazole-3-carboxylic acid. (50.0 mg, 100% yield). LCMS (ES, *m*/*z*) = 272.0 [M+1]⁺.

**Step 4:** *N-*(5-(Methylthio)-1,3,4-thiadiazol-2-yl)-5-(phenylthio)benzo[c]isoxazole-3-carboxamide was prepared using 5-(phenylthio)benzo[c]isoxazole-3-carboxylic acid following step 5 of **Example 39A.** LCMS (ES, *m*/*z*) = 401.1 [M+1]⁺. ¹H NMR (400 MHz, DMSO-*d*₆) δ 7.81 (1H, d, *J* = 9.5 Hz), 7.44-7.49 (4H, m), 7.32 (1H, d, *J* = 9.4 Hz), 2.72 (3H, s). [NH signal not observed].

### Example 43: N-(5-(methylthio)-1,3,4-thiadiazol-2-yl)-5-(phenylsulfonyl)benzo[c]isoxazole-3 carboxamide (Compound 164)

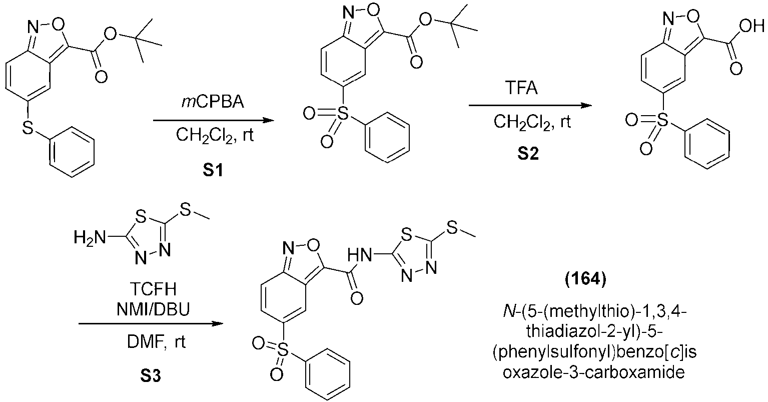

**Step 1:** *tert*-Butyl 5-(phenylthio)benzo[*c*]isoxazole-3-carboxylate (130 mg, 0.397 mmol) prepared in **Example 42** was dissolved in CH₂Cl₂ (4 mL) and 3-chloroperoxybenzoic acid (mCPBA) (151 mg, 0.874 mmol) was added slowly. The resulting solution was stirred at rt. Upon completion, the reaction mixture was diluted with ethyl acetate (EtOAc), washed with 10% Na₂S₂O₃, water, brine, dried over sodium sulfate and concentrated. The residue was purified by flash chromatography through silica gel (0 - 30% EtOAc/Hexanes) to provide *tert*-butyl 5-(phenylsulfonyl)benzo[*c*]isoxazole-3-carboxylate (140 mg, 98 % yield). LCMS (ES, *m*/*z*) = 360.1 [M+1]⁺.

**Step 2:** *tert*-Butyl 5-(phenylsulfonyl)benzo[*c*]isoxazole-3-carboxylate (60.0 mg, 0.167 mmol) was dissolved in CH₂Cl₂ (1.5 mL) and trifluoroacetic acid (TFA) (152 µL) was added dropwise. The solution was stirred at rt until completion. Solvents were then removed to provide 5-(phenylsulfonyl)benzo[c]isoxazole-3-carboxylic acid as a white solid (quant. yield) which was used directly in the next step without further purification. LCMS (ES, *m*/*z*) = 304.0 [M+1]⁺.

**Step 3:** *N*-(5-(Methylthio)-1,3,4-thiadiazol-2-yl)-5-(phenylsulfonyl)benzo[c]isoxazole-3 carboxamide was prepared using 5-(phenylsulfonyl)benzo[c]isoxazole-3-carboxylic acid following step **5** of **Example 39A.** LCMS (ES, *m*/*z*) = 433.1 [M+1]⁺. ¹H NMR (400 MHz, DMSO-*d*₆) δ 8.87 (1H, s), 8.03 (2H, d*, J =* 7.7 Hz), 7.94 (1H, d, *J =* 9.4 Hz), 7.70-7.77 (2H, m), 7.65 (2H, t, *J =* 7.5 Hz), 2.72(3H, s). [NH signal not observed].

### Example 44: N-(5-(methylthio)-1,3,4-thiadiazol-2-yl)-5-phenoxybenzoiclisoxazole-3-carboxamide (Compound 165)

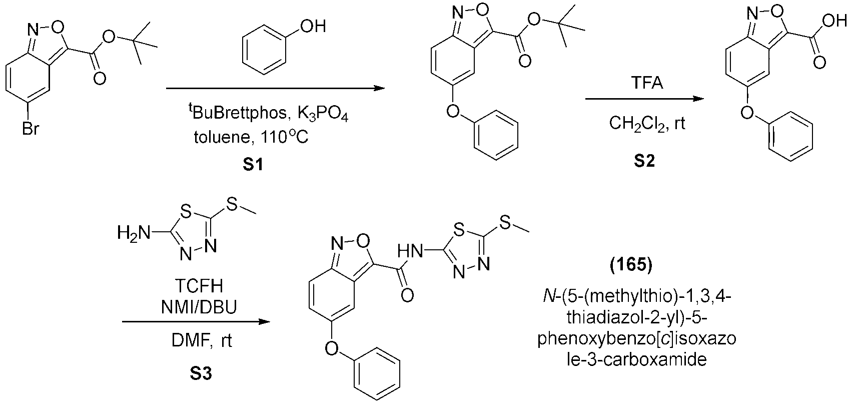

**Step 1:** *tert*-Butyl 5-bromobenzo[c]isoxazole-3-carboxylate (200 mg, 0.671 mmol) prepared in **Example 39A,** phenol (94.7 mg, 1.01 mmol), and potassium phosphate, tribasic (285 mg, 1.34 mmol) were mixed in toluene (3.35 mL). The mixture was degassed with argon before addition of [tBuBrettPhos Pd(allyl)]OTf (107 mg, 0.134 mmol) and heated at 110 °C for 3 h. The reaction mixture was allowed to cool and diluted with ethyl acetate (EtOAc). The organic phase was washed with water, brine, and dried over sodium sulfate. Solvents were then removed and the residue was purified by flash chromatography through silica gel to give *tert*-butyl 5-phenoxybenzo[c]isoxazole-3-carboxylate (50.0 mg, 24 % yield). LCMS (ES, *m*/*z*) = 312.1 [M+1]⁺.

**Step 2:** *tert*-Butyl 5-phenoxybenzo[c]isoxazole-3-carboxylate (50.0 mg, 0.161 mmol) was dissolved in CH₂Cl₂ (1.5 mL) and trifluoroacetic acid (TFA) (146 µL) was added in dropwise. The solution was kept stirring at rt overnight until completion. Solvents were then removed to give 5-phenoxybenzo[c]isoxazole-3-carboxylic acid (40mg, 98 % yield) which was used without further purification. LCMS (ES, *m*/*z*) = 256.0 [M+1]+.

**Step 3:** *N*-(5-(Methylthio)-1,3,4-thiadiazol-2-yl)-5-phenoxybenzo[c]isoxazole-3-carboxamide was prepared using 5-phenoxybenzo[c]isoxazole-3-carboxylic acid following step **5** of **Example 39A.** LCMS (ES, *m*/*z*) = 385.1 [M+1]⁺. ¹H NMR (400 MHz, DMSO-*d*₆) δ 7.92 (1H, d, *J =* 9.5 Hz), 7.50 (2H, t, *J =* 7.7 Hz), 7.43 (1H, d, *J* = 9.7 Hz), 7.30 (1H, t, *J* = 7.4 Hz), 7.21 (2H, *d, J =* 7.9 Hz), 7.05 (1H, s), 2.71 (3H, s). [NH signal not observed].

### Example 45: 5-(hydroxy(phenyl)methyl)-N-(5-(methylthio)-1,3,4-thiadiazol-2-yl)benzo[c]isoxazole-3-carboxamide (Compound 166)

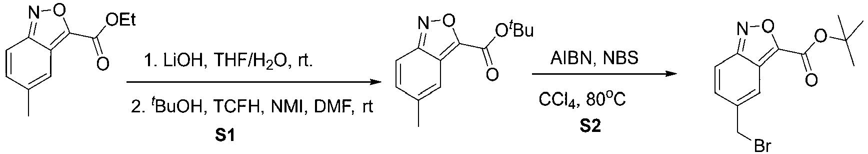

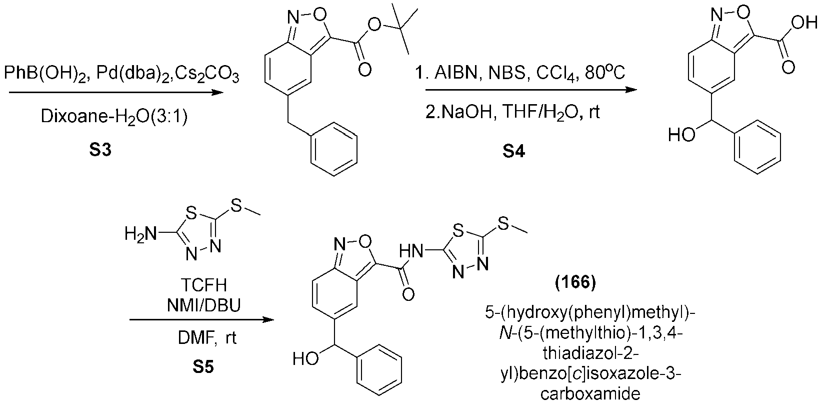

**Step 1:** Ethyl 5-methylbenzo[c]isoxazole-3-carboxylate (2.70 g, 13.2 mmol) was dissolved in tetrahydrofuran (THF) (22 mL)/H₂O (22 mL) and 1N lithium hydroxide (15.8 mL, 15.8 mmol) was added. The solution was kept stirring until complete conversion of starting material. Amberlite-120[H] was then added to acidify the reaction mixture and was then removed by filtration. The filtrate was concentrated and the residue was dissolved in anhydrous N,N-dimethylformamide (DMF) (45 mL), 'BuOH (1.94 mL, 20.3 mmol) and triethylamine (1.89 mL, 13.5 mmol). This solution was cooled in an ice-water bath. 1-Methylimidazole (3.78 mL, 47.4 mmol) and chloro-N,N,N',N'-tetramethylformamidinium hexafluorophosphate (TCFH) (4.18 g, 14.9 mmol) were added sequentially. The reaction mixture was allowed warm up to room temperature and stirred overnight. Upon completion the reaction mixture was diluted with ethyl acetate (EtOAc), washed with water, brine, dried over sodium sulfate and concentrated. The residue was purified by flash chromatography through silica gel to provide *tert*-butyl 5-methylbenzo[*c*]isoxazole-3-carboxylate (2.70 g, 85 % yield). LCMS (ES, *m*/*z*) = 234.1 [M+1]⁺.

**Step 2:** Into a solution of *tert*-butyl 5-methylbenzo[c]isoxazole-3-carboxylate (2.00 g, 8.57 mmol) in carbon tetrachloride (42 mL) was added N-bromosuccinimide (NBS) (1.83 g, 10.3 mmol) and 2,2'-azobis(2-methylpropionitrile) (AIBN) (141 mg, 0.857 mmol) at room temperature. The resulting mixture was stirred for 16 h at 80 °C. The reaction mixture was allowed to cool to rt, filtered through celite and the filtrate was concentrated under reduced pressure. The residue was purified by flash chromatography through silica gel (0 - 10% ethyl acetate (EtOAc) /hexanes) to afford *tert*-butyl 5-(bromomethyl)benzo[*c*]isoxazole-3-carboxylate (1.80 g, 67 % yield) as a white solid. LCMS (ES, *m*/*z*) = 312.0/314.0 [M+1]⁺.

**Step 3:** *tert*-Butyl 5-(bromomethyl)benzo[c]isoxazole-3-carboxylate (1.00 g, 3.20 mmol) and phenylboronic acid (391 mg, 3.20 mmol) were taken up in dioxane (26 mL) and H₂O (8.7 mL), and cesium carbonate (3.13 g, 9.61 mmol) was added. The suspension was degassed and purged with nitrogen (3 x). Bis(dibenzylideneacetone)palladium(0) (55.3 mg, 0.0961 mmol) was added and the suspension was degassed with nitrogen (3 x). The reaction mixture was heated to 80 °C and was monitored by LCMS. After overnight, the reaction mixture was cooled to rt and filtered through celite. The filtrate was diluted with ethyl acetate (EtOAc), washed with water, brine and concentrated. The residue was purified by flash chromatography through silica gel to give *tert*-butyl 5-benzylbenzo[*c*]isoxazole-3-carboxylate (760 mg, 77 % yield). LCMS (ES, *m*/*z*) = 310.1[M+1]⁺.

**Step 4:** Into a solution of *tert*-butyl 5-benzylbenzo[*c*]isoxazole-3-carboxylate (330 mg, 1.07 mmol) in carbon tetrachloride (10 mL) was added *N*-bromosuccinimide (247 mg, 1.39 mmol) and 2,2'-azobis(2-methylpropionitrile) (17.5 mg, 0.107 mmol) at room temperature. The resulting mixture was stirred for 16 h at 80 °C. The reaction mixture was then cooled down, diluted with ethyl acetate (EtOAc), washed with water, brine, dried over sodium sulfate and concentrated to give 450 mg of crude bromo-intermediates. 250 mg of the residue was taken into tetrahydrofuran (THF) (5.3 mL) and water (5.3 mL) and 2N sodium hydroxide (523 µL, 1.05 mmol) was added. The resulting mixture was stirred at rt for 2 h. After completion, the reaction mixture was acidified with Amberlite IR-120[H], filtered and concentrated. The residue was purified by C18 reverse phase chromatography to give 5-(hydroxy(phenyl)methyl)benzo[c]isoxazole-3-carboxylic acid (60.0 mg, 35 % yield). LCMS (ES, *m*/*z*) = 270.1 [M+1]⁺.

**Step 5:** 5-(Hydroxy(phenyl)methyl)*-N*-(5-(methylthio)-1,3,4-thiadiazol-2-yl)benzo[c]isoxazole-3-carboxamide was prepared using 5-(hydroxy(phenyl)methyl)benzo[c]isoxazole-3-carboxylic acid following step **5** of **Example 39A.** LCMS (ES, *m*/*z*) = 399.1 [M+1]⁺. ¹H NMR (400 MHz, DMSO-*d*₆) δ 8.11 (1H, s), 7.71 (1H, d, *J* = 9.3 Hz), 7.41 (3H, d, *J =* 6.5 Hz), 7.32 (2H, t, *J* = 7.4 Hz), 7.22 (1H, t, *J =* 7.3 Hz), 6.19 (1H, s), 5.79 (1H, s), 2.74 (3H, s). [NH signal not observed].

### Example 46: 5-benzoyl-N-(5-(methylthio)-1,3,4-thiadiazol-2-yl)benzo[c]isoxazole-3-carboxamide (Compound 167)

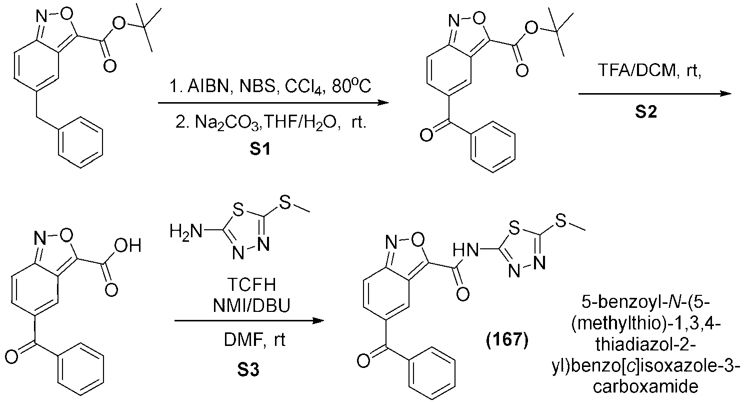

**Step 1:** To a solution of *tert*-butyl 5-benzylbenzo[*c*]isoxazole-3-carboxylate (580 mg, 1.87 mmol) in carbon tetrachloride (19 mL) was added *N*-bromosuccinimide (734 mg, 4.12 mmol) and 2,2'-azobis(2-methylpropionitrile) (30.8 mg, 0.187 mmol) at room temperature. The resulting mixture was stirred for 16 h at 80 °C. The reaction mixture was allowed to cool to rt, diluted with ethyl acetate (EtOAc), washed with water, brine, dried over sodium sulfate and concentrated. The residue was taken into tetrahydrofuran (THF) (15.0 mL) and H₂O (3.75 mL) and sodium carbonate (796 mg, 7.50 mmol) was added. The resulting mixture was stirred at rt overnight. After completion, the reaction mixture was acidified with amberlite IR-120[H], filtered and concentrated. The residue was purified by flash chromatography through silica gel to give *tert*-butyl 5-benzoylbenzo[c]isoxazole-3-carboxylate (170 mg, 28 % yield). LCMS (ES, *m*/*z*) = 324.1[M+1]⁺.

**Step 2:** *tert*-Butyl 5-benzoylbenzo[c]isoxazole-3-carboxylate (60.0 mg, 0.186 mmol) was dissolved in CH₂Cl₂ (1.7 mL) and trifluoroacetic acid (TFA) (169 µL) was added dropwise. The solution was stirred at rt for 2 days. Solvents were then removed to provide crude 5-benzoylbenzo[c]isoxazole-3-carboxylic acid (quant. yield) and the resulting residue was used without further purification. LCMS (ES, *m*/*z*) = 268.1 [M+1]⁺.

**Step 3:** 5-Benzoyl-*N*-(5-(methylthio)-1,3,4-thiadiazol-2-yl)benzo[c]isoxazole-3-carboxamide was prepared using crude 5-benzoylbenzo[c]isoxazole-3-carboxylic acid following step **5** of **Example 39A.** LCMS (ES, *m*/*z*) = 397.1 [M+1]⁺. ¹H NMR (400 MHz, DMSO-*d*₆) δ 8.36 (1H, s), 7.96 (1H, d, *J* = 9.3 Hz), 7.80-7.85 (3H, m), 7.73 (1H, *t, J =* 7.4 Hz), 7.61 (2H, *t, J =* 7.5 Hz), 2.71 (3H, s). [NH signal not observed].

### Example 47: 7-(hydroxymethyl)-N-(5-(methylthio)-1,3,4-thiadiazol-2-yl)benzole]isoxazole-3-carboxamide (Compound 168)

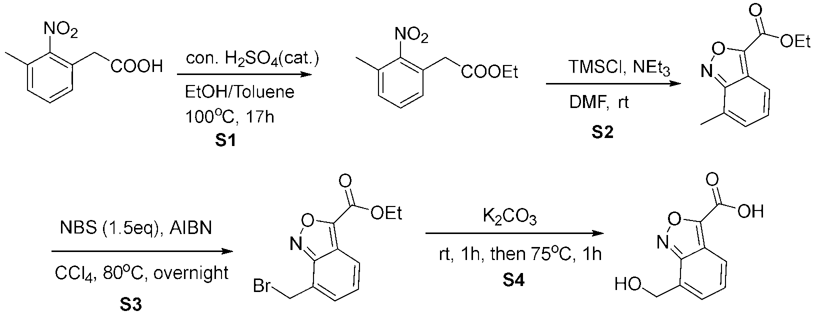

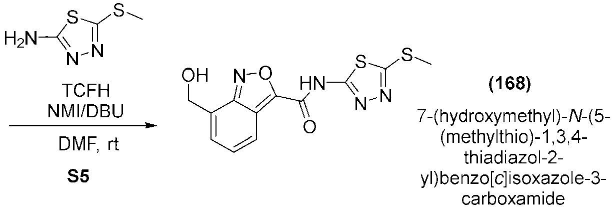

**Step 1:** A flame-dried flask was charged with 2-(3-methyl-2-nitrophenyl)acetic acid (3.00 g, 15.4 mmol), toluene (115 mL), sulfuric acid (81.9 µL, 1.54 mmol), and ethanol (EtOH) (38 mL). The flask was fitted with a condenser, and the solution was refluxed for 14 h. The solvent was removed under reduced pressure to give crude ethyl 2-(3-methyl-2-nitrophenyl)acetate (quant. yield) which was used directly in the next step without further purification. LCMS (ES, m/z) = 224.1[M+1]⁺.

**Step 2:** Ethyl 2-(3-methyl-2-nitrophenyl)acetate (3.40 g, 15.2 mmol) was dissolved in N,N-dimethylformamide (DMF) (76 mL) and treated with triethylamine (NEt₃) (10.6 mL, 76.2 mmol) and chlorotrimethylsilane (TMSCl) (9.67 mL, 76.2 mmol) sequentially. The resulting mixture was stirred at room temperature for 27 days. Upon completion, the reaction mixture was poured into ice cold diluted HCl and extracted with ethyl acetate (EtOAc), The organic phase was concentrated and purified by flash chromatography through silica gel to give ethyl 7-methylbenzo[c]isoxazole-3-carboxylate (2.90 g, 93 % yield). LCMS (ES, *m*/*z*) = 206.1 [M+1]⁺.

**Step 3:** To a solution of ethyl 7-methylbenzo[*c*]isoxazole-3-carboxylate in carbon tetrachloride (24 mL) was added *N*-bromosuccinimide (1.30 g, 7.31 mmol) and 2,2'-azobis(2-methylpropionitrile) (160 mg, 0.975 mmol) at room temperature. The resulting mixture was stirred for 16 h at 80 °C. The reaction mixture was then cooled to rt, filtered through celite and the filtrate was concentrated under reduced pressure. The residue was purified by flash chromatography through silica gel (0 - 10% ethyl acetate (EtOAc) /Hexane) to afford ethyl 7-(bromomethyl)benzo[c]isoxazole-3-carboxylate (660 mg, 48 % yield); LCMS (ES, *m*/*z*) = 284.0/286.0 [M+1]⁺, and ethyl 7-(dibromomethyl)benzo[c]isoxazole-3-carboxylate (640 mg, 36 % yield); LCMS (ES, *m*/*z*) =361.9/363.9 [M+1]⁺.

**Step 4:** Ethyl 7-(bromomethyl)benzo[*c*]isoxazole-3-carboxylate (50.0 mg, 0.176 mmol) was dissolved in tetrahydrofuran (THF) (8.8 mL) and H₂O (8.8 mL) and potassium carbonate (53.5 mg, 0.387 mmol) was added. The resulting solution was stirred at 75 °C for 1 h. The reaction mixture was cooled to rt, acidified with Amberlite IR-120[H]. The resin was filtered off and the filtrate was concentrated to give crude 7-(hydroxymethyl)benzo[c]isoxazole-3-carboxylic acid (35.0 mg, quant. yield) which was used without further purification. LCMS (ES, *m*/*z*) = 194.0 [M+1]⁺.

**Step 5:** 7-(Hydroxymethyl)-*N*-(5-(methylthio)-1,3,4-thiadiazol-2-yl)benzo[c]isoxazole-3-carboxamide was prepared using 7-(hydroxymethyl)benzo[c]isoxazole-3-carboxylic acid following step **5** of **Example 39A.** LCMS (ES, *m*/*z*) = 323.0 [M+1]⁺. ¹H NMR (400 MHz, DMSO-*d*₆) δ 7.97 (1H, s), 7.43 (1H, d, *J =* 6.3 Hz), 7.28 (1H, s), 5.48 (1H, s), 4.83 (2H, s), 2.71 (3H, s). [NH signal not observed].

### Example 48: 2-(2'-(6-((5-(methylthio)-1,3,4-thiadiazol-2-yl)carbamoyl)-2-oxo-2H-pyran-4-yl)-[1,1'-biphenyl]-4-yl)acetic acid (Compound 176)

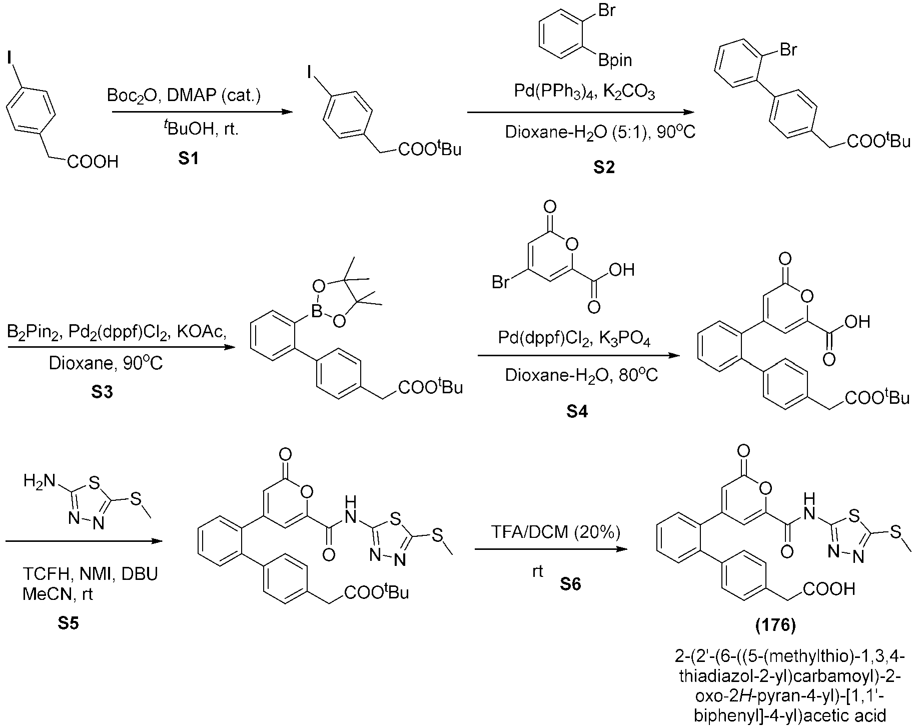

**Step 1:** Di-*tert*-butyldicarbonate (3.33 g, 15.3 mmol) and 4-dimethylaminopyridine (186 mg, 1.53 mmol) were added to a solution of 4-iodophenylacetic acid (2.00 g, 7.63 mmol) in *^{t}*BuOH (15.3 mL) under nitrogen. The mixture was stirred at room temperature for 24 hours. The reaction mixture was then concentrated to dryness and the crude material purified by flash chromatography through silica gel (0 - 10% ethyl acetate (EtOAc) /Hexane) to afford tert-butyl 2-(4-iodophenyl)acetate as a colorless oil. ¹H NMR (400 MHz, CHCl₃-d): δ 7.64 (2H, d, *J =* 8.1 Hz), 7.02 (2H, d, *J =* 7.9 Hz), 3.46 (2H, s), 1.43 (9H, s).

**Step 2:** *tert*-Butyl 2-(4-iodophenyl)acetate (400 mg, 1.26 mmol) , 2-bromophenylboronic acid (177 µL, 1.51 mmol), and potassium carbonate (348 mg, 2.51 mmol) were dissolved in dioxane (5.24 mL) and H₂O (1.05 mL). The solution was degassed with argon before addition of tetrakis(triphenylphosphine)palladium (72.6 mg, 0.0629 mmol) . The reaction mixture was degassed again with argon before being heated at 90 °C overnight. The reaction mixture was cooled to rt and diluted with ethyl acetate (EtOAc), washed with water, brine, dried over sodium sulfate and concentrated. The residue was purified by flash chromatography through silica gel (0 - 25% EtOAc/Hexane) to provide *tert*-butyl 2-(2'-bromo-[1,1'-biphenyl]-4-yl)acetate (0.150 g, 34 % yield) as a colorless oil. ¹H NMR (400 MHz, CHCl₃-*d*): δ_{H} 7.66 (1H, d, *J =* 8.0 Hz), 7.30-7.37 (6H, m), 7.17-7.21 (1H, m), 3.59 (2H, s), 1.47 (9H, s).

**Step 3:** *tert*-Butyl 2-(2'-bromo-[1,1'-biphenyl]-4-yl)acetate (550 mg, 1.58 mmol) was dissolved in dioxane (7.92 mL) and the solution was degassed with argon. Potassium acetate (466 mg, 4.75 mmol), bis(pinacolato)diboron (523 mg, 2.06 mmol) and 1,1'-bis(diphenylphosphino)ferrocene dichloropalladium (II) (116 mg, 0.158 mmol) were added sequentially. The solution was degassed again with argon and heated at 90 °C overnight. Solvents were then removed and the residue was taken up in Hexane/ ethyl acetate (EtOAc) (5:1). Black solids were filtered off through a celite pad and the filtrate was concentrated to give a brown oil which was purified by flash chromatography through silica gel (0 - 20% EtOAc/Hexane) to provide *tert*-butyl 2-(2'-(4,4,5,5-tetramethyl-1,3,2-dioxaborolan-2-yl)-[1,1'-biphenyl]-4-yl)acetate (0.58 g, 65 % yield). LCMS (ES, *m*/*z*) = 417.2 [M+23]⁺.

**Step 4:** *tert*-Butyl 2-(2'-(4,4,5,5-tetramethyl-1,3,2-dioxaborolan-2-yl)-[1,1'-biphenyl]-4-yl)acetate (200 mg, 0.507 mmol), 4-bromo-2-oxo-2*H*-pyran-6-carboxylic acid (133 mg, 0.609 mmol), and potassium phosphate, tribasic (323 mg, 1.52 mmol) were mixed in dioxane (2.11 mL) and H₂O (423 µL). The mixture was degassed with argon before addition of 1,1'-bis(diphenylphosphino)ferrocene dichloropalladium (II) (37.1 mg, 0.0507 mmol) and degassed again with argon. The resulting mixture was heated at 85 °C under argon for 5 h. Upon completion, the resulting mixture was filtered through a celite pad. Solvents were removed under reduced pressure and the crude material was purified by C18 reverse flash chromatography (0 - 50% MeCN/H₂O) to provide 4-(4'-(2-(*tert*-butoxy)-2-oxoethyl)-[1,1'-biphenyl]-2-yl)-2-oxo-2*H*-pyran-6-carboxylic acid (0.030 g, 15 % yield). LCMS (ES, *m*/*z*) = 429.1 [M+23]⁺.

**Step 5:** *tert*-Butyl 2-(2'-(6-((5-(methylthio)-1,3,4-thiadiazol-2-yl)carbamoyl)-2-oxo-2H-pyran-4-yl)-[1,1'-biphenyl]-4-yl)acetate was prepared using 4-(4'-(2-(tert-butoxy)-2-oxoethyl)-[1,1'-biphenyl]-2-yl)-2-oxo-2H-pyran-6-carboxylic acid following step **5** of **Example 39A.** LCMS (ES, *m*/*z)* = 536.1 [M+1]⁺.

**Step 6:** *tert*-Butyl 2-(2'-(6-((5-(methylthio)-1,3,4-thiadiazol-2-yl)carbamoyl)-2-oxo-2*H-*pyran-4-yl)-[1,1'-biphenyl]-4-yl)acetate (18.5 mg, 0.0344 mmol) and 1,3-dimethoxybenzene (9.02 µL, 0.0689 mmol) were dissolved in CH₂Cl₂ (626 µL) and trifluoroacetic acid (TFA) (62.6 µL) was added. The resulting solution was stirred at rt until complete consumption of starting material. Solvents were then removed and the residue was purified by C18 reverse phase chromatography to provide 10 mg of a brown solid, which was washed with MeOH to give 2-(2'-(6-((5-(methylthio)-1,3,4-thiadiazol-2-yl)carbamoyl)-2-oxo-2H-pyran-4-yl)-[1,1'-biphenyl]-4-yl)acetic acid (6.5 mg, 39 % yield) as an off-white powder. LCMS (ES, *m*/*z)* = 480.1 [M+1]⁺. ¹H NMR (400 MHz, DMSO-d₆): δ_{H} 7.48-7.60 (4H, m), 7.26 (4H, s), 7.00 (1H, s), 6.42 (1H, s), 3.56 (2H, s), 2.70 (3H, s). [COOH and NH signals not observed].

### Example 49: 1-((3-((5-(Methylthio)-1,3,4-thiadiazol-2-yl)carbamoyl)benzo[c]isoxazol-5-yl)methyl)-4-oxo-1,4-dihydroquinoline-3-carboxylic acid (Compound 184)

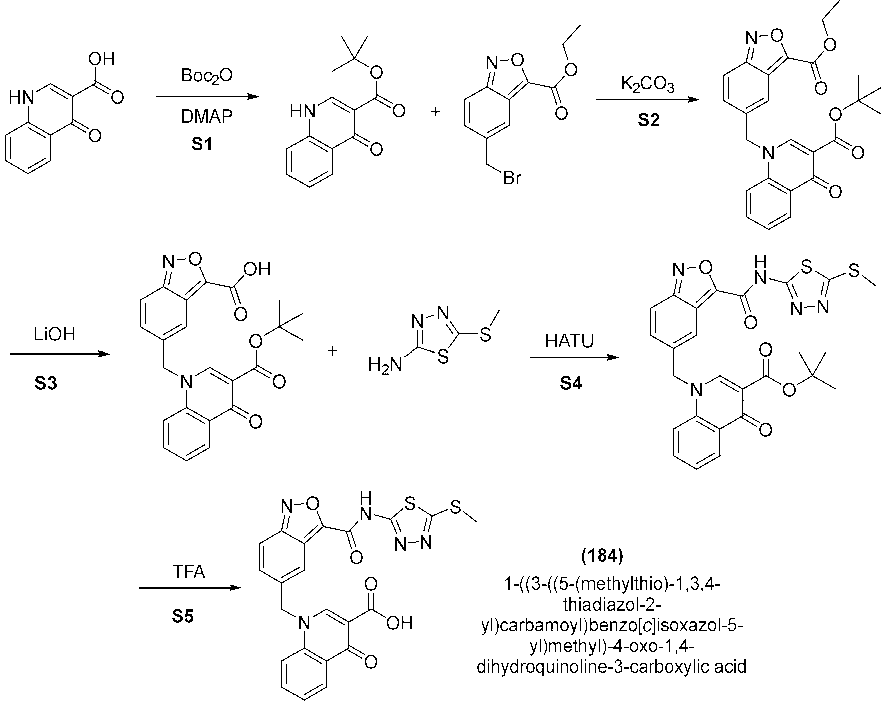

**Step 1:** A solution of 4-oxo-1,4-dihydroquinoline-3-carboxylic acid (1.0 eq, 500 mg, 2.59 mmol), di-tert-butyldicarbonate (Boc₂O) (1.5 eq, 865 mg, 3.89 mmol) and 4-dimethylaminopyridine (DMAP) (0.5 eq, 158 mg, 1.30 mmol) in anhydrous tetrahydrofuran (THF) (10 mL) under an Ar atmosphere was stirred at rt overnight. The reaction mixture was treated with 1 M HCl (28 mL) and it was stirred at rt for 10 min. The reaction mixture was brought to pH 8 with 2 M NaOH_{(aq)} sln and it was extracted with ethyl acetate (EtOAc) (3 x 20 mL). The suspension of combined organic extracts was filtered to afford crude *tert*-butyl 4-hydroxyquinoline-3-carboxylate (160 mg, 25% yield) as an off-white solid, which was used without further purification. LCMS (ES, *m*/*z*) = 244.1 [M+H]⁺.

**Step 2:** *tert*-Butyl 1-((3-((5-(methylthio)-1,3,4-thiadiazol-2-yl)carbamoyl)benzo[c]isoxazol-5-yl)methyl)-4-oxo-1,4-dihydroquinoline-3-carboxylate was prepared by following Steps **1-3** of **Example 9** using *tert*-butyl 4-hydroxyquinoline-3-carboxylate. LCMS (ES, *m*/*z*) = 550.1 [M+H]⁺.

**Step 3:** A solution of *tert*-butyl 1-((3-((5-(methylthio)-1,3,4-thiadiazol-2-*yl)carbamoyl)benzo[c]isoxazol-5-yl)methyl)-4-oxo-1,4-dihydroquinoline-3-carboxylate* (1.0 eq, 34.6 mg, 63.0 umol) and trifluoroacetic acid (207 eq, 998 uL, 13.0 mmol) in anhydrous dichloromethane (DCM) (5.00 mL) was vigorously stirred at rt for 32 h. The solvent was removed by rotary evaporation and the crude was triturated with MeCN and water. The solid was filtered and dried to afford 1-((3-((5-(methylthio)-1,3,4-thiadiazol-2-yl)carbamoyl)benzo[c]isoxazol-5-yl)methyl)-4-oxo-1,4-dihydroquinoline-3-carboxylic acid (23.7 mg, 76 % yield) as a light-yellow solid. LCMS (ES, *m*/*z)* = 494.1 [M+H]⁺. ¹H NMR (DMSO-d₆, 400 MHz): δ 9.37 (1H, s), 8.42 (1H, d, *J =* 8.1 Hz), 7.84-7.95 (4H, m), 7.64 (1H, *t, J =* 7.5 Hz), 7.43 (1H, d, *J* = 9.4 Hz), 6.00 (2H, s), 2.74 (3H, s).

### Example 50: 3-amino-N-(5-(2-chlorophenyl)-1,3,4-thiadiazol-2-yl)isoxazole-5-carboxamide (Compound 4)

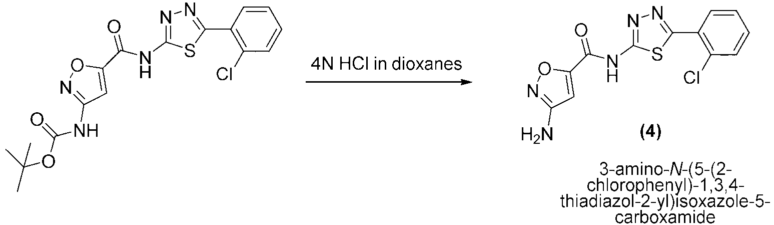

**Step 1:** *tert*-Butyl *N*-[5-[[5-(2-chlorophenyl)-1,3,4-thiadiazol-2-yl]carbamoyl]isoxazol-3-yl]carbamate was prepared according to **Example 1** using 3-(tert-butoxycarbonylamino)isoxazole-5-carboxylic acid. LCMS (ES, *m*/*z*) = 422.44 [M+H]⁺. ¹H NMR (DMSO-d₆, 400 MHz) δ 13.80 (s, 1H), 10.69 (s, 1H), 8.15 (d,J = 7.5Hz, 1H), 7.88 (s, 1H), 7.71 (dd,J = 7.8, 1.5Hz, 1H), 7.65 - 7.51 (m, 2H), 1.50 (s, 9H).

**Step 2:** *tert*-Butyl *N*-[5-[[5-(2-chlorophenyl)-1,3,4-thiadiazol-2-yl]carbamoyl]isoxazol-3-yl]carbamate (1.00 eq, 300 mg, 0.711 mmol) suspended in ethyl acetate (EtOAc) (7 mL) and 4N HCl in dioxanes (2 mL) added and stirred at rt. Mixture never became homogeneous. After 2 h, a further portion of 4N HCl in dioxanes (2 mL) was added and stirred overnight. After 16 h at rt, reaction was incomplete. Mixture was concentrated to dryness. Crude residue was re-suspended in ethyl acetate (EtOAc) (20 mL) and N,N-dimethylformamide (DMF) (1 mL) and 4N HCl in dioxanes added (2 mL) and continued stirring. After 16 h, reaction was incomplete. A further portion of 4N HCl in dioxanes (4 mL) added and placed in a 55 °C oil bath wherein mixture became homogeneous. After 2 h, LCMS shows complete deprotection. Concentrated under reduced pressure and crude material directly purified by C18 reverse phase chromatography (40 g C18, 0-100% MeCN/H₂O). Product elutes ~50 - 60% acetonitrile (MeCN). Appropriate fractions combined and concentrated to provide 3-amino-*N*-[5-(2-chlorophenyl)-1,3,4-thiadiazol-2-yl]isoxazole-5-carboxamide (68 mg, 30 % yield) as a white solid. LCMS (ES, m/z) = 322.25 [M+1]+. ¹H NMR (400 MHz, DMSO-*d*₆) δ 13.58 (s, 1H), 8.28 - 8.04 (m, 1H), 7.71 (dd,J = 7.8, 1.5Hz, 1H), 7.63 - 7.52 (m, 2H), 6.94 (s, 1H), 6.02 (s, 2H).

### Example 51: 3-acetamido-N-(5-(2-chlorophenyl)-1,3,4-thiadiazol-2-yl)isoxazole-5-carboxamide (Compound 5)

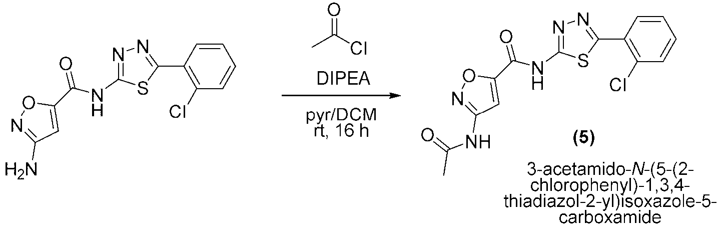

Acetyl chloride (1.10 eq, 0.0071 mL, 0.0991 mmol) was added to a solution of 3-amino-*N*-[5-(2-chlorophenyl)-1,3,4-thiadiazol-2-yl]isoxazole-5-carboxamide (1.00 eq, 29 mg, 0.0901 mmol) in a mixture of dichloromethane (DCM) (2 mL) and pyridine (Py) (1 mL). Diisopropylethylamine (DIPEA) (3.00 eq, 0.047 mL, 0.270 mmol) was added and mixture stirred at rt overnight. After 16 h, reaction appears complete as judged by LCMS. Volatiles removed under reduced pressure and crude material purified by C18 reverse phase chromatography (20 g C18, 0-100% MeCN/0.1% aqueous formic acid). Product elutes ~ 50% acetonitrile (MeCN). Appropriate fractions combined and concentrated to provide 3-acetamido-*N*-[5-(2-chlorophenyl)-1,3,4-thiadiazol-2-yl]isoxazole-5-carboxamide (5.0 mg, 13 % yield) as a white solid. LCMS (ES, m/z) = 364.15 [M+1]+. ¹H NMR (400 MHz, DMSO-*d*₆) δ 11.05 (d,J = 12.4Hz, 1H), 8.15 (s, 1H), 8.04 (s, 1H), 7.61 (d,J = 4.5 Hz, 1H), 7.51 - 7.41 (m, 2H), 7.40 - 7.15 (m, 1H), 2.09 (s, 3H).

### Example 52: N-(5-(methylthio)-1,3,4-thiadiazol-2-yl)-5-(thiophen-2-yl)-nicotinamide (Compound 96)

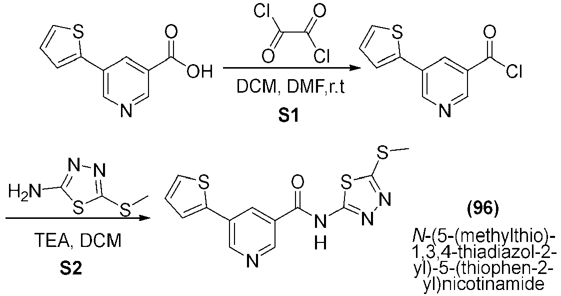

**Step 1:** To a stirred solution of 5-(thiophen-2-yl)nicotinic acid (200 mg, 0.975 mmol, 1.00 equiv) in dichloromethane (DCM) (5 mL) was added oxalyl chloride (742 mg, 5.850 mmol, 6 equiv) at 0 °C, and then N,N-dimethylformamide (DMF) (0.01 ml) was added. The resulting mixture was stirred for 2 h at room temperature. The resulting mixture was concentrated under reduced pressure to afford 5-(thiophen-2-yl)nicotinoyl chloride (200 mg) as crude product which was used in the next step directly without further purification.

**Step 2:** The crude 5-(thiophen-2-yl)nicotinoyl chloride was diluted with DCM (1 ml). triethylamine (TEA) (60 mg, 0.585 mmol, 3 equiv) and 5-(methylthio)-1,3,4-thiadiazol-2-amine (57 mg, 0.390 mmol, 2 equiv) were added at room temperature. The resulting solution was stirred for 12 h at 0 °C. The resulting mixture was filtered and the filter cake was washed with acetonitrile (MeCN) (3 x 10 mL). This resulted in N-[5-(methylsulfanyl)-1,3,4-thiadiazol-2-yl]-5-(thiophen-2-yl)pyridine-3-carboxamide (7.2 mg, 11 % yield) as a white solid. LCMS (ES, m/z) = 335.1 [M+H]⁺. ¹HNMR(400 MHz, DMSO-*d*₆) δ 13.40 (br, 1H), 9.15 (s, 1H), 9.10 (s, 1H), 8.72 (s, 1H), 7.80 (d, *J* = 4.0 Hz, 1H), 7.75 (d, *J =* 4.0 Hz, 1H), 7.26 (dd, *J =* 4.8, 3.6 Hz, 1H), 2.77 (s, 3H).

### Example 53: N5-(5-(2-chlorophenyl)-1,3,4-thiadiazol-2-yl)-N3,N3-dimethylisoxazole-3,5-dicarboxamide (Compound 87)

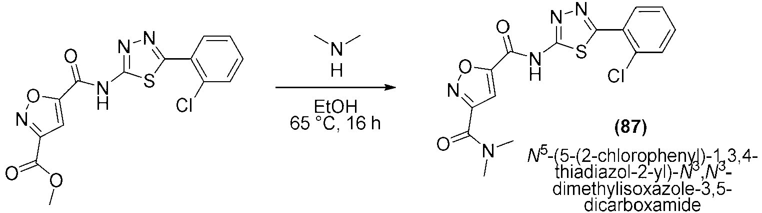

To a suspension of methyl 5-[[5-(2-chlorophenyl)-1,3,4-thiadiazol-2-yl]carbamoyl]isoxazole-3-carboxylate (1.00 eq, 40 mg, 0.110 mmol) in ethanol (EtOH) (1 mL) in a large sealed MW vial was added dimethylamine (33% solution in EtOH) (20.0 eq, 1.0 mL, 2.19 mmol) and mixture placed in a 70 °C oil bath overnight. After 16 h, volatiles removed under reduced pressure and crude purified by C18 reverse phase chromatography (30g C18, 0-100% MeCN/H₂O). Appropriate fractions combined and concentrated to provide *N*5-(5-(2-chlorophenyl)-1,3,4-thiadiazol-2-yl)-N3,N3-dimethylisoxazole-3,5-dicarboxamide (3 mg, 7 % yield) as a white solid. LCMS (ES, m/z) = 378.23 [M+H]⁺. ¹H NMR (400 MHz, DMSO-*d*₆) δ 14.08 (br.s, 1H), 8.14 (d,J = 7.4Hz, 1H), 7.81 - 7.42 (m, 4H), 3.12 (s, 3H), 3.03 (s, 3H).

### Example 54: N-(5-(2-(trifluoromethyl)phenyl)-1,3,4-thiadiazol-2-yl)benzo[c]iso-xazole-3-carboxamide (Compound 143)

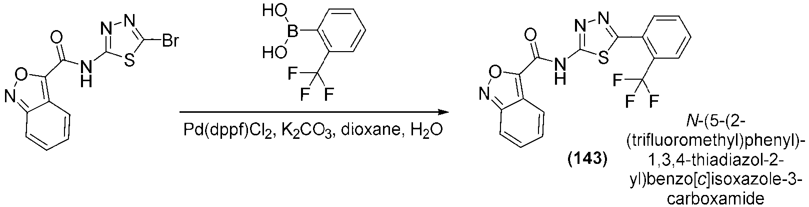

To a mixture of N-(5-bromo-1,3,4-thiadiazol-2-yl)-2,1-benzoxazole-3-carboxamide (80 mg, 0.246 mmol, 1 equiv) and 2-(trifluoromethyl) phenylboronic acid (94 mg, 0.495 mmol, 2.01 equiv) in dioxane (1 mL) and H₂O (0.2 mL) were added K₂CO₃ (102 mg, 0.738 mmol, 3.00 equiv) and [1,1'-bis(diphenylphosphino)ferrocene]dichloropalladium(II) (Pd(dppf)Cl₂) (36 mg, 0.049 mmol, 0.20 equiv) at room temperature. The reaction mixture was irradiated with microwave radiation for 30 min at 130 °C. The resulting solution was concentrated under vacuum and the residue was purified by Prep-HPLC (conditions: Xselect CSH C18 OBD Column 30*150mm 5um, n; mobile phase, acetonitrile (MeCN) and water (0.05% TFA), Gradient: 50% water (0.05% TFA) up to 58% water (0.05% TFA) in 8 min, hold 58% in 2 min) to afford N-(5-(2-(trifluoromethyl)phenyl)-1,3,4-thiadiazol-2-yl)benzo[c]iso-xazole-3-carboxamide (5.8 mg, 6 % yield) as a white solid. LCMS (ES, m/z) = 391.1[M+1]⁺. ¹H NMR (400 MHz, DMSO-*d*₆) δ 8.12 (d, *J =* 8.8 Hz, 1H), 8.01 (d, *J =* 7.6 Hz, 1H), 7.89 - 7.85 (m, 4H), 7.59-7.55 (m, 1H), 7.41-7.37 (m, 1H).

### Example 55: N-(5-(1H-pyrazol-4-yl)-1,3,4-thiadiazol-2-yl)benzo[c]isoxazole-3-carboxamide (Compound 145)

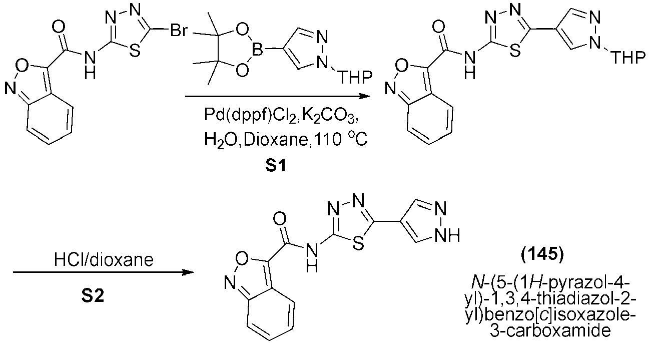

**Step 1:** Under nitrogen, a solution of *N*-[5-(1*H*-pyrazol-4-yl)-1,3,4-thiadiazol-2-yl]-2,1-benzoxazole-3-carboxamide (200 mg, 0.640 mmol, 1 equiv), 1-(oxan-2-yl)-4-(4,4,5,5-tetramethyl-1,3,2-dioxaborolan-2-yl)pyrazole (199 mg, 0.715 mmol, 1.12 equiv), [1,1'-bis(diphenylphosphino)ferrocene]dichloropalladium(II) (Pd(dppf)Cl₂) (51 mg, 0.070 mmol, 0.11 equiv) and K₂CO₃ (160 mg, 1.16 mmol, 1.81 equiv) in dioxane (9 mL) and H₂O (1.5 mL) was irradiated with microwave radiation for 45 min at 110 °C. The mixture was then concentrated under vacuum and the residue was purified by silica gel column chromatography, eluted with petroleum ether:ethyl acetate (1:1), to afford *N*-(5-(1-(Tetrahydro-2H-pyran-2-yl)-1*H*-pyrazol-4-yl)-1,3,4-thiadiazol-2-yl)benzo[c]isoxazole-3-carboxamide (180 mg, 70.90%) as a brown solid. LCMS (ES, *m*/*z)* = 397.1[M+H]⁺.

**Step 2:** A solution of *N*-(5-(1-(tetrahydro-2*H*-pyran-2-yl)-1*H*-pyrazol-4-yl)-1,3,4-thiadiazol-2-yl)benzo[c]isoxazole-3-carboxamide (150 mg, 0.378 mmol, 1 equiv) in HCl (4 M) in 1,4-dioxane (1.5 mL) was stirred for 1 h at room temperature, and then the resulting solution was concentrated under vacuum. The crude product was purified by Prep-HPLC (conditions: Xselect CSH C18 OBD Column 30*150mm 5µm, n; Mobile Phase A: acetonitrile (MeCN), Mobile Phase B: Water (0.05%TFA ); Flow rate: 60 mL/min; Gradient: 15% B to 40% B in 8 min, 40% B to 40% B in 10 min, 40% B; Wave Length: 254/220 nm) to afford N-(5-(1*H*-pyrazol-4-yl)-1,3,4-thiadiazol-2-yl)benzo[c]isoxazole-3-carboxamide(10.5 mg, 8.9 % yield) as a yellow solid. LCMS (ES, m/z) = 313.1[M+H]⁺. ¹HNMR(400 MHz, DMSO-*d*₆) δ 8.36-8.20 (br, 2H), 8.12 (d, *J =* 8.8 Hz, 1H), 7.81 (d, *J=* 8.8 Hz, 1H), 7.53 (dd, *J = 8.8,* 6.4 Hz, 1H), 7.36 (dd, *J =* 8.8, 6.4 Hz, 1H).

### Example 56: N-(5-(1-methyl-1H-pyrazol-5-yl)-1,3,4-thiadiazol-2-yl)benzo[c]isoxazole-3-carboxamide (Compound 146)

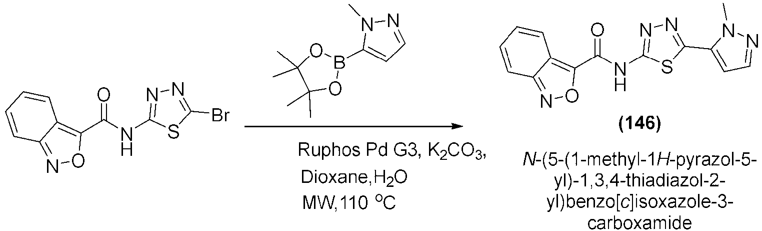

Under nitrogen, a mixture solution of *N*-(5-bromo-1,3,4-thiadiazol-2-yl)benzo[c]isoxazole-3-carboxamide (200 mg, 0.615 mmol, 1 equiv) and 1-methyl-5-(4,4,5,5-tetramethyl-1,3,2-dioxaborolan-2-yl)-1H-pyrazole (153 mg, 0.735 mmol, 1.20 equiv) in dioxane (9 mL) and H₂O (1.5 mL) was added (2-Dicyclohexylphosphino-2',6'-diisopropoxy-1,1'-biphenyl)[2-(2'-amino-1,1'-biphenyl)]palladium(II) methanesulfonate (RuPhos Palladacycle Gen.3) (57 mg, 0.068 mmol, 0.11 equiv) and K₂CO₃ (170 mg, 1.23 mmol, 2.00 equiv). The mixture was irradiated with microwave radiation for 45 min at 110°C, and then the reaction was diluted with water(15 mL) at room temperature and extracted with ethyl acetate (EtOAc) (3 x 20 mL). The combined organic layer was dried over anhydrous Na₂SO₄. After filtration, the filtrate was concentrated under reduced pressure. The crude product was purified by Prep-HPLC (conditions: X select CSH C18 OBD Column 30*150mm 5µm, n; Mobile Phase A: acetonitrile (MeCN), Mobile Phase B: Water (0.05%TFA); Flow rate: 60 mL/min; Gradient: 25% B to 55% B in 8 min, 55% B; Wave Length: 254/220 nm) to afford*N*-(5-(1-methyl-1*H*-pyrazol-5-yl)-1,3,4-thiadiazol-2-yl)benzo[c]isoxazole-3-carboxamide (5.7 mg, 2.8 % yield) as a white solid. LCMS (ES, m/z) = 327.1 [M+H]⁺. ¹HNMR (400 MHz, DMSO-*d*₆) δ 8.08 (d, *J=* 8.8 Hz, 1H), 7.83 (d, *J =* 9.2 Hz, 1H), 7.59 (d, *J =* 2.0 Hz, 1H), 7.55 (dd, *J* =9.2, 6.4 Hz, 1H), 7.38 (dd, *J* =8.8, 6.4 Hz, 1H), 6.94 (d, *J =* 2.0 Hz, 1H), 4.17 (s, 3H).

### Example 57: N-(5-(2-chlorophenyl)-1,3,4-thiadiazol-2-yl)-3-(2-hydroxyethyl)isoxazole-5-carboxamide (Compound 179)

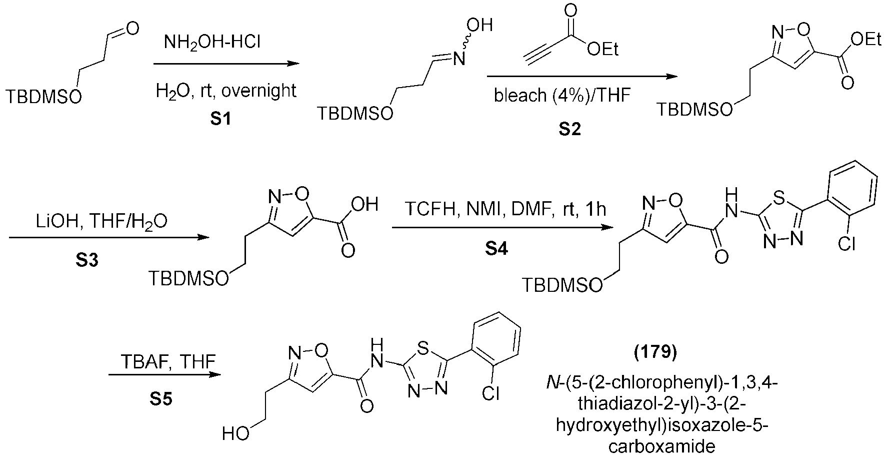

**Step 1:** To a solution of hydroxylamine hydrochloride (2.77 g, 39.8 mmol) in H₂O (66 mL) at rt was added sodium hydroxide (39.8 mL, 39.8 mmol). The mixture was stirred for 10 min before addition of 3-[(*tert*-butyldimethylsilyl)oxy]-1-propanal (5.00 g, 26.5 mmol). The mixture was stirred for 16 h at room temperature and then extracted with ethyl acetate (EtOAc). The combined organic layers were dried over sodium sulfate and evaporated to dryness to give a mixture of *cis* and *trans* isomer of 3-((*tert*-butyldimethylsilyl)oxy)propanal oxime (5.20 g, 96 % yield). The product was used without further purification. LCMS (ES, *m*/*z*) = 204.2[M+1]⁺.

**Step 2:** To a solution of 3-((*tert*-butyldimethylsilyl)oxy)propanal oxime in tetrahydrofuran (THF) (85 mL) at rt was added ethyl propiolate (5.18 mL, 51.1 mmol) and 4% sodium hypochlorite solution (42.6 mL). The mixture was stirred for 4 h at rt and then extracted with ethyl acetate (EtOAc). The combined organic layers were dried over sodium sulfate and dried. The residue was purified by flash chromatography through silica gel (0-10% EtOAc/Hexanes) to provide crude ethyl 3-(2-((*tert*-butyldimethylsilyl)oxy)ethyl)isoxazole-5-carboxylate as a pale yellow liquid. Crude ethyl 3-(2-((*tert*-butyldimethylsilyl)oxy)ethyl)isoxazole-5-carboxylate (600 mg, 2.00 mmol) was dissolved in THF (15.0 mL) and water (5.0 mL) and lithium hydroxide (48.0 mg, 2.00 mmol) was added. The mixture was stirred at rt for 2 h. Amberlite IR-120 [H] was then added to adjust pH~5. Resin was removed by filtration. The filtrate was concentrated to provide 3-(2-((*tert*butyldimethylsilyl)oxy)ethyl)isoxazole-5-carboxylic acid (510 mg, 94 % yield) as a white solid which was used without further purification. LCMS (ES, *m*/*z*) = 272.2 [M+1]⁺.

**Step 3:** 3-(2-((*tert*-Butyldimethylsilyl)oxy)ethyl)-*N*-(5-(2-chlorophenyl)-1,3,4-thiadiazol-2-yl)isoxazole-5-carboxamide was prepared using 3-(2-((*tert*-butyldimethylsilyl)oxy)ethyl)isoxazole-5-carboxylic acid according to **Example 35.** LCMS (ES, *m*/*z*) = 465.1 [M+1]⁺.

**Step 4:** To a solution of 3-(2-((*tert*-butyldimethylsilyl)oxy)ethyl)-*N*-(5-(2-chlorophenyl)-1,3,4-thiadiazol-2-yl)isoxazole-5-carboxamide (120 mg, 0.258 mmol) in tetrahydrofuran (THF) (2.6 mL) at 10 °C was added tetra-*n*-butylammonioum fluoride (TBAF) (284 µL, 0.284 mmol) and the mixture was stirred at room temperature for 1 h. The mixture was quenched with water and subsequently extracted with 2-methyl tetrahydrofuran (2-MeTHF). The combined organic layers were dried over sodium sulfate and concentrated. The residue was purified by flash chromatography through silica gel (0-2% MeOH/CH₂Cl₂) to give *N*-(5-(2-chlorophenyl)-1,3,4-thiadiazol-2-yl)-3-(2-hydroxyethyl)isoxazole-5-carboxamide (80 mg, 88 % yield) as a white powder. LCMS (ES, *m*/*z*) = 351.1 [M+1]⁺. ¹H NMR (400 MHz, DMSO-d₆): δ 13.88 (1H, br s), 8.13 (1H, d*, J =* 7.5 Hz), 7.69 (1H, d, *J =* 7.8 Hz), 7.49-7.59 (3H, m), 4.89 (1H, s), 3.71 (2H, t, *J* = 6.2 Hz), 2.86 (2H, t, *J =* 6.3 Hz).

### Example 58: 5-(3,4-Dihydroxybenzyl)-N-(5-(methylthio)-1,3,4-thiadiazol-2-yl)benzo[c]isoxazole-3-carboxamide (Compound 186)

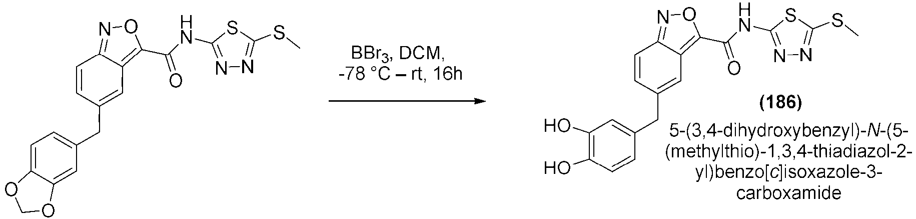

To a solution of 5-(benzo[*d*][1,3]dioxol-5-ylmethyl)-*N*-(5-(methylthio)-1,3,4-thiadiazol-2-yl)benzo[*c*]isoxazole-3-carboxamide (45.0 mg, 106 µmol) in dichloromethane (DCM) (2 mL) was added dropwise boron tribromide (BBr₃) (148 µL, 148 µmol) at -78 °C with stirring under N₂. The mixture was slowly warmed to room temperature over 0.5 h and stirred for 16 h at rt under N₂. LCMS showed presence of 25% of starting material. An additional 1 eq. of BBr₃ was added at -78 °C and slowly brought to room temperature and further stirred for 3 h. Water (10 mL) was added to quench the reaction and the DCM layer was separated. The aqueous phase was further extracted with DCM (2 x 10 mL) and the combined organics were washed with brine, and dried over Na₂SO₄. The crude product was subjected to flash chromatography through silica gel (0-10% MeOH/DCM) to give 5-(3,4-dihydroxybenzyl)-*N*-(5-(methylthio)-1,3,4-thiadiazol-2-yl)benzo[c]isoxazole-3-carboxamide (18.0 mg, 41 % yield) as a yellow solid. LCMS (ES, *m*/*z*) = 415.0 [M+1]⁺. ¹H NMR (400 MHz, DMSO-d₆): δ 8.79 (1H, s), 8.71 (1H, s), 7.72 (1H, d, *J* = 9.4 Hz), 7.34 (1H, d, *J* = 9.3 Hz), 6.65 (1H, d, *J =* 8.0 Hz), 6.59 (1H, s), 6.52 (1H, d, *J =* 8.1 Hz), 3.85 (2H, s), 2.74 (3H, s).

### Example 59: 5-(3-hydroxy-4-methoxybenzyl)-N-(5-(methylthio)-1,3,4-thiadiazol-2-yl)benzo[c]isoxazole-3-carboxamide (Compound 187)

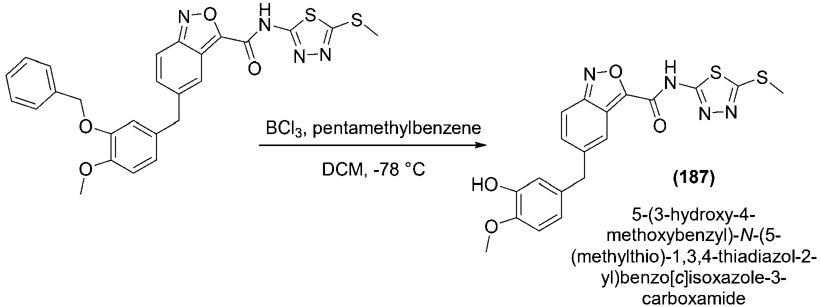

A flask under nitrogen gas was charged with 5-(3-(benzyloxy)-4-methoxybenzyl)*-*N-(5-(methylthio)-1,3,4-thiadiazol-2-yl)benzo[c]isoxazole-3-carboxamide (40.0 mg, 77.1 umol), pentamethylbenzene (39.4 µL, 231 µmol) and dichloromethane (DCM) (8.00 mL). After the reaction mixture was cooled to -78 °C (bath temperature), 1 M boron trichloride solution (154 µL, 154 µmol) is added to the flask dropwise over 5 min at -78 °C. After stirring for 2.5 h at -78 °C, the mixture was quenched by slow addition of chloroform/methanol (10/1, 2.0 mL) at -78 °C and it was allowed to warm to ambient temperature (50% conversion to desired product). The solution is concentrated on a rotary evaporator to afford crude product, which is purified by silica gel column chromatography, eluting from 100% DCM to 5% methanol (MeOH) in DCM over 20 min. The fraction containing the product were collected and concentrated in vacuo to give 5-(3-hydroxy-4-methoxybenzyl)-N-(5-(methylthio)-1,3,4-thiadiazol-2-yl)benzo[c]isoxazole-3-carboxamide (11.1 mg, 34 % yield) as a yellow solid. LCMS (ES, *m*/*z*) = 429.0 [M+1]⁺. ¹H NMR (400 MHz, DMSO-d₆): δ 8.87 (1H, s), 7.81 (1H, s), 7.73 (1H, d, *J =* 9.3 Hz), 7.34 (1H, d, *J =* 9.3 Hz), 6.84 (1H, d, *J =* 8.3 Hz), 6.65-6.67 (2H, m), 3.89 (2H, s), 3.71 (3H, s), 2.74 (3H, s).

### Example 60: 7-methyl-N-(5-(methylthio)-1,3,4-thiadiazol-2-yl)-4,5-dihydrothiazolo[5',4':5,6]benzo[1,2-c]isoxazole-3-carboxamide (Compound 181)

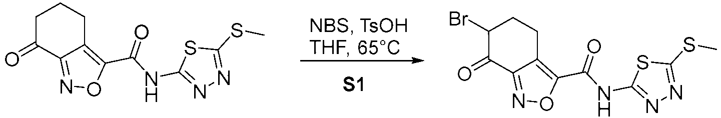

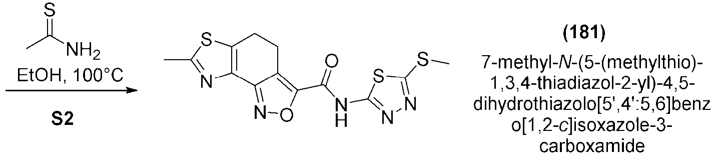

**Step 1:** A solution of *N*-(5-(methylthio)-1,3,4-thiadiazol-2-yl)-7-oxo-4,5,6,7-tetrahydrobenzo[*c*]-isoxazole-3-carboxamide (950 mg, 3.06 mmol), *N*-bromosuccinimide (NBS) (599 mg, 3.37 mmol) and *p*-toluenesulfonic acid monohydrate (TsOH) (58.2 mg, 306 µmol) in tetrahydrofuran (THF) (47 mL) was refluxed for 1h. Upon completion, the mixture was concentrated to dryness. The crude mixture was solubilized into 5 mL DMSO, then poured dropwise in 100 mL water and stirred overnight. Resulting precipitate was collected by filtration to yield 6-bromo-*N*-(5-(methylthio)-1,3,4-thiadiazol-2-yl)-7-oxo-4,5,6,7-tetrahydrobenzo[*c*]isoxazole-3-carboxamide (1.12 g, 94 % yield). ¹H NMR (CHCl₃-*d*, 400 MHz): δ 10.14 (1H, s), 4.74-4.76 (1H, m), 3.22-3.33 (2H, m), 2.80 (3H, s), 2.52-2.55 (2H, m).

**Step 2:** To a solution of 6-bromo-*N*-(5-(methylthio)-1,3,4-thiadiazol-2-yl)-7-oxo-4,5,6,7-tetrahydrobenzo[c]isoxazole-3-carboxamide (50.0 mg, 128 µmol) in ethanol (EtOH) (2 mL) was added thioacetamide (10.6 mg, 141 µmol). The mixture was heated via microwave at 100°C for 15 minutes. Upon completion, addition of water lead to a precipitate which was collected by filtration, rinsed with water and dried *in vacuo* to yield 7-methyl-N-(5-(methylthio)-,3,4-thiadiazol-2-yl)-4,5-dihydrothiazolo[5',4':5,6]benzo[,2-c]isoxazole-3-carboxamide-2 (35.0 mg, 75 % yield). LCMS (ES, *m*/*z)* = 366.0 [M+1]⁺. ¹H NMR (DMSO-d₆, 400 MHz): δ 3.18-3.19 (2H, m), 3.14-3.16 (2H, m), 2.71 (3H, s), 2.70 (3H, s).

### Example 61: 3-[[5-(methylsulfanyl)-1,3,4-thiadiazol-2-yl]carbamoyl]-2,1-benzoxazole-6-carboxylic acid (Compound 204)

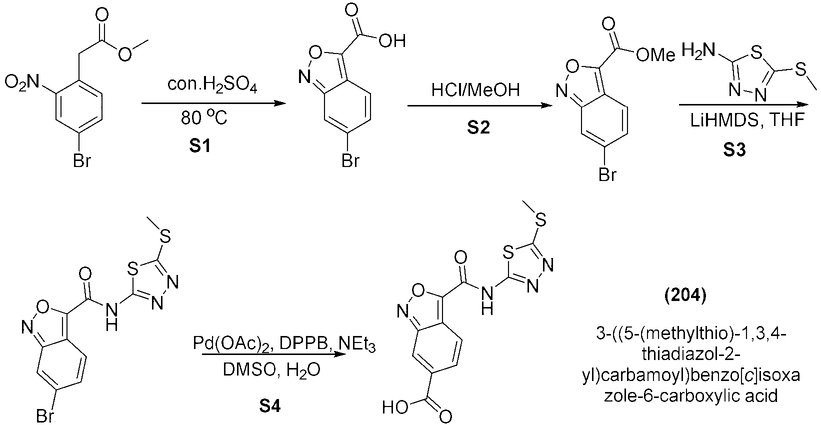

**Step 1:** A solution of methyl 2-(4-bromo-2-nitrophenyl)acetate (1.00 g, 3.649 mmol, 1.00 equiv) in H₂SO₄ (8.00 mL) was stirred for 3 h at 80 °C. The reaction was then quenched by the addition of 100 mL of water/ice. The resulting solution was extracted with ethyl acetate (EtOAc) (3 x 100 mL) and the combined organic layers were dried over anhydrous sodium sulfate. After filtration, the filtrate was concentrated under vacuum to provide 6-bromo-2,1-benzoxazole-3-carboxylic acid (800 mg, 90 % yield) as a brown solid. LCMS (ES, m/z) = 242[M+1]⁺.

**Step 2:** A solution of 6-bromo-2,1-benzoxazole-3-carboxylic acid (800.00 mg, 3.305 mmol, 1.00 equiv) in methanol (5.00 mL) and HCl (4 M) in 1,4-dioxane (5.00 mL) was stirred overnight at 25 °C. The resulting mixture was concentrated under vacuum. The residue was purified by flash chromatography through silica gel (7:13 ethyl acetate/petroleum ether) to provide methyl 6-bromo-2,1-benzoxazole-3-carboxylate (650 mg, 77 % yield) as an orange solid. LCMS (ES, m/z) = 256[M+1]⁺.

**Step 3:** Into a solution of methyl 6-bromo-2,1-benzoxazole-3-carboxylate (600.00 mg, 2.343 mmol, 1.00 equiv) in tetrahydrofuran (THF) (10.00 mL) was added 5-(methylsulfanyl)-1,3,4-thiadiazol-2-amine (414 mg, 2.81 mmol, 1.20 equiv) under nitrogen atmosphere followed by the addition of lithium bis(trimethylsilyl)amide (LiHMDS) (4.70 mL, 2.00 equiv) dropwise at 0 °C. The resulting mixture was stirred for additional 1 h at room temperature. The reaction was quenched with Water at room temperature. The resulting mixture was extracted with ethyl acetate (3 x 60 mL). The combined organic layers were washed with brine (2 x10 mL), dried over anhydrous sodium sulfate. After filtration, the filtrate was concentrated under reduced pressure. The precipitated solids were diluted by acetonitrile and stirred at rt for 3 h. The solid was collected by filtration and washed with acetonitrile (MeCN) (5 x 5 mL) to provide 6-bromo-N-[5-(methylsulfanyl)-1,3,4-thiadiazol-2-yl]-2,1-benzoxazole-3-carboxamide (570 mg, 65 % yield) as a yellow solid. LCMS (ES, m/z) = 371[M+1]⁺.

**Step 4:** Into a solution of 6-bromo-N-[5-(methylsulfanyl)-1,3,4-thiadiazol-2-yl]-2,1-benzoxazole-3-carboxamide (100 mg, 0.269 mmol, 1.00 equiv) in dimethylsulfoxide (DMSO) (3.00 mL) and H₂O (1.00 mL) were added Pd(OAc)₂ (12.10 mg, 0.054 mmol, 0.20 equiv), **1,4-bis (diphenylphosphino)butane** (DPPB) (22.98 mg, 0.054 mmol, 0.20 equiv) and triethylamine (NEt₃) (109.03 mg, 1.076 mmol, 4.00 equiv) at room temperature. The resulting solution was stirred overnight at 60 °C under carbon monoxide (CO) atmosphere. The resulting mixture was diluted with water (20 mL). The resulting mixture was extracted with EA (3 x 50 mL). The combined organic layers were washed with brine (4 mL), and dried over anhydrous Na₂SO₄. After filtration, the filtrate was concentrated under reduced pressure. The crude product was purified by Prep-HPLC with the following conditions (2#SHIMADZU (HPLC-01)): Column, Sunfire prep C18 column, 30*150, 5um; mobile phase, water (Phase A, 0.05% trifluoroacetic acid (TFA)) and acetonitrile (MeCN) (Phase B), Gradient: 25% Phase B up to 45% Phase B in 7 min; Detector, UV 254) to afford 3-[[5-(methylsulfanyl)-1,3,4-thiadiazol-2-yl]carbamoyl]-2,1-benzoxazole-6-carboxylic acid; trifluoroacetic acid (1.1 mg, 0.91 % yield) as a light yellow solid. LCMS (ES, m/z) = 337.0 [M+1]⁺. ¹H NMR (400 MHz, DMSO-d6) δ 8.41 (d, J = 5.6 Hz, 1H), 8.17 (s, 1H), 7.76 (d, J = 5.6Hz, 1H), 2.75 (s, 3H).

### Example 62: 5-(hydroxymethyl)-N-(5-(methylthio)-1,3,4-thiadiazol-2-yl)benzo[c]isoxazole-3-carboxamide (Compound 191)

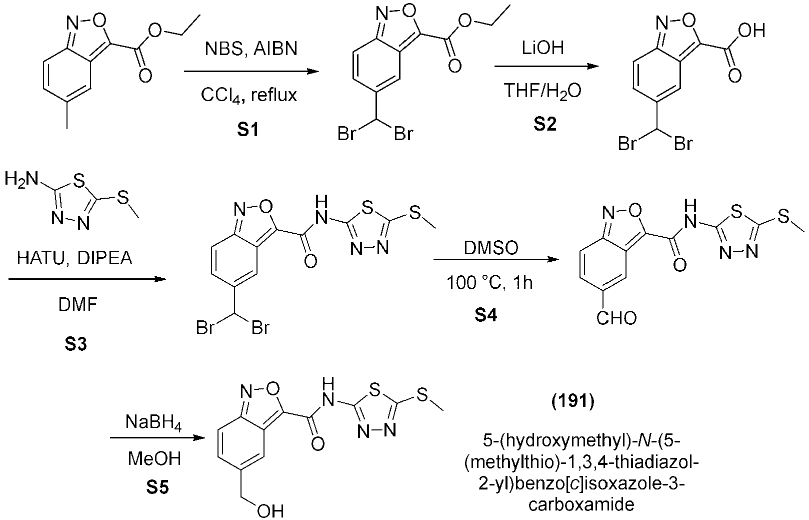

**Step 1:** To a solution of methyl 5-methylbenzo[c]isoxazole-3-carboxylate (1.96 g, 9.55 mmol) in carbon tetrachloride (CCl₄) (47 mL) was added N-bromosuccinimide (NBS) (2.04 g, 11.5 mmol) and 2,2'-azobis(2-methylpropionitrile) (AIBN) (157 mg, 955 µmol) at room temperature. The resulting mixture was stirred for 16 h at 80 °C. Additional NBS (1.6 g, 8.99 mmol) and AIBN (150 mg, 913 µmol) were added and continued stirring at 80 °C. After 35 h the reaction mixture was filtered through celite, the filtrate was concentrated under reduced pressure. The residue was purified by flash chromatography through silica gel (4:1 Hexanes/ ethyl acetate (EtOAc) to afford ethyl 5-(dibromomethyl)benzo[c]isoxazole-3-carboxylate (1.04 g, 30 % yield) as an off-white solid. LCMS (ES, *m*/*z*) = 363.9 [M+1]⁺. ¹H NMR (400 MHz, CHC13-d): δ 7.88 (1H, s), 7.81 (1H, d, *J=* 9.5 Hz), 7.74 (1H, d, *J = 9.5* Hz), 6.69 (1H, s), 4.51-4.58 (2H, m).

**Step 2:** To a solution of 5-(dibromomethyl)benzo[c]isoxazole-3-carboxylate (300 mg, 826 µmol) in tetrahydrofuran (THF) (11 mL) and H₂O (5.6 mL) at 0 °C, was added lithium hydroxide (LiOH) monohydrate (104 mg, 2.48 mmol) and the resulting mixture was stirred for 1 h at 0-10 °C. The reaction mixture was acidified with an acidic resin (Amberlist IR-120) , followed by filtration to remove resin. The resin was further rinsed with ethyl acetate (EtOAc) /MeOH and the filtrate was concentrated to give 5-(dibromomethyl)benzo[c]isoxazole-3-carboxylic acid (277 mg, 100% yield) as a yellow solid which was used in the next step without further purification. LCMS (ES, *m*/*z)* = 355.9 [M+1]⁺.

**Step 3:** *N*,*N*-Diisopropylethylamine (360 µL, 2.07 mmol) and 1-[Bis(dimethylamino)methylene]-1H-1,2,3-triazolo[4,5-b]pyridinium 3- oxid hexafluorophosphate (HATU) (377 mg, 992 umol) were added sequentially to a solution of 5-(dibromomethyl)benzo[*c*]isoxazole-3-carboxylic acid (277 mg, 827 µmol) in N,N-dimethylformamide (DMF) (7.2 mL) at 0 °C. The resulting mixture was stirred for 10 min. To the resultant solution, 2-amino-5-(methylthio)-1,3,4-thiadiazole (122 mg, 827 µmol) was added, the mixture was stirred at 0 °C for 10 min and then at rt for 90 min. The reaction was diluted with water and extracted with ethyl acetate (3 x). Combined organic layers were washed with brine and concentrated. The crude product was subjected to flash chromatography through silica gel (0-10% MeOH/ dichloromethane (DCM)) to give 5-(dibromomethyl)-*N*-(5-(methylthio)-1,3,4-thiadiazol-2-yl)benzo[*c*]isoxazole-3-carboxamide (180 mg, 43 % yield) as a yellow solid. LCMS (ES, *m*/*z*) = 464.9 [M+1]⁺. ¹H NMR (400 MHz, DMSO-*d*₆): δ 8.22 (1H, s), 7.96 (1H, d, *J =* 9.4 Hz), 7.76 (1H, d, *J =* 9.5 Hz), 7.58 (1H, s), 2.74 (3H, s).

**Step 4:** A solution of 5-(dibromomethyl)-*N*-(5-(methylthio)-1,3,4-thiadiazol-2-yl)benzo[*c*]isoxazole-3-carboxamide in DMSO (9 mL) was heated at 100 °C for 5 h. The reaction mixture was poured into water (30 mL), and the resulting mixture was extracted with ethyl acetate (60 mL x 2). The combined organic layers were washed with water, brine, dried over anhydrous sodium sulfate and concentrated under reduced pressure. The crude product was purified by flash chromatography through silica gel (0-10% MeOH/ dichloromethane (DCM) to give 5-formyl-*N*-(5-(methylthio)-1,3,4-thiadiazol-2-yl)benzo[c]isoxazole-3-carboxamide (80.0 mg, 58 % yield) as a light yellow solid. LCMS (ES, *m*/*z*) = 321.0 [M+1]⁺. ¹H NMR (400 MHz, DMSO-*d*₆): δ 10.08 (1H, s), 8.81 (1H, s), 7.93 (1H, d, *J =* 9.4 Hz), 7.83 (1H, d, *J =* 9.4 Hz), 2.74 (3H, s).

**Step 5:** 5-Formyl-*N*-(5-(methylthio)-1,3,4-thiadiazol-2-yl)benzo[c]isoxazole-3-carboxamide (20.0 mg, 62.4 µmol) was dissolved in methanol (MeOH) (5.0 mL) and sodium borohydride (NaBH₄) (7.1 mg, 187 µmol) was added to this solution in 3 portions at 0 °C at 1 h intervals. The mixture was stirred at 0-10 °C over the course of the reaction. After 3.5 h, saturated NH₄Cl solution was added, the mixture was extracted with ethyl acetate (EtOAc) (3 × 20 mL), the combined organic phases were washed with brine and dried over Na₂SO₄. The solvent was evaporated and the resulting residue was purified by flash chromatography through silica gel (0-10% MeOH/ dichloromethane (DCM)) to give 5-(hydroxymethyl)-*N*-(5-(methylthio)-1,3,4-thiadiazol-2-yl)benzo[c]isoxazole-3-carboxamide (14.0 mg, 70 % yield) as a light yellow solid. LCMS (ES, *m*/*z*) = 323.0 [M+1]⁺. ¹H NMR (400 MHz, DMSO-*d*₆): δ 7.96 (1H, s), 7.77 (1H, d, *J =* 9.3 Hz), 7.44 (1H, *d, J =* 9.3 Hz), 4.57 (2H, s), 2.74 (3H, s).

### Example 63: 7-Hydroxy-N-(5-(methylthio)-1,3,4-thiadiazol-2-yl)-4,5,6,7-tetrahydrobenzo[c]isoxazole-3-carboxamide (Compound 196)

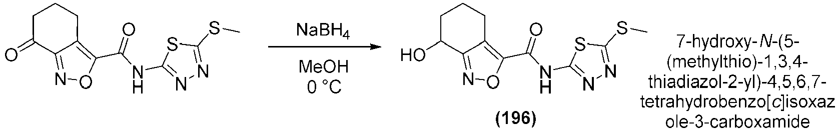

To a solution of *N*-(5-(methylthio)-1,3,4-thiadiazol-2-yl)-7-oxo-4,5,6,7-tetrahydrobenzo[*c*]isoxazole-3-carboxamide (25.0 mg, 80.6 umol) in MeOH (1 mL) at 0 °C was added sodium borohydride (4.0 mg, 106 umol). The reaction was stirred at 0 °C for 20 minutes. The reaction was poured in water (5 mL) and extracted with dichloromethane (DCM) (3 x 5 mL). The organic layers were combined, washed with brine, dried over MgSO₄ and concentrated. The compound was purified via C18 reverse phase chromatography (10-55% MeCN/water + 0.1% HCl) to give 7-hydroxy-*N*-(5-(methylthio)-1,3,4-thiadiazol-2-yl)-4,5,6,7-tetrahydrobenzo[*c*]isoxazole-3-carboxamide (12.0 mg, 48 % yield). LCMS (ES, *m*/*z*) = 313.0 [M+1]+. ¹H NMR (CHCl₃-*d*, 400 MHz): δ 5.11 (1H, dd, *J =* 8.6, 5.6 Hz), 2.98-3.04 (1H, m), 2.82-2.87 (1H, m), 2.79 (3H, s), 2.25-2.30 (1H, m), 2.06-2.12 (1H, m), 1.88 (1H, q, *J* = 11.0 Hz), 1.69-1.78 (1H, m).

### Example 64: 6-Methoxy-N-(5-(methylthio)-1,3,4-thiadiazol-2-yl)-4,5-dihydronaphtho[1,2-c]isoxazole-3-carboxamide (Compound 172)

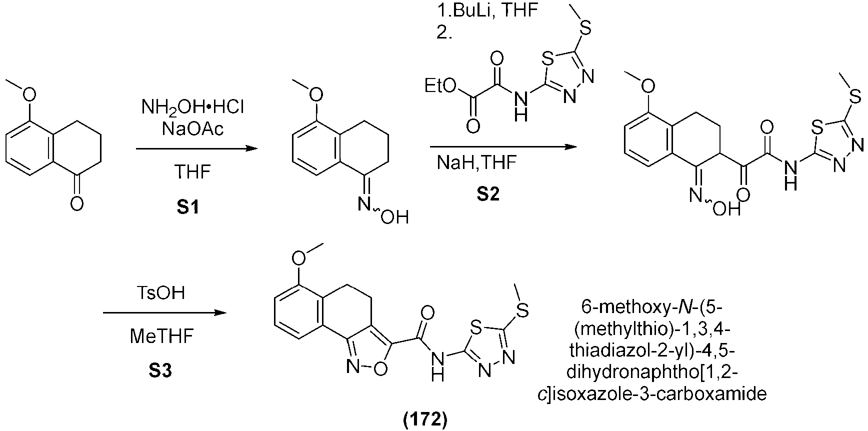

**Step 1:** In a 25 mL flask was introduced 5-methoxy-1-tetralone (2.00 g, 11.3 mmol), hydroxylamine hydrochloride (1.03 g, 14.8 mmol) and sodium acetate (1.12 g, 13.6 mmol) which were dissolved in methanol (MeOH) (8.4 mL). The reaction was reflux for 1 h. The reaction was quenched by addition of water (25 mL) at room temperature. The reaction was then extracted with ethyl acetate (EtOAc) (25 mL). The organic phase was washed with ammonium chloride (sat. solution), the combined aqueous phases were then back extracted with ethyl acetate (EtOAc), dried over magnesium sulfate and the solvent was removed *in vacuo* to afford 5-methoxy-3,4-dihydronaphthalen-1(2*H*)-one oxime (1.55 g, 71 % yield) as a grey solid. LCMS (ES, *m*/*z*) = 192.1 [M+H]⁺. ¹H NMR (DMSO-*d6*, 400 MHz) δ 11.04 (s; 1H); 7.45 (d; *J=* 7.97 Hz; 1H); 7.13 (t; *J=* 8.04 Hz; 1H); 6.89 (d; *J =* 8.09 Hz; 1H); 3.76 (s; 3H); 2.58-2.62 (m; 4H); 1.70 (t; *J =* 6.46 Hz; 2H).

**Step 2:** In a 50 mL round bottom flask was introduced 5-methoxy-3,4-dihydronaphthalen-1(2*H*)-one oxime (100 mg, 523 µmol) which was dissolved in tetrahydrofuran (THF) (3 mL). The solution was cooled down to -78 °C and butyl lithium (889 µL of 1.47 M in hexanes, 1.31 mmol) was added dropwise over 15 minutes while keeping the internal temperature below -65 °C. After addition of the first equivalent, solution turns bright orange. The solution was stirred for 45 minutes at -78 °C and 15 minutes at room temperature. In a second 25 mL flask equipped with a magnetic stirrer was added sodium hydride (58.6 mg of 60% oil disp., 1.46 mmol) which was then dissolved in THF (3 mL). Ethyl 2-((5-(methylthio)-1,3,4-thiadiazol-2-yl)amino)-2-oxoacetate (142 mg, 575 µmol) was added portion wise at 0 °C to the light yellow clear solution and the mixture was stirred for 30 minutes at room temp, giving a cloudy off-white suspension. The first solution was then cooled to -78 °C and the second cloudy white suspension of mixed oxalate was cannulated dropwise. Stirring was continued at -78 °C for 10 min. and then at room temperature for 30 minutes. The reaction mixture was cooled to -50 °C and acetic acid (1.50 mL, 26.1 mmol) in THF (5 mL) was added dropwise (gas evolution). The reaction mixture was allowed to warm up to room temperature, it was diluted with ammonium acetate solution (2M, 10 mL) and 2-methyl tetrahydrofuran (2-MeTHF) (10 mL). The organic phase was washed with brine, the combined aqueous phases were then back extracted with 2-MeTHF, dried over magnesium sulfate and the solvent was removed *in vacuo.* The crude product was purified by flash chromatography through silica gel (2:8 to 100% ethyl acetate (EtOAc) /hexanes) to afford 2-(1-(hydroxyimino)-5-methoxy-1,2,3,4-tetrahydronaphthale*N*-2-yl)-*N*-(5-(methylthio)-1,3,4-thiadiazol-2-yl)-2-oxoacetamide (97.0 mg, 47 % yield) as an off-white solid. LCMS (ES, *m*/*z*) = 393.1 [M+H]⁺. ¹H NMR (400 MHz, DMSO-*d6*,): δ 12.78 (bs; 1H); 7.98 (bs; 1H);7.30-7.31 (m; 1H); 7.24 (m; 1H); 7.01 (m, 1H); 3.84 (dd; *J =* 13.45; 4.95 Hz; 1H); 3.76 (s; 3H); 2.88 (m; 2H) 2.71 (s; 3H); 2.23 (m; 2H).

**Step 3:** A microwave vial was charged with molecular sieves (4 Å) and 2-(1-(hydroxyimino)-5-methoxy-1,2,3,4-tetrahydronaphthalen-2-yl)-*N*-(5-(methylthio)-1,3,4-thiadiazol-2-yl)-2-oxoacetamide (40.0 mg, 102 µmol) in 2-methyl tetrahydrofuran (2-MeTHF) (2.5 mL). *p-*Toluenesulfonic acid monohydrate (TsOH) (96.9 mg, 510 µmol) was then added and the reaction was heated for 75 minutes at 125 °C by microwave irradiation. Reaction was then quenched with saturated NaHCO₃ solution (3 mL). The organic phase was washed with saturated NaHCO₃ solution, the combined aqueous phases were then back extracted with 2-MeTHF, dried over magnesium sulfate and the solvent was removed *in vacuo.* The crude brown solid was redissolved in 0.7 mL of DMSO and purified by C18 reverse phase chromatography on semi prep HPLC using 0.1% Formic acid)/MeCN (9:1 to 2:8) to afford 6-methoxy-*N*-(5-(methylthio)-1,3,4-thiadiazol-2-yl)-4,5-dihydronaphtho[1,2-c]isoxazole-3-carboxamide (8.0 mg, 21 % yield) as a white fluffy solid. LCMS (ES, *m*/*z*) = 375.1. [M+H]⁺. ¹H NMR (400 MHz, DMSO) δ 7.48 (d; *J =* 7.69 Hz; 1H); 7.36 (t; *J =* 8.06 Hz; 1H); 7.14 (d; *J =* 8.24 Hz; 1H); 3.84 (s; 3H); 3.05 (t; *J =* 7.16 Hz; 2H); 2.92 (t; *J =* 7.16 Hz; 2H); 2.71 (s ; 3H).

### Example 65: N-(5-(methylthio)-1,3,4-thiadiazol-2-yl)-7-oxo-4,5,6,7-tetrahydrobenzo[c]isoxazole-3-carboxamide (Compound 195)

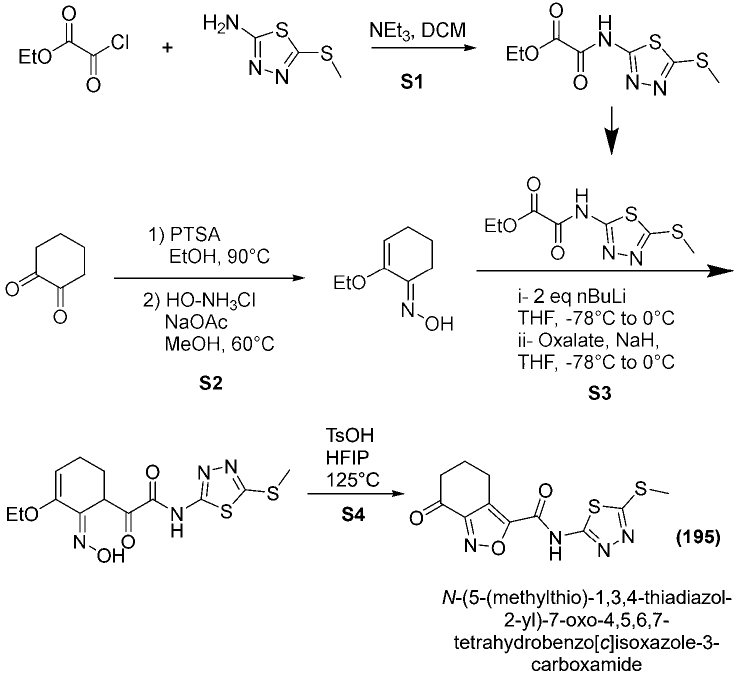

**Step 1:** To a solution of 2-amino-5-(methylthio)-1,3,4-thiadiazole (10.0 g, 67.9 mmol) and triethylamine (14.2 mL, 102 mmol) in dichloromethane (DCM) (160 mL) at 0 °C was added ethyl oxalyl chloride (7.97 mL, 71.3 mmol) dropwise. The solution was stirred for 1 h at rt. The organic layer was washed with 1N HCl (100 mL), dried on MgSO4 and concentrated to give ethyl 2-((5-(methylthio)-1,3,4-thiadiazol-2-yl)amino)-2-oxoacetate (16.4 g, 98 % yield). ¹H NMR (DMSO-*d*₆, 400 MHz): δ 4.30 (2H, q, *J=* 7.1 Hz), 2.72 (3H, s), 1.29 (3H, t, *J =* 7.1 Hz).

**Step 2:** A solution of 1,2-cyclohexanedione (25.0 g, 212 mmol) and *p*-toluenesulfonic acid monohydrate (4.03 g, 21.2 mmol) in ethanol (EtOH) (500 mL) was refluxed for 16 h. The reaction was cooled to rt and solvent was removed *in vacuo* to yield crude 2-ethoxycyclohex-2-en-1-one (34.5g, 116 %, contains residual EtOH). ¹H NMR (DMSO-*d*₆, 400 MHz): δ_{H} 5.97 (1H, t, *J=* 4.6 Hz), 3.66 (2H, q, *J =* 7.0 Hz), 2.31-2.38 (4H, m), 1.81-1.87 (2H, m), 1.21 (3H, t, *J =* 7.0 Hz). The crude 2-ethoxycyclohex-2-en-1-one was then diluted in MeOH (500 mL). Hydroxylamine hydrochloride (17.7 g, 254 mmol) and sodium acetate (17.2 g, 210 mmol) were added, and the mixture was heated to 65 °C. After 30 minutes, the mixture was concentrated down to approximately 150 mL, then sat. NaHCO₃ was added until pH 8. This mixture was extracted 6 x with dichloromethane (DCM). Combined organic layers were washed with brine, dried over MgSO₄ and concentrated to give 2-ethoxycyclohex-2-en-1-one oxime-1 (27.6 g, 84 % yield). The crude material was used as such without further purification. ¹H NMR (DMSO-d₆, 400 MHz): δ 10.87 (1H, s), 5.23 (1H, t, *J =* 4.6 Hz), 3.68 (2H, q, *J =* 7.0 Hz), 2.45 (2H, d, *J =* 6.7 Hz), 2.12 (2H, q, *J =* 5.5 Hz), 1.53-1.59 (2H, m), 1.19 (3H, t, *J =* 7.0 Hz).

**Step 3:** To a solution of 2-ethoxycyclohex-2-en-1-one oxime (5.00 g, 32.2 mmol) in tetrahydrofuran (THF) (140 mL) at -78 °C was added n-butyl lithium (27.1 mL, 67.7 mmol) dropwise over 15 minutes, keeping the internal temperature below -40 °C. The stirring was continued at -78 °C for 10 minutes, then the reaction mixture was allowed to warm up to 0 °C over 15 minutes. In a separate flask, ethyl 2-((5-(methylthio)-1,3,4-thiadiazol-2-yl)amino)-2-oxoacetate (8.76 g, 35.4 mmol) was solubilized in THF (55 mL), then cooled to 0 °C and treated with sodium hydride portion wise (1.93 g, 48.3 mmol) for 15 minutes. The oxime reaction mixture was then cooled to -78 °C, and the oxalate mixture was cannulated in the oxime mixture, keeping the internal temperature below -40 °C. The mixture was then stirred at -78 °C for 10 minutes, then warmed to 0 °C and stirred to 1.5 h. The mixture was quenched with 25% aqueous NH₄OAc until pH < 10 and stirred vigorously. THF was removed by evaporation and 300 mL 2-methyl tetrahydrofuran (2-MeTHF) was added. The mixture was stirred vigorously for 10 minutes and then filtered on a Buchner funnel. The filtrate separated in two layers and the aqueous layer was extracted with an extra 4 x 75 mL 2-MeTHF. Organic layers were combined, washed with brine, dried over MgSO₄ and concentrated *in vacuo* to give an orange foam. This crude material was purified by flash chromatography through silica gel (0-10% MeOH/ dichloromethane (DCM) to provide 2-(3-ethoxy-2-(hydroxyimino)cyclohex-3-en-1-yl)-*N*-(5-(methylthio)-1,3,4-thiadiazol-2-yl)-2-oxoacetamide (280 mg, 2.4 % yield). ¹H NMR (DMSO-*d*₆, 400 MHz): δ 5.41-5.43 (1H, m), 3.77-3.87 (2H, m), 3.73 (1H, dd, *J =* 13.9, 5.1 Hz), 2.68 (3H, s), 2.28-2.35 (2H, m), 1.60-1.72 (2H, m), 1.25 (3H, t, *J =* 7.0 Hz).

**Step 4:** A microwave was charged with molecular sieves, 2-(3-ethoxy-2-(hydroxyimino)cyclohex-3-en-1-yl)-*N*-(5-(methylthio)-1,3,4-thiadiazol-2-yl)-2-oxoacetamide (280 mg, 786 umol) and *p*-toluenesulfonic acid monohydrate (747 mg, 3.93 mmol) , HFIP (8.6 mL) and the resulting mixture was stirred for 10 minutes at 125 °C under microwave irradiation. Solvent was removed *in vacuo* and the crude product was purified by C18 reverse phase chromatography (0-100% MeCN/1% aqueous formic acid) to afford *N*-(5-(methylthio)-1,3,4-thiadiazol-2-yl)-7-oxo-4,5,6,7-tetrahydrobenzo[c]isoxazole-3-carboxamide (149 mg, 61 % yield) as a beige solid. LCMS (ES, *m*/*z*) = 311.0 [M+H] ⁺. ¹H NMR (400 MHz, CHCl₃-*d*): δ 3.14 (2H, t, *J =* 6.21 Hz;); 2.80 (3H, s); 2.77 (2H, t, *J =* 6.57 Hz); 2.23 (2H, t, *J =* 6.40 Hz).

### Example 66: 8-Methyl-N-(5-(methylthio)-1,3,4-thiadiazol-2-yl)-4,5-dihydroisoxazolo[4,3-h]quinazoline-3-carboxamide (Compound 174)

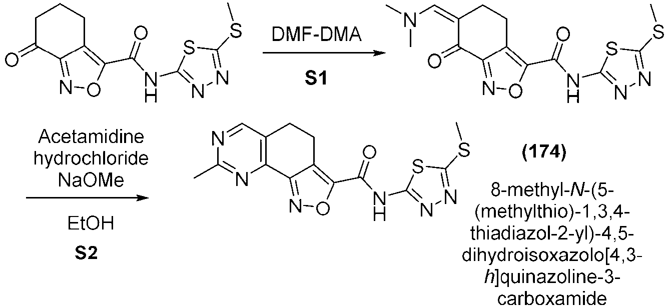

**Step 1:** In a 20 mL vial was introduced *N*-(5-(methylthio)-1,3,4-thiadiazol-2-yl)-7-oxo-4,5,6,7-tetrahydrobenzo[c]isoxazole-3-carboxamide (185 mg, 596 µmol) which was dissolved in DMSO (1.5 mL). *N*,*N*-Dimethylformamide dimethyl acetal (1.5 mL, 11.2 mmol) was then added and the reaction was left to stir at rt. After 6 hours, the reaction was quenched by addition of water followed by nBuOH. The organic phase was washed with water, the combined aqueous phases were then back extracted with nBuOH, dried over magnesium sulfate and the solvent was removed *in vacuo* to provide 6-((dimethylamino)methylene)-*N*-(5-(methylthio)-1,3,4-thiadiazol-2-yl)-7-oxo-4,5,6,7-tetrahydrobenzo[c]isoxazole-3-carboxamide (150 mg, 69 % yield) which was used in subsequent steps without further purification. LCMS (ES, *m*/*z*) = 366.0 [M+H] ⁺. ¹H NMR (400 MHz, DMSO-d6) δ 7.67 (s; 1H); 3.17 (s; 6 H); 3.00 (d; *J =* 6.70 Hz; 2H); 2.92 (d; *J =* 6.70 Hz; 2H); 2.71 (s; 3H).

**Step 2:** In a 10 mL microwave flask was introduced 6-((dimethylamino)methylene)-*N*-(5-(methylthio)-1,3,4-thiadiazol-2-yl)-7-oxo-4,5,6,7-tetrahydrobenzo[c]isoxazole-3-carboxamide (50.0 mg, 137 umol) which was dissolved in ethanol (EtOH) (2 mL). Sodium methoxide (29.6 mg, 547 umol) was then added followed by acetamidine hydrochloride (25.9 mg, 274 umol). The vial was capped and the solution was heated at 80 °C overnight. The brown cloudy mixture was quenched by addition of water, diluted with nBuOH (2 mL) and transferred in a separatory funnel. The organic phase was washed with NH₄OAc sat. solution, the combined aqueous phases were then back extracted with *n*BuOH, dried over magnesium sulfate and the solvent was removed *in vacuo.* The crude reddish solid was purified by C18 reverse phase chromatography (MeCN/ 1% aqueous formic acid; 1:9 to 6:4). Selected fractions were concentrated and the resulting residue was suspended in ethanol (EtOH) (1 mL). The suspension was triturated for two hours, collected by filtration on a Buchner funnel and dried under high vacuum overnight to afford 8-Methyl-*N*-(5-(methylthio)-1,3,4-thiadiazol-2-yl)-4,5-dihydroisoxazolo[4,3-h]quinazoline-3-carboxamide (3.90 mg, 6.9 % yield) as a white solid. LCMS (ES, m/z) = 361.0 [M+H] ⁺. ¹H NMR (400 MHz, DMSO-d6) δ 8.80 (s; 1H); 3.13 (t; *J =* 6.2Hz; 2H); 3.00 (t; *J =* 6.2Hz; 2H); 2.66 (s; 3H); 2.52 (s; 3H).

Additional compounds prepared following the above Examples are provided in Table A.

| **Table A. Additional compounds prepared** | | | |
|---|---|---|---|
| # | **Structure and Name** | **m/z [M+H]⁺** | **Preparation** |
| 3 | *tert*-butyl (5-((5-(2-chlorophenyl)-1,3,4-thiadiazol-2-yl)carbamoyl)isoxazol-3-yl)carbamate | 422.44 | Example **1** |
| 6 | *N*-(5-(2-chlorophenyl)-1,3,4-thiadiazol-2-yl)isoxazole-5-carboxamide | 307.18 | Example **1** |
| 8 | *N*-(5-(2,6-difluorophenyl)-1,3,4-thiadiazol-2-yl)isoxazolo[4,3-h]quinoline-3-carboxamide | 410.23 | Example **5** |
| 9 | *N*-(5-cyano-1,3,4-thiadiazol-2-yl)isoxazolo[4,3-h]quinoline-3-carboxamide | 323.25 | Example **5** |
| 10 | *N*-(5-(pyridin-2-yl)-1,3,4-thiadiazol-2-yl)isoxazolo[4,3-h]quinoline-3-carboxamide | 375.15 | Example **5** |
| 11 | *N*-(3-(methylthio)-1,2,4-thiadiazol-5-yl)isoxazolo[4,3-h]quinoline-3-carboxamide | 344.23 | Example **5** |
| 12 | *N*-(5-(trifluoromethyl)-1,3,4-thiadiazol-2-yl)isoxazolo[4,3-h]quinoline-3-carboxamide | 366.27 | Example **5** |
| 13 | *N*-(1,3,4-thiadiazol-2-yl)isoxazolo[4,3-h]quinoline-3-carboxamide | 298.22 | Example **5** |
| 14 | *N*-(5-(dimethylamino)-1,3,4-thiadiazol-2-yl)isoxazolo[4,3-h]quinoline-3-carboxamide | 341.31 | Example **4** |
| 15 | methyl 5-((5-(methylthio)-1,3,4-thiadiazol-2-yl)carbamoyl)isoxazole-3-carboxylate | 301.20 | Example **1** |
| 16 | 5-((5-(methylthio)-1,3,4-thiadiazol-2-yl)carbamoyl)isoxazole-3-carboxylic acid | 287.19 | Example **2** |
| 17 | 3-cyclopropyl-N-(5-(methylthio)- 1,3,4-thiadiazol-2-yl)isoxazole-5-carboxamide | 283.14 | Example **18** |
| 18 | *N*-(5-(difluoromethyl)-1,3,4-thiadiazol-2-yl)benzo[c]isoxazole-3-carboxamide | 297.09 | Example **18** |
| 19 | *N*-(5-phenyl-1,3,4-thiadiazol-2-yl)benzo[c]isoxazole-3-carboxamide | 368.27 | Example **1** |
| 20 | *N-*(5-propyl-1,3,4-thiadiazol-2-yl)benzo[c]isoxazole-3-carboxamide | 289.25 | Example **1** |
| 21 | *N-*(5-(pyridin-2-yl)-1,3,4-thiadiazol-2-yl)benzo[c]isoxazole-3-carboxamide | 324.18 | Example **1** |
| 22 | *N*-(5-(2-chlorophenyl)-1,3,4-thiadiazol-2-yl)benzo[c]isoxazole-3-carboxamide | 357.18 | Example **1** |
| 23 | *N*-(5-(3-chlorophenyl)-1,3,4-thiadiazol-2-yl)benzo[c]isoxazole-3-carboxamide | 357.18 | Example **1** |
| 24 | *N*-(5-(2-methoxyphenyl)-1,3,4-thiadiazol-2-yl)benzo[c]isoxazole-3-carboxamide | 353.19 | Example **1** |
| 25 | *N*-(5-(3-fluorophenyl)-1,3,4-thiadiazol-2-yl)benzo[c]isoxazole-3-carboxamide | 341.31 | Example **1** |
| 26 | *N*-(5-(2-fluorophenyl)-1,3,4-thiadiazol-2-yl)benzo[c]isoxazole-3-carboxamide | 341.31 | Example **1** |
| 27 | *N*-(5-(o-tolyl)-1,3,4-thiadiazol-2-yl)benzo[c]isoxazole-3-carboxamide | 337.32 | Example **1** |
| 28 | *N*-(5-(m-tolyl)-1,3,4-thiadiazol-2-yl)benzo[c]isoxazole-3-carboxamide | 337.26 | Example **1** |
| 29 | *N*-(5-(p-tolyl)-1,3,4-thiadiazol-2-yl)benzo[c]isoxazole-3-carboxamide | 337.32 | Example **1** |
| 30 | *N*-(5-(3-hydroxyphenyl)-1,3,4-thiadiazol-2-yl)benzo[c]isoxazole-3-carboxamide | 339.38 | Example **18** |
| 31 | *N*-(5-(2-chlorophenyl)-1,3,4-thiadiazol-2-yl)-3-methoxyisoxazole-5-carboxamide | 337.32 | Example **18** |
| 32 | *N*5-(5-(2-chlorophenyl)-1,3,4-thiadiazol-2-yl)isoxazole-3,5-dicarboxamide | 350.27 | Example **18** |
| 33 | *N*-(5-(2-chlorophenyl)-1,3,4-thiadiazol-2-yl)-3-ethylisoxazole-5-carboxamide | 335.27 | Example **18** |
| 34 | *N*-(5-(2-chlorophenyl)-1,3,4-thiadiazol-2-yl)-3-(trifluoromethyl)isoxazole-5-carboxamide | 375.17 | Example **18** |
| 35 | *N*-(5-(2-chlorophenyl)-1,3,4-thiadiazol-2-yl)-3-(difluoromethyl)isoxazole-5-carboxamide | 357.24 | Example **18** |
| 36 | *N*-(5-(2-chlorophenyl)-1,3,4-thiadiazol-2-yl)-3-methylisoxazole-5-carboxamide | 321.26 | Example **18** |
| 37 | *N*-(5-(2-chlorophenyl)-1,3,4-thiadiazol-2-yl)-3-cyanoisoxazole-5-carboxamide | 332.28 | Example **18** |
| 38 | *N*-(5-(2-chlorophenyl)-1,3,4-thiadiazol-2-yl)-3-(tetrahydro-2H-pyran-4-yl)isoxazole-5-carboxamide | 391.24 | Example **18** |
| 39 | *N*-(5-(2-chlorophenyl)- 1,3,4-thiadiazol-2-yl)-3-cyclopropylisoxazole-5-carboxamide | 347.28 | Example **18** |
| 40 | *N*-(5-(2-chlorophenyl)-1,3,4-thiadiazol-2-yl)-3-isopropylisoxazole-5-carboxamide | 349.28 | Example **18** |
| 41 | *N*-(5-(2-chlorophenyl)-1,3,4-thiadiazol-2-yl)-3-(3-methyloxetan-3-yl)isoxazole-5-carboxamide | 377.16 | Example **18** |
| 42 | 1-benzyl-N-(5-(methylthio)-1,3,4-thiadiazol-2-yl)-1*H*-1,2,3-triazole-4-carboxamide | 333.21 | Example **1** |
| 43 | *tert*-butyl 3-((5-(methylthio)-1,3,4-thiadiazol-2-yl)carbamoyl)-6,7-dihydroisoxazolo[4,3-c]pyridine-5(4*H*)-carboxylate | 398.14 | Example **1** |
| 44 | 5-(3-chlorobenzyl)-*N*-(5-(methylthio)-1,3,4-thiadiazol-2-yl)-1,3,4-oxadiazole-2-carboxamide | 368.27 | Example **18** |
| 45 | 5-(2,6-difluorophenyl)-*N*-(5-(methylthio)-1,3,4-thiadiazol-2-yl)-1,3,4-oxadiazole-2-carboxamide | 356.18 | Example **18** |
| 46 | 3-(2,6-difluorophenyl)*-*N-(5-(methylthio)-1,3,4-thiadiazol-2-yl)isoxazole-5-carboxamide | 355.19 | Example **18** |
| 47 | 3-(2,5-difluorophenyl)-*N*-(5-(methylthio)-1,3,4-thiadiazol-2-yl)isoxazole-5-carboxamide | 355.12 | Example **18** |
| 48 | 3-(2,4-difluorophenyl)-*N*-(5-(methylthio)-1,3,4-thiadiazol-2-yl)isoxazole-5-carboxamide | 355.19 | Example **18** |
| 49 | 5-(2-chlorobenzyl)-*N*-(5-(methylthio)-1,3,4-thiadiazol-2-yl)-1,3,4-oxdiazole-2-carboxamide | 368.20 | Example **18** |
| 50 | 3-(4-chlorophenyl)-*N*-(5-(methylthio)-1,3,4-thiadiazol-2-yl)isoxazole-5-carboxamide | 353.19 | Example **18** |
| 51 | *N*-(5-(2-chlorophenyl)-1,3,4-thiadiazol-2-yl)-8-hydroxy-4-oxo-1,4-dihydroquinoline-2-carboxamide | 399.27 | Example **18** |
| 52 | *N*-(5-(2-chlorophenyl)-1,3,4-thiadiazol-2-yl)-3-(1-methyl-1H-pyrazol-4-yl)isoxazole-5-carboxamide | 387.32 | Example **18** |
| 53 | *N*-(5-(2-chlorophenyl)-1,3,4-thiadiazol-2-yl)-3-(1,5-dimethyl-1H-pyrazol-4-yl)isoxazole-5-carboxamide | 401.33 | Example **18** |
| 54 | *N*-(5-(2-chlorophenyl)- 1,3,4-thiadiazol-2-yl)-3-(1,3-dimethyl-1H-pyrazol-4-yl)isoxazole-5-carboxamide | 401.40 | Example **18** |
| 55 | *N*-(5-(2-chlorophenyl)- 1,3,4-thiadiazol-2-yl)-3-hydroxyisoxazole-5-carboxamide | 323.18 | Example **1** |
| 56 | *N*-(5-(2-chlorophenyl)-1,3,4-thiadiazol-2-yl)-6-hydroxypyridazine-3-carboxamide | 334.20 | Example **18** |
| 57 | *N*-(5-(2,3-dichlorophenyl)-1,3,4-thiadiazol-2-yl)-3-hydroxyisoxazole-5-carboxamide | 357.18 | Example **1** |
| 58 | *N*-(5-(2-chlorophenyl)-1,3,4-thiadiazol-2-yl)-1H-1,2,4-triazole-3-carboxamide | 307.25 | Example **18** |
| 59 | *N*-(5-(2-chlorophenyl)-1,3,4-thiadiazol-2-yl)-1H-1,2,3-triazole-4-carboxamide | 307.25 | Example **18** |
| 60 | *N*-(5-(2-chlorophenyl)-1,3,4-thiadiazol-2-yl)-1H-tetrazole-5-carboxamide | 306.30 | Example **1** |
| 61 | *N*-(5-(2-chlorophenyl)-1,3,4-thiadiazol-2-yl)-2-oxo-2,3-dihydrooxazole-5-carboxamide | 323.18 | Example **18** |
| 62 | *N*-(5-(2-chlorophenyl)-1,3,4-thiadiazol-2-yl)-2-oxo-2,3-dihydropyrimidine-4-carboxamide | 334.20 | Example **18** |
| 63 | *N*-(5-(2-chlorophenyl)-1,3,4-thiadiazol-2-yl)-5-oxo-4,5-dihydro-1H-1,2,4-triazole-3-carboxamide | 323.12 | Example **18** |
| 65 | *N*-(5-(methylthio)-1,3,4-thiadiazol-2-yl)-4-phenylthiazole-2-carboxamide | 335.20 | Example **1** |
| 66 | *N*-(5-(methylthio)-1,3,4-thiadiazol-2-yl)-2-phenyl-2H-tetrazole-5-carboxamide | 320.13 | Example **1** |
| 67 | *N*-(5-(methylthio)-1,3,4-thiadiazol-2-yl)-4,5,6,7-tetrahydrobenzo[c]isoxazole-3-carboxamide | 296.95 | Example **1** |
| 68 | 3-(2,3-dihydro-1*H*-inden-4-yl)-*N*-(5-(methylthio)-1,3,4-thiadiazol-2-yl)isoxazole-5-carboxamide | 359.24 | Example **1** |
| 69 | *N*-(5-ethyl-1,3,4-thiadiazol-2-yl)benzo[c]isoxazole-3-carboxamide | 274.84 | Example **1** |
| 70 | *N*-(5-(ethylthio)-1,3,4-thiadiazol-2-yl)benzo[c]isoxazole-3-carboxamide | 307.05 | Example **1** |
| 71 | *N-*(5-(methylthio)-1,3,4-thiadiazol-2-yl)-3-(pyrazin-2-yl)-1,2,4-oxadiazole-5-carboxamide | 322.19 | Example **26** |
| 72 | *N*-(5-(methylthio)-1,3,4-thiadiazol-2-yl)-[2,3'-bipyridine]-5'-carboxamide | 329.95 | Example **1** |
| 74 | *N*-(5-(methylthio)-1,3,4-thiadiazol-2-yl)-5-(2-phenylazetidin-1-yl)-1,3,4-oxadiazole-2-carboxamide | 375.24 | Example **20** |
| 75 | *N*-(5-(methylthio)-1,3,4-thiadiazol-2-yl)-3-(thiophen-2-yl)isoxazole-5-carboxamide | 324.95 | Example **1** |
| 76 | *N*-(5-(methylthio)-1,3,4-thiadiazol-2-yl)-2-(thiophen-2-yl)thiazole-5-carboxamide | 341.15 | Example **1** |
| 77 | 5-methyl-*N*-(5-(methylthio)-1,3,4-thiadiazol-2-yl)-2-(thiophen-2-yl)oxazole-4-carboxamide | 339.03 | Example **1** |
| 78 | 6-fluoro-4-methyl-*N*-(5-(methylthio)-1,3,4-thiadiazol-2-yl)-1*H*-indole-2-carboxamide | 323.25 | Example **1** |
| 79 | *N*-(5-(methylthio)-1,3,4-thiadiazol-2-yl)benzo[c]isoxazole-3-carboxamide | 293.17 | Example **1** |
| 80 | *N*-(5-(methylthio)-1,3,4-thiadiazol-2-yl)-3-(tetrahydro-2*H-*pyran-4-yl)isoxazole-5-carboxamide | 327.23 | Example **1** |
| 81 | *N*-(5-methyl-1,3,4-thiadiazol-2-yl)benzo[c]isoxazole-3-carboxamide | 261.16 | Example **1** |
| 82 | 3-isopropyl-*N*-(5-(methylthio)-1,3,4-thiadiazol-2-yl)isoxazole-5-carboxamide | 285.20 | Example **1** |
| 83 | *N*-(5-(methylthio)-1,3,4-thiadiazol-2-yl)-3-(3-(trifluoromethoxy)phenvl)isoxazole-5-carboxamide | 402.99 | Example **24** |
| 84 | 3-cyclohexyl-*N*-(5-(methylthio)-1,3,4-thiadiazol-2-yl)isoxazole-5-carboxamide | 324.97 | Example **24** |
| 85 | 3-cyclopentyl-*N*-(5-(methylthio)-1,3,4-thiadiazol-2-yl)isoxazole-5-carboxamide | 311.03 | Example **24** |
| 86 | 3-cyclobutyl-*N*-(5-(methylthio)-1,3,4-thiadiazol-2-yl)isoxazole-5-carboxamide | 297.09 | Example **24** |
| *8*8 | *N*5-(5-(2-chlorophenyl)-1,3,4-thiadiazol-2-yl)-*N*3-(2-methoxyethyl)isoxazole-3,5-dicarboxamide | 408.30 | Example **53** |
| 89 | *N*5-(5-(2-chlorophenyl)-1,3,4-thiadiazol-2-yl)-*N*3-methylisoxazole-3,5-dicarboxamide | 364.28 | Example **53** |
| 90 | *N*5-(5-(2-chlorophenyl)-1,3,4-thiadiazol-2-yl)-*N*3-hydroxyisoxazole-3,5-dicarboxamide | 366.27 | Example **53** |
| 92 | *N*5-(5-(2-chlorophenyl)-1,3,4-thiadiazol-2-yl)-*N*3-phenylisoxazole-3,5-dicarboxamide | 426.23 | Example **3** |
| 100 | 5-(3,3-dimethylpiperidin-1-yl)-*N*-(5-(methylthio)-1,3,4-thiadiazol-2-yl)-1,2,4-oxadiazole-3-carboxamide | 355.2 | Example **25** |
| 101 | *N*-[5-(methylthio)-1,3,4 -thiadiazol-2-yl]-5-(piperidin-1-yl)-1,2,4-oxadiazole-3-carboxamide | 327.1 | Example **25** |
| 102 | 5-(4,4-dimethylpiperidin-1-yl)-*N*-(5-(methylthio)-1,3,4-thiadiazol-2-yl)-1,2,4-oxadiazole-3-carboxamide | 355.2 | Example **25** |
| 103 | *N*-(5-(methylthio)-1,3,4-thiadiazol-2-yl)-5-(2-phenylpiperidin-1-yl)-1,2,4-oxadiazole-3-carboxamide | 403.2 | Example **25** |
| 105 | *N*-[5-(methylsulfanyl)-1,3,4-thiadiazol-2-yl]-5-(pyridin-3-yl)-1,2,4-oxadiazole-3-carboxamide | 321.0 | Example **26** |
| 107 | *N*-[5-(methylsulfanyl)-1,3,4-thiadiazol-2-yl]-5-(pyridin-3-yl)-1,3,4-oxadiazole-2-carboxamide | 321.0 | Example **26** |
| 109 | 5-(3-hydroxypiperidin-1-yl)-*N*-(5-(methylthio)-1,3,4-thiadiazol-2-yl)-1,3,4-oxadiazole-2-carboxamide | 343.1 | Example **23** |
| 111 | 1-(5-{[5-(methylsulfanyl)-1,3,4-thiadiazol-2-yl]carbamoyl}-1,3,4-oxadiazol-2-yl)pyrrolidine-2-carboxylic acid | 357.0 | Example **21** |
| 112 | 1-(5-{[5-(methylsulfanyl)-1,3,4-thiadiazol-2-yl]carbamoyl}-1,3,4-oxadiazol-2-yl)pyrrolidine-3-carboxylic acid | 357.0 | Example **21** |
| 113 | 5-[benzyl(methyl)amino]-*N*-[5-(methylsulfanyl)-1,3,4-thiadiazol-2-yl]-1,3,4-oxadiazole-2-carboxamide | 363.1 | Example **23** |
| 114 | 5-[methyl(1-phenylethyl)amino]-*N*-[5-(methylsulfanyl)-1,3,4-thiadiazol-2-yl]-1,3,4-oxadiazole-2-carboxamide | 377.0 | Example **23** |
| 115 | 5-[2-(hydroxymethyl)pyrrolidin-1-yl]-*N*-[5-(methylsulfanyl)-1,3,4-thiadiazol-2-yl]-1,3,4-oxadiazole-2-carboxamide | 343.0 | Example **23** |
| 116 | 5-(3-hydroxypyrrolidin-1-yl)-*N*-[5-(methylsulfanyl)-1,3,4-thiadiazol-2-yl]-1,3,4-oxadiazole-2-carboxamide | 329.0 | Example **23** |
| 117 | *(R)*-*N*-(5-(2-chlorophenyl)-1,3,4-thiadiazol-2-yl)-5-(2-(pyridin-2-yl)pyrrolidin-1-yl)-1,3,4-oxadiazole-2-carboxamide | 454.2 | Example **23** |
| 118 | (*R*)-*N*-(5-(2-chlorophenyl)-1,3,4-thiadiazol-2-yl)-5-(2-phenylpyrrolidin-1-yl)-1,3,4-oxadiazole-2-carboxamide | 453.1 | Example **23** |
| 119 | 5-(3,3-dimethylpiperidin-1-yl)-*N*-(5-(methylthio)-1,3,4-thiadiazol-2-yl)-1,3,4-oxadiazole-2-carboxamide | 355.1 | Example **23** |
| 120 | 5-(4,4-dimethylpiperidin-1-yl)-N-(5-(methylthio)-1,3,4-thiadiazol-2-yl)-1,3,4-oxadiazole-2-carboxamide | 355.1 | Example **23** |
| 123 | *N*-(5-(methylthio)-1,3,4-thiadiazol-2-yl)-5-(2-phenylpyrrolidin-1-yl)-1,3,4-oxadiazole-2-carboxamide | 389.1 | Example **23** |
| 124 | *N*-(5-(methylthio)-1,3,4-thiadiazol-2-yl)-5-(3-oxopiperazin-1-yl)-1,3,4-oxadiazole-2-carboxamide | 342.0 | Example **23** |
| 125 | (*R*)-*N*-(5-(methylthio)-1,3,4-thiadiazol-2-yl)-5-(2-phenylpyrrolidin-1-yl)-1,3,4-oxadiazole-2-carboxamide | 389.2 | Example **23** |
| 126 | (*S*)-*N*-(5-(methylthio)-1,3,4-thiadiazol-2-yl)-5-(2-phenylpyrrolidin-1-yl)-1,3,4-oxadiazole-2-carboxamide | 389.1 | Example **23** |
| 128 | *N*-(5-(thiazol-5-yl)-1,3,4-thiadiazol-2-yl)benzo[c]isoxazole-3-carboxamide | 330.1 | Example **37** |
| 129 | *N*-[5-(1*H*-imidazol-4-yl)-1,3,4-thiadiazol-2-yl]-2,1-benzoxazole-3-carboxamide | 313.1 | Example **37** |
| 131 | *N*-(5-(thiazol-2-yl)-1,3,4-thiadiazol-2-yl)benzo[c]isoxazole-3-carboxamide | 330.0 | Example **37** TFA salt isolated |
| 134 | 2-benzyl -*N*-(5-(methylthio)-1,3,4-thiadiazol-2-yl)-2*H-*tetrazole-5-carboxamide | 334.1 | Example **29** |
| 135 | *N*-(5-(methylthio)-1,3,4-thiadiazol-2-yl)-2-(pyridin-3-ylmethyl)-2*H*-tetrazole-5-carboxamide | 335.0 | Example **29** |
| 138 | 2-(2-fluorophenyl)-*N*-[5-(methylsulfanyl)-1,3,4-thiadiazol-2-yl]-1,2,3,4-tetrazole-5-carboxamide | 338.0 | Example **31** |
| 140 | *N*-[5-(methylsulfanyl)-1,3,4-thiadiazol-2-yl]-3-(1,2-thiazol-5-yl)-1,2-oxazole-5-carboxamide | 326.0 | Example **24** |
| 141 | *N*-(5-(methylthio)-1,3,4-thiadiazol-2-yl)-3-(thiazol-5-yl)isoxazole-5-carboxamide | 325.9 | Example **24** TFA salt isolated |
| 142 | *N*-(5-(methylthio)-1,3,4-thiadiazol-2-yl)-3-(thiazol-2-yl)isoxazole-5-carboxamide | 326.0 | Example **24** |
| 144 | *N*-[5-(2-bromophenyl)-1,3,4-thiadiazol-2-yl]- 2,1-benzoxazole-3-carboxamide | 401.0 | Example **54** |
| 147 | *N*-(5-(2-(difluoromethyl)phenyl)-1,3,4-thiadiazol-2-yl)benzo[c]isoxazole-3-carboxamide | 373.1 | Example **54** |
| 149 | *N*-(5-(methylthio)-1,3,4-thiadiazol-2-yl)-5-(2-phenylpiperidin-1-yl)-1,3,4-oxadiazole-2-carboxamide | 403.1 | Example **23** |
| 151 | 3-(2,3-Difluorophenyl)-*N*-(5-(methylthio)-1,3,4-thiadiazol-2-yl)isoxazole-5-carboxamide | 355.0 | Example **10** |
| 152 | *N*-(5-(Methylthio)-1,3,4-thiadiazol-2-yl)-3-(pyrimidin-2-yl)isoxazole-5-carboxamide | 321.0 | Example **10** |
| 153 | *N*-(5-(Methylthio)-1,3,4-thiadiazol-2-yl)-3-(quinazolin-2-yl)isoxazole-5-carboxamide | 371.0 | Example **10** |
| 154 | *N*-(5-(2-Chlorophenyl)-1,3,4-thiadiazol-2-yl)-3-(pyrimidin-2-yl)isoxazole-5-carboxamide | 385.0 | Example **10** |
| 155 | *N*-(5-(2-Chlorophenyl)-1,3,4-thiadiazol-2-yl)-3-(quinazolin-2-yl)isoxazole-5-carboxamide | 435.0 | Example **10** |
| 156 | *N*-(5-(2-Chlorophenyl)-1,3,4-thiadiazol-2-yl)-3-(5-fluoropyrimidin-2-yl)isoxazole-5-carboxamide | 403.0 | Example **10** |
| 157 | *N*-(5-(2-Chlorophenyl)-1,3,4-thiadiazol-2-yl)-3-(4-methylpyrimidin-2-yl)isoxazole-5-carboxamide | 399.0 | Example **10** |
| 158 | *N*-(5-(2-Chlorophenyl)-1,3,4-thiadiazol-2-yl)-3-(5-methylpyrimidin-2-yl)isoxazole-5-carboxamide | 399.0 | Example **10** |
| 159 | 5-Benzyl-*N*-(5-(methylthio)-1,3,4-thiadiazol-2-yl)benzo[c]isoxazole-3-carboxamide | 383.0 | Example **11** |
| 160 | 5-(4-Methoxybenzyl)-*N*-(5-(methylthio)-1,3,4-thiadiazol-2-yl)benzo[*c*]isoxazole-3-carboxamide | 413.0 | Example **11** |
| 161 | 5-(3-methoxybenzyl)-*N*-(5-(methylthio)-1,3,4-thiadiazol-2-yl)benzo[*c*]isoxazole-3-carboxamide | 413.1 | Example **11** |
| 171 | *N*-(5-(methylthio)-1,3,4-thiadiazol-2-yl)-4,5-dihydrothiazolo[5',4':5,6]benzo[1,2-c]isoxazole-3-carboxamide | 352.0 | Example **60** |
| 173 | *N*-(5-(Methylthio)-1,3,4-thiadiazol-2-yl)-4,5-dihydroisoxazolo[4,3-*h*]quinoline-3-carboxamide | 346.0 | Example **64** |
| 177 | 2-(2'-(6-((5-(methylthio)-1,3,4-thiadiazol-2-yl)carbamoyl)-2-oxo-2*H*-pyran-4-yl)-[1,1'-biphenyl]-3-yl)acetic acid | 480.0 | Example **48** |
| 188 | 5-(4-hydroxybenzyl)-*N*-(5-(methylthio)-1,3,4-thiadiazol-2-yl)benzo[*c*]isoxazole-3-carboxamide | 399.0 | Example **59** |
| 189 | 5-((5-Hydroxy-6-oxo-1,6-dihydropyridin-3-yl)methyl)-*N*-(5-(methylthio)-1,3,4-thiadiazol-2-yl)benzo[*c*]isoxazole-3-carboxamide | 416.1 | Example **59** |
| 193 | *N*6-methyl-*N*3-(5-(methylthio)-1,3,4-thiadiazol-2-yl)benzo[c]isoxazole-3,6-dicarboxamide | 350.1 | Example **13** |
| 197 | *N*-(5-ethyl-1,3,4-thiadiazol-2-yl)isoxazolo[4,3-h]quinoline-3-carboxamide | 326.24 | Example **5** |
| 198 | 1-(3,3-dimethylbutyl)-*N*-(5-(methylthio)-1,3,4-thiadiazol-2-yl)-1*H*-1,2,3-triazole-4-carboxamide | 326.97 | Example **1** |
| 199 | 8-hydroxy-*N*-(5-(methylthio)-1,3,4-thiadiazol-2-yl)-4-oxo-1,4-dihydroquinoline-2-carboxamide | 333.21 [M-1]- | Example **1** |
| 200 | 4-hydroxy-*N*-(5-(methylthio)-1,3,4-thiadiazol-2-yl)-6-phenylpicolinamide | 345.23 | Example **1** |
| 201 | *N*-(5-(methylthio)-1,3,4-thiadiazol-2-yl)-6-phenylpyrimidine-4-carboxamide | 330.22 | Example **1** |
| 207 | *N*-(5-(methylthio)-1,3,4-thiadiazol-2-yl)-1-oxo-1*H-*isochromene-3-carboxamide | 320.05 | Example **16** |
| 208 | *N*-[5-(methylsulfanyl)-1,3,4-thiadiazol-2-yl]-3-phenyl-1,2-oxazole-5-carboxamide | 319.02 | Example **1** |
| 209 | 3-(3-chlorophenyl)-*N*-[5-(methylsulfanyl)-1,3,4-thiadiazol-2-yl]-1,2-oxazole-5-carboxamide | 352.95 | Example **1** |
| 210 | 3-(4-methoxyphenyl)-*N*-[5-(methylsulfanyl)-1,3,4-thiadiazol-2-yl]-1,2-oxazole-5-carboxamide | 349.05 | Example **1** |
| 211 | 3-(3-fluorophenyl)-*N*-[5-(methylsulfanyl)-1,3,4-thiadiazol-2-yl]-1,2-oxazole-5-carboxamide | 337.01 | Example **1** |
| 212 | 3-(3-methoxyphenyl)-*N*-[5-(methylsulfanyl)-1,3,4-thiadiazol-2-yl]-1,2-oxazole-5-carboxamide | 349.10 | Example **1** |
| 213 | *N*-[5-(methylsulfanyl)-1,3,4-thiadiazol-2-yl]-6-oxo-4-phenylpyran-2-carboxamide | 346.0 | Example **16** |
| 214 | 3-(3-cyanophenyl)-*N*-[5-(methylsulfanyl)-1,3,4-thiadiazol-2-yl]-1,2-oxazole-5-carboxamide | 344.0 | Example **1** |
| 215 | 3-(4-methylphenyl)-*N*-[5-(methylsulfanyl)-1,3,4-thiadiazol-2-yl]-1,2-oxazole-5-carboxamide | 333.40 | Example **1** |
| 216 | 3-(2-chlorophenyl)-*N*-[5-(methylsulfanyl)- 1,3,4-thiadiazol-2-yl]-1,2-oxazole-5-carboxamide | 353.0 | Example **1** |
| 217 | 3-(2-fluorophenyl)-*N*-(5-(methylthio)-1,3,4-thiadiazol-2-yl)isoxazole-5-carboxamide | 337.10 | Example **1** |
| 218 | 3-(2-methylphenyl)-*N*-[5-(methylsulfanyl)-1,3,4-thiadiazol-2-yl]-1,2-oxazole-5-carboxamide | 333.0 | Example **1** |
| 219 | 3-(3-methylphenyl)-*N*-[5-(methylsulfanyl)-1,3,4-thiadiazol-2-yl]-1,2-oxazole-5-carboxamide | 333.0 | Example **1** |
| 220 | *N*-[5-(methylsulfanyl)-1,3,4-thiadiazol-2-yl]naphtho[1,2-c][1,2]oxazole-3-carboxamide | 343.0 | Example **5** |
| 221 | 6-methyl-*N*-[5-(methylsulfanyl)-1,3,4-thiadiazol-2-yl]-2,1-benzoxazole-3-carboxamide | 307.0 | Example **1** |
| 222 | 6-fluoro-*N*-[5-(methylsulfanyl)-1,3,4-thiadiazol-2-yl]-2,1-benzoxazole-3-carboxamide | 311.32 | Example **1** |
| 223 | 5-fluoro-*N*-[5-(methylsulfanyl)-1,3,4-thiadiazol-2-yl]-2,1-benzoxazole-3-carboxamide | 310.9 | Example **1** |
| 224 | 5-methoxy-*N*-[5-(methylsulfanyl)-1,3,4-thiadiazol-2-yl]-2,1-benzoxazole-3-carboxamide | 323.1 | Example **1** |
| 225 | *N*-[5-(methylsulfanyl)-1,3,4-thiadiazol-2-yl]-6-oxo-5-phenylpyran-2-carboxamide | 346.0 | Example **16** |
| 226 | 7-methyl-*N*-[5-(methylsulfanyl)-1,3,4-thiadiazol-2-yl]-2,1-benzoxazole-3-carboxamide | 307.0 | Example **1** |
| 227 | 5-methyl-*N*-[5-(methylsulfanyl)-1,3,4-thiadiazol-2-yl]-2,1-benzoxazole-3-carboxamide | 307.0 | Example **1** |
| 228 | 7-chloro-*N*-[5-(methylsulfanyl)-1,3,4-thiadiazol-2-yl]-2,1-benzoxazole-3-carboxamide | 327.0 | Example **1** |
| 229 | 5-chloro-*N*-[5-(methylsulfanyl)-1,3,4-thiadiazol-2-yl]-2,1-benzoxazole-3-carboxamide | 327.0 | Example **1** |
| 230 | 7-methoxy-*N*-[5-(methylsulfanyl)-1,3,4-thiadiazol-2-yl]-2,1-benzoxazole-3-carboxamide | 323.0 | Example **1** |
| 231 | 6-methoxy-*N*-[5-(methylsulfanyl)-1,3,4-thiadiazol-2-yl]-2,1-benzoxazole-3-carboxamide | 323.05 | Example **1** |
| 232 | *N*-[5-(methylsulfanyl)-1,3,4-thiadiazol-2-yl]-4-phenyl-1,2-oxazole-5-carboxamide | 319.05 | Example **1** |
| 233 | 3-benzyl-*N*-[5-(methylsulfanyl)-1,3,4-thiadiazol-2-yl]-1,2-oxazole-5-carboxamide | 333.0 | Example **1** |
| 234 | *N*-[5-(methylsulfanyl)-1,3,4-thiadiazol-2-yl]-3-phenyl-1,2,4-oxadiazole-5-carboxamide | 320.05 | Example **1** |
| 235 | *N*-[5-(methylsulfanyl)-1,3,4-thiadiazol-2-yl]-5-phenyl-1,3,4-oxadiazole-2-carboxamide | 320.05 | Example **1** |
| 236 | *N*-(5-methylsulfanyl-1,3,4-thiadiazol-2-yl)-5-phenyl-1,2,4-oxadiazole-3-carboxamide | 320.05 | Example **1** |
| 237 | 4-(2-fluorophenyl)-*N*-(5-methylsulfanyl-1,3,4-thiadiazol-2-yl)-6-oxo-pyran-2-carboxamide | 364.00 | Example **16** |
| 238 | 4-(3-fluorophenyl)-*N*-(5-methylsulfanyl-1,3,4-thiadiazol-2-yl)-6-oxo-pyran-2-carboxamide | 364.00 | Example **16** |
| 239 | 4-(4-fluorophenyl)-*N*-(5-methylsulfanyl-1,3,4-thiadiazol-2-yl)-6-oxo-pyran-2-carboxamide | 363.90 | Example **16** |
| 240 | 4-(3-chlorophenyl)-*N*-(5-methylsulfanyl-1,3,4-thiadiazol-2-yl)-6-oxo-pyran-2-carboxamide | 379.95 | Example **16** |
| 241 | 4-(3-methoxyphenyl)-*N*-(5-methylsulfanyl-1,3,4-thiadiazol-2-yl)-6-oxo-pyran-2-carboxamide | 375.95 | Example **16** |
| 242 | 3,4-dimethyl-*N*-(5-methylsulfanyl-1,3,4-thiadiazol-2-vl)isoxazole-5-carboxamide | 270.90 | Example **1** |
| 243 | 4-(4-chlorophenyl)-*N*-(5-methylsulfanyl-1,3,4-thiadiazol-2-yl)-6-oxo-pyran-2-carboxamide | 380.1 | Example **16** |
| 244 | 4-(2-methoxyphenyl)-*N*-(5-methylsulfanyl-1,3,4-thiadiazol-2-yl)-6-oxo-pyran-2-carboxamide | 376.15 | Example **16** |
| 245 | *N*-(5-methylsulfanyl-1,3,4-thiadiazol-2-yl)-4-(2-naphthyl)-6-oxo-pyran-2-carboxamide | 396.00 | Example **16** |
| 246 | *N*-(5-methylsulfanyl-1,3,4-thiadiazol-2-yl)-6-oxo-4-(8-quinolyl)pyran-2-carboxamide | 397.05 | Example **16** |
| 247 | *N*-(5-methylsulfanyl-1,3,4-thiadiazol-2-yl)-6-oxo-4-(4-quinolyl)pyran-2-carboxamide | 397.15 | Example **16** |
| 248 | *N*-(5-methylsulfanyl-1,3,4-thiadiazol-2-yl)-4-(o-tolyl)-6-oxo-pyran-2-carboxamide | 360.05 | Example **16** |
| 249 | *N*-(5-methylsulfanyl- 1,3,4-thiadiazol-2-yl)-4-(m-tolyl)-6-oxo-pyran-2-carboxamide | 359.95 | Example **16** |
| 250 | *N*-(5-methylsulfanyl-1,3,4-thiadiazol-2-yl)-6-oxo-4-(p-tolvl)pvran-2-carboxamide | 360.05 | Example **16** |
| 251 | 4-(2-chlorophenyl)-*N*-(5-methylsulfanyl-1,3,4-thiadiazol-2-yl)-6-oxo-pyran-2-carboxamide | 380.0 | Example **16** |
| 252 | 4-(4-methoxyphenyl)-*N*-(5-methylsulfanyl-1,3,4-thiadiazol-2-yl)-6-oxo-pyran-2-carboxamide | 376.00 | Example **16** |
| 253 | *N*-(5-methylsulfanyl-1,3,4-thiadiazol-2-yl)-4-(1-naphthyl)-6-oxo-pyran-2-carboxamide | 396.05 | Example **16** |
| 254 | *N*-(5-methylsulfanyl-1,3,4-thiadiazol-2-yl)-6-oxo-4-(2-quinolyl)pyran-2-carboxamide | 397.05 | Example **16** |
| 255 | *N*-(5-methylsulfanyl-1,3,4-thiadiazol-2-yl)-6-oxo-4-(3-quinolyl)pyran-2-carboxamide | 397.00 | Example **16** |
| 256 | 4-(4-isoquinolyl)-*N*-(5-methylsulfanyl-1,3,4-thiadiazol-2-yl)-6-oxo-pyran-2-carboxamide | 397.00 | Example **16** |
| 257 | 5-anilino-*N*-(5-methylsulfanyl-1,3,4-thiadiazol-2-yl)-1,3,4-oxadiazole-2-carboxamide | 335.00 | Example **1** |
| 258 | 5-cyclohexyl-*N-*(5-methylsulfanyl-1,3,4-thiadiazol-2-yl)-1,3,4-oxadiazole-2-carboxamide | 326.00 | Example **1** |
| 259 | 5-benzyl-*N*-(5-methylsulfanyl-1,3,4-thiadiazol-2-yl)-1,3,4-oxadiazole-2-carboxamide | 334.00 | Example **1** |
| 260 | *N*-(5-methylsulfanyl-1,3,4-thiadiazol-2-yl)-[1,2,4]triazolo[4,3-a]pyridine-3-carboxamide | 292.95 | Example **1** |
| 261 | 3-benzyl-*N*-(5-methylsulfanyl-1,3,4-thiadiazol-2-yl)-1,2,4-oxadiazole-5-carboxamide | 334.00 | Example **1** |
| 262 | 3-cyclohexyl-*N*-(5-(methylthio)-1,3,4-thiadiazol-2-yl)-1,2,4-oxadiazole-5-carboxamide | 326.15 | Example **1** |
| 263 | 5-cyclohexyl-*N*-(5-(methylthio)-1,3,4-thiadiazol-2-yl)-1,2,4-oxadiazole-3-carboxamide | 326.05 | Example **1** |
| 264 | 4-(isoquinolin-7-yl)-*N*-(5-(methylthio)-1,3,4-thiadiazol-2-yl)-2-oxo-2*H*-pyran-6-carboxamide | 397.00 | Example **16** |
| 265 | *N*-(5-(methylthio)-1,3,4-thiadiazol-2-yl)-3-(phenylamino)-1,2,4-oxadiazole-5-carboxamide | 335.00 | Example **1** |
| 266 | *N*-(5-(methylthio)-1,3,4-thiadiazol-2-yl)-2-phenylisonicotinamide | 329.10 | Example **1** |
| 267 | 4-(4-methylquinolin-8-yl)-*N*-(5-(methylthio)-1,3,4-thiadiazol-2-yl)-2-oxo-2*H*-pyran-6-carboxamide | 411.05 | Example **16** |
| 268 | *N*-(5-(methylthio)-1,3,4-thiadiazol-2-yl)-2-oxo-4-(phenylamino)-2*H*-pyran-6-carboxamide | 360.95 | Example **17A** |
| 269 | 4-(1-methyl-1*H*-indazol-4-yl)-*N*-(5-(methylthio)-1,3,4-thiadiazol-2-yl)-2-oxo-2*H*-pyran-6-carboxamide | 400.00 | Example **16** |
| 270 | *N*-(5-(methylthio)-1,3,4-thiadiazol-2-yl)-2-oxo-4-(quinolin-7-yl)-2*H*-pyran-6-carboxamide | 397.15 | Example **16** |
| 271 | *N*-(5-(methylthio)-1,3,4-thiadiazol-2-yl)isoxazolo[3,4-f]quinoline-3-carboxamide | 341.85 [M-1]- | Example **5** |
| 272 | *N*-(5-(methylthio)-1,3,4-thiadiazol-2-yl)-2-oxo-4-(6-oxo-1,6-dihydropyridin-3-yl)-2*H*-pyran-6-carboxamide | 360.85 [M-1]- | Example **16** |
| 273 | *N*-(5-(methylthio)-1,3,4-thiadiazol-2-yl)isoxazolo[3,4-f]isoquinoline-3-carboxamide | 344.00 | Example **5** |
| 274 | 4-(1-methyl-1*H*-benzo[d]imidazol-4-yl)-N-(5-(methylthio)-1,3,4-thiadiazol-2-yl)-2-oxo-2*H*-pyran-6-carboxamide | 400.00 | Example **16** |
| 275 | 4-(3,4-dihydro-2*H*-benzo[b][1,4]oxazin-8-yl)-*N*-(5-(methylthio)-1,3,4-thiadiazol-2-yl)-2-oxo-2*H*-pyran-6-carboxamide | 403.20 | Example **16** |
| 276 | *N*-(5-(methylthio)-1,3,4-thiadiazol-2-yl)-2-oxo-4-(6-oxo-1,6-dihydropyridin-2-yl)-2*H*-pyran-6-carboxamide | 362.90 | Example **16** |
| 277 | 4-(isoquinolin-8-yl)-*N*-(5-(methylthio)-1,3,4-thiadiazol-2-yl)-2-oxo-2*H*-pyran-6-carboxamide | 397.15 | Example **16** |
| 278 | methyl 3-((5-(methylthio)-1,3,4-thiadiazol-2-yl)carbamoyl)benzo[c]isoxazole-6-carboxylate | 351.10 | Example **61** |
| 279 | *N*-(5-(methylthio)-1,3,4-thiadiazol-2-yl)isoxazolo[4,3-h]isoquinoline-3-carboxamide | 343.95 | Example **5** |
| 280 | *N*-(5-(methylamino)-1,3,4-thiadiazol-2-yl)isoxazolo[4,3-h]quinoline-3-carboxamide | 327.05 | Example **5** |

### Example 67: Biological Activity

### hcGAS Kinase-Glo assay

Compounds were tested for their human-cGAS (h-cGAS) inhibition activity using the methodology reported in Lama et al., "Development of human cGAS-specific small molecule inhibitors for repression of double stranded DNA (dsDNA)-triggered interferon expression", Nature Communications 10, Article number: 2261 (2019), which is herein incorporated by reference in its entirety, with slight changes to some conditions as shown in **Table B.** The results of the assays, expressed in IC₅₀ ranges are reported in **Table C** with the following designations: A represents an IC₅₀ value < 0.1 µM; B represents an IC₅₀ value ≥ 0.1 µM and < 0.5 µM; C represents an IC₅₀ value ≥ 0 .5 µM and < 1.0 µM; D represents an IC₅₀ value ≥ 1.0 µM.

| **Table B. Summary of assay conditions** | | **Lama et al. 2019** | **Present Disclosure** |
|---|---|---|---|
| **Enzyme** | human-cGAS h-cGAS (nM) | 100 | 40 |
| **Buffer** | Tris(hydroxymethyl)aminomethane hydrochloride (Tris-HCl) pH 7.4 (mM) | 20 | 20 |
| | MgCl₂ (mM) | 5 | 10 |
| | NaCl (mM) | 150 | 25 |
| | Tween^{™}-20 (%) | 0.01 | 0.01 |
| | ZnCl₂ (µM) | 1 | 1 |
| | Dithiothreitol (DTT) (mM) | 1 | 1 |
| | Dimethylsulfoxide (DMSO) (%) | 0.5 | 5 |
| **Substrates** | Adenosine triphosphate (ATP) (µM) | 100 | 100 |
| | Guanosine triphosphate (GTP) (µM) | 100 | 100 |
| | double stranded DNA (dsDNA) (nM) | 25 | 25 |
| **Assay** | Plate (wells) | 384 | 384 |
| | Incubation length (h) | 7 | 3 |
| | Total volume (µL) | 20 | 20 |
| | Kinase-Glo Max (µL) | 20 | 20 |

| **Table C. Activity data** | | | |
|---|---|---|---|
| **Compound No.** | **hcGAS potency IC₅₀** | **Compound No.** | **hcGAS potency IC₅₀** |
| 1 | B | 142 | C |
| 2 | A | 143 | B |
| 3 | B | 144 | B |
| 4 | A | 145 | D |
| 5 | B | 146 | D |
| 6 | B | 147 | C |
| 7 | A | 148 | D |
| 8 | B | 149 | C |
| 9 | B | 150 | C |
| 10 | B | 151 | C |
| 11 | B | 152 | B |
| 12 | B | 153 | B |
| 13 | C | 154 | B |
| 14 | D | 155 | B |
| 15 | C | 156 | B |
| 16 | B | 157 | B |
| 17 | C | 158 | B |
| 18 | D | 159 | C |
| 19 | D | 160 | C |
| 20 | D | 161 | C |
| 21 | C | 162 | C |
| 22 | B | 163 | D |
| 23 | D | 164 | C |
| 24 | C | 165 | C |
| 25 | D | 166 | D |
| 26 | B | 167 | D |
| 27 | C | 168 | C |
| 28 | D | 169 | A |
| 29 | D | 170 | B |
| 30 | D | 171 | D |
| 31 | B | 172 | D |
| 32 | B | 173 | C |
| 33 | B | 174 | D |
| 34 | C | 175 | B |
| 35 | C | 176 | C |
| 36 | B | 177 | C |
| 37 | A | 178 | A |
| 38 | B | 179 | B |
| 39 | B | 180 | C |
| 40 | B | 181 | C |
| 41 | B | 182 | C |
| 42 | D | rac-183 | D |
| 43 | D | 184 | D |
| 44 | D | 185 | D |
| 45 | C | 186 | C |
| 46 | B | 187 | D |
| 47 | C | 188 | C |
| 48 | C | 189 | D |
| 49 | C | 190 | D |
| 50 | C | 191 | D |
| 51 | C | 192 | D |
| 52 | B | 193 | D |
| 53 | B | 194 | C |
| 54 | B | 195 | D |
| 55 | A | 196 | D |
| 56 | B | 197 | B |
| 57 | A | 198 | D |
| 58 | C | 199 | D |
| 59 | C | 200 | D |
| 60 | D | 201 | D |
| 61 | B | 202 | C |
| 62 | D | 203 | B |
| 63 | C | 204 | B |
| 64 | A | 205 | B |
| 65 | D | 206 | A |
| 66 | C | 207 | D |
| 67 | C | 208 | C |
| 68 | C | 209 | C |
| 69 | C | 210 | D |
| 70 | D | 211 | C |
| 71 | C | 212 | D |
| 72 | D | 213 | B |
| 73 | C | 214 | D |
| 74 | D | 215 | C |
| 75 | C | 216 | C |
| 76 | D | 217 | B |
| 77 | D | 218 | C |
| 78 | C | 219 | C |
| 79 | C | 220 | B |
| 80 | C | 221 | B |
| 81 | D | 222 | C |
| 82 | C | 223 | C |
| 83 | D | 224 | D |
| 84 | B | 225 | D |
| 85 | C | 226 | D |
| 86 | C | 227 | C |
| 87 | C | 228 | C |
| 88 | C | 229 | C |
| 89 | C | 230 | B |
| 90 | B | 231 | B |
| 91 | A | 232 | C |
| 92 | B | 233 | C |
| 93 | C | 234 | C |
| 94 | B | 235 | C |
| 95 | C | 236 | C |
| 96 | C | 237 | B |
| 97 | D | 238 | B |
| 98 | D | 239 | B |
| 99 | D | 240 | B |
| 100 | D | 241 | B |
| 101 | D | 242 | C |
| 102 | D | 243 | C |
| 103 | C | 244 | B |
| 104 | C | 245 | B |
| 105 | D | 246 | B |
| 106 | C | 247 | C |
| 107 | C | 248 | B |
| 108 | C | 249 | B |
| 109 | D | 250 | B |
| 110 | C | 251 | B |
| 111 | B | 252 | B |
| 112 | B | 253 | B |
| 113 | C | 254 | B |
| 114 | C | 255 | C |
| 115 | C | 256 | C |
| 116 | C | 257 | C |
| 117 | B | 258 | C |
| 118 | B | 259 | C |
| 119 | C | 260 | D |
| 120 | C | 261 | C |
| 121* | C | 262 | C |
| 122* | B | 263 | D |
| 123 | B | 264 | B |
| 124 | D | 265 | C |
| 125 | B | 266 | D |
| 126 | C | 267 | B |
| 127 | C | 268 | B |
| 128 | D | 269 | B |
| 129 | D | 270 | B |
| 130 | C | 271 | C |
| 131 | B | 272 | C |
| 132 | D | 273 | D |
| 133 | D | 274 | B |
| 134 | D | 275 | B |
| 135 | D | 276 | B |
| 136 | C | 277 | C |
| 137 | D | 278 | C |
| 138 | C | 279 | C |
| 139 | C | 280 | D |
| 140 | D | | |
| 141 | D | | |

### Equivalents

The details of one or more embodiments of the disclosure are set forth in the accompanying description above. Although any methods and materials similar or equivalent to those described herein can be used in the practice or testing of the present disclosure, the preferred methods and materials are now described. Other features, objects, and advantages of the disclosure will be apparent from the description and from the claims. In the specification and the appended claims, the singular forms include plural referents unless the context clearly dictates otherwise. Unless defined otherwise, all technical and scientific terms used herein have the same meaning as commonly understood by one of ordinary skill in the art to which this disclosure belongs. All patents and publications cited in this specification are incorporated by reference.

The foregoing description has been presented only for the purposes of illustration and is not intended to limit the disclosure to the precise form disclosed, but by the claims appended hereto.

### EMBODIMENTS

The present disclosure provides the following Embodiments of the invention:
1. A compound of Formula (I): 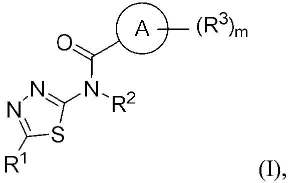 or a pharmaceutically acceptable salt, hydrate, solvate, prodrug, stereoisomer, labeled isotope, or tautomer thereof, wherein:
   Ring A is a 5- to 10-membered heteroaryl or 3- to 10-membered heterocyclyl;
   R¹ is H, -SR⁴, -S(O)R⁴, -S(O)₂R⁴, -N(R⁴)₂, -CN, C₁-C₆ alkyl, C₆-C₁₀ aryl, or 5- to 10-membered heteroaryl, wherein the alkyl, aryl or heteroaryl is optionally substituted with one or more R⁵, provided R¹ is not pyridinyl when Ring A is 1-isoquinolinyl;
   R² is H or C₁-C₆ alkyl;
   each R³ is independently halogen, -CN, -OR⁸, -NH₂, -NH(R⁶), -N(R⁶)(R⁷), -NHC(O)R⁸, - CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶, or
   two R³, together with the atoms to which they are attached, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷, or
   two geminal R³, together with the carbon atom to which they attached, form an oxo;
   R⁴ is H, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl;
   each R⁵ is independently -OH, halogen, -C(O)OH, C₁-C₆ alkyl, C₁-C₆ alkoxy, C₂-C₆ alkenyl, or C₂-C₆ alkynyl, wherein the alkyl, alkoxy, alkenyl, or alkynyl is optionally substituted with halogen, OH, or NH₂;
   each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, -
   (CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ-C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl), or
   two R⁶, together with the atoms to which they are attached, form a C₃-C₈ cycloalkyl, C₆-C₁₀ aryl, 5- to 10-membered heteroaryl, or 3- to 10-membered heterocyclyl, or
   two geminal R⁶, together with the carbon atom to which they are attached, form an oxo;
   each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, or
   two R⁷, together with the atoms to which they are attached, form a C₃-C₈ cycloalkyl, C₆-C₁₀ aryl, 5- to 10-membered heteroaryl, or 3- to 10-membered heterocyclyl, or
   two geminal R⁷, together with carbon atom to which they are attached, form oxo;
   R³ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, or heteroaryl is optionally substituted with one or more R⁹;
   R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₐ-NHC(O)R¹⁰, -(CH₂)ₙ-NHC(O)-(CH₂)ₚ-OR¹⁰, -(CH₂)ₙ-NHR¹⁰, -(CH₂)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl);
   R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, heteroaryl, or aryl is optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, 3- to 10-membered heterocyclyl, 5- to 10-membered heteroaryl, or C₆-C₁₀ aryl;
   m is an integer from 0 to 4;
   n is an integer from 0 to 6; and
   p is an integer from 0 to 6.
2. The compound of Embodiment 1, wherein the compound is of Formula **(I-a):** 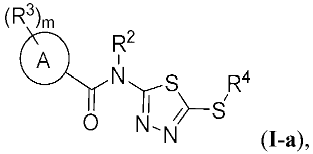 or a pharmaceutically acceptable salt, hydrate, solvate, prodrug, stereoisomer, labeled isotope, or tautomer thereof.
3. The compound of Embodiment 1 or 2, wherein Formula **(I-a)** is Formula (**I-a-1**) 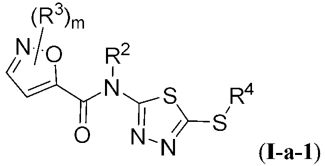 or a pharmaceutically acceptable salt, hydrate, solvate, prodrug, stereoisomer, labeled isotope, or tautomer thereof.
4. The compound of any one of Embodiments 1-3, wherein Formula **(I-a)** is Formula (**I-a-1-i**) 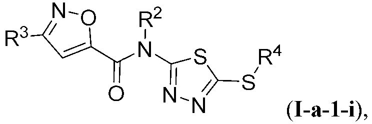 or a pharmaceutically acceptable salt, hydrate, solvate, prodrug, stereoisomer, labeled isotope, or tautomer thereof.
5. The compound of any one of Embodiments 1-3, wherein Formula **(I-a)** is Formula (**I-a-1-ii**) 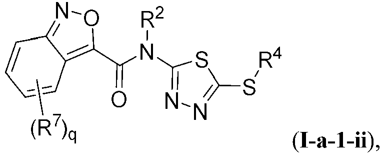 wherein q is 0, 1, 2, 3, or 4, or a pharmaceutically acceptable salt, hydrate, solvate, prodrug, stereoisomer, labeled isotope, or tautomer thereof.
6. The compound of Embodiment 1 or 2, wherein Formula **(I-a)** is Formula (**I-a-2**): 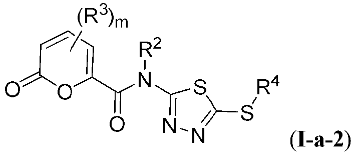 wherein m is 0, 1, 2, 3, or 4, or a pharmaceutically acceptable salt, hydrate, solvate, prodrug, stereoisomer, labeled isotope, or tautomer thereof.
7. The compound of any one of Embodiments 1-2 and 6, wherein Formula **(I-a)** is Formula (**I-a-**2-i): 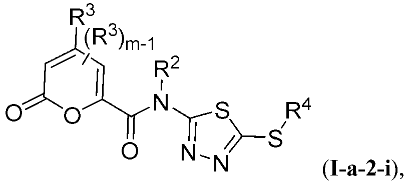 wherein m is 1, 2, or 3, or a pharmaceutically acceptable salt, hydrate, solvate, prodrug, stereoisomer, labeled isotope, or tautomer thereof.
8. The compound of any one of Embodiments 1-2 and 6-7, wherein Formula **(I-a)** is Formula **(I-a-2-ii**) 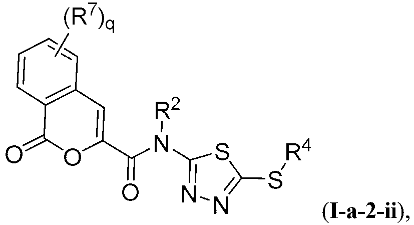 wherein q is 0, 1, 2, 3, or 4, or a pharmaceutically acceptable salt, hydrate, solvate, prodrug, stereoisomer, labeled isotope, or tautomer thereof.
9. The compound of Embodiment 1 or 2, wherein Formula **(I-a)** is any one of Formula (**I-a-5**) to Formula (**I-a-13**): 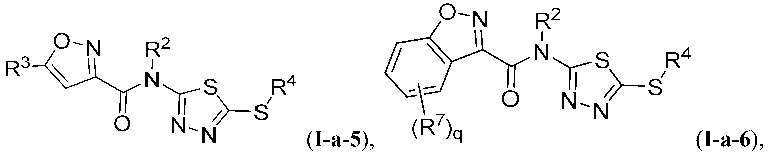 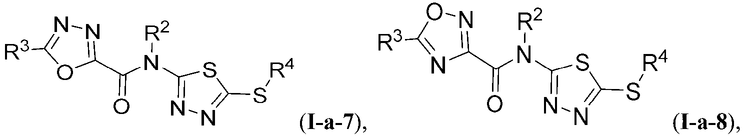 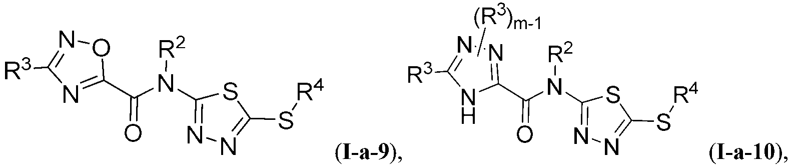 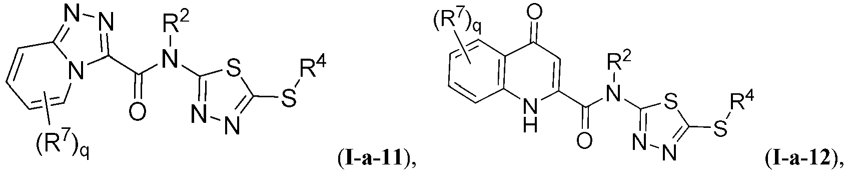 or 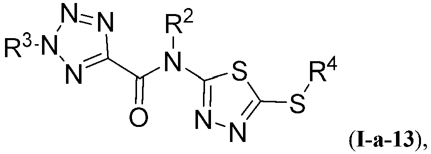 wherein q is 0, 1, 2, 3, or 4, or a pharmaceutically acceptable salt, hydrate, solvate, prodrug, stereoisomer, labeled isotope, or tautomer thereof.
10. The compound, or a pharmaceutically acceptable salt, isomer, solvate, prodrug, or tautomer thereof, of Embodiment 1, wherein R¹ is hydrogen, -SCH₃, -SCH₂CH₃, -CH₃, -CHF₂, -CF₃, -CH₂CH₃, -(CH₂)₂CH₃, -(CH₂)₄CH₃, -CN, -CH₂CH₂OCH₃, -NH₂, -NH(CH₃), -N(CH₃)₂, -CH(CH₂CH₃), - CH(C₆H₆)(CH₂CH₃), 3-chlorophenyl, 3-fluorophenyl, 3-methoxyphenyl, 2-fluoropyridinyl.
11. The compound, or a pharmaceutically acceptable salt, hydrate, solvate, prodrug, stereoisomer, labeled isotope, or tautomer thereof, of Embodiment 10, wherein R¹ is -SCH₃.
12. The compound, or a pharmaceutically acceptable salt, hydrate, solvate, prodrug, stereoisomer, labeled isotope, or tautomer thereof, of any of the preceding Embodiments, wherein R² is H.
13. The compound, or a pharmaceutically acceptable salt, hydrate, solvate, prodrug, stereoisomer, labeled isotope, or tautomer thereof, of any of the preceding Embodiments wherein R² is methyl.
14. The compound, or a pharmaceutically acceptable salt, hydrate, solvate, prodrug, stereoisomer, labeled isotope, or tautomer thereof, of any of the preceding Embodiments, wherein Ring A is 5- or 6-membered heterocyclyl or 5- or 6-membered heteroaryl.
15. The compound, or a pharmaceutically acceptable salt, hydrate, solvate, prodrug, stereoisomer, labeled isotope, or tautomer thereof, of claim any of the preceding Embodiments, wherein Ring A is: 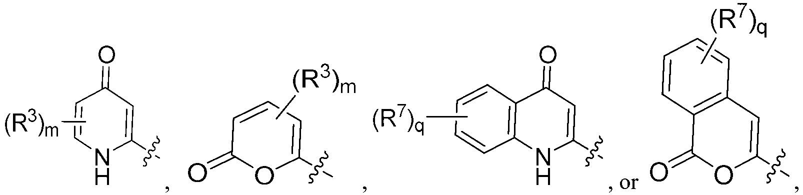 wherein q is 0, 1, 2, 3, or 4.
16. The compound, or a pharmaceutically acceptable salt, hydrate, solvate, prodrug, stereoisomer, labeled isotope, or tautomer thereof, of Embodiment 14, wherein Ring A is a 5- or 6-membered heteroaryl.
17. The compound, or a pharmaceutically acceptable salt, hydrate, solvate, prodrug, stereoisomer, labeled isotope, or tautomer thereof, of Embodiment 16, wherein Ring A is 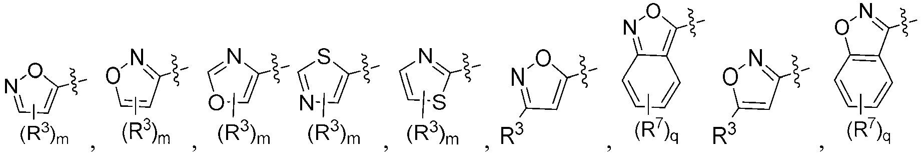 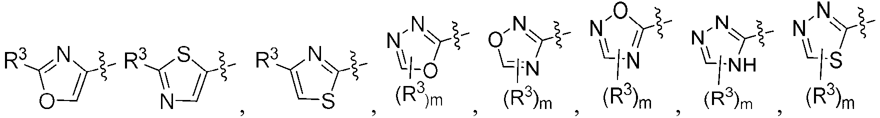 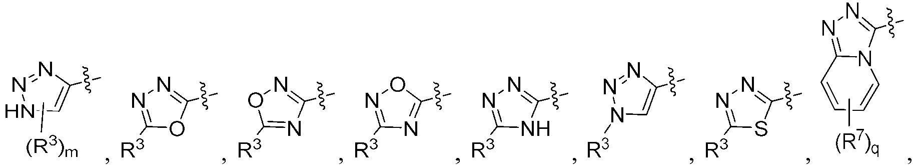 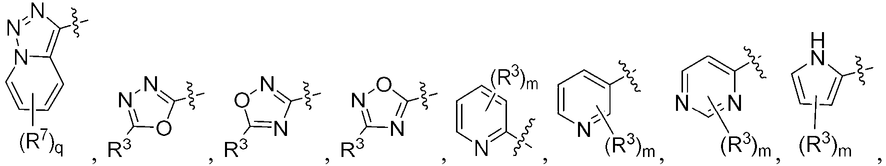 or 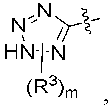 wherein m is 0, 1, 2, 3, or 4, and q is 0, 1, 2, 3, or 4.
18. The compound, or a pharmaceutically acceptable salt, hydrate, solvate, prodrug, stereoisomer, labeled isotope, or tautomer thereof, of any of the preceding Embodiments, wherein each R³ is independently halogen, -CN, -OR⁸, -NH₂, -NH(R⁶), -N(R⁶)(R⁷), -NHC(O)R⁸, -CO(OR⁸), -C(O)R⁸, - C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl or heteroaryl is optionally substituted with one or more R⁶.
19. The compound, or a pharmaceutically acceptable salt, hydrate, solvate, prodrug, stereoisomer, labeled isotope, or tautomer thereof, of any one of Embodiments 1-17, wherein two R³, together with the intervening atoms, form a C₃-C₈ cycloalkyl, C₆-C₁₀ aryl, 5- to 10-membered heteroaryl, or 3- to 10-membered heterocyclyl, wherein the cycloalkyl, aryl, heterocyclyl, or heteroaryl is optionally substituted with one or more R⁷.
20. The compound, or a pharmaceutically acceptable salt, hydrate, solvate, prodrug, stereoisomer, labeled isotope, or tautomer thereof, of any one of Embodiments 1-17, wherein two geminal R³, together with the carbon atom to which they attached, form an oxo.
21. The compound, or a pharmaceutically acceptable salt, hydrate, solvate, prodrug, stereoisomer, labeled isotope, or tautomer thereof, of Embodiment 31, wherein two R³, together with the atoms to which they are attached, forms 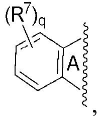 wherein, q is 0, 1, 2, 3, or 4.
22. The compound, or a pharmaceutically acceptable salt, hydrate, solvate, prodrug, stereoisomer, labeled isotope, or tautomer thereof, of any one of the preceding Embodiments, wherein R⁴ is C₁-C₆ alkyl.
23. The compound, or a pharmaceutically acceptable salt, hydrate, solvate, prodrug, stereoisomer, labeled isotope, or tautomer thereof, of Embodiment 22, wherein R⁴ is methyl or ethyl.
24. A compound selected from Table 1, or a pharmaceutically acceptable salt, hydrate, solvate, prodrug, stereoisomer, labeled isotope, or tautomer thereof.
25. A pharmaceutical composition comprising the compound of any one of the preceding Embodiments and one or more pharmaceutically acceptable carriers.
26. A method of treating a cGAS-related disease or disorder, the method comprising administering to the subject a compound of any one of the preceding Embodiments.
27. A method of modulating cGAS, the method comprising administering to the subject a compound of any one of the preceding Embodiments.
28. The compound of any one of the preceding Embodiments for use in treating a cGAS-related disease or disorder.
29. Use of the compound of any one of the preceding Embodiments, in the manufacture of a medicament, for the treatment of a cGAS-related disease or disorder.
30. The method, compound, or use of any one of any one of Embodiments 26-29, wherein the subject is a human.
31. The method, compound, or use of any one of Embodiments 26-30, wherein the cGAS-related disease or disorder is inflammation, an auto-immune disease, a cancer, an infection, a disease or disorder of the central nervous system, a metabolic disease, a cardiovascular disease, a respiratory disease, a kidney disease, a liver disease, an ocular disease, a skin disease, a lymphatic disease, a rheumatic disease, a psychological disease, graft versus host disease, allodynia, or an cGAS-related disease in a subject that has been determined to carry a germline or somatic non-silent mutation in cGAS.
32. The method, compound, or use of any one of Embodiments 26-31, wherein the disease or disorder of the central nervous system is Parkinson's disease, Alzheimer's disease, traumatic brain injury, spinal cord injury, amyotrophic lateral sclerosis, or multiple sclerosis.
33. The method, compound, or use of any one of Embodiments 26-31, wherein the kidney disease is an acute kidney disease, a chronic kidney disease, or a rare kidney disease.
34. The method, compound, or use of any one of Embodiments 26-31, wherein the skin disease is psoriasis, hidradenitis suppurativa (HS), or atopic dermatitis.
35. The method, compound, or use of any one of Embodiments 26-31, wherein the rheumatic disease is dermatomyositis, Still's disease, or juvenile idiopathic arthritis.
36. The method, compound, or use of any one of Embodiments 26-31, wherein the cGAS-related disease in a subject that has been determined to carry a germline or somatic non-silent mutation in cGAS is cryopyrin-associated auto inflammatory syndrome.
37. The method, compound, or use of any one of Embodiments 26-31, wherein the cryopyrin-associated auto inflammatory syndrome is familial cold autoinflammatory syndrome, Muckle-Wells syndrome, or neonatal onset multisystem inflammatory disease.

## Claims

1. A compound of Formula (I): 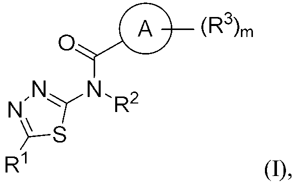 or a pharmaceutically acceptable salt, hydrate, solvate, prodrug, stereoisomer, labeled isotope, or tautomer thereof, wherein:
Ring A is 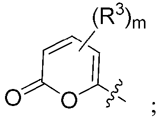
R¹ is H, -SR⁴, -S(O)R⁴, -S(O)₂R⁴, -N(R⁴)₂, -CN, C₁-C₆ alkyl, C₆-C₁₀ aryl, or 5- to 10-membered heteroaryl, wherein the alkyl, aryl or heteroaryl is optionally substituted with one or more R⁵;
R² is H or C₁-C₆ alkyl;
each R³ is independently halogen, -CN, -OR⁸, -NH₂, -NH(R⁶), -N(R⁶)(R⁷), -NHC(O)R⁸, - CO(OR⁸), -C(O)R⁸, -C(O)N(R⁸)₂, -SR⁸, -S(O)₂R⁸, C₁-C₆ alkyl, C₃-C₈ cycloalkyl, C₆-C₁₀ aryl, 5- to 10-membered heteroaryl, or 3- to 10-membered heterocyclyl, wherein the alkyl, cycloalkyl, aryl, heterocyclyl, or heteroaryl is optionally substituted with one or more R⁶;
R⁴ is H, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl;
each R⁵ is independently -OH, halogen, -C(O)OH, C₁-C₆ alkyl, C₁-C₆ alkoxy, C₂-C₆ alkenyl, or C₂-C₆ alkynyl, wherein the alkyl, alkoxy, alkenyl, or alkynyl is optionally substituted with halogen, OH, or NH₂;
each R⁶ is independently halogen, -OH, -CO(OR⁸), -(CH₂)ₙ-NHC(O)-(CH₂)ₚ-OR⁸, CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR⁸, - (CH₂)ₙ-NHC(O)R⁸, -(CH₂)ₙ-NHC(O)-(CH₂)ₚ-OR⁸, -(CH₂)ₙ-NHR⁸, -(CH₂)ₙ-NHS(O)R⁸, -(CH₂)ₙ-NHS(O)₂R⁸, -(CH₂)ₙ-C(O)R⁸, -(CH₂)ₙ₋C(O)OR⁸, -(CH₂)ₙ-OR⁸, -(CH₂)ₙO(CH₂)ₙC(O)NHR⁸, C₁-C₆ alkyl, C₁-C₆ alkoxy, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl), or
two R⁶, together with the atoms to which they are attached, form a C₃-C₈ cycloalkyl, C₆-C₁₀ aryl, 5- to 10-membered heteroaryl, or 3- to 10-membered heterocyclyl, or
two geminal R⁶, together with the carbon atom to which they are attached, form an oxo;
each R⁷ is independently halogen, -OH, -C(O)OR⁸, C₁-C₆ alkyl, C₁-C₆ hydroxyalkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, or
two R⁷, together with the atoms to which they are attached, form a C₃-C₈ cycloalkyl, C₆-C₁₀ aryl, 5- to 10-membered heteroaryl, or 3- to 10-membered heterocyclyl, or
two geminal R⁷, together with carbon atom to which they are attached, form oxo;
R⁸ is H, -OH, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, or heteroaryl is optionally substituted with one or more R⁹;
R⁹ is -(CH₂)ₙ-NHC(O)-(CH₂)ₚ-OR¹⁰, -CN, C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, aryl, heterocyclyl, or heteroaryl is optionally substituted with halogen, -OH, -NH₂, -NHC(O)OR¹⁰, -(CH₂)ₙ-NHC(O)R¹⁰, -(CH₂)ₙ-NHC(O)-(CH²)ₚ-OR¹⁰, -(CH₂)ₙ-NHR¹⁰, -(CH²)ₙ-NHS(O)R¹⁰, -(CH₂)ₙ-NHS(O)₂R¹⁰, -(CH₂)ₙ-C(O)R¹⁰, - (CH₂)ₙ-C(O)OR¹⁰, C₁-C₆ alkyl, C₃-C₈ cycloalkyl, C₆-C₁₀ aryl, 5- to 10-membered heteroaryl, 3- to 10-membered heterocyclyl, or -(C₁-C₆ alkyl)-(C₆-C₁₀ aryl);
R¹⁰ is C₁-C₆ alkyl, C₁-C₆ alkoxy, C₂-C₆ alkenyl, C₂-C₆ alkynyl, C₃-C₈ cycloalkyl, C₆-C₁₀ aryl, 5- to 10-membered heteroaryl, or 3- to 10-membered heterocyclyl, wherein the alkyl, alkoxy, alkenyl, alkynyl, cycloalkyl, heterocyclyl, heteroaryl, or aryl is optionally substituted with one or more halogen, -OH, -CO(OH), -(CH₂)ₙ-NHC(O)-(CH₂)ₚ-OR⁸, -CN, C₁-C₆ alkyl, C₁-C₆ alkoxy, C₂-C₆ alkenyl, C₂-C₆ alkynyl, C₃-C₈ cycloalkyl, 3- to 10-membered heterocyclyl, 5- to 10-membered heteroaryl, or C₆-C₁₀ aryl;
m is an integer from 0, 1, 2, or 3;
n is an integer from 0 to 6; and
p is an integer from 0 to 6.

2. The compound of claim 1, or a pharmaceutically acceptable salt, hydrate, solvate, prodrug, stereoisomer, labeled isotope, or tautomer thereof, wherein R¹ is C₆-C₁₀ aryl substituted with one or more R⁵.

3. The compound of claim 1, or a pharmaceutically acceptable salt, hydrate, solvate, prodrug, stereoisomer, labeled isotope, or tautomer thereof, wherein R¹ is 5- to 10-membered heteroaryl substituted with one or more R⁵.

4. The compound of claim 1 of Formula (**I-a-2**): 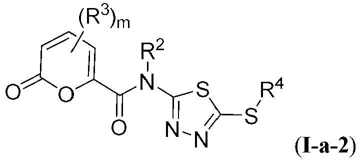 wherein m is 0, 1, 2, or 3, or a pharmaceutically acceptable salt, hydrate, solvate, prodrug, stereoisomer, labeled isotope, or tautomer thereof.

5. The compound, or a pharmaceutically acceptable salt, hydrate, solvate, prodrug, stereoisomer, labeled isotope, or tautomer thereof, of any one of claims 1-4, wherein R² is H.

6. The compound of claim 1 selected from the group consisting of:
| | | | |
|---|---|---|---|
| **0001.** | 64 | **0002.** | 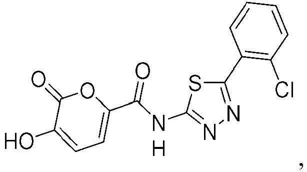 |
| **0003.** | 176 | **0004.** | 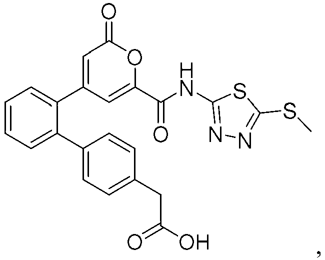 |
| **0005.** | 177 | **0006.** | 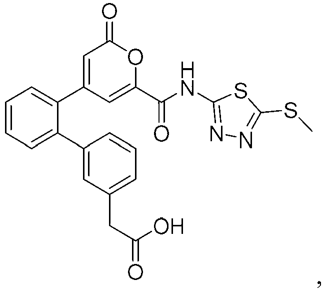 |
| **0007.** | 182 | **0008.** | 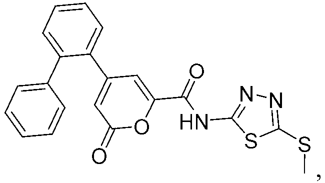 |
| **0009.** | 202 | **00010.** | 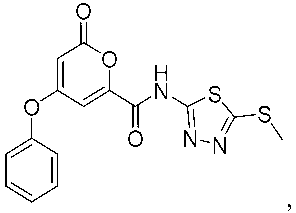 |
| **00011.** | 203 | **00012.** | 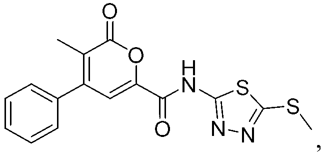 |
| **00013.** | 205 | **00014.** | 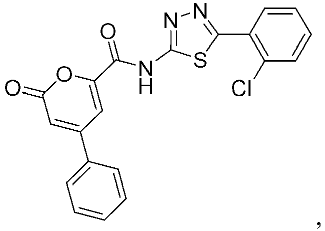 |
| **00015.** | 206 | **00016.** | 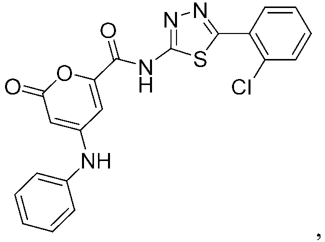 |
| **00017.** | 213 | **00018.** | 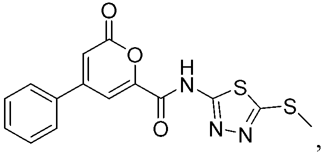 |
| **00019.** | 225 | **00020.** | 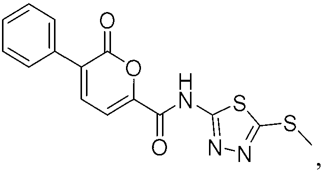 |
| **00021.** | 237 | **00022.** | 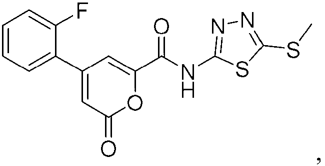 |
| **00023.** | 238 | **00024.** | 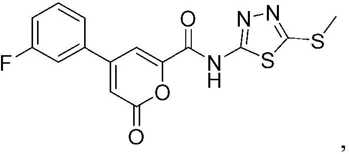 |
| **00025.** | 239 | **00026.** | 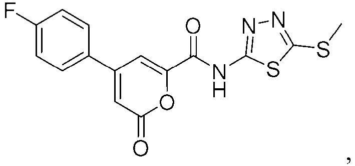 |
| **00027.** | 240 | **00028.** | 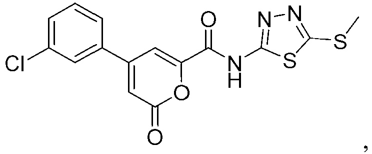 |
| **00029.** | 241 | **00030.** | 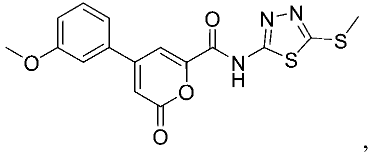 |
| **00031.** | 243 | **00032.** | 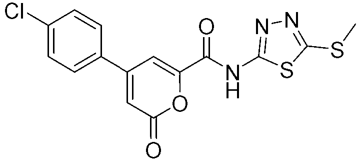 |
| **00033.** | 244 | **00034.** | 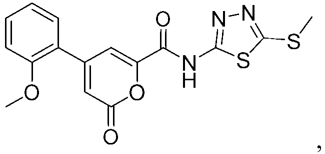 |
| **00035.** | 245 | **00036.** | 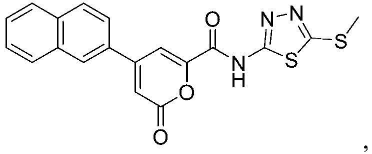 |
| **00037.** | 246 | **00038.** | 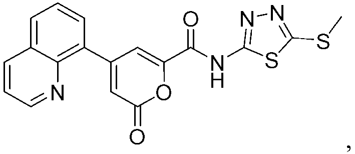 |
| **00039.** | 247 | **00040.** | 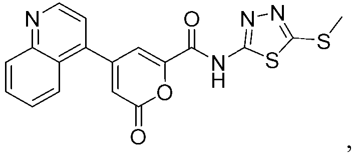 |
| **00041.** | 248 | **00042.** | 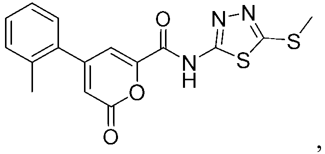 |
| **00043.** | 249 | **00044.** | 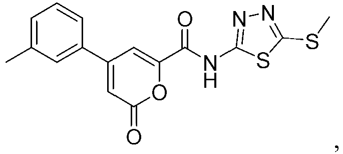 |
| **00045.** | 250 | **00046.** | 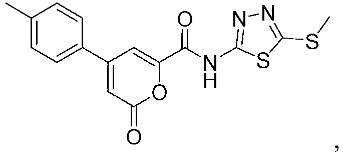 |
| **00047.** | 251 | **00048.** | 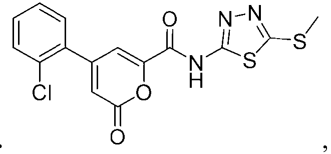 |
| **00049.** | 252 | **00050.** | 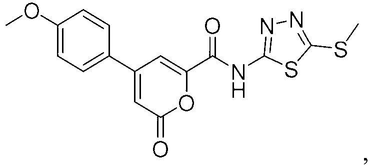 |
| **00051.** | 253 | **00052.** | 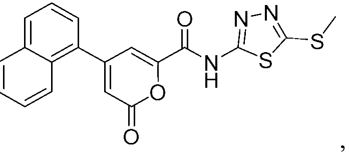 |
| **00053.** | 254 | **00054.** | 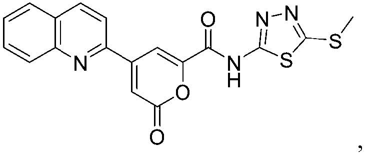 |
| **00055.** | 255 | **00056.** | 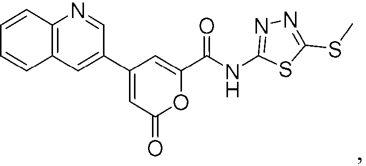 |
| **00057.** | 256 | **00058.** | 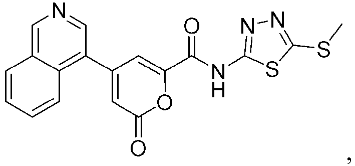 |
| **00059.** | 264 | **00060.** | 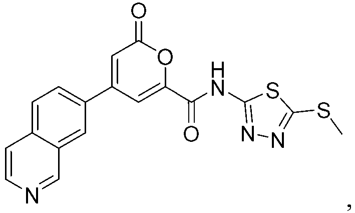 |
| **00061.** | 267 | **00062 .** | 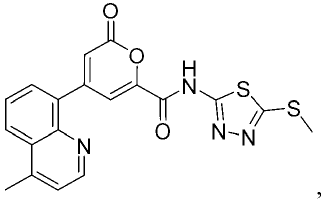 |
| **00063.** | 268 | **00064.** | 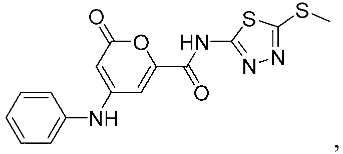 |
| **00065.** | 269 | **00066.** | 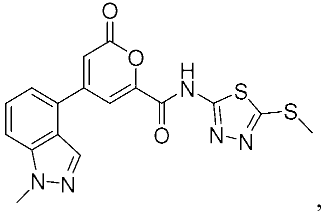 |
| **00067.** | 270 | **00068.** | 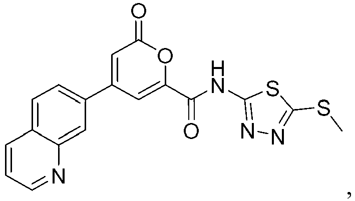 |
| **00069.** | 272 | **00070.** | 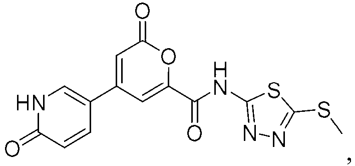 |
| **00071.** | 274 | **00072.** | 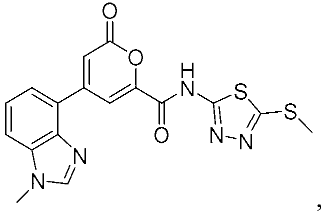 |
| **00073.** | 275 | **00074.** | 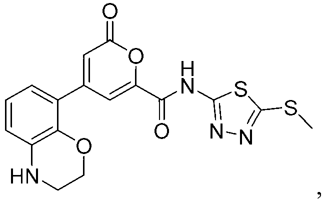 |
| **00075.** | 276 | **00076.** | 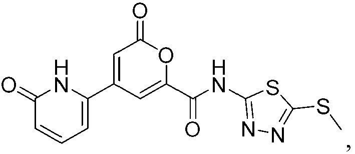 and |
| **00077.** | 277 | **00078.** | 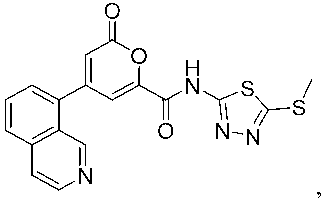 |
or a pharmaceutically acceptable salt, hydrate, solvate, prodrug, stereoisomer, labeled isotope, or tautomer of any of the foregoing.

7. A pharmaceutical composition comprising the compound of any one claims 1-6, or a pharmaceutically acceptable salt, hydrate, solvate, prodrug, stereoisomer, labeled isotope, or tautomer thereof, and one or more pharmaceutically acceptable carriers.

8. The compound of any one of claims 1-6, or the pharmaceutical composition of claim 7, for use in treating a cGAS-related disease or disorder in a subject.

9. The compound or pharmaceutical composition for use according to claim 8, wherein the cGAS-related disease or disorder is inflammation, an auto-immune disease, a cancer, an infection, a disease or disorder of the central nervous system, a metabolic disease, a cardiovascular disease, a respiratory disease, a kidney disease, a liver disease, an ocular disease, a skin disease, a lymphatic disease, a rheumatic disease, a psychological disease, graft versus host disease, allodynia, or a cGAS-related disease in a subject that has been determined to carry a germline or somatic non-silent mutation in cGAS.

10. The compound or pharmaceutical composition for use according to claim 8, wherein the cGAS-related disease or disorder is systemic lupus erythematosus (SLE), cutaneous lupus erythematosus (CLE), scleroderma, psoriasis, Aicardi Goutières syndrome, Sjogren's syndrome, rheumatoid arthritis, inflammatory bowel diseases, multiple sclerosis, diabetes, cardiovascular, and neurodegenerative diseases.

11. The compound or pharmaceutical composition for use according to claim 8, wherein the cGAS-related disease or disorder is systemic lupus erythematosus (SLE), cutaneous lupus erythematosus (CLE), psoriasis, insulin-dependent diabetes mellitus (IDDM), scleroderma, Aicardi Goutières syndrome, dermatomyositis, inflammatory bowel diseases, multiple sclerosis, rheumatoid arthritis and Sjogren's syndrome (SS).
